# EUROPÄISCHE PATENTSCHRIFT

(11) **EP 0 631 575 B1**
(45) Veröffentlichungstag und Bekanntmachung des Hinweises auf die Patenterteilung: **04.10.2001**
(21) Anmeldenummer: 93906495.2
(22) Anmeldetag: 10.03.1993
(51) Int. Cl.: C07D 239/46, C07D 239/42, C07D 239/52, C07D 239/34, C07D 239/94, C07D 239/88, C07D 401/12, C07D 495/04

(54) **SUBSTITUIERTE PYRIMIDINE UND IHRE VERWENDUNG ALS SCHÄDLINGSBEKÄMPFUNGSMITTEL**
SUBSTITUTED PYRIMIDINES AND THEIR USE AS PESTICIDES
PYRIMIDINES SUBSTITUEES ET LEUR UTILISATION COMME PESTICIDES

(30) Priorität: 14.03.1992 DE 4208254
(43) Veröffentlichungstag der Anmeldung: 04.01.1995
(73) Patentinhaber: Aventis CropScience GmbH, 65929 Frankfurt am Main (DE)
(72) Erfinder: SCHAPER, Wolfgang, D-8901 Diedorf (DE); FREUSS, Rainer, D-6238 Hofheim/Taunus (DE); SALBECK, Gerhard, D-6239 Kriftel/Taunus (DE); BRAUN, Peter, D-6500 Mainz (DE); KNAUF, Werner, D-6239 Eppstein/Taunus (DE); SACHSE, Burkhard, D-6233 Kelkheim/Taunus (DE); WALTERSDORFER, Anna, D-6000 Frankfurt am Main (DE); KERN, Manfred, D-6501 Lörzweiler (DE); LÜMMEN, Peter, Dr., D-65510 Idstein (DE); BONIN, Werner, D-6233 Kelkheim/Taunus (DE)
(86) Internationale Anmeldenummer: EP9300536
(87) Internationale Veröffentlichungsnummer: WO9319050

(56) Entgegenhaltungen:
- EP-A- 0 323 757
- EP-A- 0 447 891
- EP-A- 0 452 002
- EP-A- 0 519 211
- US-A- 3 470 182
- US-A- 4 196 207

## Beschreibung

Die Erfindung betrifft substituierte 4-Amino- und 4-Alkoxypyrimidine, Verfahren zu ihrer Herstellung und ihre Verwendung als Schädlingsbekämpfungsmittel, insbesondere als Insektizide, Akarizide und Fungizide.

Es ist bereits bekannt, daß bestimmte substituierte 4-Aminopyrimidine gute fungizide, akarizide und insektizide Wirkung zeigen (vgl. EP-A-57 440, EP-A-196 524, EP-A-264 217, EP-A-276 406, EP-A-323 757, EP-A-326 328, EP-A-326 329, EP-A-326 330, EP-A-356 158, EP-A-370 704, EP-A-411 634, EP-A-424 125, EP-A-452 002, EP-A-459 611, EP-A-447 891). Die biologische Wirkung dieser Verbindungen ist jedoch insbesondere bei niedrigen Aufwandmengen und Konzentrationen nicht in allen Anwendungsbereichen zufriedenstellend.

Es wurden substituierte 4-Amino- und 4-Alkoxypyrimidine der allgemeinen Formel I gefunden, 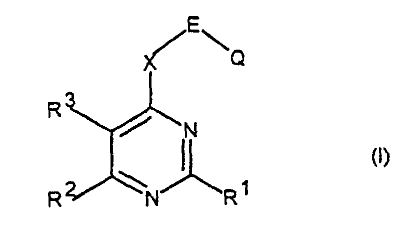 worin
- R¹: Wasserstoff, Halogen, (C₁-C₄)-Alkyl oder (C₃-C₆)-Cycloalkyl bedeutet,
- R²: Wasserstoff, (C₁-C₄)-Alkyl, Halogen, (C₁-C₄)-Halogenalkyl, (C₁-C₄)-Alkoxy, (C₁-C₄)-Halogenalkoxy, (C₁-C₄)-Alkoxy-(C₁-C₄)-alkyl, (C₁-C₄)-Alkylthio, (C₁-C₄)-Alkylthio-(C₁-C₄)-alkyl, (C₁-C₄)-Alkylamino oder Di-(C₁-C₄)-alkylamino bedeutet,
- R³: Wasserstoff, (C₁-C₄)-Alkyl, (C₁-C₄)-Alkoxy, (C₁-C₄)-Halogenalkoxy, Halogen, (C₁-C₄)-Alkylthio, Amino, (C₁-C₄)-Alkylamino oder Di-(C₁-C₄)-alkylamino bedeutet oder
- R² und R³: zusammen mit den Kohlenstoffatomen, an die sie gebunden sind, einen ungesättigten 6-gliedrigen isocyclischen Ring bilden, der gegebenenfalls durch 1, 2 oder 3 gleiche oder verschiedene Reste substituiert ist und diese Reste (C₁-C₄)-Alkyl, (C₁-C₄)-Alkoxy, (C₁-C₄)-Halogenalkyl, (C₁-C₄)-Halogenalkoxy undloder Halogen bedeuten,
oder
- R² und R³: zusammen mit den Kohlenstoffatomen, an die sie gebunden sind, einen gesättigten 6-gliedrigen isocyclischen Ring bilden, der gegebenenfalls durch 1, 2 oder 3 (C₁-C₄)-Alkylgruppen substituiert ist,
- X: NH oder Sauerstoff bedeutet,
- E: für eine direkte Bindung oder eine geradkettige oder verzweigte (C₁-C₄)-Alkandiylgruppe steht,
- Q: die Bedeutung Q¹ hat und
- Q¹: eine Cycloalkylgruppe der allgemeinen Formel II bedeutet 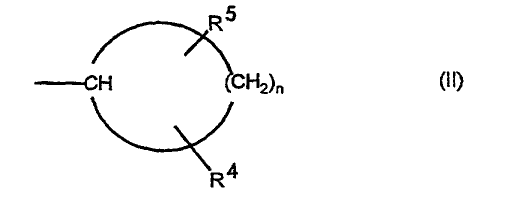 worin n eine ganze Zahl von 2 bis 7 ist,
- R⁴ und R⁵: gleich oder verschieden sind und jeweils Wasserstoff, (C₁-C₁₂)-Alkyl, (C₃-C₈)-Cycloalkyl, (C₃-C₈)-Cycloalkyl-(C₁-C₄)-alkyl, (C₁-C₈)-Alkoxy, (C₃-C₈)-Cycloalkoxy, (C₁-C₄)-Alkoxy-(C₁-C₄)-alkyl, (C₃-C₈)-Cycloalkyl-(C₁-C₄)-alkoxy, Tri-(C₁-C₈)-alkylsilyl, Di-(C₁-C₈)-alkyl-(C₃-C₈)-cycloalkylsilyl, Di-(C₁-C₈)-alkyl-(phenyl-(C₁-C₄)-alkyl)-silyl, Di-(C₁-C₈)-alkyl-(C₁-C₄)-halogenalkylsilyl, Dimethylphenylsilyl, (C₁-C₄)-Halogenalkyl, Halogen, (C₁-C₄)-Halogenalkoxy, Heteroaryl, Phenyl, Phenyl-(C₁-C₄)-alkyl, Benzyloxy, Benzyloxy-(C₁-C₄-)-alkyl, Benzylthio, Phenylthio oder Phenoxy bedeuten, wobei die Phenylringe in den sieben letztgenannten Resten unsubstituiert oder mit einem oder zwei Substituenten versehen sein können und diese Substituenten gleich oder verschieden sind und jeweils (C₁-C₈)-Alkyl, (C₃-C₈)-Cycloalkyl, (C₁-C₄)-Halogenalkyl, Halogen, (C₁-C₄)-Dialkylamino (C₁-C₄)-Alkylthio, (C₁-C₈)-Alkoxy, (C₁-C₄)-Halogenalkoxy, (C₁-C₄)-Alkoxy-(C₁-C₄)-alkoxy, H₅C₂-O-[CH₂-CH₂-O-]ₓ, 2-(Tetrahydro-2H-pyran-2-yloxy)-ethoxy, (C₂-C₈)-Alkenyl, (C₂-C₈)-Alkinyl, Benzyloxy, welches im Phenylrest gegebenenfalls einen oder zwei gleiche oder verschiedene Substituenten aus der Reihe (C₁-C₄)-Alkyl, (C₁-C₄)-Halogenalkyl, (C₁-C₄)-Alkoxy, (C₁-C₄)-Halogenalkoxy und Halogen trägt, Tri-(C₁-C₄)-alkylsilylmethoxy, (C₃-C₈)-Cycloalkyl-(C₁-C₄)-alkoxy, 1,3-Dioxolan-2-ylmethoxy, Tetrahydrofuran-2-ylmethoxy oder Tetrahydro-2H-pyran-2-ylmethoxy bedeuten können, wobei R⁴ und R⁵ nicht beide gleichzeitig Wasserstoff bedeuten können und wobei in zwei benachbarten Substituenten, die gleich oder verschieden sind und aus (C₁-C₈)-Alkyl und (C₁-C₈)-Alkoxy ausgewählt sind, jeweils ein Wasserstoffatom durch eine gemeinsame, diese beiden Substituenten verknüpfende C-C-Bindung ersetzt sein kann, und worin unter dem Ausdruck "Heteroaryl" einen Arylrest, worin mindestens eine CH-Gruppe durch N ersetzt ist und/oder mindestens zwei benachbarte CH-Gruppen durch S, NH oder 0 ersetzt sind, oder
- R⁴ und R⁵: zusammen mit der Cycloalkylgruppe ein 3-8-gliedriges spirocyclisches Ringsystem bilden, das an Stelle einer oder zweier CH₂-Gruppen Sauerstoff enthalten kann, oder
- R⁴ und R⁵: zusammen mit den diese tragenden Kohlenstoffatomen einen ankondensierten 5- oder 6-gliedrigen Isocyclus bilden,
- x: 2, 3 oder 4, vorzugweise 2 ist,
oder
- Q: die Bedeutung Q² hat und
- Q²: eine Gruppe der allgemeinen Formel III bedeutet 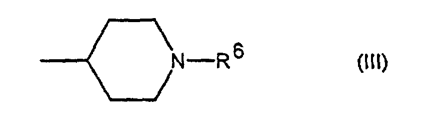
worin R⁶ eine Gruppe der allgemeinen Formel Z-W bedeutet und Z eine direkte Verbindung oder Carbonyl oder Sulfonyl bedeutet und W eine Phenyl, Naphthyl Biphenylyl oder eine dieser Gruppen worin mindestens eine CH-Gruppe durch N ersetzt ist und/oder mindestens zwei benachbarte CH-Gruppen durch S, NH oder O ersetzt sind bedeutet, die unsubstituiert oder mit einem oder zwei Substituenten versehen sein konnen und diese Substituenten gleich oder verschieden sind und jeweils (C₁-C₈)-Alkyl, (C₃-C₈)-Cycloalkyl, Trifluormethyl, Halogen, (C₁-C₄)-Alkoxy, (C₁-C₄)-Dialkylamino oder (C₁-C₄)-Alkylthio bedeuten,
oder deren Säureadditionssalze.

Falls Q die Bedeutung Q¹ hat, R² und R³ zusammen mit den diese tragenden Kohlenstoffatomen einen ungestättigten 6-gliedrigen isocyclischen Ring bilden, und R⁴ und R⁵ einen ankondensierten 5- oder 6-gliedrigen Ring bilden, so ist für die beiden letztgenannten Reste die Bedeutung 5-gliedriger Ring bevorzugt.

Bevorzugt sind solche Verbindungen der Formel I, für die R¹ Wasserstoff, Methyl oder Cyclopropyl sind,
- R²: (C₁-C₄)-Alkyl, Chlor, Methoxy, Ethoxy oder Methoxymethyl bedeuten,
- R³: Wasserstoff, (C₁-C₃)-Alkyl, Methoxy, Ethoxy oder Halogen bedeuten oder
- R² und R³: zusammen mit den Kohlenstoffatomen, an die sie gebunden sind, einen ungesättigten oder gesättigten 6-gliedrigen Ring bilden,
- Q: die Bedeutung Q¹ oder Q² hat, insbesondere solche Verbindungen der Formel I, für die
- R¹: Wasserstoff oder Methyl bedeutet,
- R²: Methyl, Ethyl, Methoxy, Ethoxy oder Methoxymethyl bedeutet,
- R³: Methyl, Ethyl, Methoxy, Chlor oder Brom bedeutet, oder
- R² und R³: zusammen mit den Kohlenstoffatomen, an die sie gebunden sind, das Chinazolin-System bilden, oder
- R² und R³: zusammen mit den Kohlenstoffatomen, an die sie gebunden sind, einen gesättigten 6-gliedrigen Ring bilden, und
- Q: die Bedeutung Q¹ oder Q² hat, vorzugsweise solche Verbindungen der Formel I, worin
- E: eine direkte Bindung bedeutet
- R¹: Wasserstoff bedeutet,
- R²: Methyl, Ethyl oder Methoxymethyl bedeutet,
- R³: Chlor, Brom oder Methoxy bedeutet oder
- R² und R³: zusammen mit den Kohlenstoffatomen, an die sie gebunden sind, das Chinazolin-System bilden, das mit Fluor, Chlor, Brom oder Methyl substituiert sein kann oder
- R² und R³: x zusammen mit dem Pyrimidin-Ring das 5,6,7,8-Tetrahydrochinazolin-System bilden und
- Q: die Bedeutung Q¹ oder Q² hat.

Ganz besonders bevorzugt sind solche Verbindungen der Formel I, worin
- E: eine direkte Bindung bedeutet,
- R¹: Wasserstoff bedeutet,
- R²: Methoxymethyl und R³ Methoxy oder
- R²: Methyl oder Ethyl und R³ Chlor oder Brom bedeutet oder
- R² und R³: zusammen mit dem Kohlenstoffatom, an das sie gebunden sind, ein Chinazolin-System bilden, das mit Fluor, Chlor oder Methyl substituiert ist oder ein 5,6,7,8-Tetrahydrochinazolin-System bilden und
- Q: die Bedeutung Q¹ hat, insbesondere solche Verbindungen der Formel I, für die
- E: eine direkte Verbindung bedeutet
- R¹: Wasserstoff bedeutet,
- R²: Methoxymethyl und R³ Methoxy bedeutet oder
- R²: Ethyl und R³ Chlor bedeutet oder
- R² und R³: zusammen mit dem Kohlenstoffatom, an das sie gebunden sind, ein Chinazolin oder ein 5,6,7,8-Tetrahydrochinazolin-System bilden und
- Q¹: eine in Position 3 oder 4 substituierte Cycloalkylgruppe der allgemeinen Formel II bedeutet, worin
- n: 4 oder 5 bedeutet,
- R⁴: (C₃-C₈)-Alkyl, Cyclopentyl, Cyclohexyl, Phenyl oder Phenoxy bedeutet, wobei die beiden letztgenannten Reste unsubstituiert oder mit einem oder zwei Substituenten versehen sein können die gleich oder verschieden sein können und diese Fluor, Chlor, Brom, (C₁-C₄)-Alkyl, (C₁-C₄)-Alkoxy, Trifluormethyl, (C₁-C₂)-Halogenalkoxy, Cyclohexyl, 2-Ethoxyethoxy, Methylthio oder Dimethylamino bedeuten und
- R⁵: Wasserstoff bedeutet.

Ganz besonders bevorzugt sind auch Verbindungen der Formel I, worin
- E: eine direkte Bindung bedeutet,
- R¹: Wasserstoff bedeutet,
- R²: Methoxymethyl und R³ Methoxy oder
- R²: Ethyl und R³ Chlor bedeuten, oder
- R² und R³: zusammen mit den Kohlenstoffatomen, an die sie gebunden sind, ein Chinazolin- oder ein 5,6,7,8-Tetrahydrochinazolin-System bilden,
- Q: die Bedeutung Q¹ hat und
- Q¹: in 4-Position substituiertes Cyclohexyl bedeutet, und E und der Substituent in 4-Position des Cyclohexyl zueinander cis-ständig sind,
- R⁴: wie oben definiert ist und
- R⁵: vorzugsweise Wasserstoff bedeutet.

In der obigen Formel I ist unter "Halogen" ein Fluor-, Chlor-, Brom- oder lodatom vorzugsweise ein Fluor-, Chlor- oder Bromatom zu verstehen,
unter dem Ausdruck "(C₁-C₄)-Alkyl" ein unverzweigter oder verzweigter Kohlenwasserstoffrest mit 1-4 Kohlenstoffatomen, wie z.B. der Methyl-, Ethyl-, Propyl-, 1-Methylethyl-, 1-Methylpropyl-, 2-Methylpropyl- oder 1,1-Dimethylethylrest,
unter dem Ausdruck "(C₁-C₈)-Alkyl" die vorgenannten Alkylreste sowie z.B. der Pentyl, 2-Methylbutyl- oder der 1,1-Dimethylpropylrest, der Hexyl-, Heptyl-, Octylrest oder 1,1,3,3-Tetramethylbutyl;
unter dem Ausdruck "(C₁-C₁₂)-Alkyl" die vorgenannten Alkylreste sowie z.B. der Nonyl-, Decyl-, Undecyl- oder Dodecylrest;
unter dem Ausdruck "(C₃-C₈)-Cycloalkyl" die Cyclopropyl-, Cyclobutyl-, Cyclopentyl-, Cyclohexyl-, Cycloheptyl- oder Cyclooctylgruppe;
unter dem Ausdruck "(C₁-C₄)-Alkoxy" eine Alkoxygruppe, deren Kohlenwasserstoffrest die unter dem Ausdruck "(C₁-C₄)-Alkyl" angegebene Bedeutung hat;
unter dem Ausdruck "(C₃-C₈)-Cycloalkoxy" eine Cycloalkoxygruppe, deren Kohlenwasserstoffrest die unter "(C₃-C₈)-Cycloalkyl" angegebene Bedeutung hat; unter dem Ausdruck "(C₁-C₄)-Alkylthio" eine Alkylthiogruppe, deren Kohlenwasserstoffrest die unter dem Ausdruck "(C₁-C₄)-Alkyl" angegebene Bedeutung hat;
unter dem Ausdruck "(C₁-C₄)-Halogenalkoxy" eine Halogenalkoxygruppe, deren Halogen-Kohlenwasserstoffrest die unter dem Ausdruck "(C₁-C₄)-Halogenalkyl" angegebene Bedeutung hat,
unter dem Ausdruck "(C₁-C₄)-Alkoxy-(C₁-C₄)alkyl" beispielsweise eine 1-Methoxyethylgruppe, eine 2-Methoxyethylgruppe, eine 2-Ethoxyethylgruppe, eine Methoxymethyl- oder Ethoxymethylgruppe, eine 3-Methoxypropylgruppe oder eine 4-Butoxybutylgruppe;
unter dem Ausdruck "(C₁-C₄)-Alkylthio-(C₁-C₄)alkyl" beispielsweise Methylthiomethyl, Ethylthiomethyl, Propylthiomethyl, 2-Methylthioethyl, 2-Ethylthioethyl oder 3-Methylthiopropyl;
unter dem Ausdruck "(C₁-C₄)-Alkylamino" eine Alkylaminogruppe, deren Kohlenwasserstoffrest die unter dem Ausdruck "(C₁-C₄)-Alkyl" angegebene Bedeutung hat, bevorzugt die Ethyl- und Methylaminogruppe;
unter dem Ausdruck "Di-(C₁-C₄)-alkylamino" eine Dialkylaminogruppe, deren Kohlenwasserstoffreste die unter dem Ausdruck "(C₁-C₄)-Alkyl" angegebene Bedeutung haben, bevorzugt die Dimethyl- und Diethylaminogruppe;
unter dem Ausdruck "(C₁-C₄)-Halogenalkyl" eine unter dem Ausdruck "(C₁-C₄)-Alkyl" genannte Alkylgruppe, in der eines oder mehrere Wasserstoffatome durch die obengenannten Halogenatome, bevorzugt Chlor oder Fluor, ersetzt wird, wie beispielsweise die Trifluormethylgruppe, die 2,2,2-Trifluorethylgruppe, die Chlormethyl-, Fluormethylgruppe, die Difluormethylgruppe oder die 1,1,2,2-Tetrafluorethylgruppe;
unter dem Ausdruck "C₃-C₈)-Cycloalkyl-(C₂-C₄)-alkyl" eine der oben genannten (C₁-C₄)-Alkylgruppen, die mit einer der oben genannten (C₃-C₈)-Cycloalkylgruppen substituiert ist, beispielsweise Cyclohexylmethyl, Cyclohexylethyl, Cyclohexylpropyl, Cyclohexylbutyl oder 1-Cyclohexyl-1-methyl-ethyl;
unter dem Ausdruck "Phenyl-(C₁-C₄)-alkyl" eine der oben genannten (C₁-C₄)-Alkylgruppen, die mit einer Phenylgruppe subsitutiert ist, beispielsweise die Benzyl-, die 2-Phenylethyl-, die 1-Phenylethyl-, die 1-Methyl-1-phenylethylgruppe, die 3-Phenylpropyl-, die 4-Phenylbutylgruppe oder die 2-Methyl-2-phenyl-ethylgruppe;
unter dem Ausdruck "Aryl" beispielsweise, Phenyl, Naphthyl oder Biphenylyl, vorzugsweise Phenyl;
unter dem Ausdruck "Heteroaryl" einen Arylrest, worin mindestens eine CH-Gruppe durch N ersetzt ist und/oder mindestens zwei benachbarte CH-Gruppen durch S, NH oder O ersetzt sind;
unter dem Ausdruck "Benzyloxy-(C₁-C₄)-alkyl" eine (C₁-C₄)-Alkylgruppe mit den oben angegebenen Bedeutungen die mit einer Benzyloxygruppe substituiert ist, z.B. die Benzyloxymethyl- oder die 2-(Benzyloxy)-ethyl-Gruppe;
unter dem Ausdruck "(C₃-C₈)-Cycloalkyl-(C₁-C₄)-alkoxy" eine (C₁-C₄)-Alkoxygruppe mit den oben angegebenen Bedeutungen, die mit einer (C₃-C₈)-Cycloalkylgruppe mit den oben angegebenen Bedeutungen substituiert ist, z.B. die Cyclopropylmethyl- oder die Cyclohexylmethylgruppe;
unter dem Ausdruck "Tri-(C₁-C₈)-alkylsilyl" eine Trialkylsilylgruppe die vorzugsweise zwei Methylgruppen und eine (C₁-C₈)-Alkylgruppe mit den oben angegebenen Bedeutungen trägt, z.B. die Trimethylsilyl-, die Dimethylethylsilyl oder die Dimethyloctylsilylgruppe;
unter dem Ausdruck "Di-(C₁-C₈)-alkyl-(C₁-C₄)-halogenalkylsilyl" einen Silylrest, der vorzugsweise zwei Methylgruppen und einen (C₁-C₄)-Halogenalkylrest mit den
unter dem Ausdruck (C₁-C₄)-Halogenalkyl angegebenen Bedeutungen trägt, z.B. der Dimethyl-3,3,3-trifluorpropylsilyl-Rest;
unter dem Ausdruck "(C₁-C₄)-Alkoxy-(C₁-C₄)-alkoxy" z.B. die Ethoxymethoxy-, 2-Ethoxy-ethoxy-, 2-Butoxyethoxy- oder 2-Methoxyethoxy-Gruppe;
unter dem Ausdruck "(C₂-C₈)-Alkenyl" z.B. die Allyl-, 1-Methylallyl-, 2-Butenyl- oder 3-Methyl-2-butenyl-Gruppe;
unter dem Ausdruck "(C₂-C₈)-Alkinyl" z.B. die Propargyl-, 2-Butinyl- oder 2-Pentinylgruppe;
unter dem Ausdruck "Tri-(C₁-C₄)-alkylsilylmethoxy" ein Trialkylsilylmethoxyrest mit vorzugsweise 2 Methylgruppen, worin die (C₁-C₄)-Alkylgruppe die oben angegebenen Bedeutungen hat;
unter dem Ausdruck "Di-(C₁-C₈)-alkyl-phenyl-(C₁-C₄)-alkylsilyl ein Trialkylsilylrest mit vorzugsweise zwei Methylgruppen, bei dem eine Alkylgruppe die oben für den Ausdruck "Phenyl-(C₁-C₄)-alkyl" angegebenen Bedeutungen hat, vorzugsweise die Dimethylbenzylsilyl-Gruppe.

Die oben gegebene Erläuterung gilt entsprechend für Homologe bzw. deren abgeleitete Reste.

Die vorliegende Erfindung betrifft die Verbindungen der Formel I in Form der freien Base oder eines Säureadditionssalzes. Säuren, die zur Salzbildung herangezogen werden können, sind anorganische Säuren, wie Salzsäure, Bromwasserstoffsäure, Salpetersäure, Schwefelsäure, Phosphorsäure oder organische Säuren wie Ameisensäure, Essigsäure, Propionsäure, Malonsäure, Oxalsäure, Fumarsäure, Adipinsäure, Stearinsäure, Ölsäure, Methansulfonsäure, Benzolsulfonsäure oder Toluolsulfonsäure.

Die Verbindungen der Formel I weisen zum Teil ein oder mehrere asymmetrische Kohlenstoffatome auf. Es können daher Racemate und Diastereomere auftreten. Die Erfindung umfaßt sowohl die reinen Isomeren als auch deren Gemische. Die Gemische von Diastereomeren können nach gebräuchlichen Methoden, z.B. durch selektive Kristallisation aus geeigneten Lösungsmitteln oder durch Chromatographie in die Komponenten aufgetrennt werden. Racemate können nach üblichen Methoden in die Enantiomeren aufgetrennt werden, so z.B. durch Salzbildung mit einer optisch aktiven Säure, Trennung der diasteromeren Salze und Freisetzung der reinen Enantiomeren mittels einer Base.

Falls Q¹ eine Cycloalkylgruppe der allgemeinen Formel II bedeutet und n= 4,5 oder 6, vorzugsweise 5 ist, so sind E und ein für n=5 vorzugsweise in 4-Position stehender Rest R⁴ oder R⁵, zueinander vorzugsweise in der cis-Konfiguration.

Die Erfindung betrifft weiterhin ein Verfahren zur Herstellung von Verbindungen der Formel I, das dadurch gekennzeichnet ist, daß man eine Verbindung der Formel IV 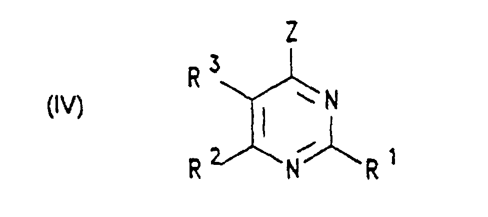 worin R¹, R² und R³ die unter Formel I angegebenen Bedeutungen haben und Z eine Abgangsgruppe, beispielsweise Halogen, Alkylthio, Alkansulfonyloxy oder Arylsulfonyloxy, Alkylsulfonyl oder Arylsulfonyl bedeutet, mit einem Nucleophil der Formel V

HX - E - Q (V)

worin X, E und Q die unter Formel I angegebenen Bedeutungen haben, umsetzt und die so erhaltenen Verbindungen der Formel I gegebenenfalls, falls R³ Wasserstoff bedeutet, an C⁵ des Pyrimidins chloriert oder bromiert.

Die oben beschriebene Substitutionsreaktion ist im Prinzip bekannt. Die Abgangsgruppe Z ist in weiten Grenzen variierbar und kann beispielsweise ein Halogenatom wie Fluor, Chlor, Brom oder lod bedeuten oder Alkylthio wie Methyl- oder Ethylthio, oder Alkansulfonyloxy wie Methan-, Trifluormethan- oder Ethansulfonyloxy oder Arylsulfonyloxy, wie Benzolsulfonyloxy oder Toluolsulfonyloxy oder Alkylsulfonyl wie Methyl- oder Ethylsulfonyl oder Arylsulfonyl wie Phenyl- oder Toluolsulfonyl.

Die vorgenannte Reaktion wird in einem Temperaturbereich von 20-150°C, zweckmäßig in Anwesenheit einer Base und gegebenenfalls in einem inerten organischen Lösungsmittel wie N,N-Dimethylformamid, N,N-Dimethylacetamid, Dimethylsulfoxid, N-Methylpyrrolidin-2-on, Dioxan, Tetrahydrofuran, 4-Methyl-2-pentanon, Methanol, Ethanol, Butanol, Ethylenglykol, Ethylenglykoldimethylether, Toluol, Chlorbenzol oder Xylol durchgeführt. Es können auch Gemische der genannten Lösungsmittel verwendet werden.

Geeignete Basen für den Fall, daß X Sauerstoff bedeutet, sind beispielsweise Alkali- oder Erdalkalimetallcarbonate,-hydrogencarbonate, -amide oder -hydride wie Natriumcarbonat, Natriumhydrogencarbonat, Kaliumcarbonat, Natriumamid oder Natriumhydrid, für den Fall, daß X NH bedeutet, sind dies beispielsweise Alkali- oder Erdalkalimetallcarbonate, -hydrogencarbonate, -hydroxide, -amide oder - hydride wie Natriumcarbonat, Natriumhydrogencarbonat, Kaliumcarbonat, Natriumhydroxid, Natriumamid oder Natriumhydrid oder organische Basen wie Triethylamin oder Pyridin. Auch ein zweites Äquivalent eines Amins V kann als Hilfsbase eingesetzt werden.

Die Ausgangsverbindungen der Formel IV können nach zu bekannten Verfahren analogen Verfahren hergestellt werden. Als Ausgangsprodukte dienen Acetessigester-Derivate, die über die entsprechenden Hydroxypyrimidine in die Halogenpyrimidine überführt werden:

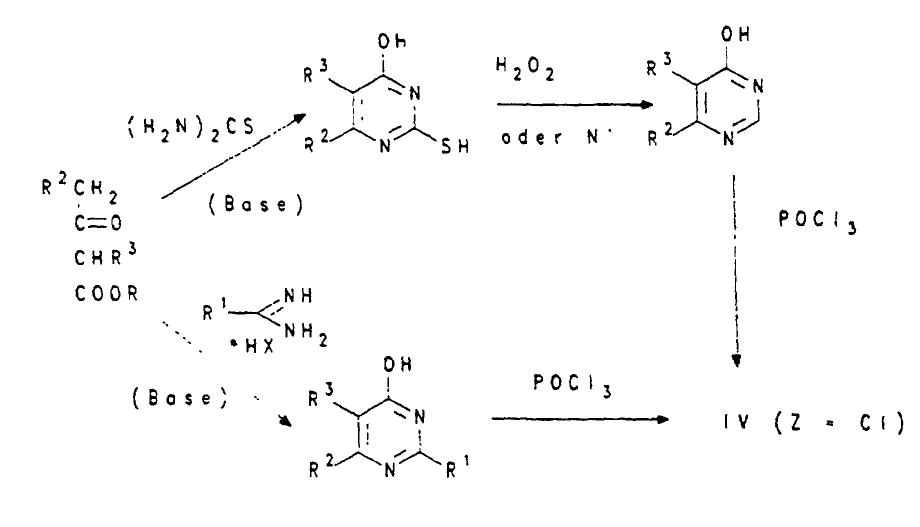

Die Ausgangsverbindungen der Formel IV können weiterhin in Analogie zu bekannten Verfahren aus Malonester-Derivaten erhalten werden:

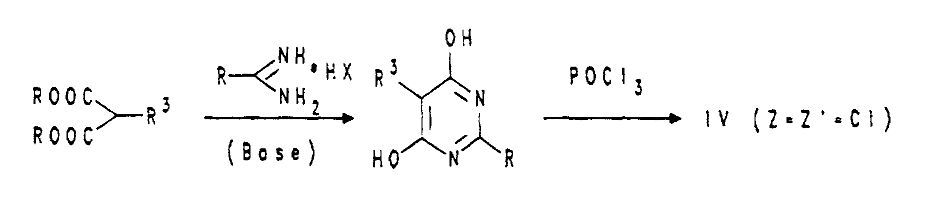

Die als Ausgangsprodukte benötigten Nucleophile der Formel V können für den Fall, daß X Sauerstoff bedeutet, nach bekannten Verfahren hergestellt werden, beispielsweise durch Reduktion einer Carbonylgruppe mit einem geeigneten Reduktionsmittel, beispielsweise einem komplexen Metallhydrid oder im Falle eines Aldehyds oder Ketons auch mit Wasserstoff und einem Hydrierkatalysator. Weitere Möglichkeiten sind die Umsetzung einer metallorganischen Verbindung mit einer Carbonylgruppe oder einem Oxiran. Zur Darstellung von Cyclohexanol-Derivaten können auch geeignete substituierte Phenole mit Wasserstoff in Gegenwart eines Hydrierkatalysators umgesetzt werden.

Die als Ausgangsprodukte benötigten Nucleophile der Formel V können für den Fall, daß X NH bedeutet, ebenfalls nach bekannten Methoden hergestellt werden, beispielsweise Reduktion eines Oxims oder eines Nitrils mit einem geeigneten Reduktionsmittel, beispielsweise einem komplexen Metallhydrid oder Wasserstoff in Gegenwart eines Hydrierkatalysators, reduktive Aminierung oder Leuckart-Wallach-Reaktion eines Aldehyds oder Ketons oder Gabriel-Reaktion eines Alkylhalogenids oder -Tosylats. Zur Darstellung von Cyclohexylamin-Derivaten können auch geeignete substituierte Aniline mit Wasserstoff in Gegenwart eines Hydrierkatalysators umgesetzt werden.

Die Verbindungen der Formel I, worin R³ Halogen bedeutet, können nach bekannten Verfahren halogeniert werden.

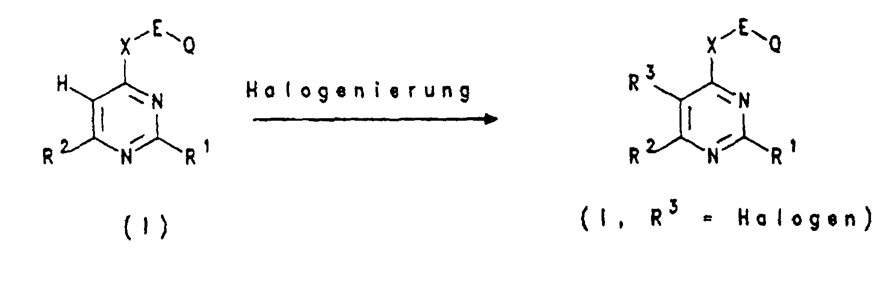

Im Falle der 5-Chlorderivate können z.B. elementares Chlor, Natriumhypochlorit, Sulfurylchlorid oder N-Chlorsuccinimid zum Einsatz kommen, zur Bromierung eignen sich besonders elementares Brom oder N-Bromsuccinimid. Geeignete Lösungsmittel sind z.B. Dichlormethan, Chloroform oder Eisessig.

Die Wirkstoffe eignen sich bei guter Pflanzenverträglichkeit und günstiger Warrnblütertoxizität zur Bekämpfung von tierischen Schädlingen, insbesondere Insekten, Spinnentieren, Helminthen und Mollusken, ganz besonders bevorzugt zur Bekämpfung von Insekten und Spinnentieren, die in der Landwirtschaft, bei der Tierzucht, in Forsten, im Vorrats- und Materialschutz sowie auf dem Hygienesektor vorkommen. Sie sind gegen normal sensible und resistente Arten sowie alle oder einzelne Entwicklungsstadien wirksam. Zu den oben erwähnten Schädlingen gehören:
Aus der Ordnung der Acarina z.B. Acarus siro, Agras spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Eotetranychus spp., Oligonychus spp., Eutetranychus spp.
Aus der Ordnung der Isopoda, z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spp.
Aus der Ordnung der Symphyla z.B. Scutigerella immaculata.
Aus der Ordung der Thysanura z.B. Lepisma saccharina.
Aus der Ordnung der Collembola z.B. Onychiurus armatus.
Aus der Ordnung der Orthoptera z.B. Blatta orientalis,
Periplaneta americana, Leucophaea maderae, Blatlella germanica, Acheta domesticus, Gryllotalpa spp., Locusta migratoria migratorioides, Melanoplus differentialis,
Schistocerca gregaria.
Aus der Ordnung des Isoptera z.B. Reticulitermes spp..
Aus der Ordnung der Anoplura z.B. Phylloxera vastatrix, Pemphigus spp., Pediculus humanus corporis, Haematopinus spp., Linognathus spp..
Aus der Ordnung der Mallophaga z.B. Trichodectes spp., Damalinea spp..
Aus der Ordnung der Thysanoptera z.B. Hercinothrips femoralis, Thrips tabaci.
Aus der Ordnung der Heteroptera z.β. Eurygaster spp., Dysdercus intermedius, Piesma quadrata, Cimex lectularius, Rhodnius prolixus, Triatoma spp..
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelus bilobatus, Nephotettix cincticeps, Lecanium corni, Saissetia oleae, Laodelphax striatellus, Nilaparvata lugens, Aonidiella aurantii, Aspidiotus hederae, Pseudococcus spp., Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp., Bucculatrix thurberiella, Phyllocnistis citrella, Agrotis spp., Euxoa spp., Feltia spp., Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylioides chrysocephala, Epilachna varivestis, Atomaria spp., Oryzaephilus surinamensis, Anthonumus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp..
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hypoposca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.
Aus der Ordnung der Siphonaptera z.B. Xenopsylla cheopis, Ceratophyllus spp..
Aus der Ordnung der Arachnida z.B. Scorpio maurus, Latrodectus mactans.
Aus der Klasse der Helminthen z.B. Haemonchus, Trichostrongulus, Ostertagia. Cooperia, Chabertia, Strongyloides, Oesophagostomum, Hyostrongulus, Ancylostoma, Ascaris und Heterakis sowie Fasciola und pflanzenschädigende Nematoden z.B. solche der Gattungen Meloidogyne, Heterodera, Ditylenchus, Aphelenchoides, Radopholus, Globodera, Pratylenchus, Longidorus und Xiphinema.
Aus der Klasse der Gastropoda z.B. Deroceras spp., Arion spp., Lymnaea spp., Galba spp., Succinea spp., Biomphalaria spp., Bulinus spp., Oncomelania spp.. Aus der Klasse der Bivalva z.B. Dreissena spp..

Die Erfindung betrifft auch insektizide und akarizide Mittel, die die Verbindungen der Formel I neben geeigneten Formulierungshilfsmitteln enthalten.

Die erfindungsgemäßen Mittel enthalten die Wirkstoffe der Formeln I im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher infrage:
Spritzpulver (WP), emulgierbare Konzentrate (EC), wäßrige Lösungen (SC),
Emulsionen, versprühbare Lösungen, Dispersionen auf Öl- oder Wasserbasis (SC),
Suspoemulsionen (SC), Stäubemittel (DP), Beizmittel, Granulate in Form von Mikro-, Sprüh-, Aufzugs- und Adsorptionsgranulaten, wasserdispergierbare Granulate (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C. Hauser Verlag München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zussatzstoffe sind ebenfalls bekannt und werden beispielsweise beschrieben in: Watkins, "Handbook of Insecticide Dust Diluents and Carriers", 2nd Ed., Darland Books, Caldwell N.J.; H.v.Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's, "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Auf der Basis dieser Formulierungen lassen sich auch Kombinationen mit anderen pestizid wirksamen Stoffen, Düngemitteln und/oder Wachstumsregulatoren herstellen, z.B. in Form einer Fertigformulierung oder als Tankmix. Spritzpulver sind in Wasser gleichmäßig dispergierbare Präparate, die neben dem Wirkstoff außer einem Verdünnungs- oder Inertstoff noch Netzmittel, z.B. polyoxethylierte Alkylphenole, polyoxethylierte Fettalkohole, Alkyl- oder Alkylphenol-sulfonate und Dispergiermittel, z.B. ligninsulfonsaures Natrium, 2,2'-dinaphthylmethan-6,6'-disulfonsaures Natrium, dibutylnaphthalin-sulfonsaures Natrium oder auch oleylmethyltaurinsaures Natrium enthalten.
Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren können beispielsweise verwendet werden: Alkylarylsulfonsaure Calcium-Salze wie Cadodecylbenzol-sulfonat oder nichtionische Emulgatoren wie Fettsäurepolyglykolester, Alkylarylpolyglykolether, Fettalkoholpolyglykolether, Propylenoxid-Ethylenoxid-Kondensationsprodukte, Alkylpolyether, Sorbitanfettsäureester, Polyoxyethylensorbitan-Fettsäureester oder Polyoxethylensorbitester.

Stäubemittel erhält man durch Vermahlen des Wirkstoffes mit fein verteilten festen Stoffen, z.B. Talkum, natürlichen Tonen wie Kaolin, Bentonit, Pyrophillit oder Diatomeenerde. Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln, z.B. Polyvinylalkohol, polyacrylsaurem Natrium oder auch Mineralölen, auf die Oberfläche von Trägerstoffen wie Sand, Kaolinite oder von granuliertem Inertmaterial. Auch können geeignete Wirkstoffe in der für die Herstellung von Düngemittelgranulaten üblichen Weise - gewünschtenfalls in Mischung mit Düngemitteln - granuliert werden.

In Spritzpulvern beträgt die Wirkstoffkonzentration z.B. etwa 10 bis 90 Gew.-%, der Rest zu 100 Gew.-% besteht aus üblichen Formulierungsbestandteilen. Bei emulgierbaren Konzentraten kann die Wirkstoffkonzentration etwa 5 bis 80 Gew.-% betragen. Staubförmige Formulierungen enthalten meistens 5 bis 20 Gew.-% an Wirkstoff, versprühbare Lösungen etwa 2 bis 20 Gew.-%. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Daneben enthalten die genannten Wirkstofformulierungen gegebenenfalls die jeweils üblichen Haft-, Netz-, Dispergier-, Emulgier-, Penetrations-, Lösungsmittel, Füll- oder Trägerstoffe.

Zur Anwendung werden die in handelsüblicher Form vorliegenden Konzentrate gegebenenfalls in üblicher Weise verdünnt, z.B. bei Spritzpulvern, emulgierbaren Konzentraten, Dispersionen und teilweise auch bei Mikrogranulaten mittels Wasser. Staubförmige und granulierte Zubereitungen sowie versprühbare Lösungen werden vor der Anwendung üblicherweise nicht mehr mit weiteren inerten Stoffen verdünnt.

Mit den äußeren Bedingungen wie Temperatur, Feuchtigkeit u.a. variiert die erforderliche Aufwandmenge. Sie kann innerhalb weiter Grenzen schwanken, z.B. zwischen 0,001 und 10,0 kg/ha oder mehr Aktivsubstanz, vorzugsweise liegt sie jedoch zwischen 0,005 und 5 kg/ha.

Die erfindungsgemäßen Wirkstoffe können in ihren handelsüblichen Formulierungen sowie in den aus diesen Formulierungen bereiteten Anwendungsformen in Mischungen mit anderen Wirkstoffen, wie Insektiziden, Lockstoffen, Sterilantien, Akariziden, Nematiziden, Fungiziden, wachstumsregulierenden Stoffen oder Herbiziden vorliegen.

Zu den Schädlingsbekämpfungsmitteln zählen beispielsweise Phosphorsäureester, Carbamate, Carbonsäureester, Formamidine, Zinnverbindungen, durch Mikroorganismen hergestellte Stoffe u.a..
Bevorzugte Mischungspartner sind
1. aus der Gruppe der Phosphorverbindungen
   Acephate, Azamethiphos, Azinphos-ethyl, Azinphosmethyl, Bromophos, Bromophos-ethyl, Chlorfenvinphos, Chlormephos, Chlorpyrifos, Chlorpyrifos-methyl, Demeton, Demeton-S-methyl, Demeton-S-methyl Sulphone, Dialifos, Diazinon, Dichlorvos, Dicrotophos,
   0,0-1,2,2,2-tetrachlorethylphosphorthioate (SD 208 304), Dimethoate, Disutfoton, EPN, Ethion, Ethoprophos, Etrimfos, Famphur, Fenamiphos, Fenitrothion, Fensulfothion, Fenthion, Fonofos, Formothion, Heptenophos, lsazophos, Isothioate, Isoxathion, Malathion, Methacrifos, Methamidophos, Methidathion, Salithion, Mevinphos, Monocrotophos, Naled, Omethoate, Oxydemeton-methyl, Parathion, Parathion-methyl, Phenthoate, Phorate, Phosalone, Phosfolan, Phosmet, Phosphamidon, Phoxim, Pirimiphos-ethyl, Pirimiphos-methyl, Profenofos, Propaphos, Proetamphos, Prothiofos, Pyraclofos, Pyridapenthion, Quinalphos, Sulprofos, Temephos, Terbufos, Tetrachlorvinphos, Thiometon, Triazophos, Trichlorphon, Vamidothion;
2. aus der Gruppe der Carbamate
   Aldicarb, 2-sec.-Butylphenylmethylcarbamate (BPMC), Carbaryl, Carbofuran, Carbosulfan, Cloethocarb, Benfuracarb, Ethiofencarb, Furathiocarb, lsoprocarb, Methomyl, 5-Methyl-m-cu-menylbutyryl(methyl)carbamate, Oxamyl, Pirimicarb, Propoxur, Thiodicarb, Thiofanox, Ethyl 4,6,9-triaza-4-benzyl-6, 10-dimethyl-8-oxa7-oxo-5,11-dithia-9-dodecenoate (OK 135), 1-Methylthio(ethylideneamino)-N-methyl-N-(morpholinothio)carbamate (UC 51717);
3. aus der Gruppe der Carbonsäureester
   Allethrin, Alphametrin, 5-Benzyl-3-furylmethyl-(E)-(1R)-cis 2,2-di-methyl-3-(2-oxothiolan-3-ylidenemethyl)cyclopropanecarboxylate, Bioallethrin, Bioallethrin((S)-cyclopentylisomer), Bioresmethrin, Biphenate, (RS)-1-Cyano-1-(6-phenoxy-2-pyridyl)methyl-(1RS)-trans-3-(4-tert.butylphenyl)-2,2-dimethylcyclopropanecarboxylate (NCI 85193), Cycloprothrin, Cyhalothrin, Cypermethrin, Cyphenothrin, Deltamethrin, Empenthrin, Esfenvalerate, Fenfluthrin, Fenpropathrin, Fenvalerate, Flucythrinate, Flumethrin, Fluvalinate (D-isomer), Permethrin, Pheothrin ((R)-Isomer), d-Pralethrin, Pyrethrine (natürliche Produkte), Resmethrin, Tefluthrin, Tetramethrin, Tralomethrin;
4. aus der Gruppe der Amidine
   Amitraz, Chlordimeform;
5. aus der Gruppe der Zinnverbindungen
   Cyhexatin, Fenbutatinoxide;
6. Sonstige
   Abamectin, Bacillus thuringiensis, Bensultap, Binapacryl, Bromopropylate, Buprofezin, Camphechlor, Cartap, Chlorobenzilate, Chlorfluazuron, 2-(4-(Chlorphenyl)-4,5-diphenylthiophen (UBI-T 930), Chlorfentezine, Cyclopropancarbonsäure-(2-naphthylmethyl)ester (Ro12-0470), Cyromazin, N-(3,5-Dichlor-4-(1,1,2,3,3,3-hexafluor-1-propyloxy)phenyl)carbamoyl)-2-chlorbenzcarboximidsäureethylester, DDT, Dicofol, N-(N-(3,5-Di-chlor-4-(1,1,2,2-tetrafluorethoxy)phenylamino)carbonyl)-2,6-difluorbenzamid (XRD 473), Diflubenzuron, N-(2,3-Dihydro-3-methyl-1,3-thiazol-2-ylidene)-2,4-xylidine, Dinobuton, Dinocap, Endosulfan, Ethofenprox, (4-Ethoxyphenyl) (dimethyl)(3-(3-phenoxyphenyl)propyl)silan, (4-Ethoxyphenyl) (3-(4-fluoro-3-phenoxyphenyl)propyl)dimethylsilan, Fenoxycarb, 2-Fluoro-5-(4-(4-ethoxyphenyl)-4-methyl-1-pentyl)diphenylether (MTI 800), Granulose- und Kernpolyederviren, Fenthiocarb, Flubenzimine, Flucycloxuron, Flufenoxuron, Gamma-HCH, Hexythiazox, Hydramechylnon (AC 217300), Ivermectin, 2-Nitromethyl-4,5-dihydro-6H-thiazin (SD 52618), 2-Nitromethyl-3,4-dihydrothiazol (SD 35651), 2-Nitromethylene-1,2-thiazinan-3-ylcarbamaldehyde (WL 108477), Propargite, Teflubenzuron, Tetradifon, Tetrasul, Thiocyclam, Triflumuron.

Der Wirkstoffgehalt der aus den handelsüblichen Formulierungen bereiteten Anwendungsformen kann von 0,00000001 bis zu 95 Gew.-% Wirkstoff, vorzugsweise zwischen 0,00001 und 1 Gew.-% liegen.

Die Anwendung geschieht in einer den Anwendungsformen angepaßten üblichen Weise.

Die erfindungsgemäßen Wirkstoffe eignen sich auch zur Bekämpfung von Endo- und Ektoparasiten auf dem veterinärmedizinischen Gebiet bzw. auf dem Gebiet der Tierhaltung.

Die Anwendung der erfindungsgemäßen Wirkstoffe geschieht hier in bekannter Weise wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießen (pour-on and spot-on) und des Einpuderns sowie durch parenterale Anwendung in Form beispielsweise der Injektion.

Die erfindungsgemäßen neuen Verbindungen der Formel I können demgemäß auch besonders vorteilhaft in der Viehhaltung (z.B. Rinder, Schafe, Schweine und Geflügel wie Hühner, Gänse usw.) eingesetzt werden. In einer bevorzugten Ausführungsform der Erfindung werden den Tieren die neuen Verbindungen, gegebenenfalls in geeigneten Formulierungen (vgl. oben) und gegebenenfalls mit dem Trinkwasser oder Futter oral verabreicht. Da eine Ausscheidung im Kot in wirksamer Weise erfolgt, läßt sich auf diese Weise sehr einfach die Entwicklung von Insekten im Kot der Tiere verhindern. Die jeweils geeigneten Dosierungen und Formulierungen sind insbesondere von der Art und dem Entwicklungsstadium der Nutztiere und auch vom Befallsdruck abhängig und lassen sich nach den üblichen Methoden leicht ermitteln und festlegen. Die neuen Verbindungen können bei Rindern z.B. in Dosierungen von 0,01 bis 1 mg/kg Körpergewicht eingesetzt werden.

Die erfindungsgemäßen Verbindungen der Formel I zeichnen sich auch durch eine hervorragende fungizide Wirkung aus. Bereits in das pflanzliche Gewebe eingedrungene pilzliche Krankheitserreger lassen sich erfolgreich kurativ bekämpfen. Dies ist besonders wichtig und vorteilhaft bei solchen Pilzkrankheiten, die nach eingetretener Infektion mit den sonst üblichen Fungiziden nicht mehr wirksam bekämpft werden können. Das Wirkungsspektrum der beanspruchten Verbindungen erfaßt eine Vielzahl verschiedener wirtschaftlich bedeutender, phytopathogener Pilze, wie z.B. Pyricularia oryzae, Leptosphaeria nodorum, Drechslera teres, echte Mehltauarten, Venturia inaequalis, Botrytis cinerea, Pseudocercosporella herpotrichoides, Rostpilze sowie Vertreter der Oomyceten wie z. B. Phytophthora infestans und Plasmopara viticola.

Die erfindungsgemäßen Verbindungen eignen sich daneben auch für den Einsatz in technischen Bereichen, beispielsweise als Holzschutzmittel, als Konservierungsmittel in Anstrichfarben, in Kühlschmiermittel für die Metallbearbeitung oder als Konservierungsmittel in Bohr- und Schneidölen.

Die Erfindung betrifft auch Mittel, die die Verbindungen der Formel I neben geeigneten Formulierungshilfsmitteln enthalten. Die erfindungsgemäßen Mittel enthalten die Wirkstoffe der Formel I im allgemeinen zu 1 bis 95 Gew.-%.

Sie können auf verschiedene Art formuliert werden, je nachdem wie es durch die biologischen und/oder chemisch-physikalischen Parameter vorgegeben ist. Als Formulierungsmöglichkeiten kommen daher in Frage: Spritzpulver (WP), emulgierbare Konzentrate (EC), wäßrige Dispersionen auf Öl- oder Wasserbasis (SC), Suspoemulsionen (SC), Stäubemittel (DP), Beizmittel, Granulate in Form von wasserdispergierbaren Granulaten (WG), ULV-Formulierungen, Mikrokapseln, Wachse oder Köder.

Diese einzelnen Formulierungstypen sind im Prinzip bekannt und werden beispielsweise beschrieben in:
Winnacker-Küchler, "Chemische Technologie", Band 7, C-Hauser Verlag, München, 4. Aufl. 1986; van Falkenberg, "Pesticides Formulations", Marcel Dekker N.Y., 2nd Ed. 1972-73; K. Martens, "Spray Drying Handbook", 3rd Ed. 1979, G. Goodwin Ltd. London.

Die notwendigen Formulierungshilfsmittel wie Inertmaterialien, Tenside, Lösungsmittel und weitere Zusatzstoffe sind ebenfalls bekannt und werden beispielsweise beschrieben in:
Watkins, "Handbood of insecticide Dust Diluents and Carriers", 2nd Ed., Darland Books, Caldwell N.J.; H.v. Olphen, "Introduction to Clay Colloid Chemistry", 2nd Ed., J. Wiley & Sons, N.Y.; Marschen, "Solvents Guide", 2nd Ed., Interscience, N.Y. 1950; McCutcheon's "Detergents and Emulsifiers Annual", MC Publ. Corp., Ridgewood N.J.; Sisley and Wood, "Encyclopedia of Surface Active Agents", Chem. Publ. Co. Inc., N.Y. 1964; Schönfeldt, "Grenzflächenaktive Äthylenoxidaddukte", Wiss. Verlagsgesell., Stuttgart 1976; Winnacker-Küchler, "Chemische Technologie", Band 7, C. Hauser Verlag München, 4. Aufl. 1986.

Die erfindungsgemäßen Wirkstoffe können in ihren handelsüblichen Formulierungen entweder allein oder in Kombination mit weiteren, literaturbekannten Fungiziden angewendet werden.

Als literaturbekannte Fungizide, die erfindungsgemäß mit den Verbindungen der Formel (I) kombiniert werden können, sind z. B. folgende Produkte zu nennen:
Anilazine, Benalaxyl, Benodanil, Benomyl, Binapacryl, Bitertanol, Buthiobat, Captafol, Captan, Carbendazim, Carboxin, CGD-94240 F, Chlobenzthiazone, Chlorthalonil, Cymoxanil, Cyproconazole, Cyprofuram, Dichlofluanid, Dichlomezin, Didobutrazol, Diethofencarb, Difluconazole, Dimethirimol, Dimethomorph, Diniconazole, Dinocap, Dithianon, Dodemorph, Dodine, Edifenfos, Ethirimol, Etridiazol, Fenarimol, Fenfuram, Fenpiclonil, Fenpropidin, Fenpropimorph, Fentinacetate, Fentinhydroxide, Fluaziram, Fluobenzimine, Fluorimide, Flusilazole, Flutolanil, Flutriafol, Folpet, Fosetylaluminium, Fuberidazole, Furalaxyl, Furmecyclox, Guazatine, Hexaconazole, Imazalil, Iprobenfos, Iprodione, Isoprothiolane, Kupferverbindungen wie Cu-oxychlorid, Oxine-Cu, Cu-oxide, Mancozeb, Maneb, Mepronil, Metalaxyl, Methasulfocarb, Methfuroxam, Myclobutanil, Nabam, Nitrothalidopropyl, Nuarimol, Ofurace, Oxadixyl, Oxycarboxin, Penconazol, Pencycuron, PP 969, Probenazole, Probineb, Prochloraz, Procymidon, Propamocarb, Propiconazol, Prothiocarb, Pyracarbolid, Pyrifenox, Pyroquilon, Rabenzazole, Sulfur, Tebuconazole, Thiabendazole, Thiofanatemethyl, Thiram, Tolclofos-methyl, Tolylfluanid, Triadimefon, Triadimenol, Tricyclazole, Tridemorph, Triflumizol, Triforine, Vinchlozolin, Zineb, Natriumdodecylsulfonate, Natrium-dodecylsulfat, Natrium-C13/C15-alkoholethersulfonat, Natrium-cetostearylphosphatester, Dioctyl-natrium-sulfosuccinat, Natrium-isopropylnaphthalenesulfonat, Natrium-methylenebisnaphthalenesulfonat, Cetyl-trimethyl-ammoniumchlorid, Salze von langkettigen primären, sekundären oder tertiären Aminen, Alkylpropyleneamine, Lauryl-pyrimidiniumbromid, ethoxilierte quarternierte Fettamine, Alkyl-dimethyl-benzyl-ammoniumchlorid und 1-Hydroxyethyl-2-alkyl-imidazolin.

Die oben genannten Kombinationspartner stellen bekannte Wirkstoffe dar, die zum großen Teil in GH.R Worthing, U.S.B. Walker, The Pesticide Manual, 7. Auflage (1983), British Crop Protection Council beschrieben sind.

Der Wirkstoffgehalt der aus den handelsüblichen Formulierungen bereiteten Anwendungsformen kann in weiten Bereichen variieren, die Wirkstoffkonzentration der Anwendungsformen kann zwischen 0,0001 und 95 Gew.-% Wirkstoff, vorzugsweise zwischen 0,001 und 1 Gew.-% liegen. Die Anwendung geschieht in einer den Anwendungsformen angepaßten üblichen Weise.

Nachfolgende Beispiele dienen zur Erläuterung der Erfindung.

### A. Formulierungsbeispiele

a) Ein Stäubemittel wird erhalten, indem man 10 Gew.-Teile Wirkstoff und 90 Gew.-Teile Talkum als Inertstoff mischt und in einer Schlagmühle zerkleinert.
b) Ein in Wasser leicht dispergierbares, benetzbares Pulver wird erhalten, indem man 25 Gew.-Teile Wirkstoff, 65 Gew.-Teile kaolinhaltige Quarz als Inertstoff, 10 Gew.-Teile ligninsulfonsaures Kalium und 1 Gew.-Teil oleoylmethyltaurinsaures Natrium als Netz- und Dispergiermittel mischt und in einer Stiftmühle mahlt.
c) Ein in Wasser leicht dispergierbares Dispersionskonzentrat stellt man her, indem man 40 Gew.-Teile Wirkstoff mit 7 Gew.-Teilen eines Sulfobernsteinsäurehalbesters, 2 Gew.-Teilen einers Ligninsuifonsäure-Natriumsalzes und 51 Gew.-Teilen Wasser mischt und in einer Reibkugelmühle auf eine Feinheit von unter 5 Mikron vermahlt.
d) Ein emulgierbares Konzentrat läßt sich herstellen aus 15 Gew.-Teilen Wirkstoff, 75 Gew.-Teilen Cyclohexanon als Lösungsmittel und 10 Gew.-Teilen oxethyliertem Nonylphenol (10 AeO) als Emulgator.
e) Ein Granulat läßt sich herstellen aus 2 bis 15 Gew.-Teilen Wirkstoff und einem inerten Granulatträgermaterial wie Attapulgit, Bimsgranulat und/oder Ouarzsand. Zweckmäßigerweise verwendet man eine Suspension des Spritzpulvers aus Beispiel b) mit einem Feststoffanteil von 30 % und spritzt diese auf die Oberfläche eines Attapulgitgranulats, trocknet und vermischt innig. Dabei beträgt der Gewichtsanteil des Spritzpulvers ca. 5 % und der des inerten Trägermaterials ca. 95 % des fertigen Granulats.

### B Biologische Beispiele (Verwendung als Fungizid)

### Beispiel 1

Gerstenpflanzen werden im 3-Blattstadium mit Konidien des Gerstenmehltaus (Erysiphe graminis f. sp. hordei) stark inokuliert und in einem Gewächshaus bei 20 °C und einer relativen Luftfeuchte von 90-95 % aufgestellt. 24 Stunden nach Inokulation werden die Pflanzen mit den in Tabelle 1 aufgeführten Verbindungen in den angegebenen Wirkstoffkonzentrationen gleichmäßig benetzt. Nach einer Inkubationszeit von 10 Tagen werden die Pflanzen auf Befall mit Gerstenmehltau untersucht. Der Befallsgrad wird ausgedrückt in % befallener Blattfläche, bezogen auf unbehandelte, zu 100 % infizierte Kontrollpflanzen.

Bei 500 mg Wirkstoff/I Spritzbrühe zeigen die folgenden Substanzen eine vollständige Befallsunterdrückung.
Verbindungen gemäß Beispiel Nr.
9, 17, 25, 30, 55, 80, 93, 99, 100

### Beispiel 2

Gerstenpflanzen der Sorte "Igri" werden im 2-Blatt-Stadium mit einer wäßrigen Suspension der beanspruchten Verbindungen tropfnaß behandelt.

Nach dem Antrocknen des Spritzbelages werden die Pflanzen mit einer wäßrigen Sporensuspension von Pyrenophora teres inokuliert und für 16 h in einer Klimakammer bei 100 % rel. Luftfeuchte inkubiert. Anschließend werden die infizierten Pflanzen im Gewächshaus bei 25 °C und 80 % rel. Luftfeuchte weiterkultiviert.

Ca. 1 Woche nach Inokulation wird der Befall ausgewertet und der Befallsgrad in befallener Blattfläche im Vergleich zur unbehandelten, zu 100 % infizierten Kontrolle bonitiert.

Bei 500 mg/l Spritzbrühe zeigen die folgenden Substanzen eine vollständige Befallsunterdrückung.
Verbindungen gemäß Beispiel Nr.
2, 9, 10, 13, 14, 17, 25, 55, 80, 93, 106.

### Beispiel 3

Weizen der Sorte "Jubilar' wird im 2-Blatt-Stadium mit wässrigen Suspensionen der beanspruchten Verbindungen tropfnaß behandelt.

Nach dem Antrocknen des Spritzbelages werden die Pflanzen mit einer wässrigen Sporensuspension von Puccinia recondita inokuliert. Die Pflanzen werden für ca. 16 Stunden tropfnaß in eine Klimakammer mit 20 °C und ca. 100 % rel. Luftfeuchte gestellt. Anschließend werden sie in einem Gewächshaus bei einer Temperatur von 22 - 25 °C und 50 - 70 % rel. Luftfeuchte weiterkultiviert.

Nach einer Inkubationszeit von ca. 2 Wochen sporuliert der Pilz auf der gesamten Blattoberfläche der nicht behandelten Kontrollpflanzen (100 % Infektion), so daß eine Befallsauswertung der Versuchspflanzen vorgenommen werden kann. Der Befallsgrad wird in befallener Blattfläche im Vergleich zu den unbehandelten, infizierten Kontrollpflanzen ausgedrückt.

Bei 500 mg/l Spritzbrühe zeigen die folgenden Substanzen eine vollständige Befallsunterdrückung.
Verbindungen gemäß Beispiel Nr.
2, 9, 12, 17, 25, 30, 80, 93, 99.

### Biologische Beispiele (Verwendung als Akarizid/Insektizid)

### Beispiel 1

Mit schwarzer Bohnenblattlaus (Aphis fabae) stark besetzte Ackerbohnen (Vicia faba) werden mit wäßrigen Verdünnungen von Spritzpulverkonzentraten mit 250 ppm Wirkstoffgehalt bis zum Stadium des beginnenden Abtropfens besprüht. Die Mortalität der Blattläuse wird nach 3 Tagen bestimmt. Eine 100 %ige Abtötung kann mit den Verbindungen gemäß Beispiel Nr. 2, 9, 13, 17, 19, 25, 30, 55, 80, 93, 99, 106, 1741, 1749, 1750 erzielt werden.

### Beispiel 2

Mit Bohnenspinnmilben (Tetranychus urticae, Vollpopulation) stark befallene Bohnenplanzen (Phaseolus v.) wurden mit der wässrigen Verdünnung eines Spritzpulverkonzentrates, das 250 ppm des jeweiligen Wirkstoffes enthielt, gespritzt.

Die Mortalität der Milben wurde nach 7 Tagen kontrolliert. 100 % Abtötung wurde mit den foglenden Verbindungen erreicht.
Verbindungen gemäß Beispiel Nr.
2, 9, 10, 17, 19, 25, 30, 55, 80, 93, 94, 99, 106, 1741.

### Beispiel 3

Filterpapierscheiben mit aufliegenden Eiern von Baumwollwanzen (Oncopeltus fasciatus) werden mit jeweils 0,5 ml wässriger Verdünnung der zu testenden Formulierung behandelt. Nach Antrocknen des Belages wird die Petrischale verschlossen und der Innenraum auf max. Luftfeuchtigkeit gehalten. Nach Aufbewahrung bei Raumtemperatur werden nach 7 Tagen die ovizide und larvizide Wirkung ermittelt. Mit 250 ppm Wirkstoffgehalt wurde für die folgenden Verbindungen 100 % Mortalität erzielt.
Verbindungen gemäß Beispiel Nr.
2, 9, 10, 13, 17, 19, 25, 30, 55, 80, 93, 95, 99, 106, 312, 1741, 1749, 1750.

### Beispiel 4

Auf die Innenseite des Deckels und des Bodens einer Petrischale werden jeweils 1 ml der zu testenden Formulierung emulgiert in Wasser gleichmäßig aufgetragen und nach dem Antrocknen des Belages jeweils 10 Imagines der Hausfliege (Musca domestica) eingegeben. Nach dem Verschließen der Schalen werden diese bei Raumtemperatur aufbewahrt und nach 3 Stunden die Mortalität der Versuchstiere bestimmt. Bei 250 ppm (bezogen auf den Gehalt an Wirkstoff) zeigen die folgenden Präparate eine gute Wirkung (100 % Mortalität) gegenüber der Stubenfliege.
Verbindungen gemäß Beispiel Nr.
9, 10, 17, 18, 19, 25, 55, 80, 93, 99, 106, 1741, 1750

### Beispiel 5

Reissaatgut wird auf Watte in Zuchtgläsern feucht zur Keimung gebracht und nach dem Heranwachsen auf ca. 8 cm Halmlänge mit den Blättern in die zu prüfende Testlösung gegeben. Nach dem Abtropfen werden die so behandelten Reispflanzen getrennt nach Prüfkonzentration in Zuchtbehälter gegeben und mit je 10 Larven (L3) der Art Nilaparvata lugens besetzt. Nach Aufbewahren der verschlossenen Zuchtbehälter bei 21 °C kann nach 4 Tagen die Mortalität der Zikadenlarven bestimmt werden.
Unter diesen Versuchsbedingungen zeigen die Verbindungen gemäß Beispiel Nr. 9, 25, 30, 93, 99, 432 bei 250 ppm a.i. Testkonzentration eine 100 %ige Wirkung.

### Beispiel 6

Weizensaatgut wird unter Wasser 6 Stunden vorgekeimt, danach in 10 ml Glas-Prüfröhrchen gegeben und mit je 2 ml Erde abgedeckt. Nach Zugabe von 1 ml Wasser bleiben die Pflanzen in den Zuchtgläschen bis zum Erreichen einer Wuchshöhe von ca. 3 cm unter Raumtemperatur (21°C) stehen. Anschließend werden mittlere Diabrotica-Larvenstadien (je 10 Stück) in die Gläschen auf die Erde gegeben und nach 2 Stunden 1 ml der zu überprüfenden Konzentration an Testflüssigkeit auf die Erdoberfläche in die Gläschen pipettiert.

Nach 5 Tagen Standzeit unter Laborbedingung (21°C) wird die Erde bzw. die Wurzelteile auf lebende Diabrotica-Larven durchsucht und die Mortalität festgestellt.

Die Verbindungen gemäß Beispiel Nr. 2, 17, 19, 25, 93, 99 erwiesen sich unter den genannten Versuchsbedingungen bei 250 ppm a.i. Testkonzentration bis 100 % wirksam.

### Beispiel 7

In vitro-Test an tropischen Rinderzecken (Boophilus microplus) In folgender Versuchsanordnung ließ sich die Wirksamkeit der beanspruchten Verbindungen gegen Zecken nachweisen:

Zur Herstellung einer geeigneten Wirkstoffzubereitung wurden die Wirkstoffe 10 %ig (G/V) in einer Mischung, bestehend aus Dimethylformamid (85 g), Nonylphenolpolyglykolether (3 g) und oxethyliertes Rizinusöl (7 g), gelöst und die so erhaltenen Emulsionskonzentrate mit Wasser auf eine Prüfkonzentration von 500 ppm verdünnt.

In diese Wirkstoffverdünnungen wurden jeweils zehn vollgesogene Weibchen der tropischen Zecke, Boophilus microplus, für fünf Minuten eingetaucht. Die Zecken wurden anschließend auf Filterpapier getrocknet und dann zum Zwecke der Eiablage mit der Rückseite auf einer Klebefolie befestigt. Die Aufbewahrung der Zecken erfolgte im Wärmeschrank bei 28°C und einer Luftfeuchtigkeit von 90 %.

Zur Kontrolle wurden Zeckenweibchen lediglich in Wasser eingetaucht.
Zur Bewertung der Wirksamkeit wurde zwei Wochen nach der Behandlung die Hemmung der Eiablage herangezogen. Dabei besagen 100 %, daß keine, 0 daß alle Zecken Eier abgelegt haben. In diesem Test bewirkten die Verbindungen 10, 19, 30 und 106 in einer Wirkstoffkonzentration von 500 ppm jeweils eine 100 %ige Hemmung der Eiablage.

### Herstellungsbeispiele

### Beispiel A

### 4-(4-tert-Butylcyclohexylamino)-5-Chlor-6-ethyl-pyrimidin

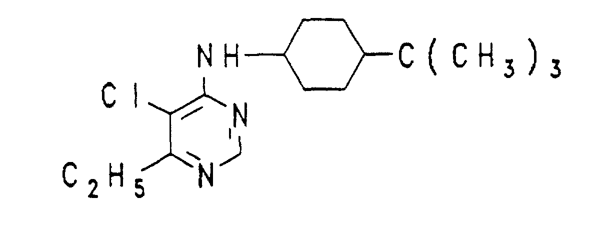

3,5 g (0,02 mol) 4,5-Dichlor-6-ethyl-pyrimidin und 7,8 g (0,05 mol) 4-tert-Butylcyclohexylamin wurden ohne Lösungsmittel zwei Stunden auf 100°C erwärmt. Nach Abkühlen auf Raumtemperatur wurde mit Methylenchlorid/Wasser aufgearbeitet, die organische Phase getrocknet und eingeengt. Zur weiteren Reinigung und zur Trennung der cis/trans Isomeren wurde an Kieselgel mit Petrolether/Ethylacetat 7:3 chromatographiert.

Es wird zunächst das trans-Cyclohexylamino-Derivat eluiert (0,8 g gelbes Öl erstarrt, Fp. 94 - 96°). Nach einer Mischfraktion, die verworfen wurde, wurde zuletzt reines cis-Cyclohexylamino-Derivat erhalten (3,0 g gelbes Öl).

### Herstellung von 4-tert-Butylcyclohexylamin

312 g 4-tert-Butylcyclohexanon wurden in 500 ml mit Ammoniak gesättigtem Methanol in Gegenwart von 10 g Raney-Nickel bei 100°C und 100 bar hydriert. Nach Abfiltrieren des Katalysators wurde eingeengt und das Rohprodukt am Dünnschichtverdampfer gereinigt (105°/0,5 mm). Man erhielt 303 g farblose Flüssigkeit. Das Produkt ist ein Isomerengemisch, in dem das cis-Cyclohexylamin-Derivat überwiegt.

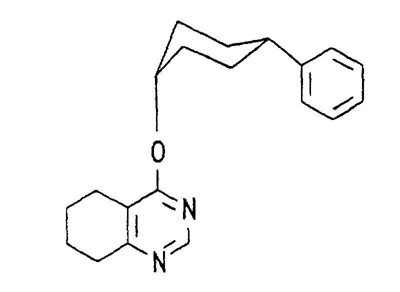

### 4-(cis-4-Phenyl-cyclohexyloxy)-5,6,7,8-tetrahydrochinazolin

Zu einer Lösung von 1,85 g (105 mmol) cis-4-Phenylcyclohexanol in 30 ml abs. THF gab man portionsweise 0,5 g (16,7 mmol) 80% NaH. Danach wurde 1 Stunde auf 50°C erwärmt und 1,5 g (8,75 mmol) 4-Chlor-5,6,7,8-tetrahydrochinazolin, gelöst in 15 ml abs. THF, zugetropft. Das Reaktionsgemisch wurde anschließend 2 Stunden auf Rückfluß erhitzt. Nach Abkühlen auf RT wurde auf ges. NH₄Cl-Lösung gegossen, mit Ether extrahiert und die vereinigten organischen Phasen über MgSO₄ getrocknet. Das Lösemittel wurde i.Vak. abgedampft und der Rückstand (2,7 g) durch Flash-Chromatographie über Kieselgel mit n-Hexan/Ethylacetat (2:1) gereinigt. Nach Einengen erhielt man 1,5 g (50,2 % d.Th), farblose Kristalle, Fp. 109°C.

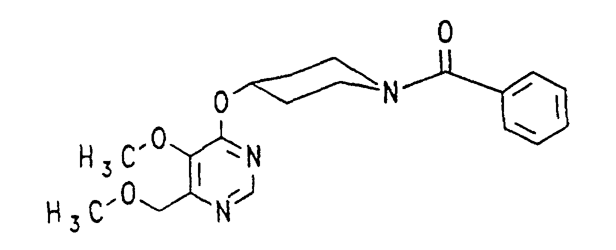

### 4-(N-Benzoyl-piperidyl-4-oxy)-5-methoxy-6-methoxymethylpyrimidin

Zu einer Suspension von 0,66 g (22 mmol) 80% NaH in THF wurden 2,9 g (14,3 mmol) N-Benzoyl-4-hydroxypiperidin (hergestellt durch NaBH₄-Reduktion von N-Benzoyl-piperidin-4-on) zugegeben. Danach wurde 1 Stunde auf 35-40°C erwärmt und anschließend 2,50 g (13,3 mmol) 4-Chlor-5-methoxy-6-methoxymethylpyrimidin unverdünnt zugegeben. Das Reaktionsgemisch wurde 5 Stunden auf 40°C erwärmt, auf wenig ges. NH₄CI-Lösung gegossen und 5 mal mit Ethylacetat extrahiert. Die vereinigten organischen Phasen wurden über MgSO₄ getrocknet und der Rückstand (3,4 g) über Kieselgel durch Flash-Chromatographie mit Ethylacetat gereinigt. Nach Einengen erhielt man 0,6 g (13 % d. T) gelbes Öl, n_{D}²⁰=1.5815.

Weitere Beispiele finden sich in den folgenden Tabellen I - IV.

Verwendete Abkürzungen

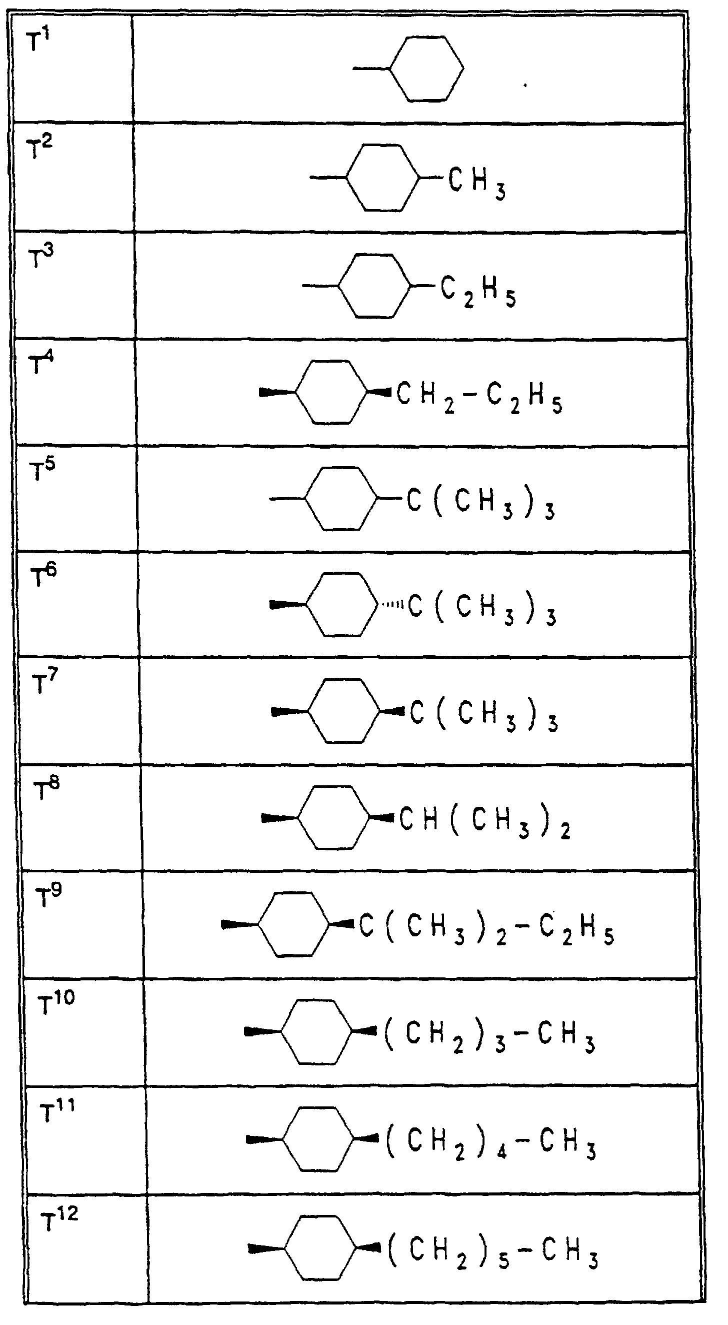

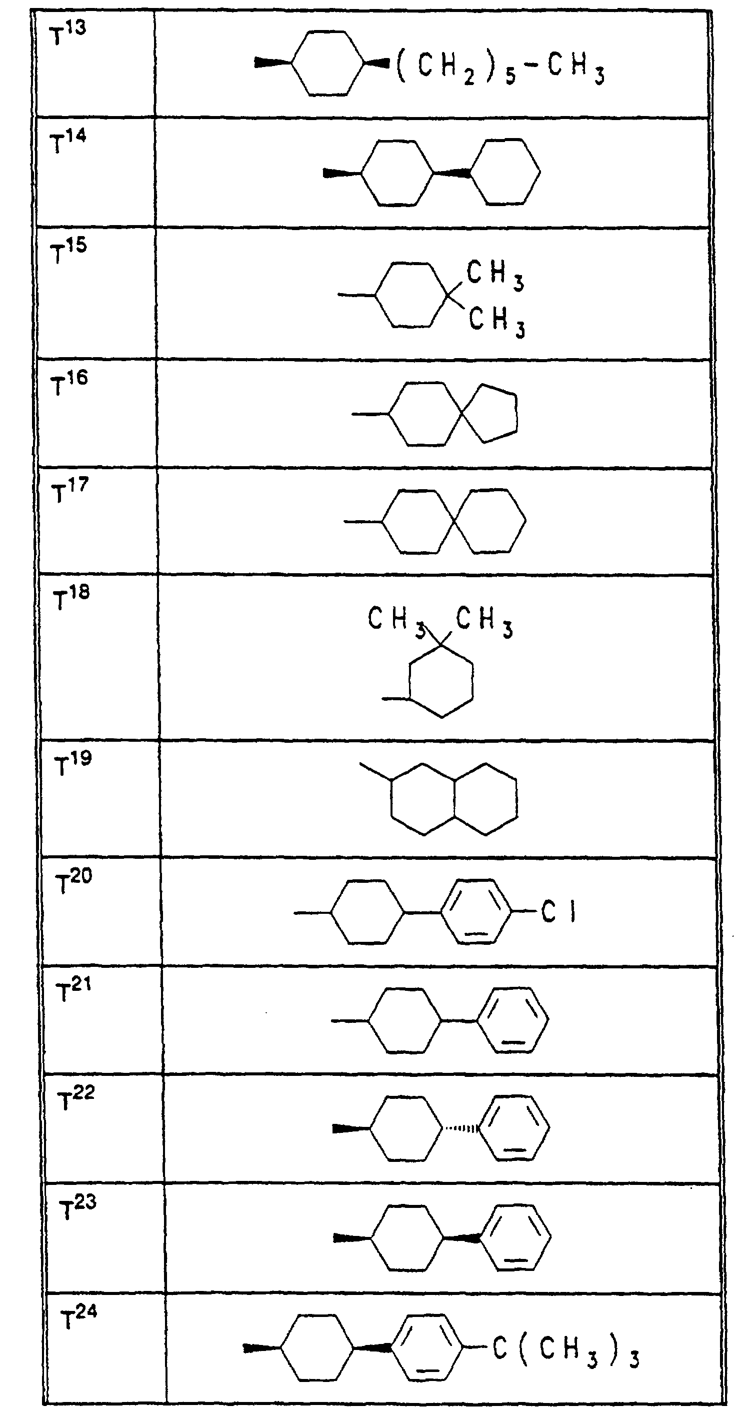

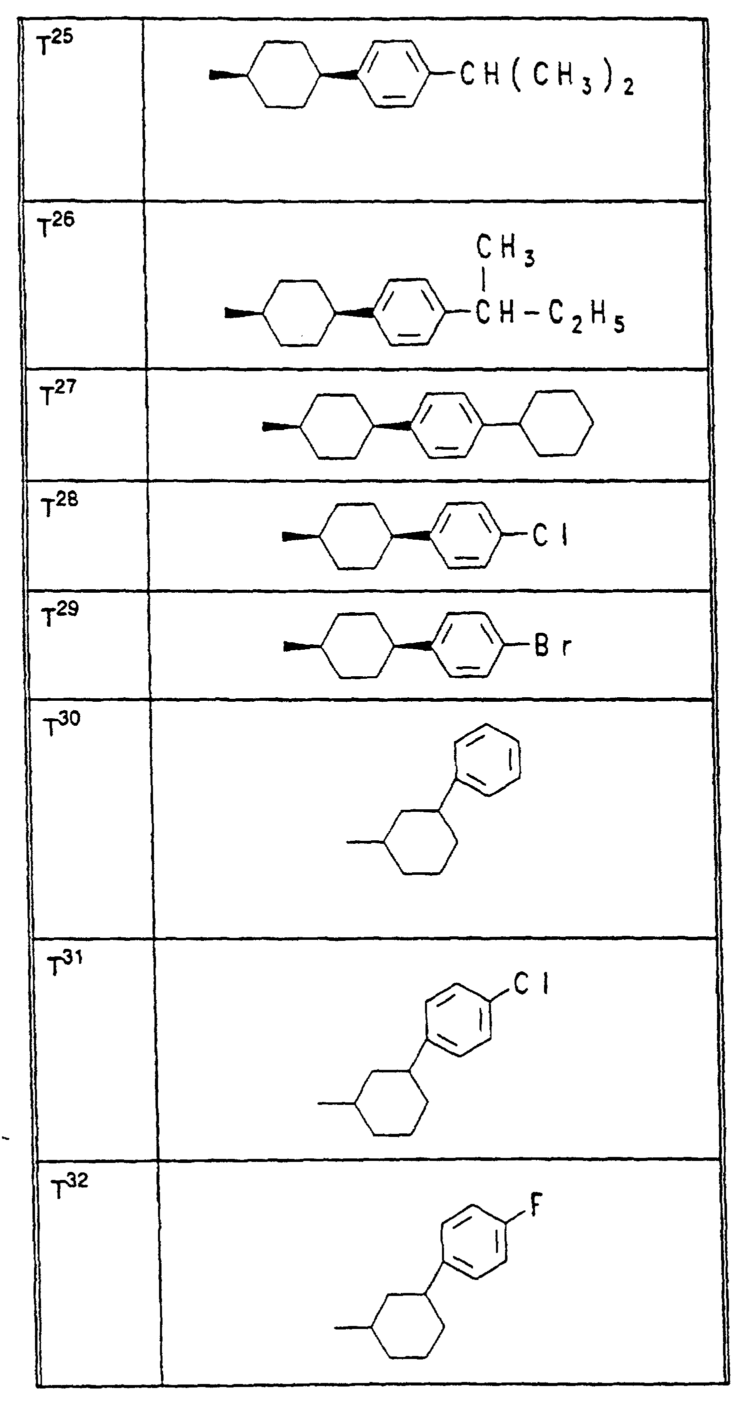

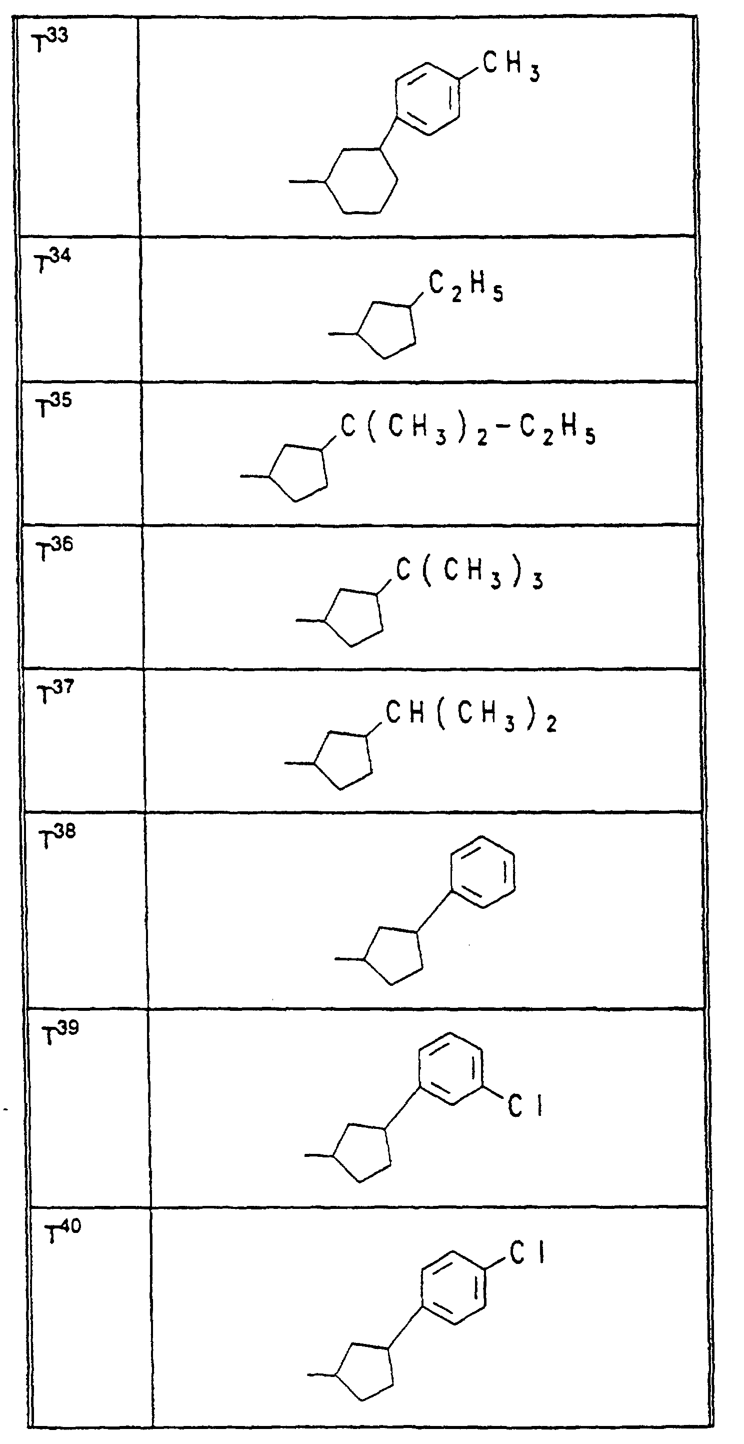

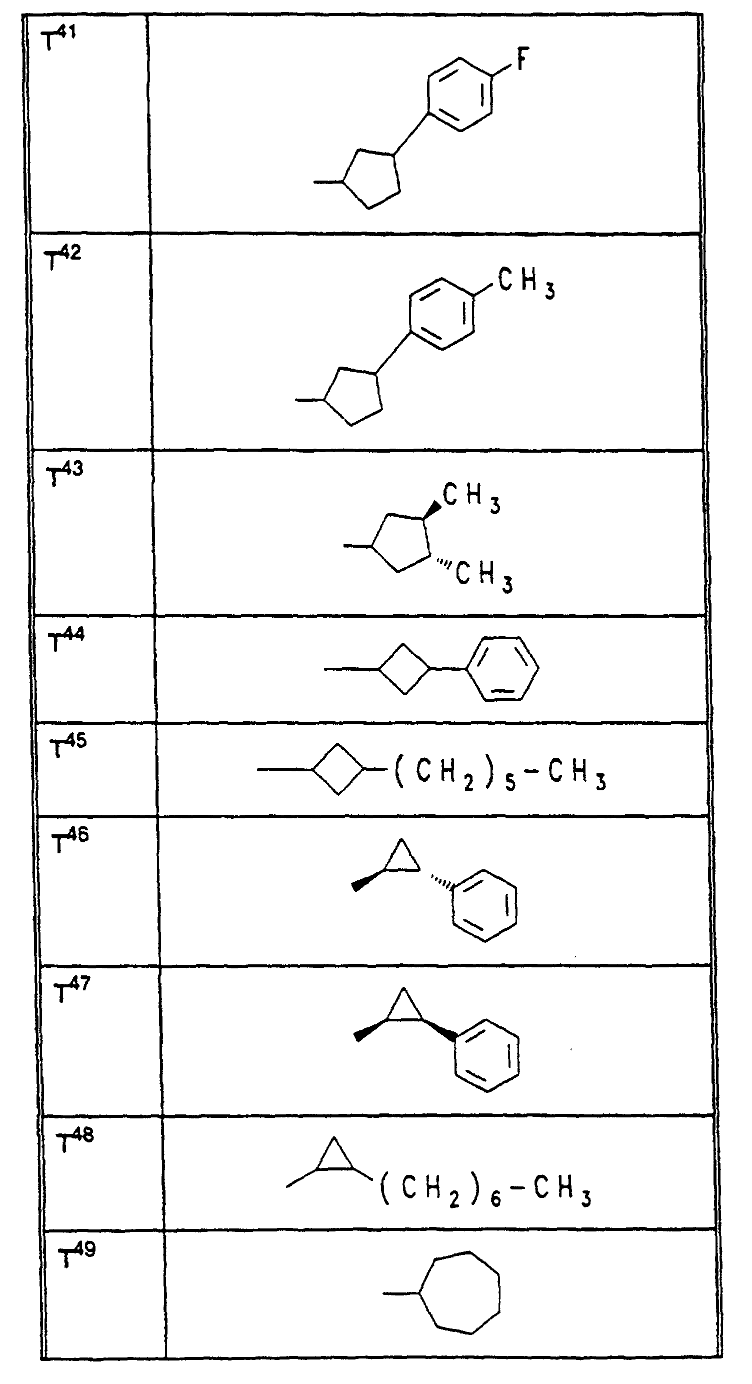

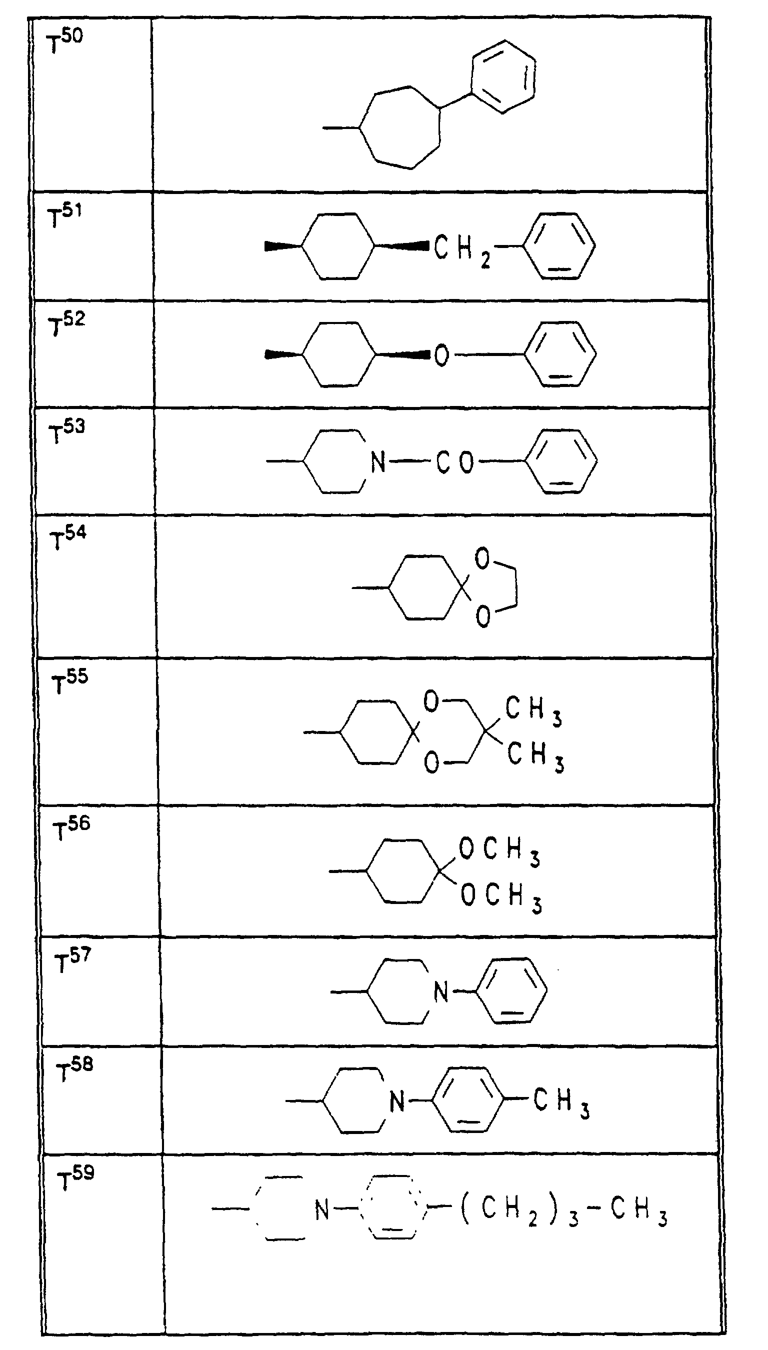

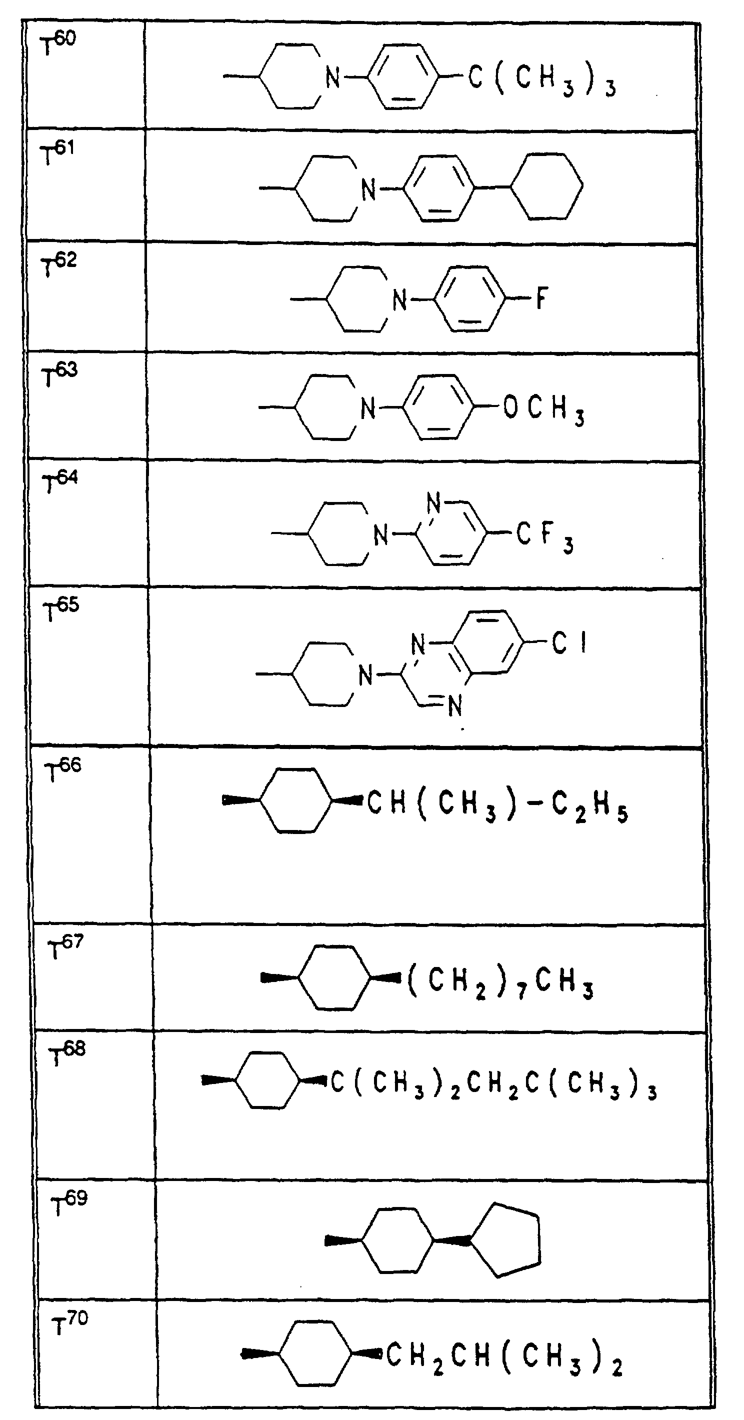

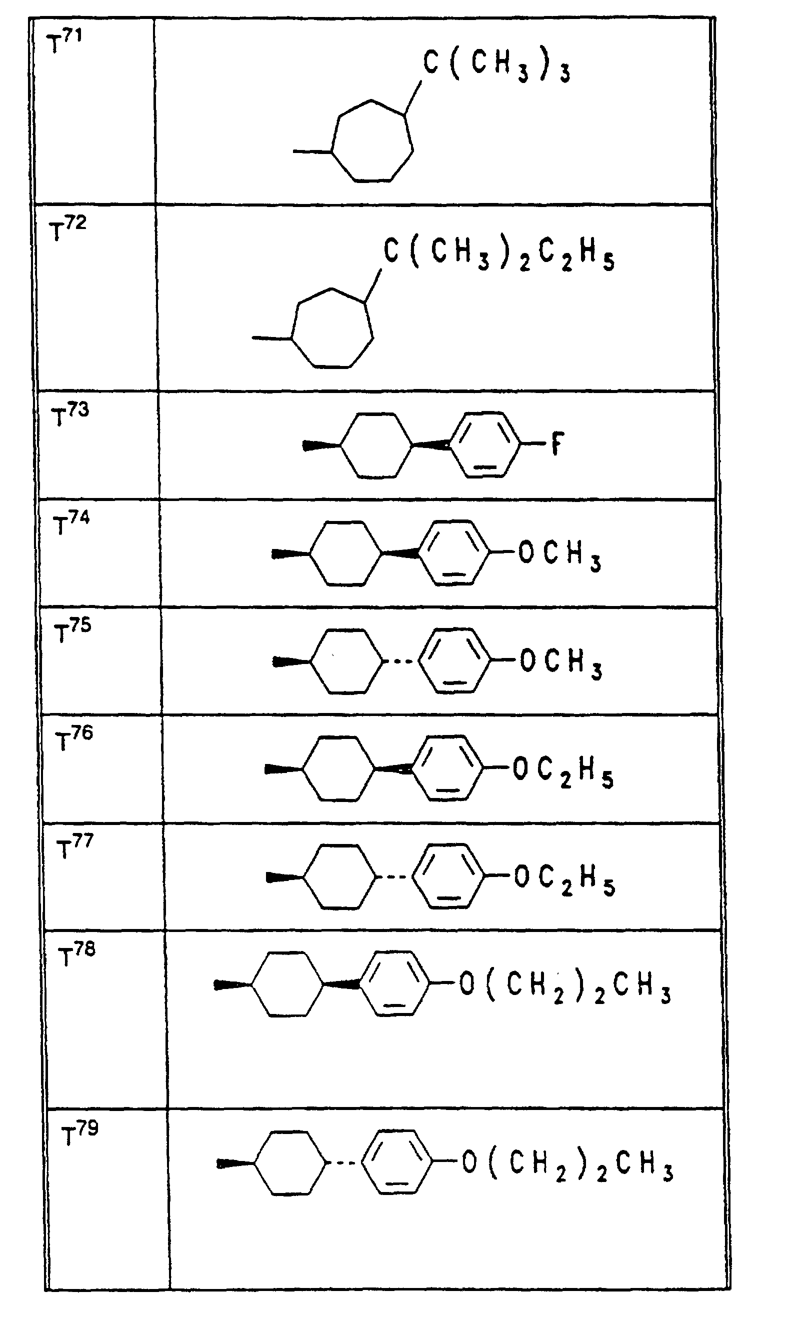

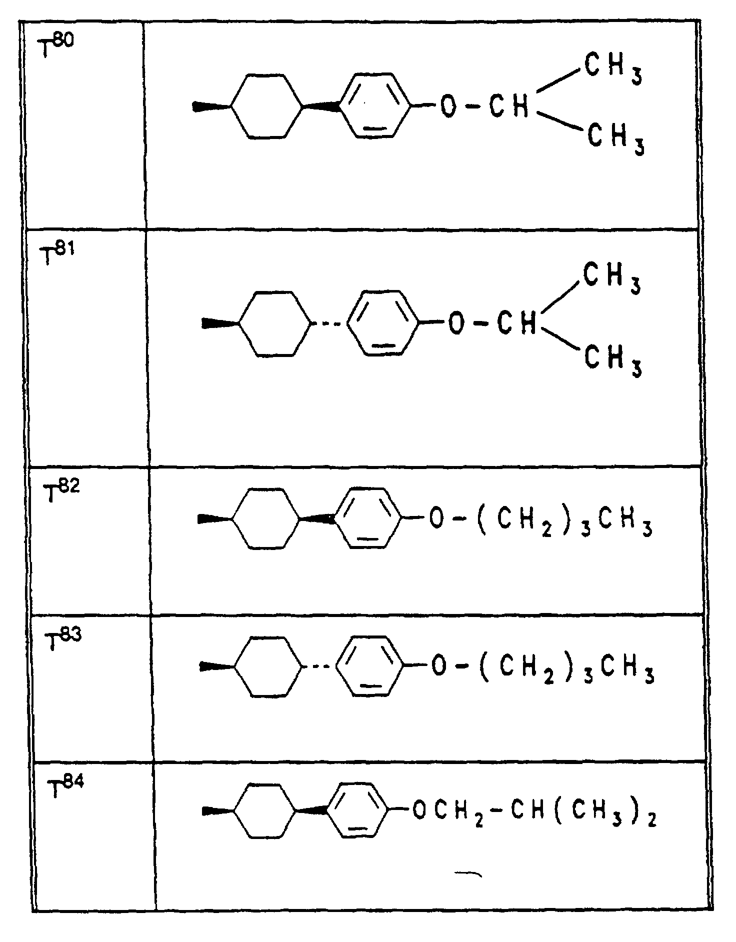

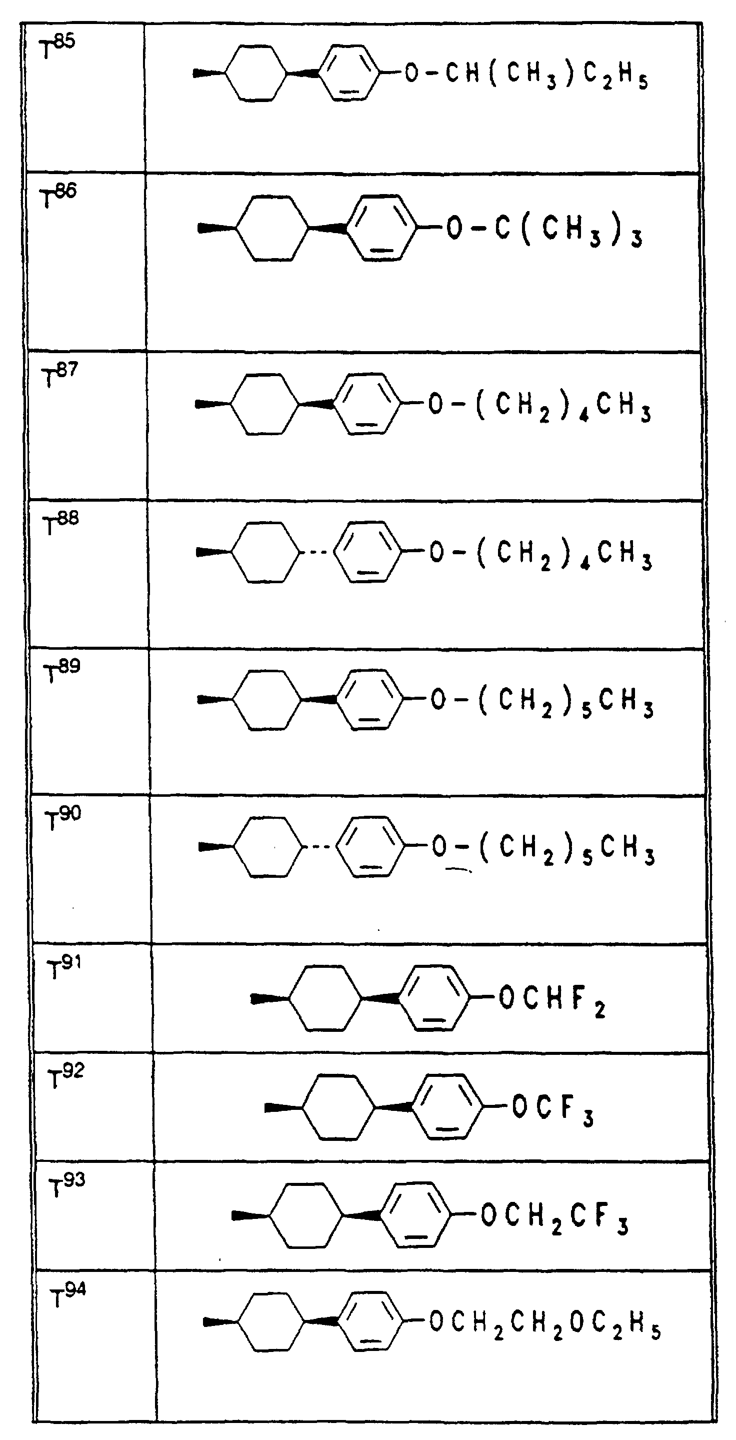

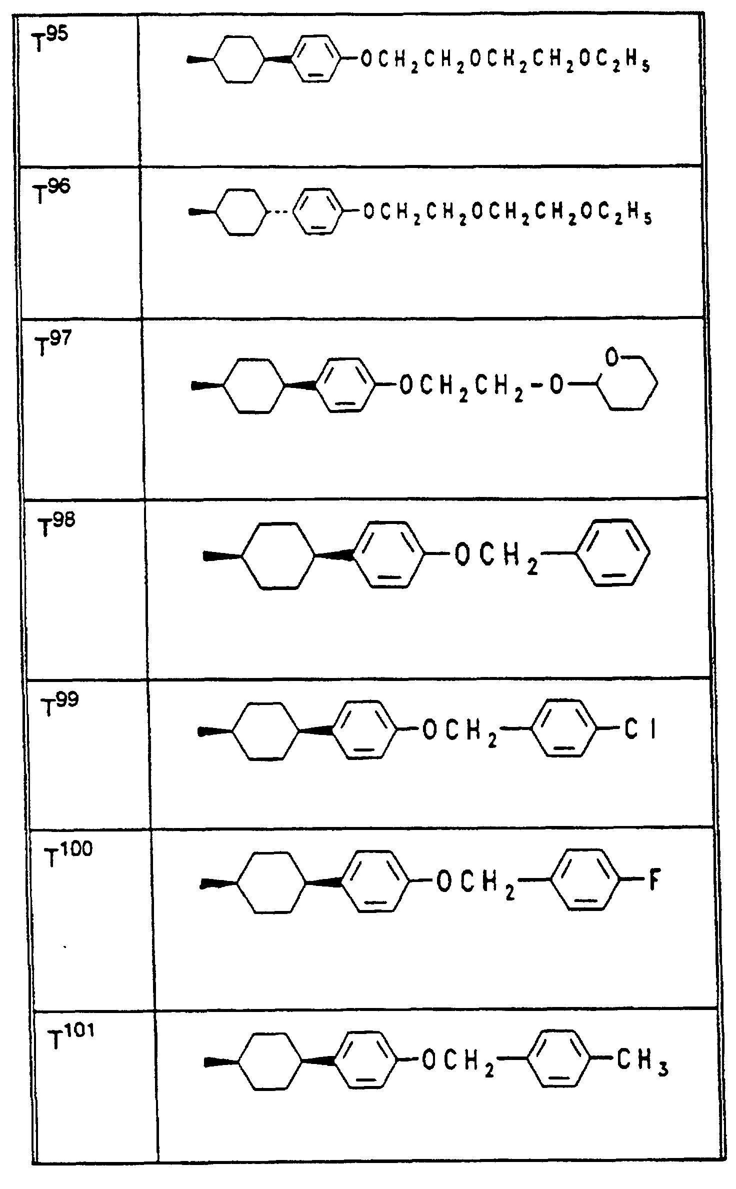

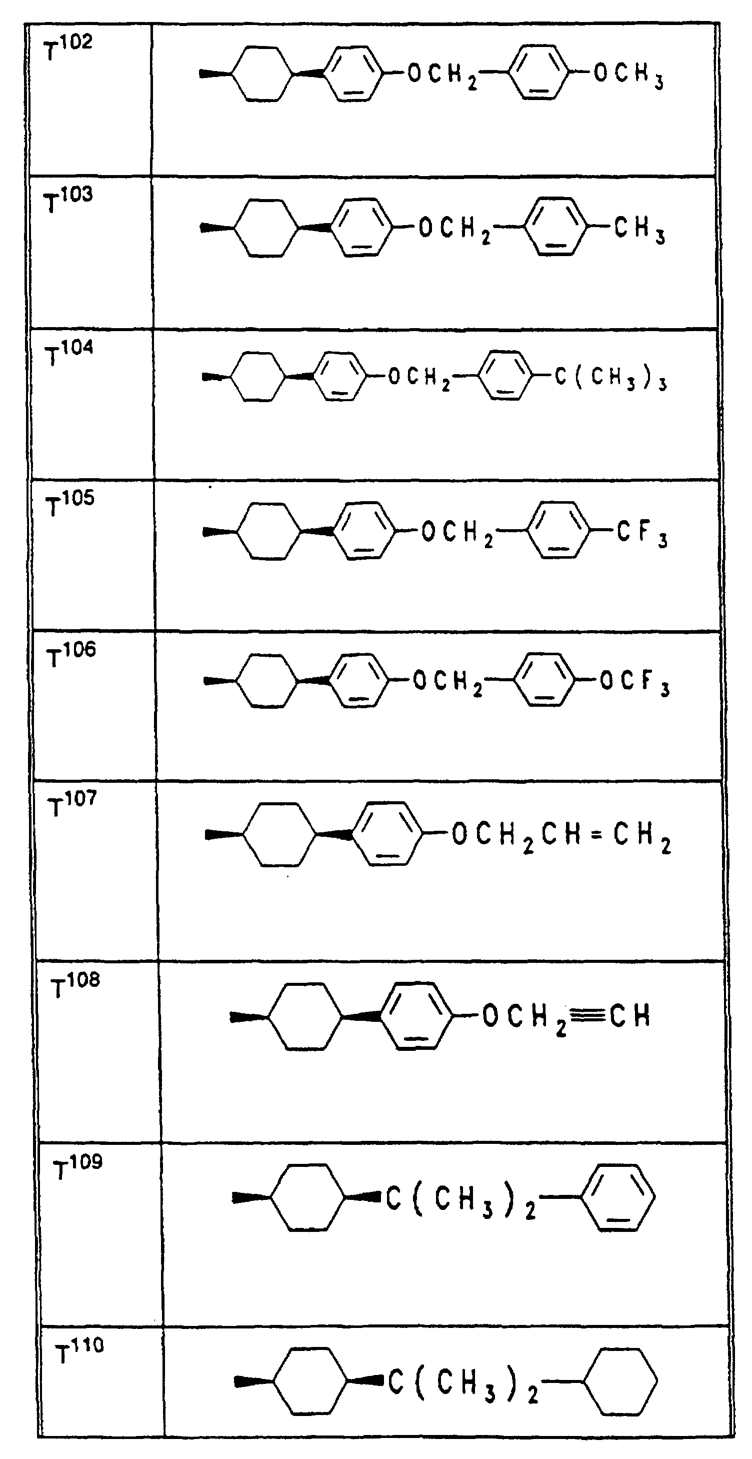

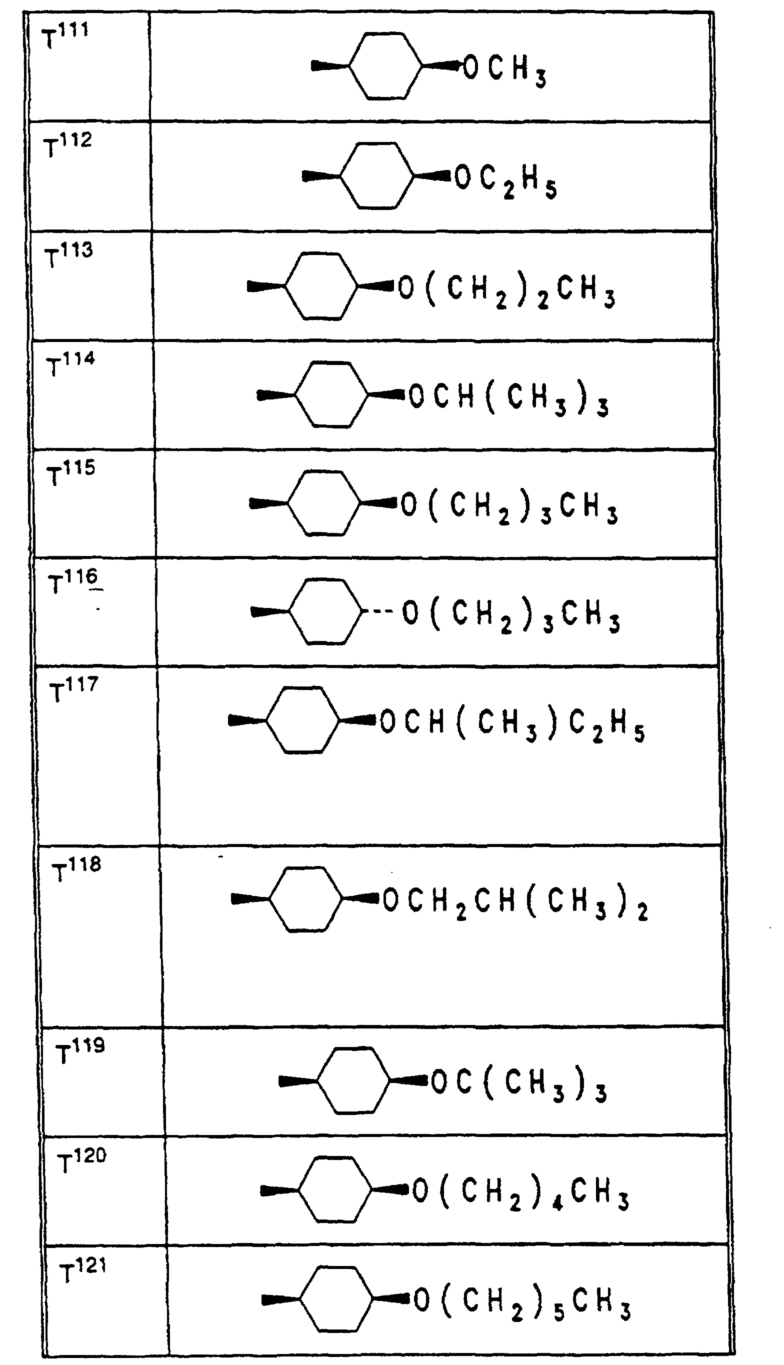

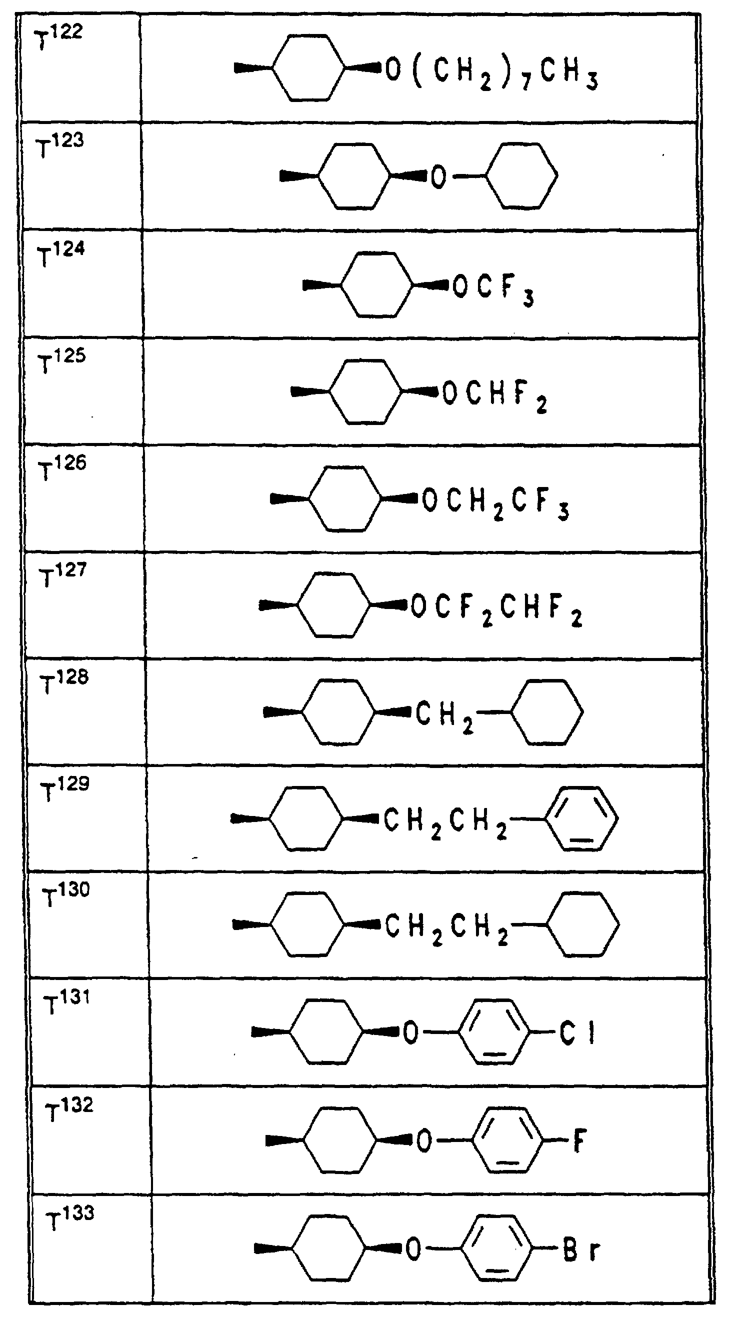

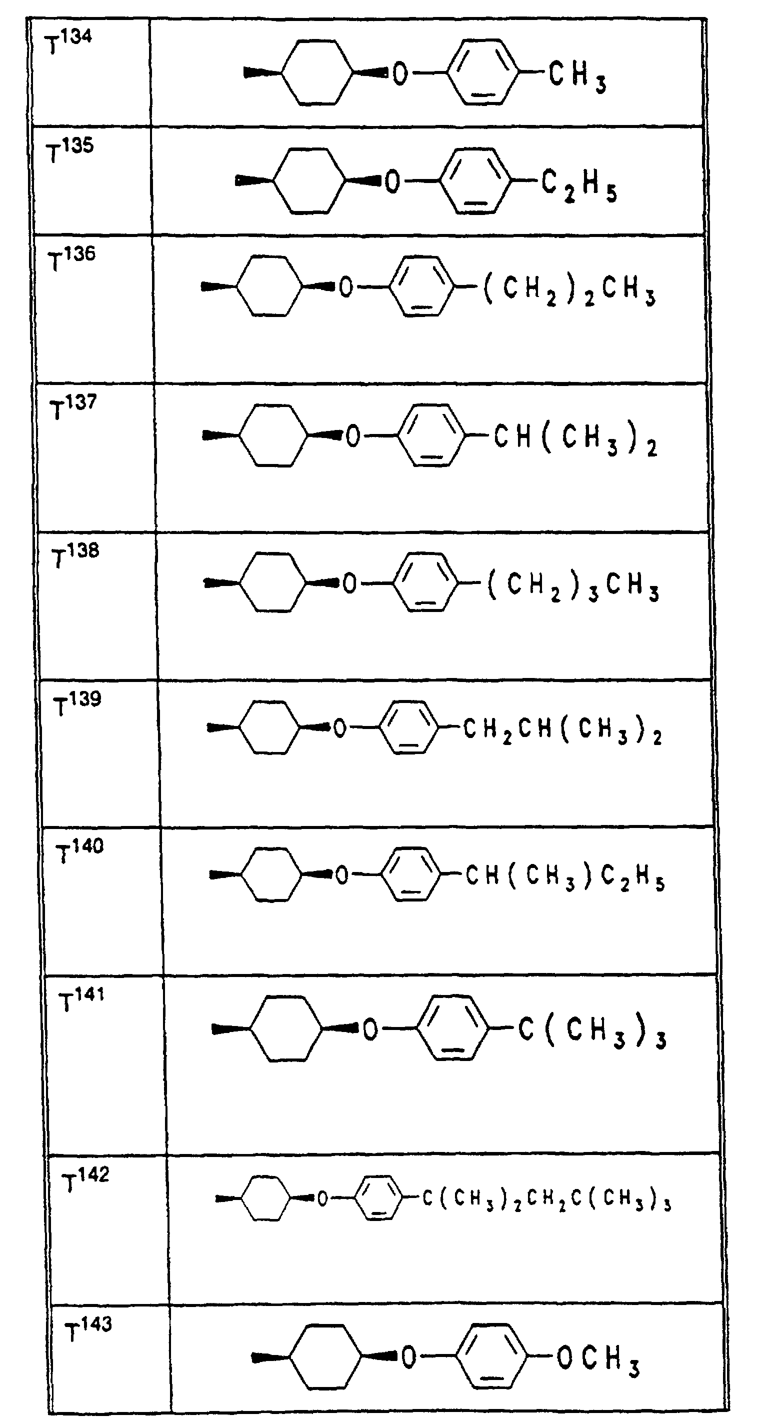

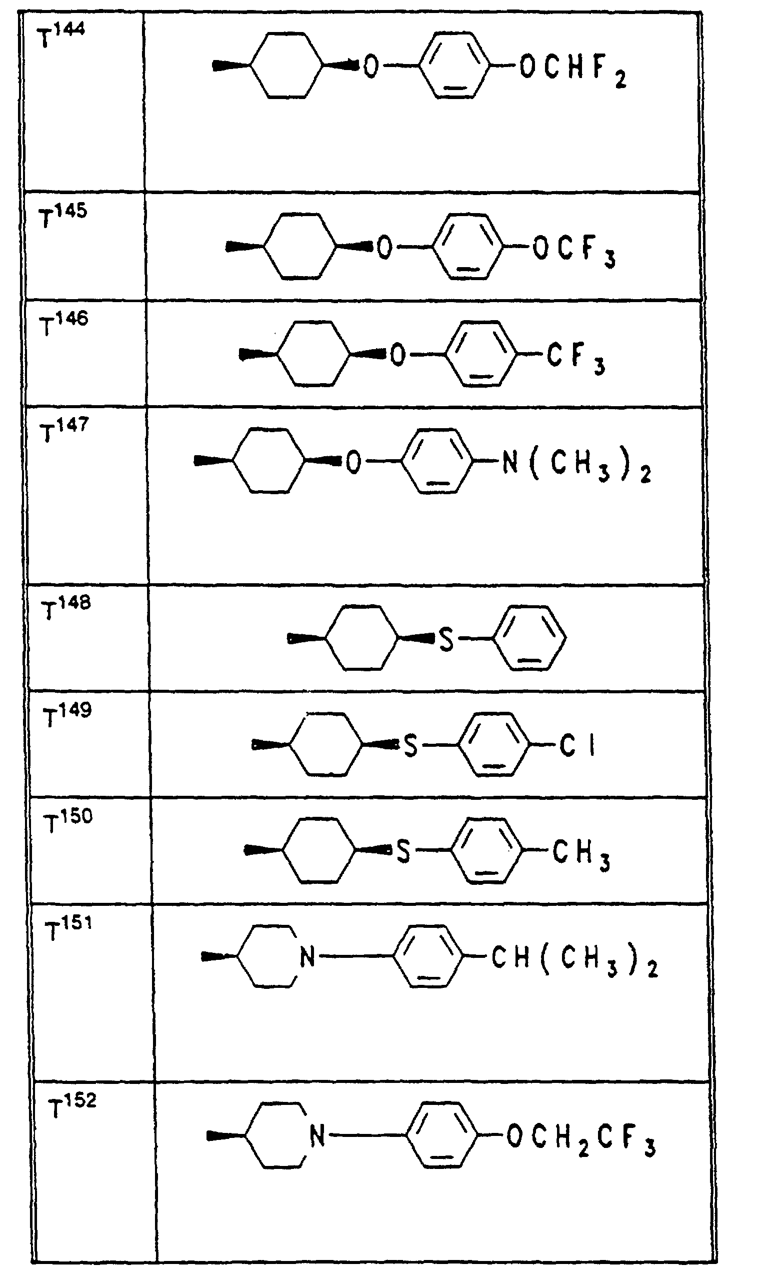

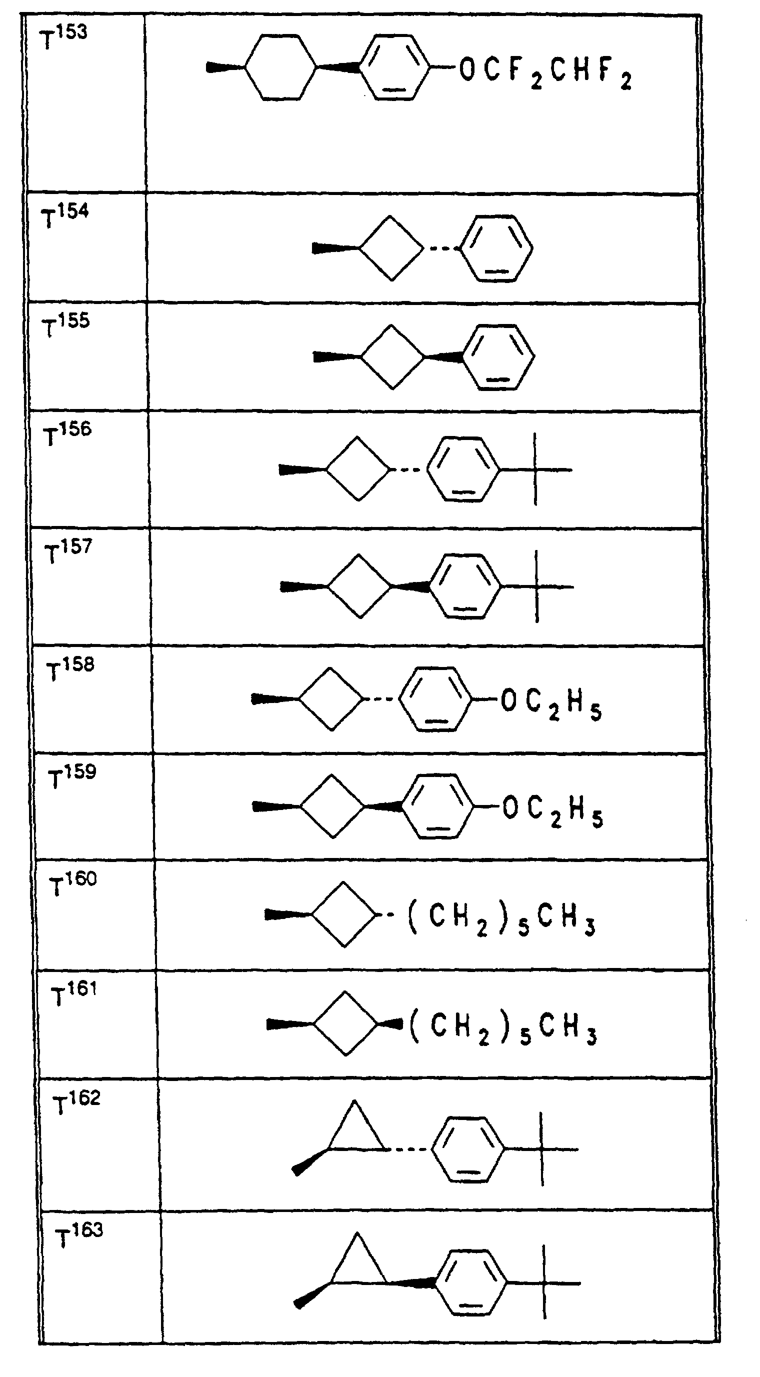

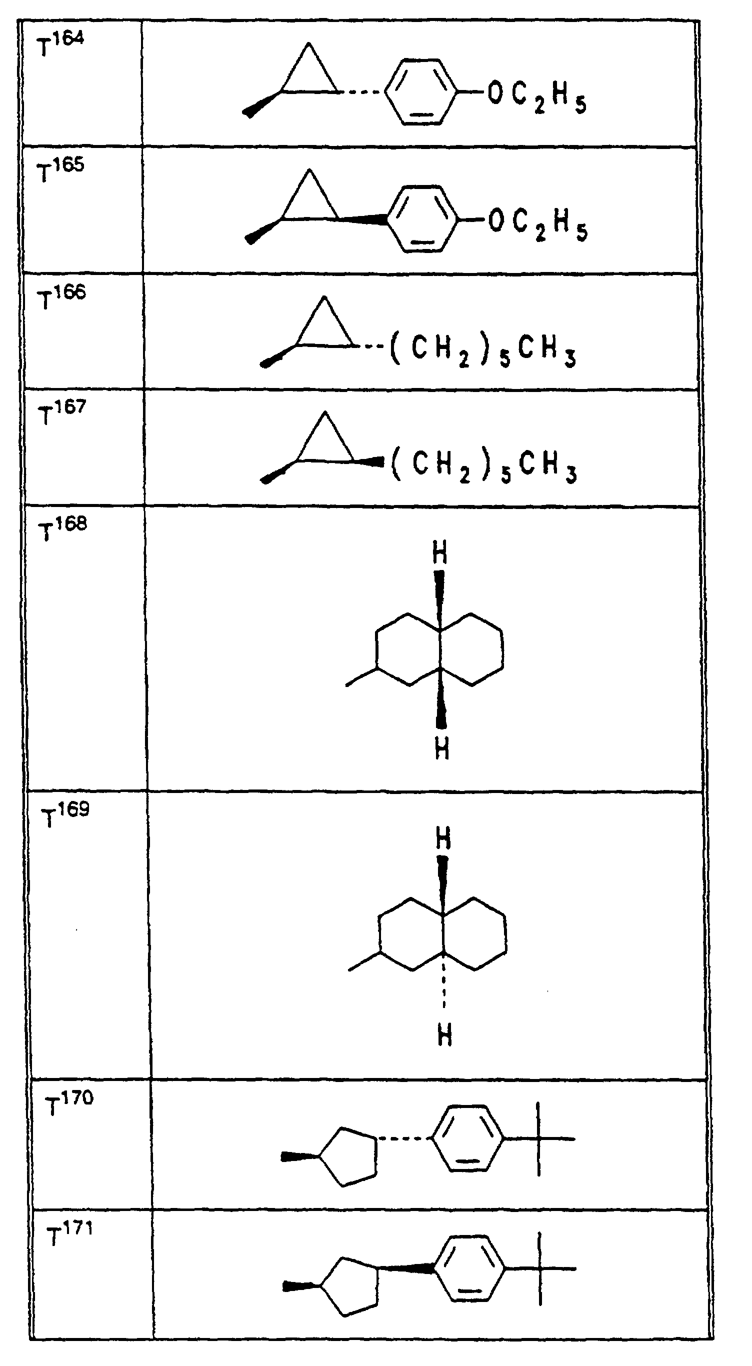

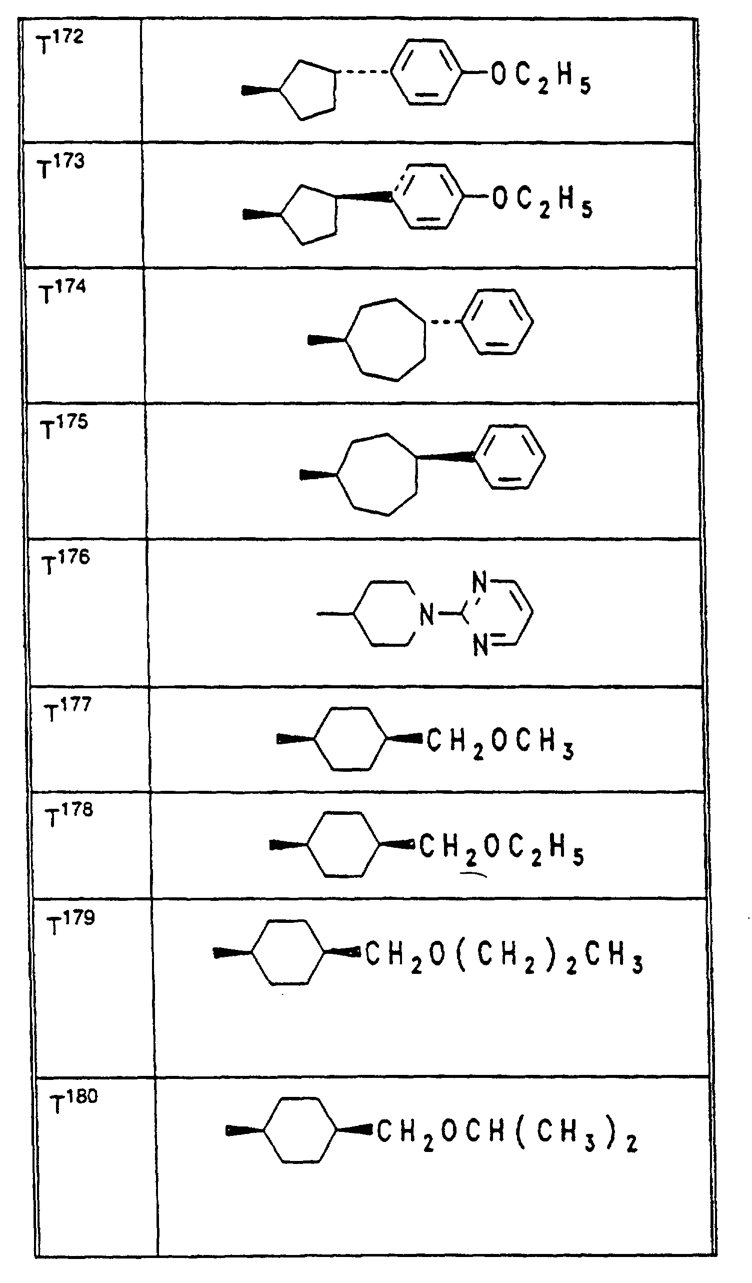

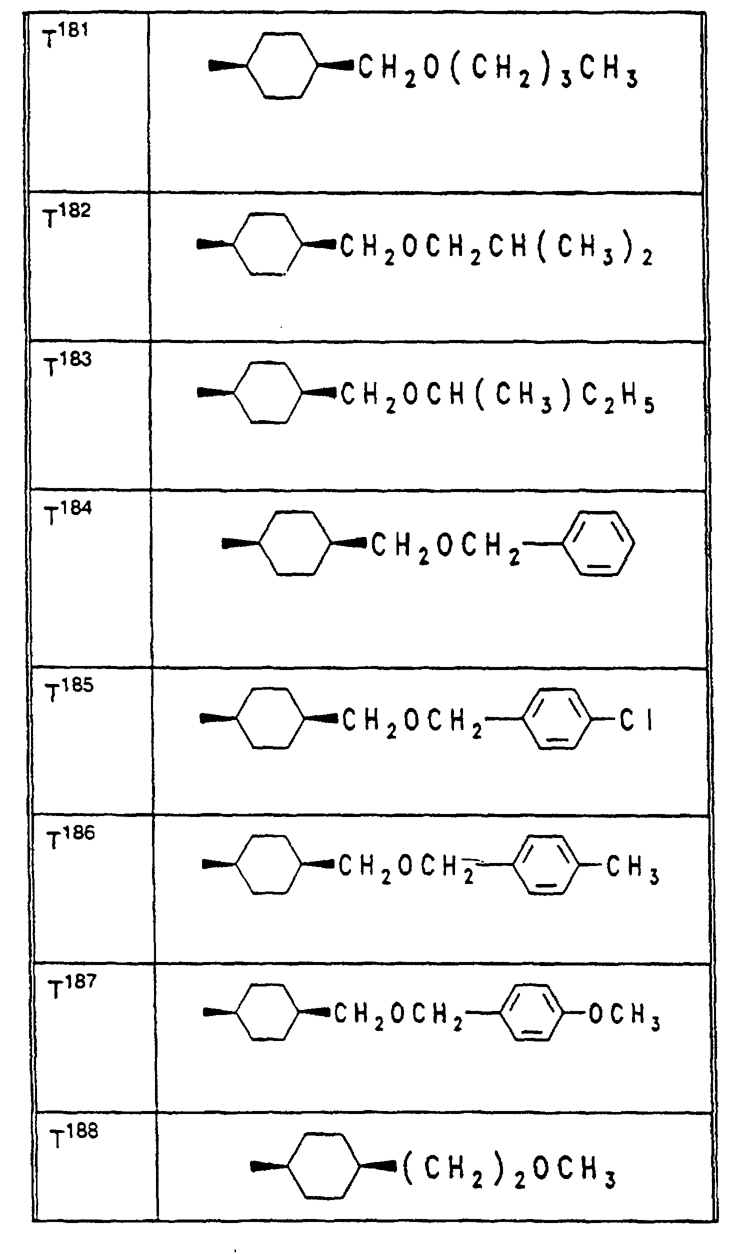

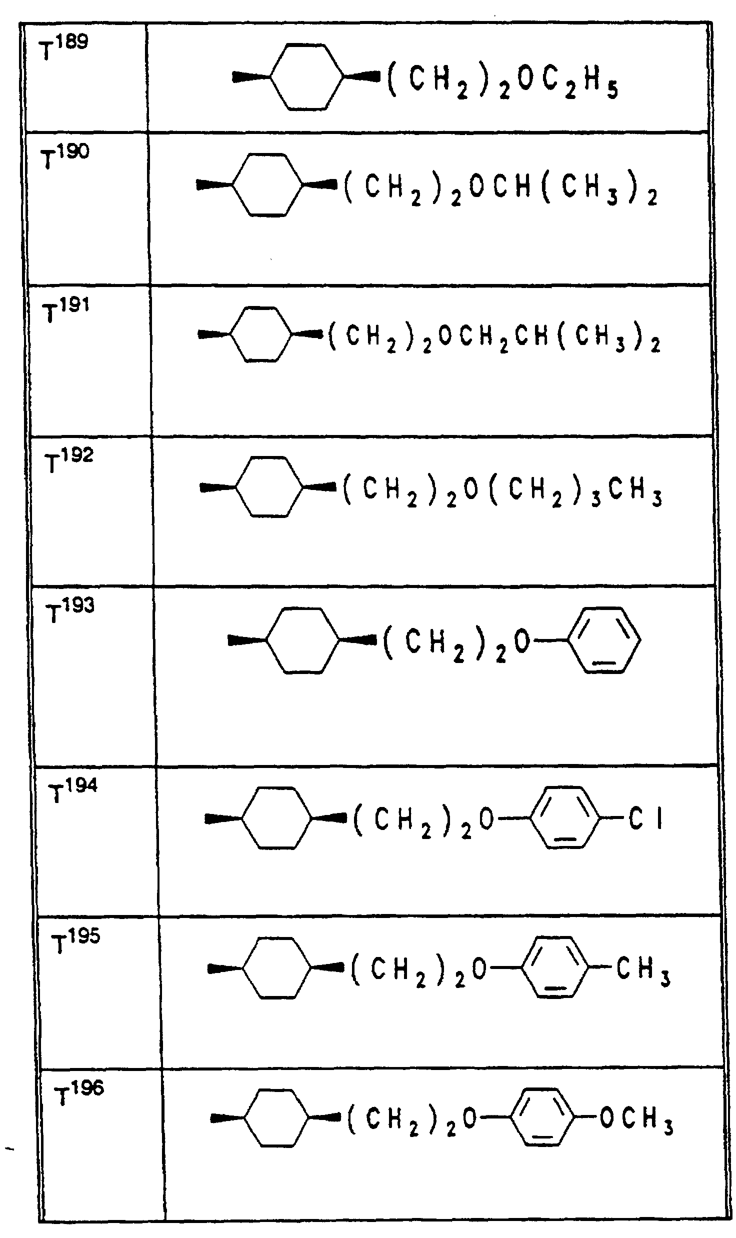

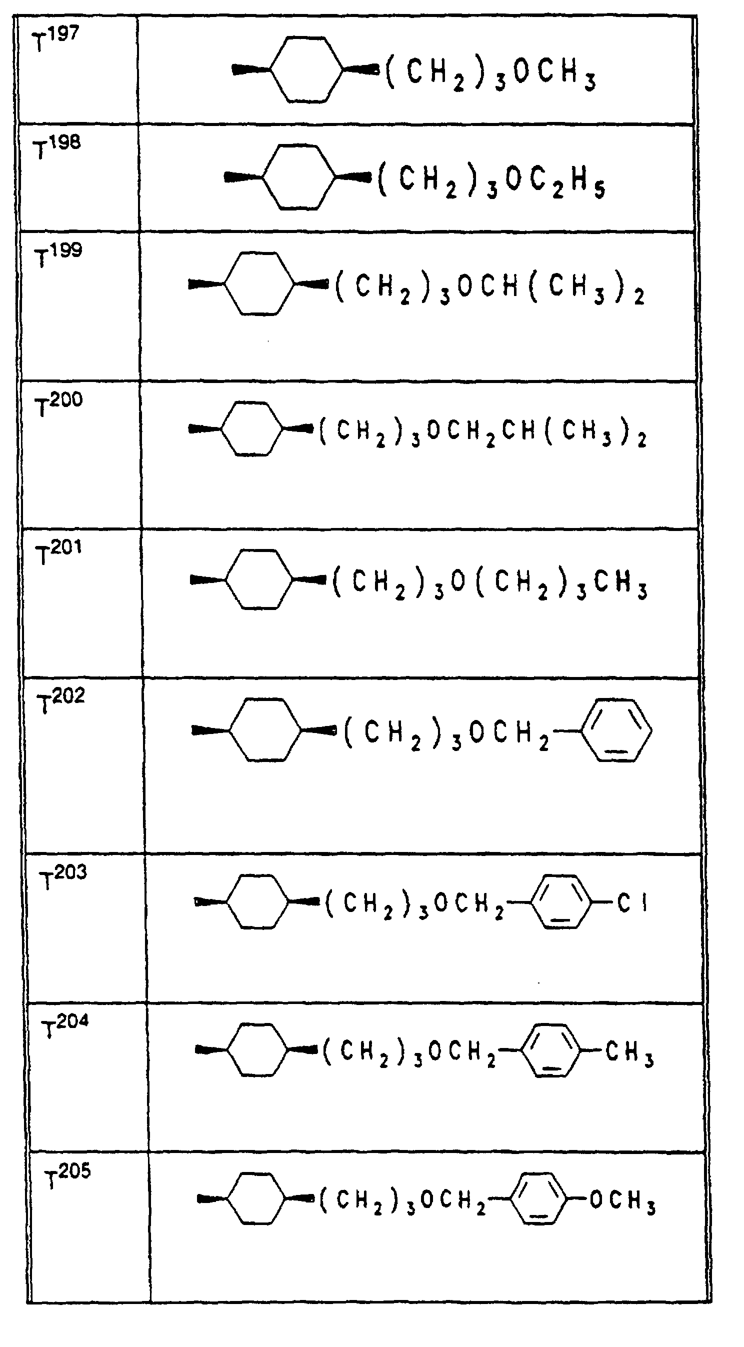

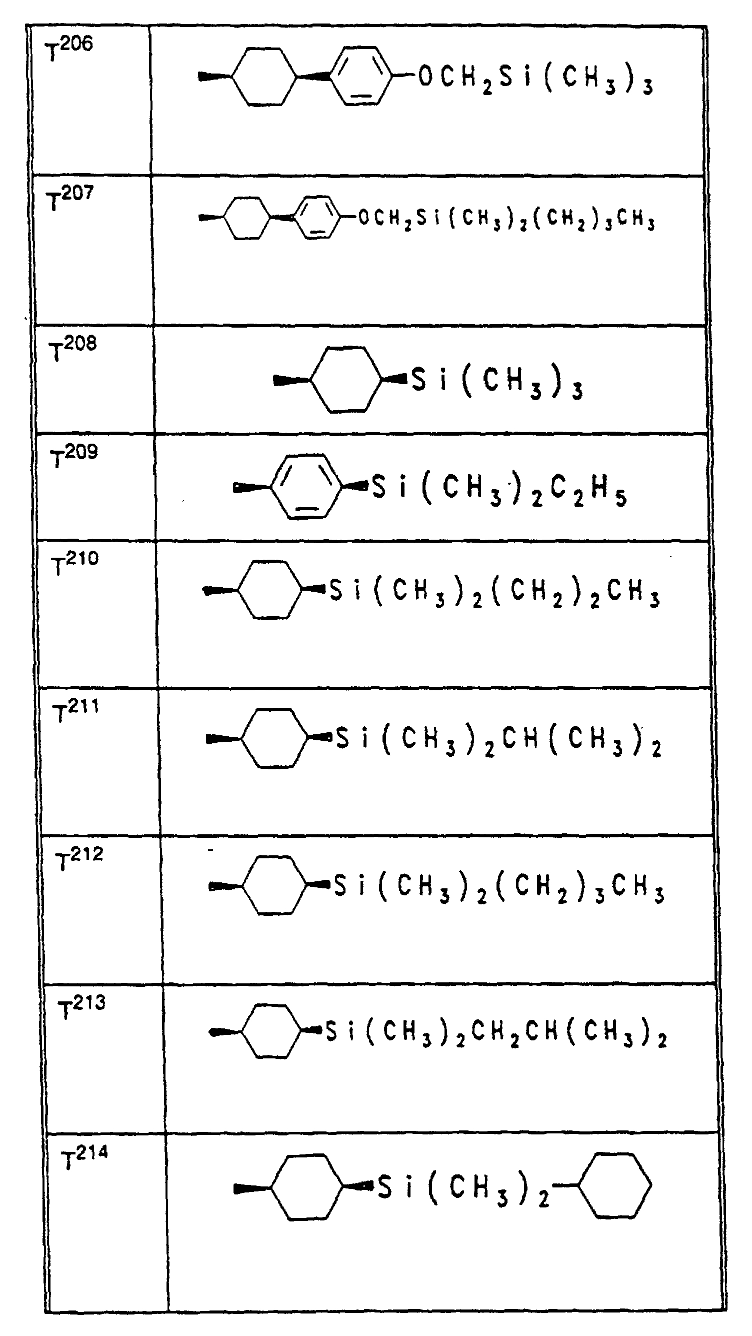

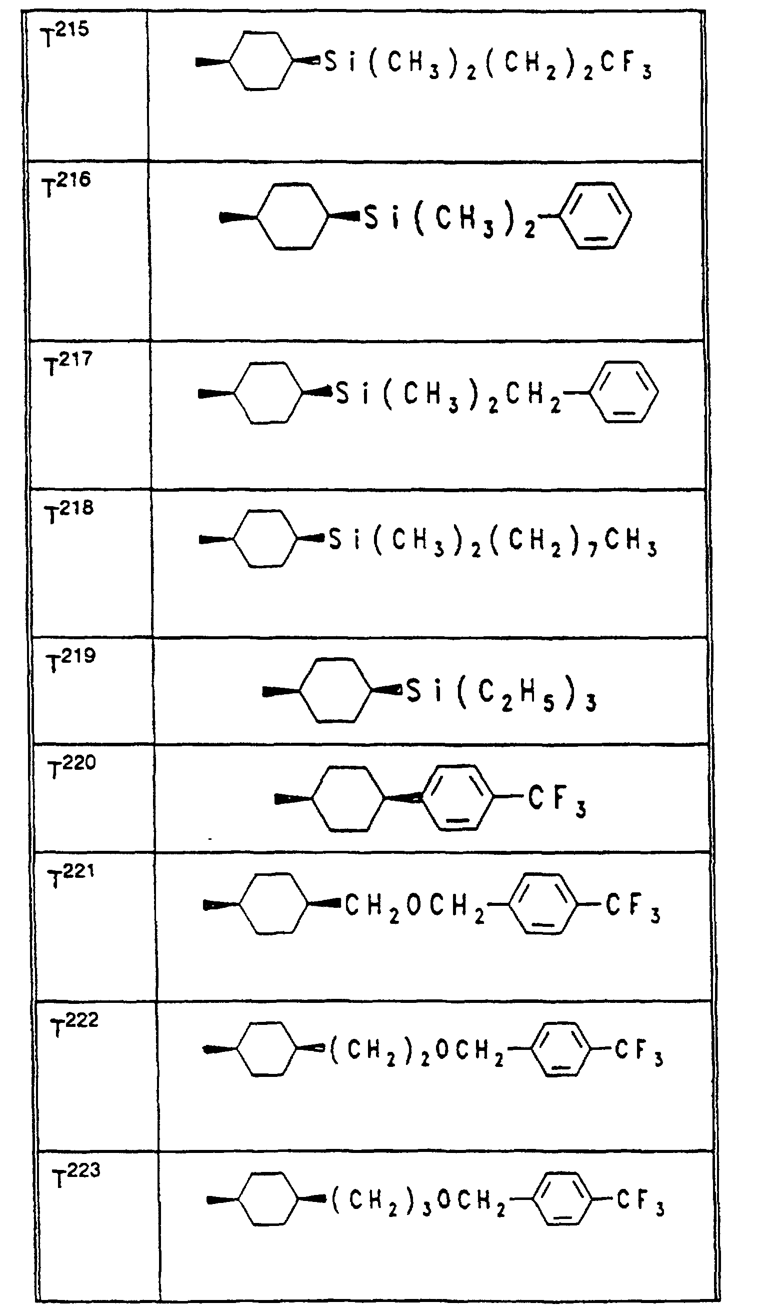

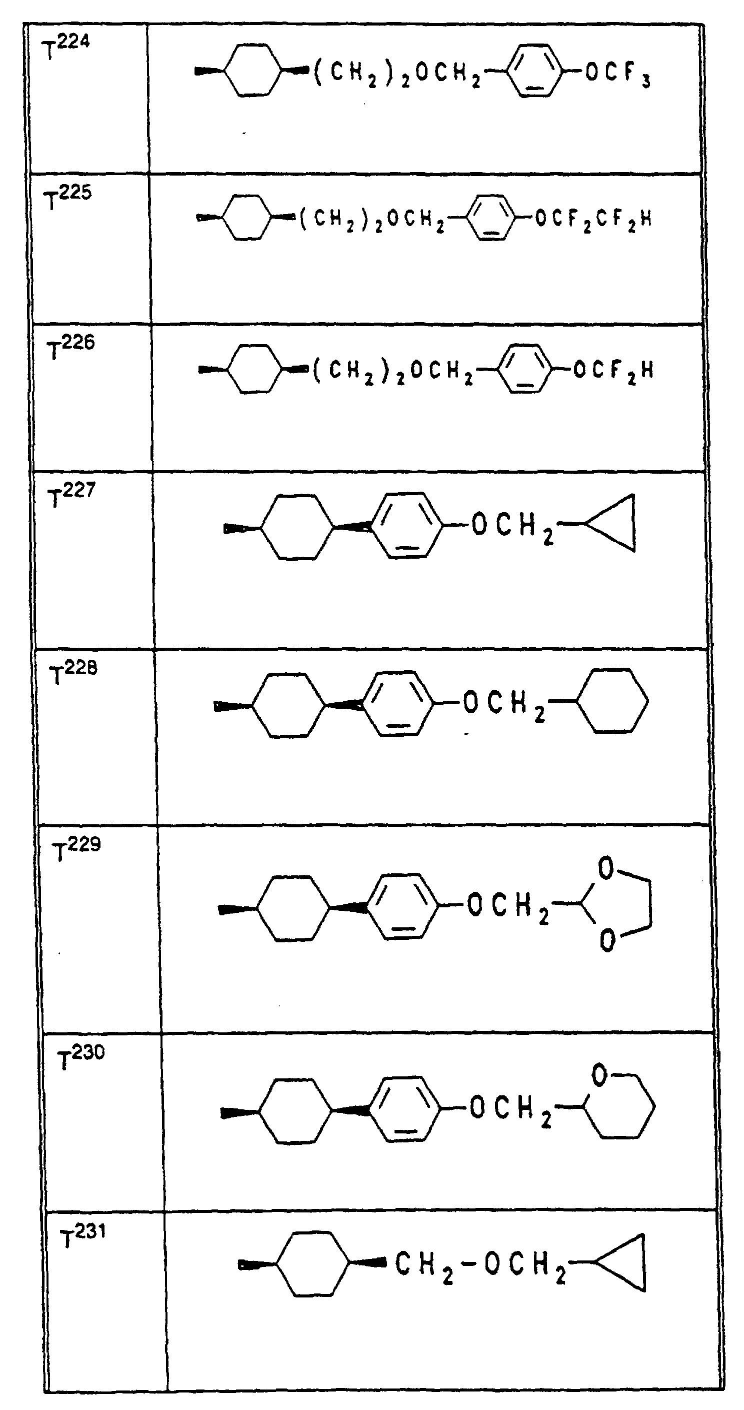

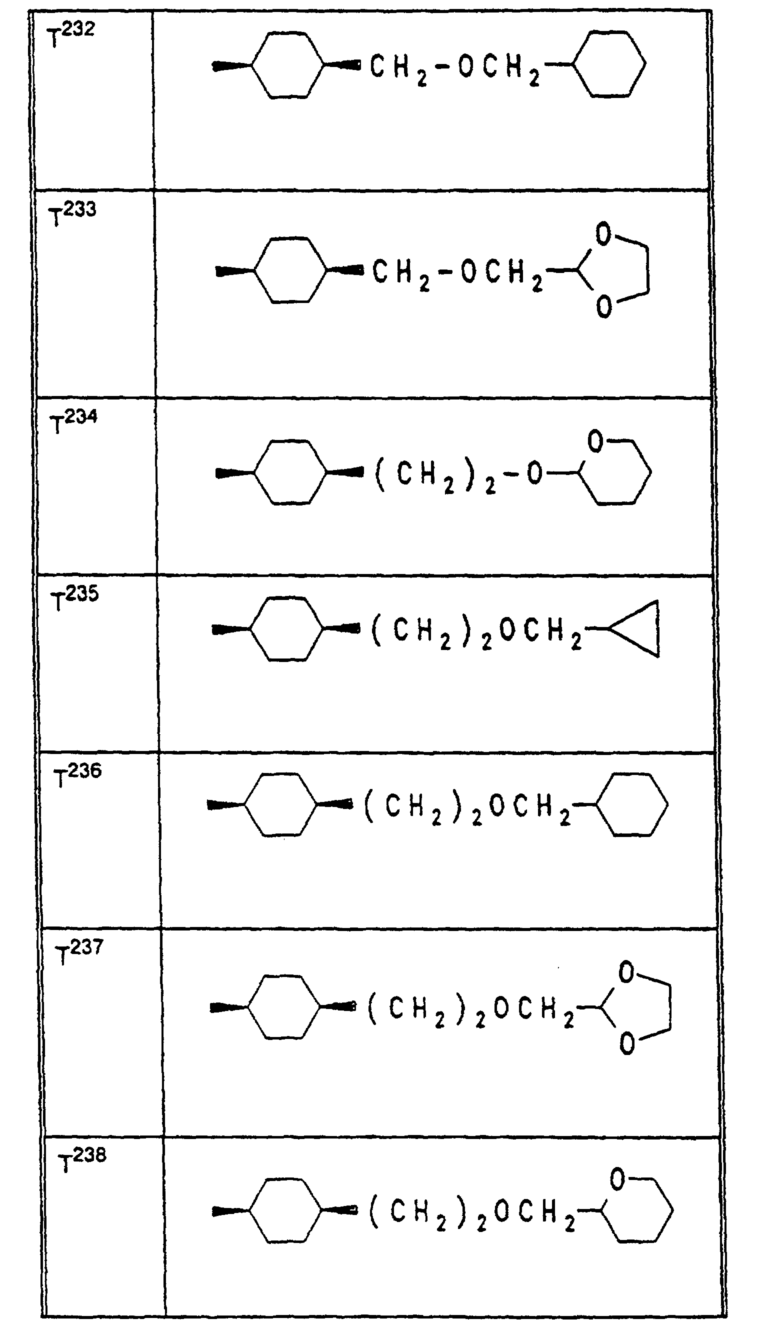

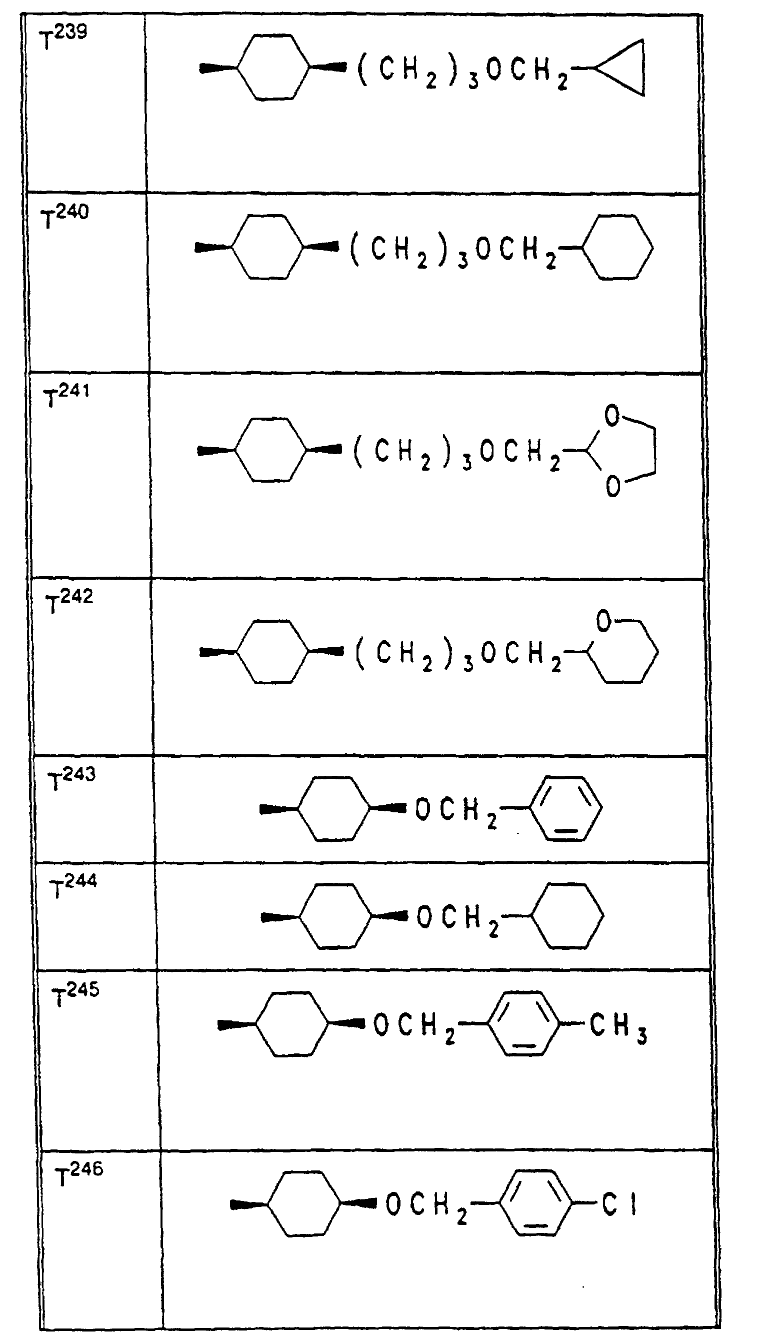

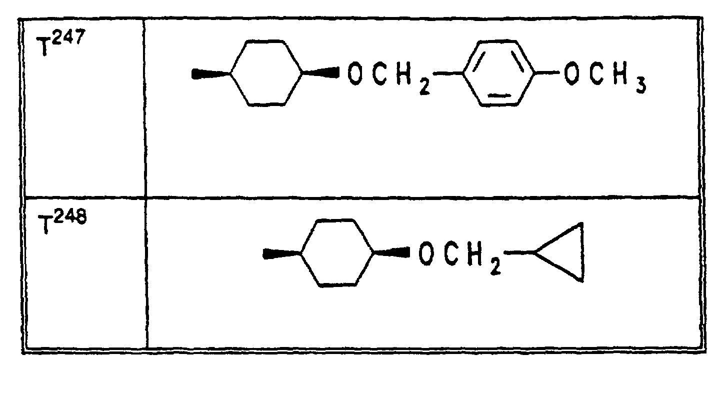

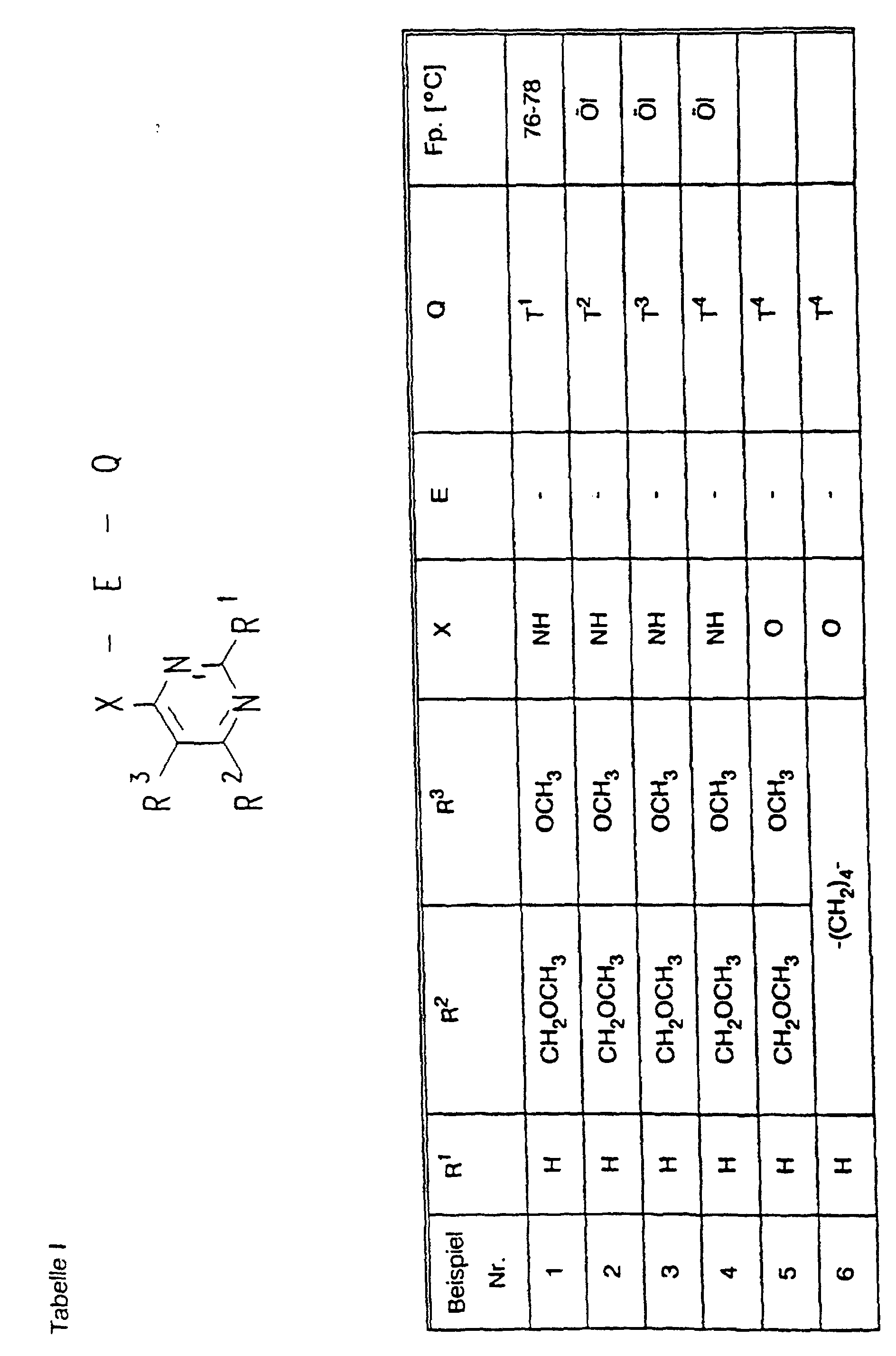

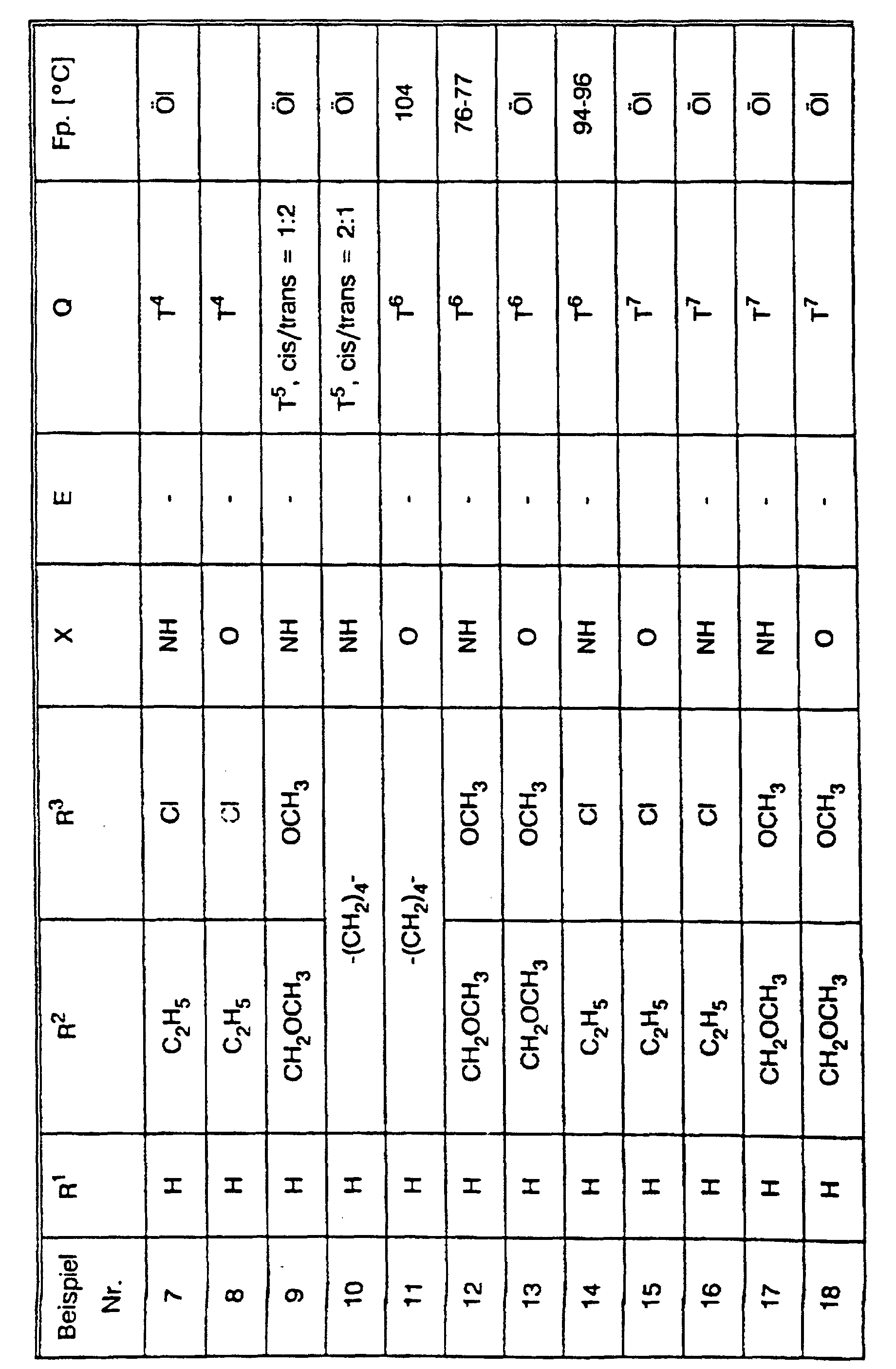

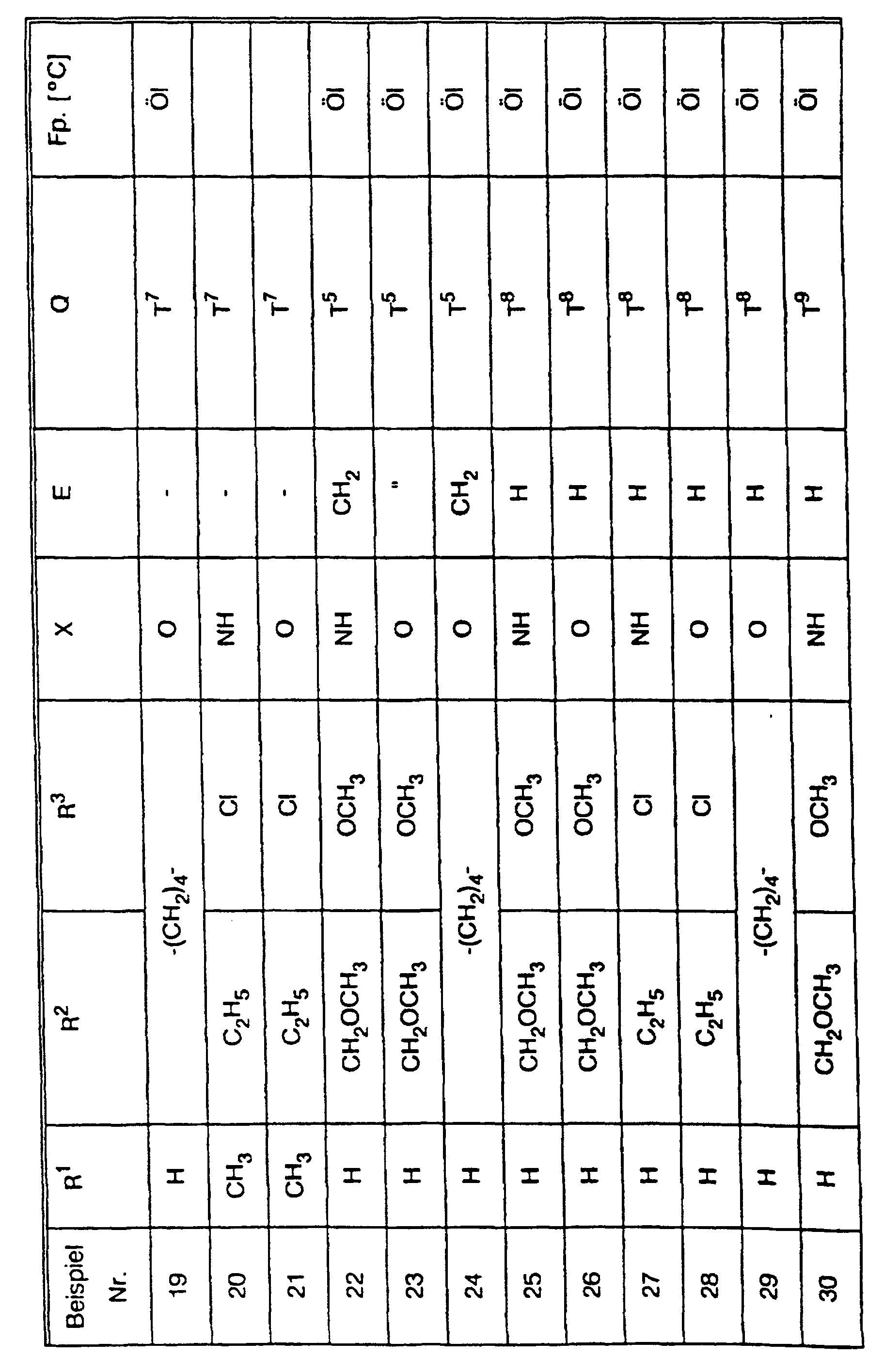

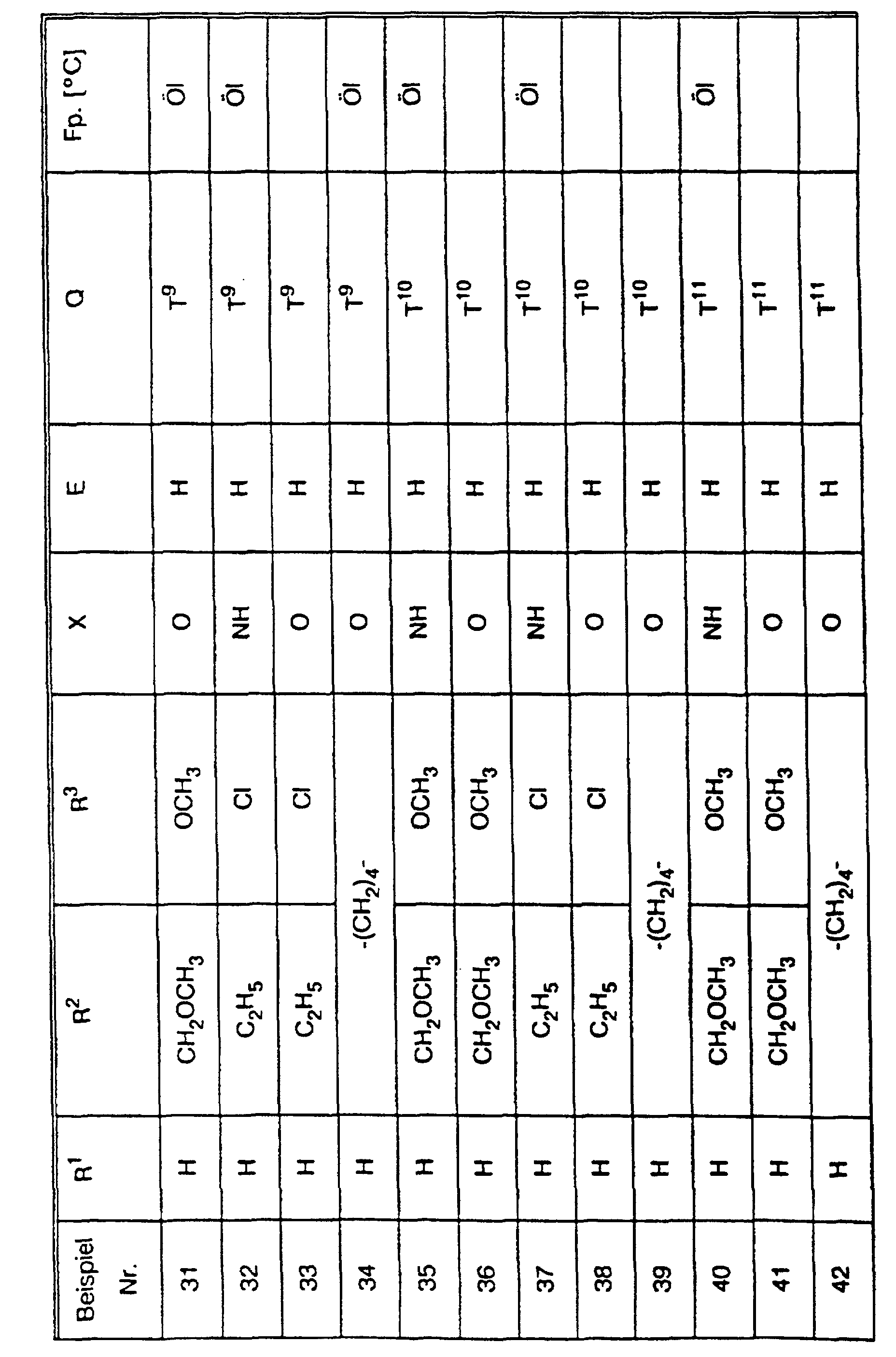

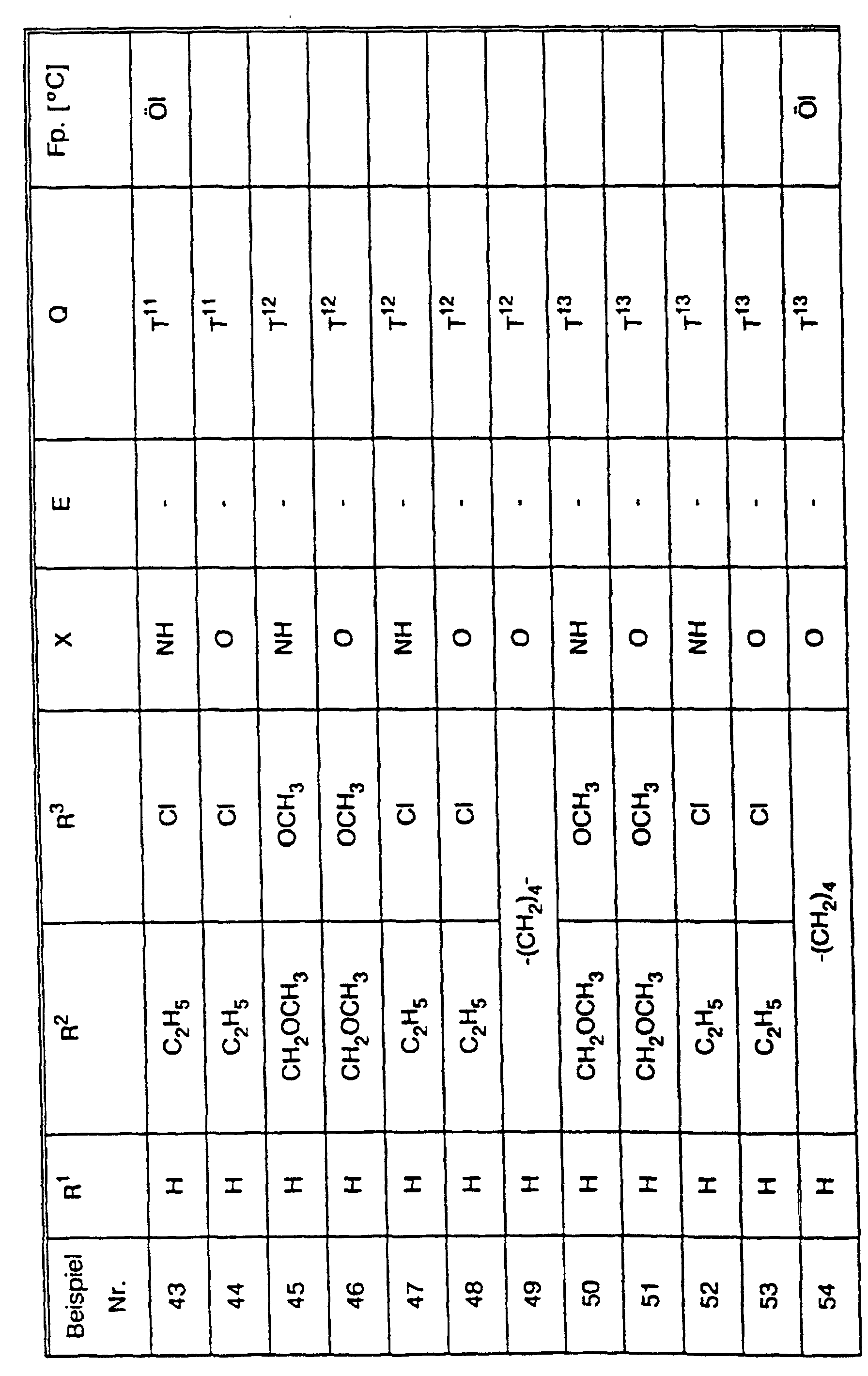

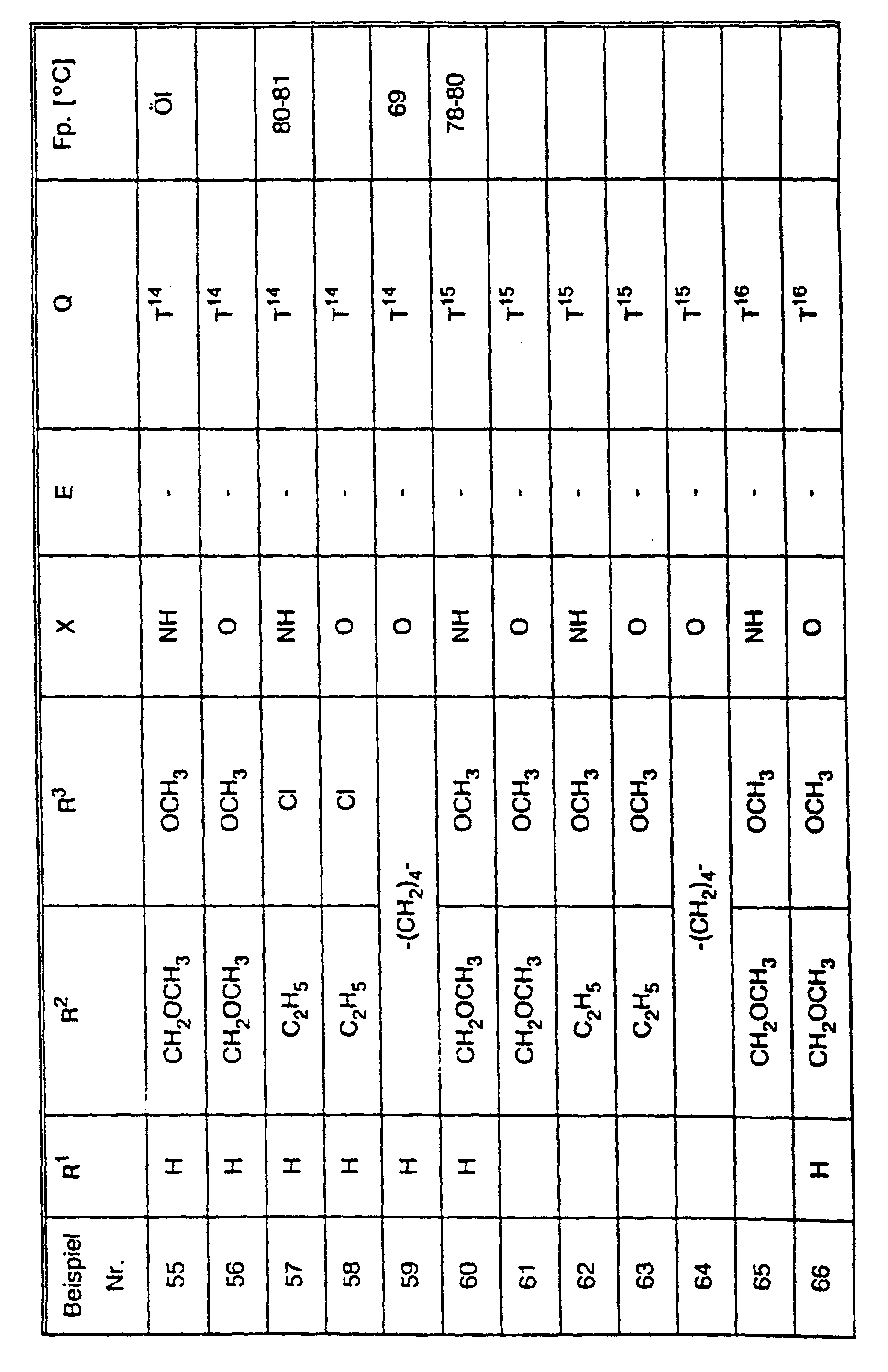

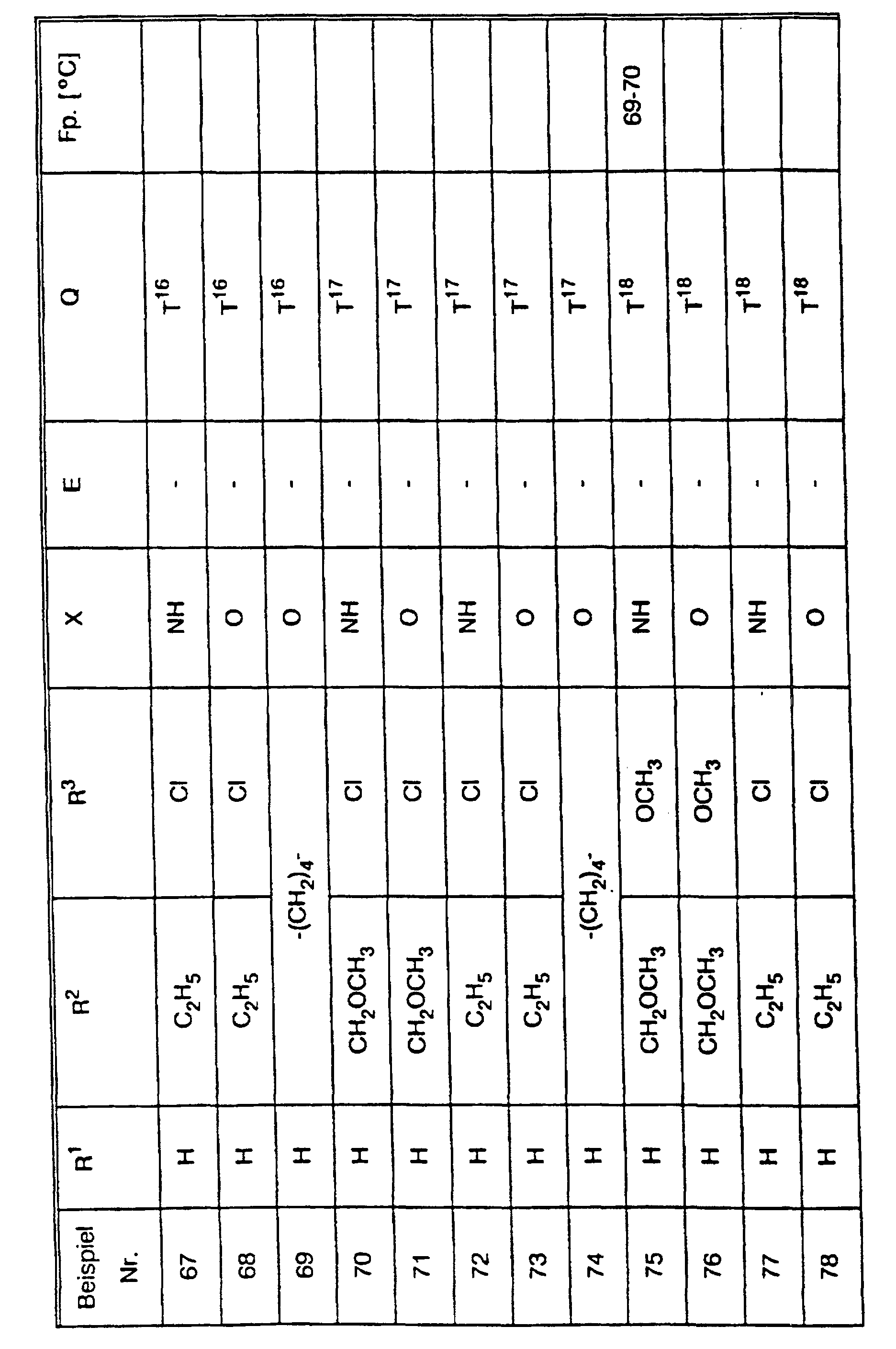

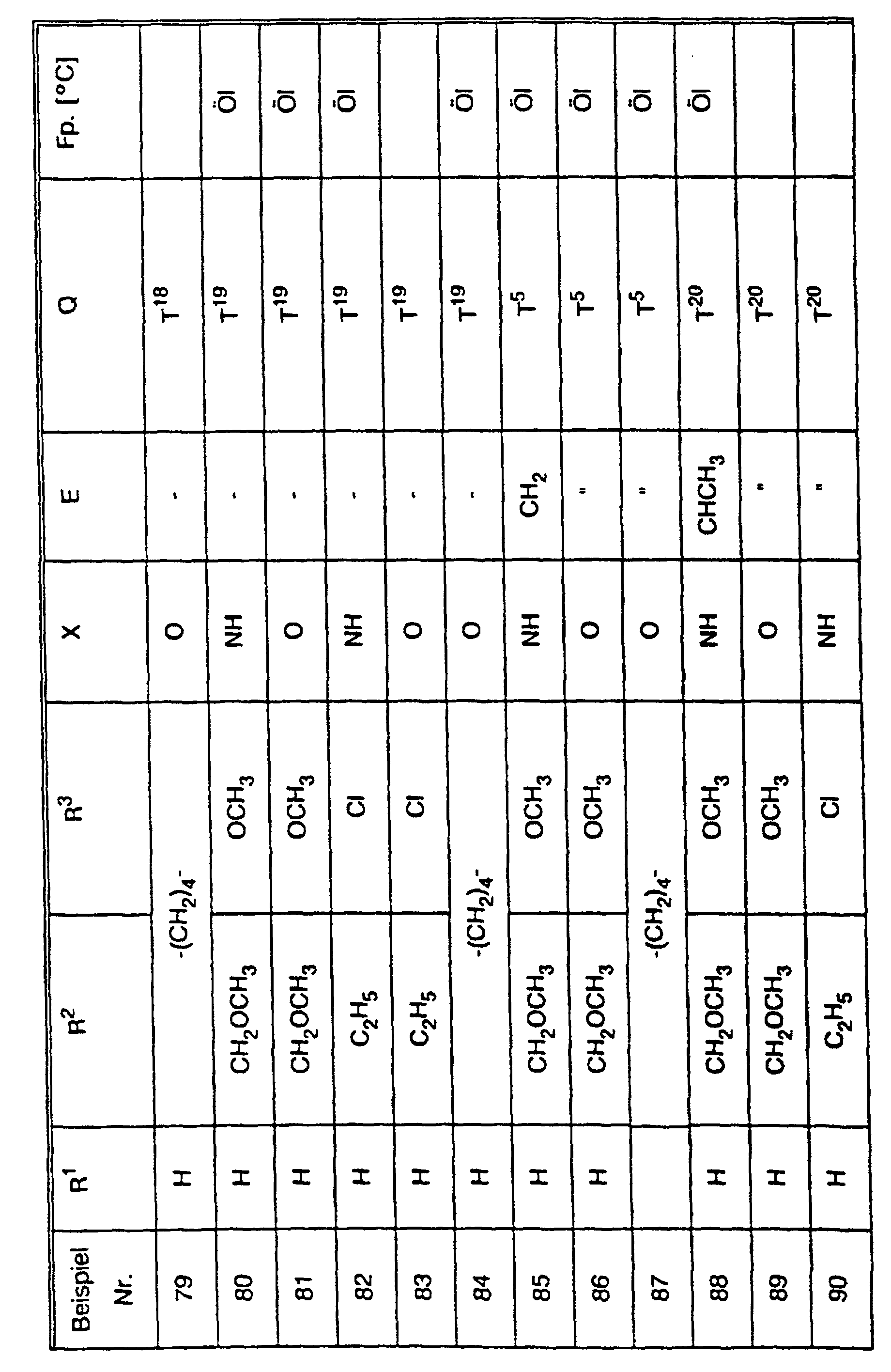

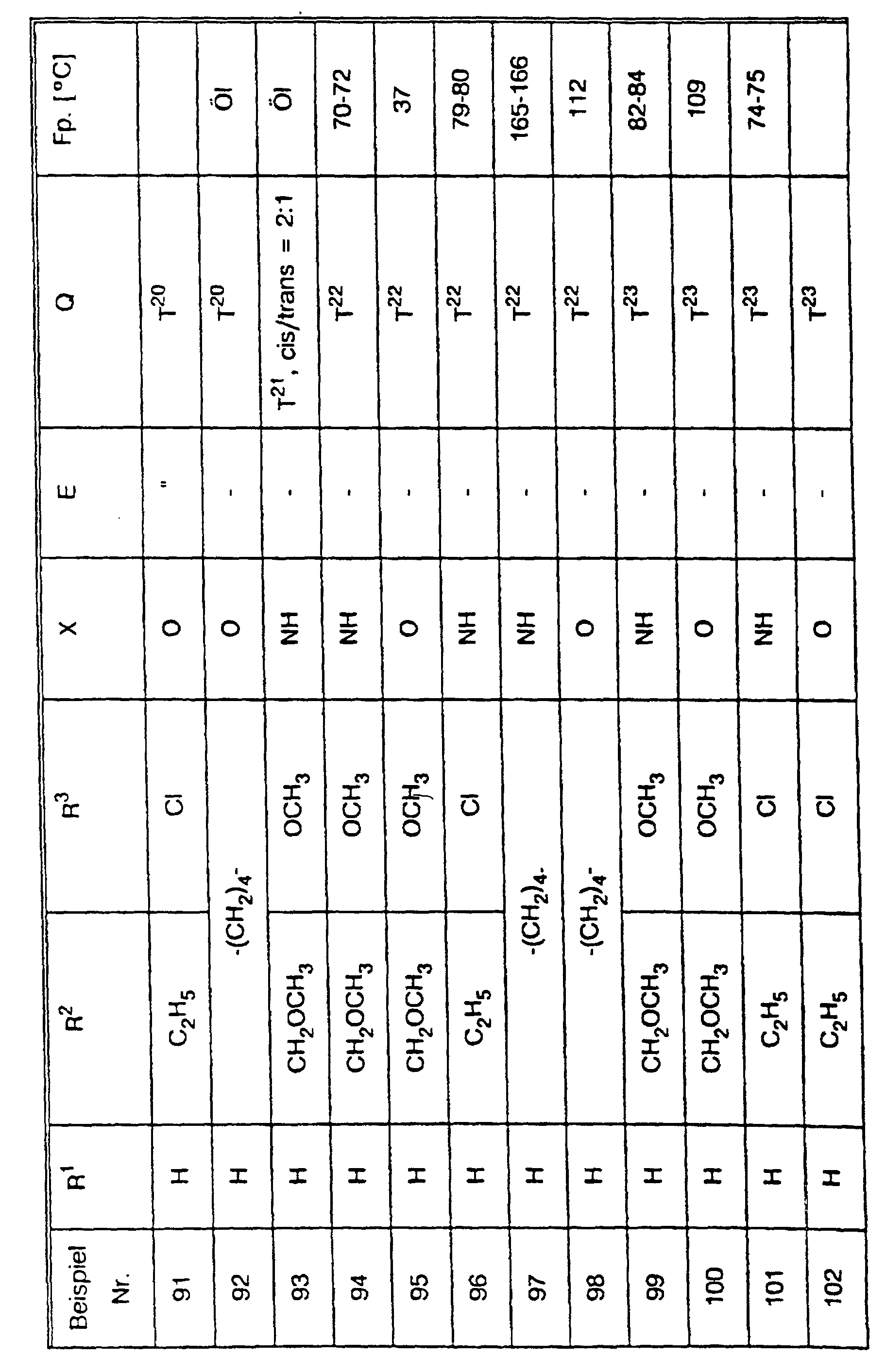

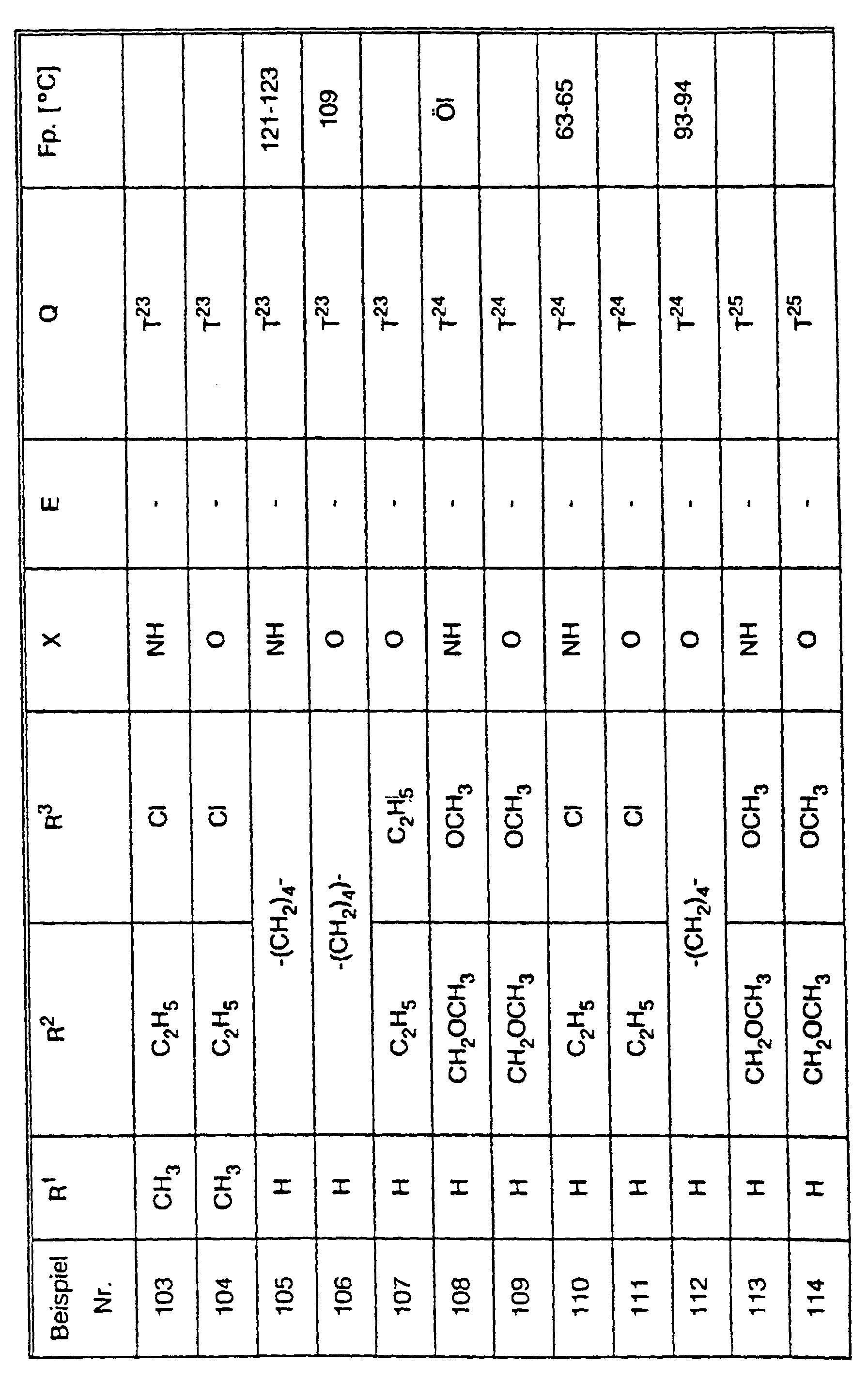

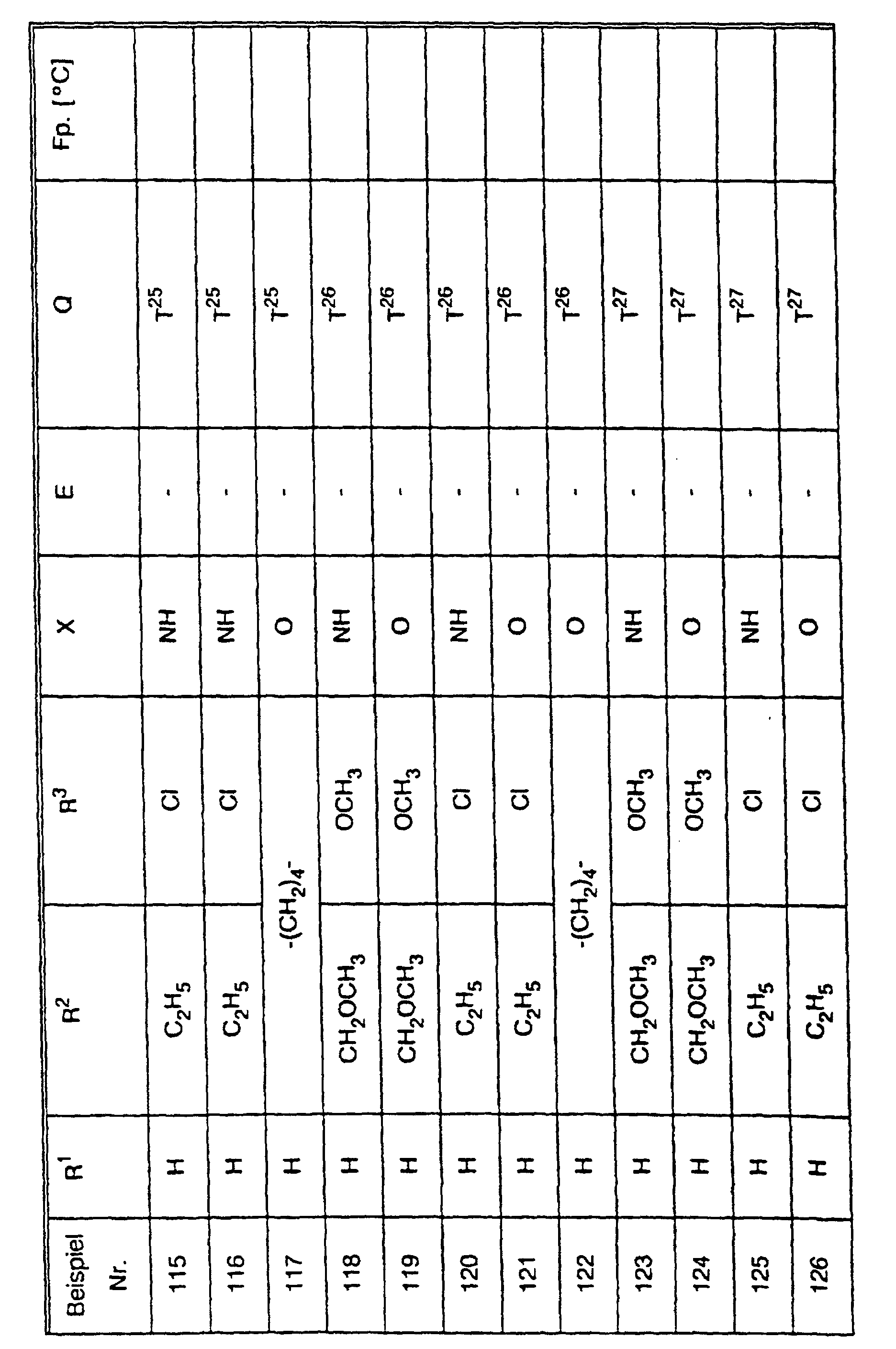

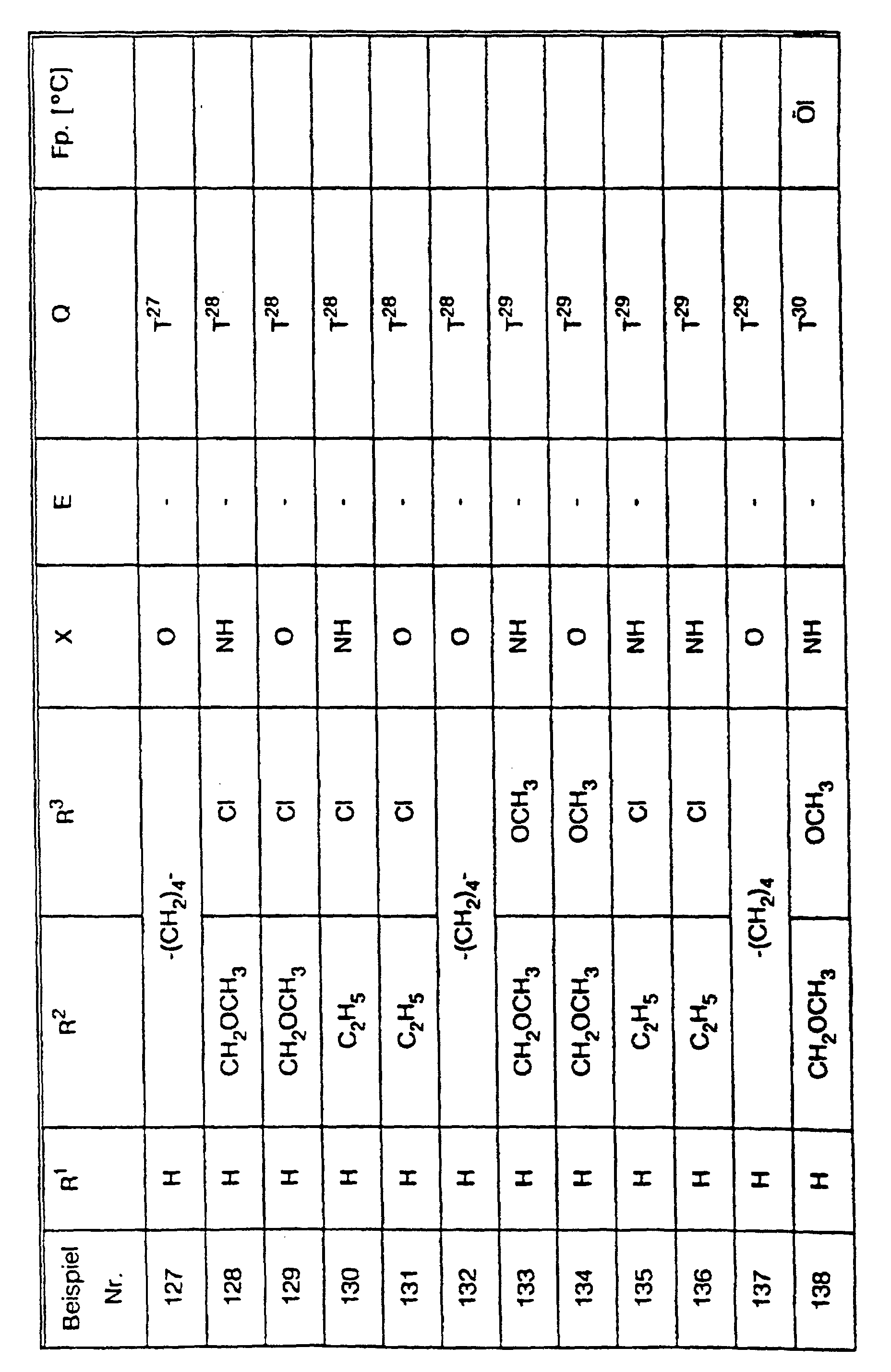

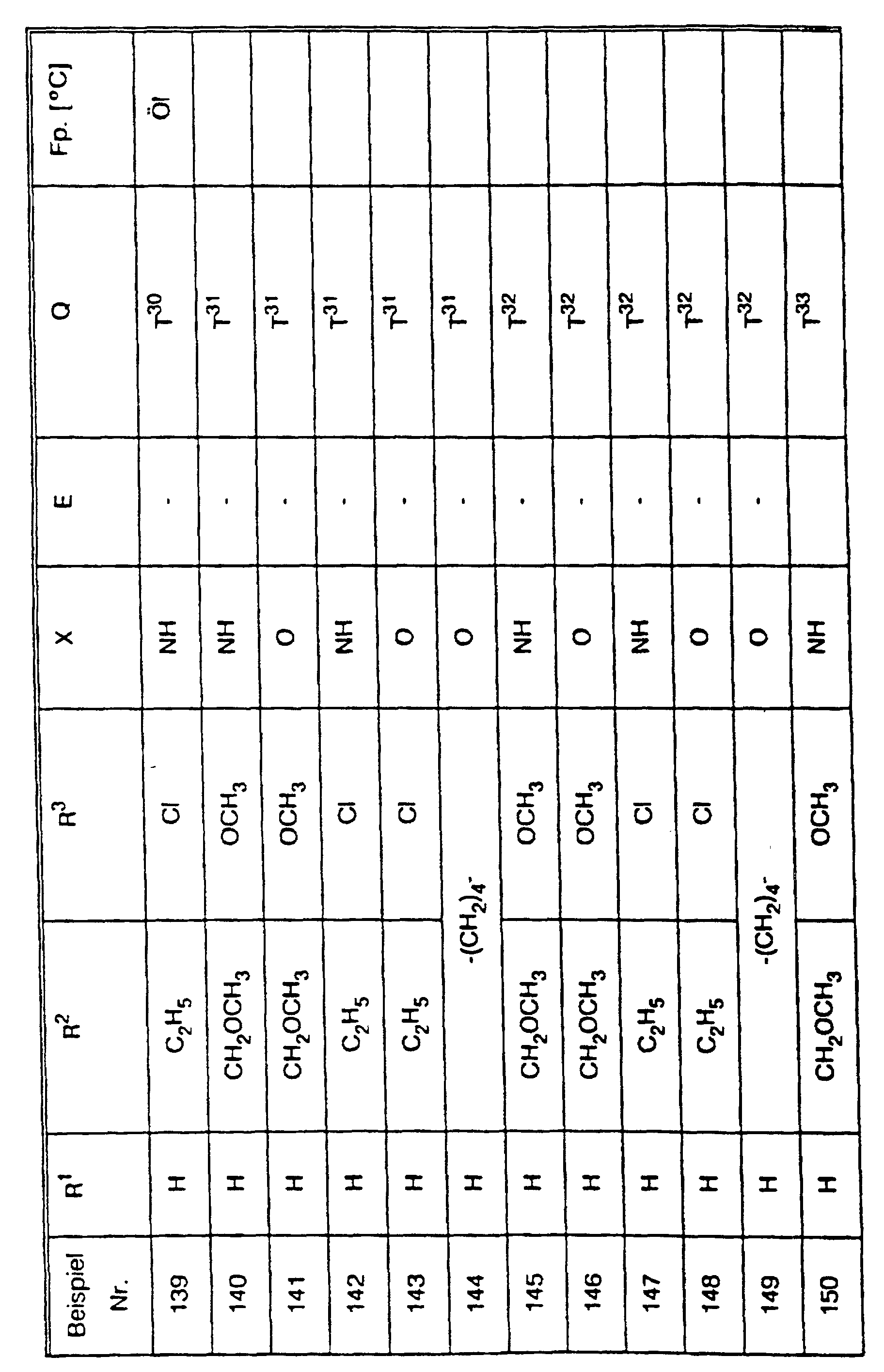

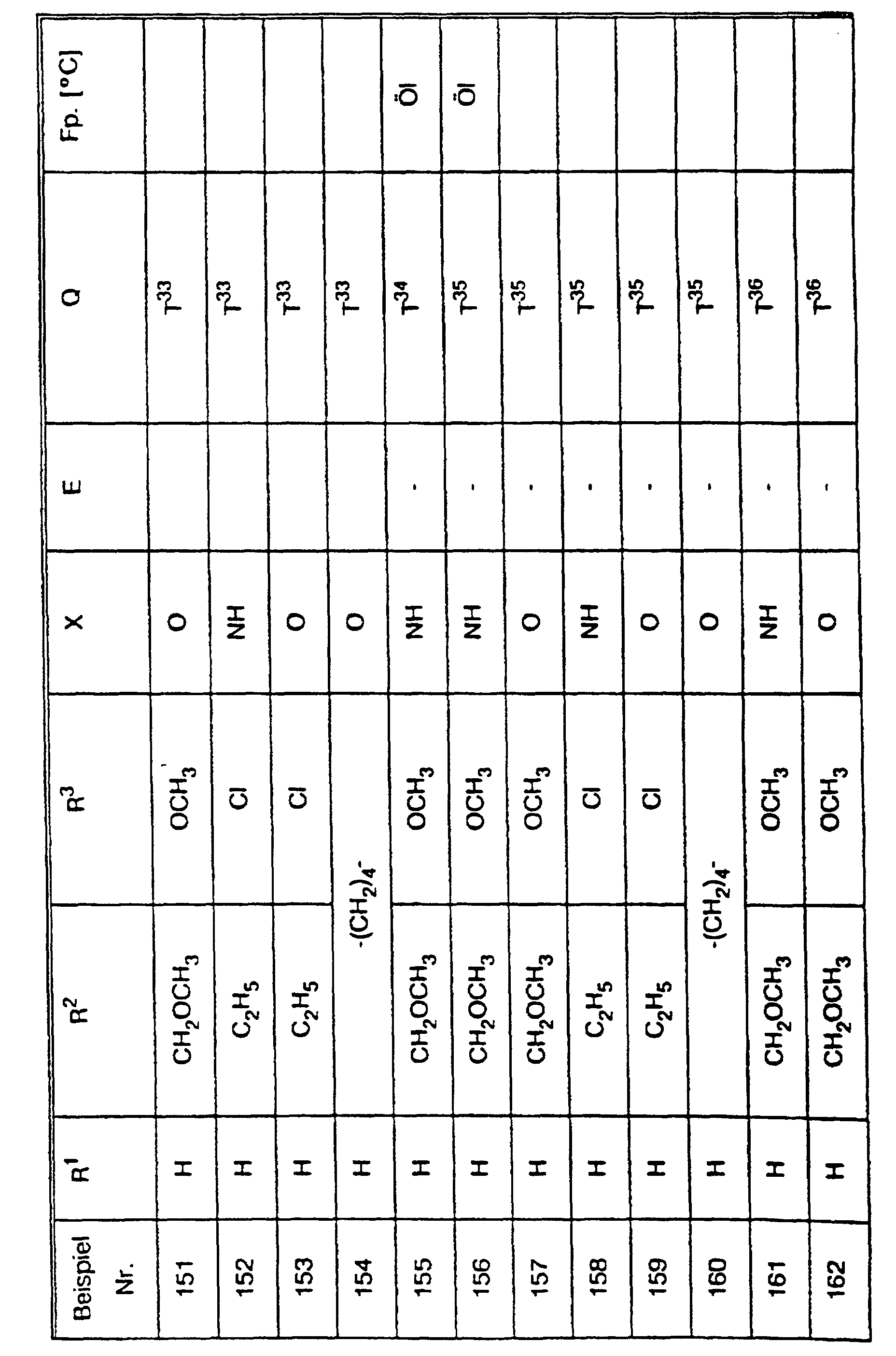

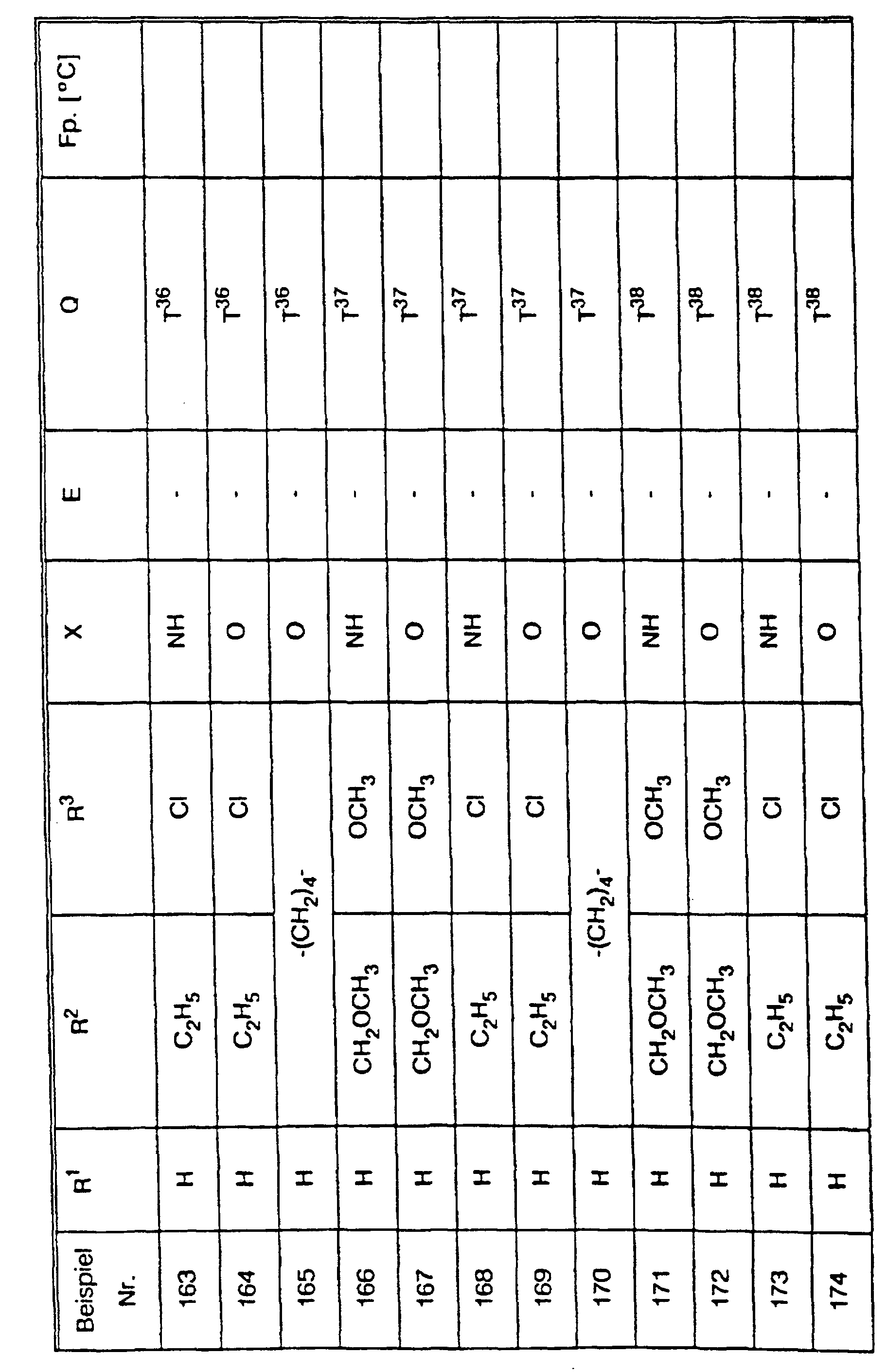

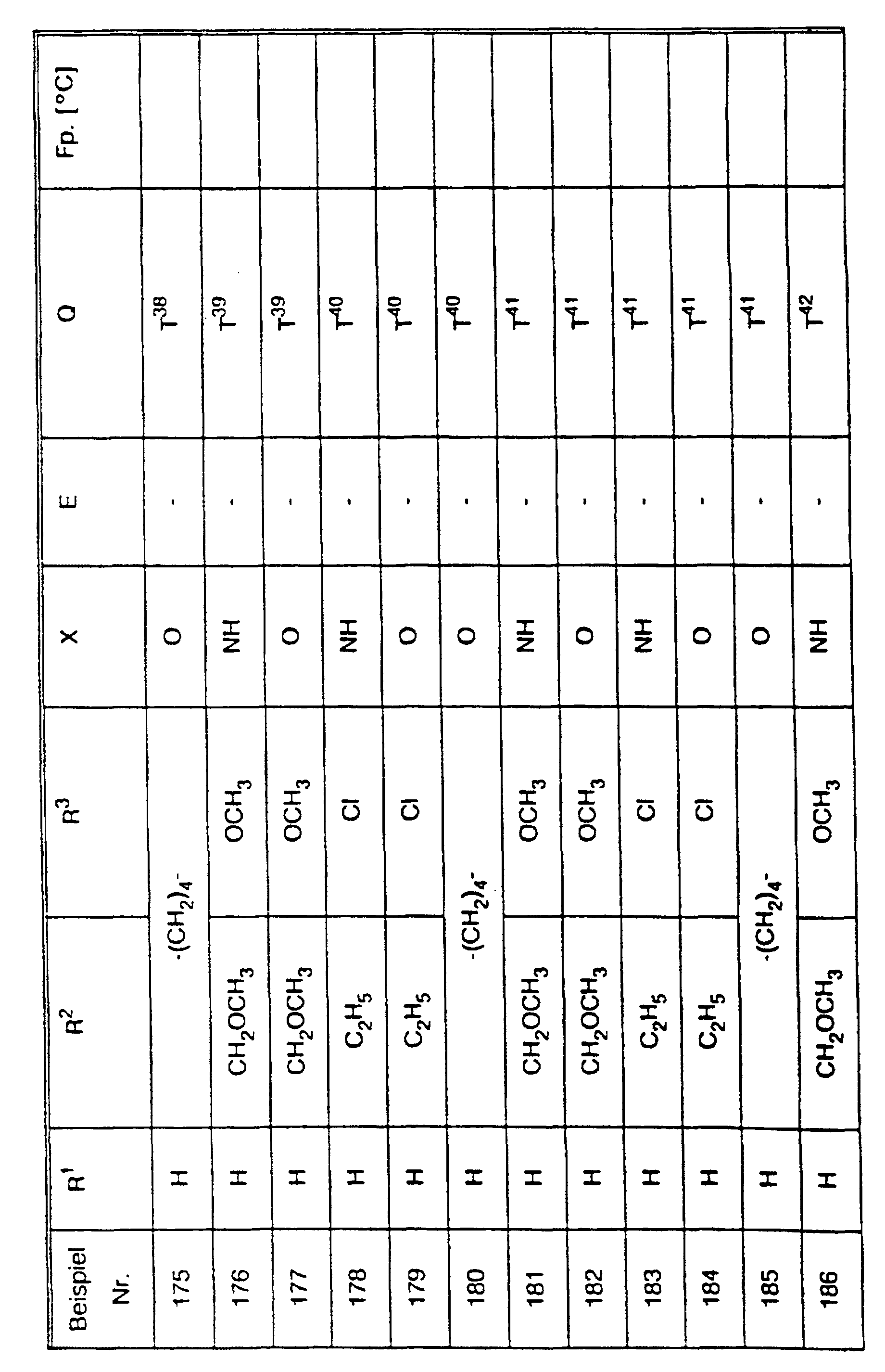

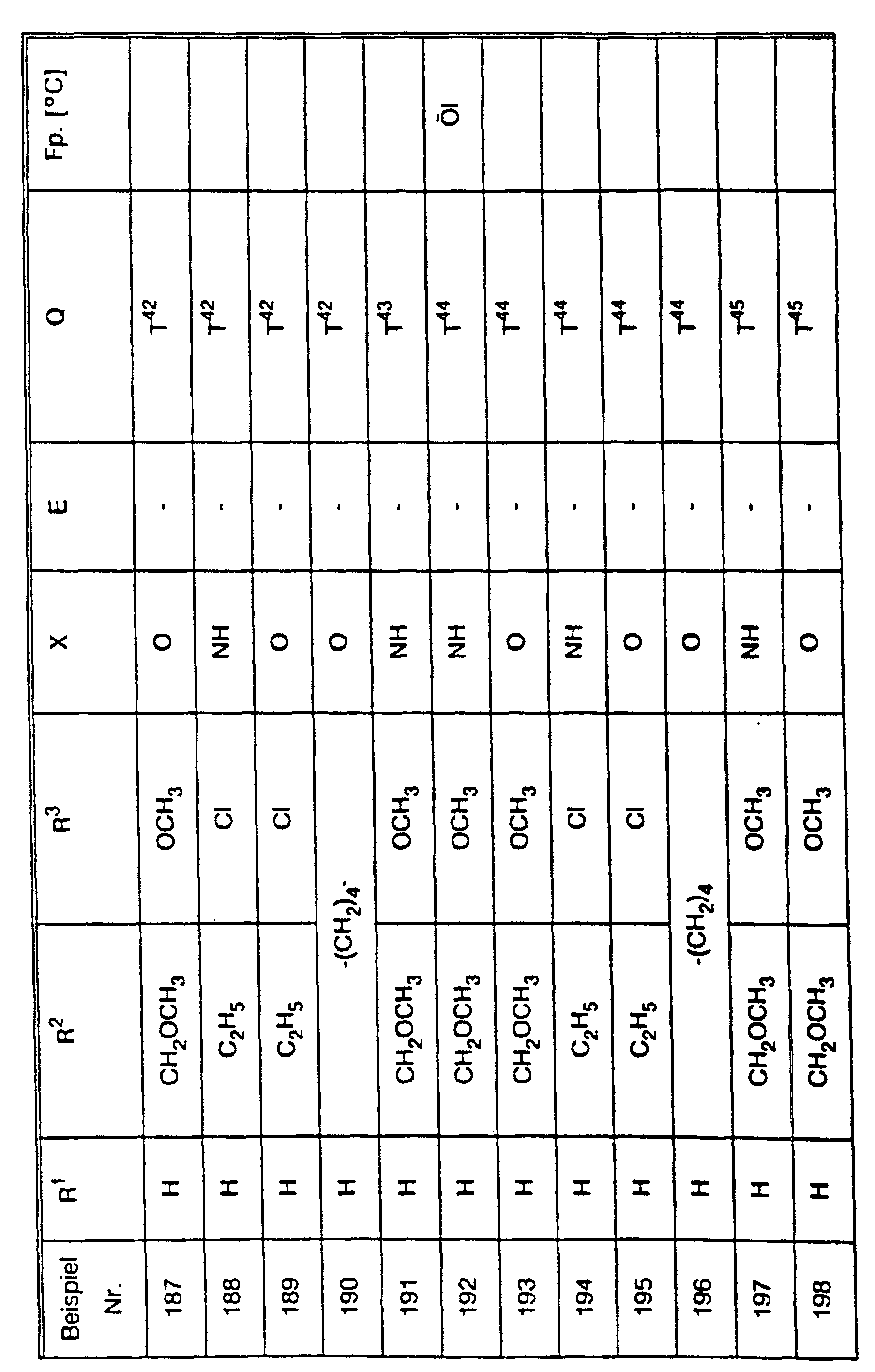

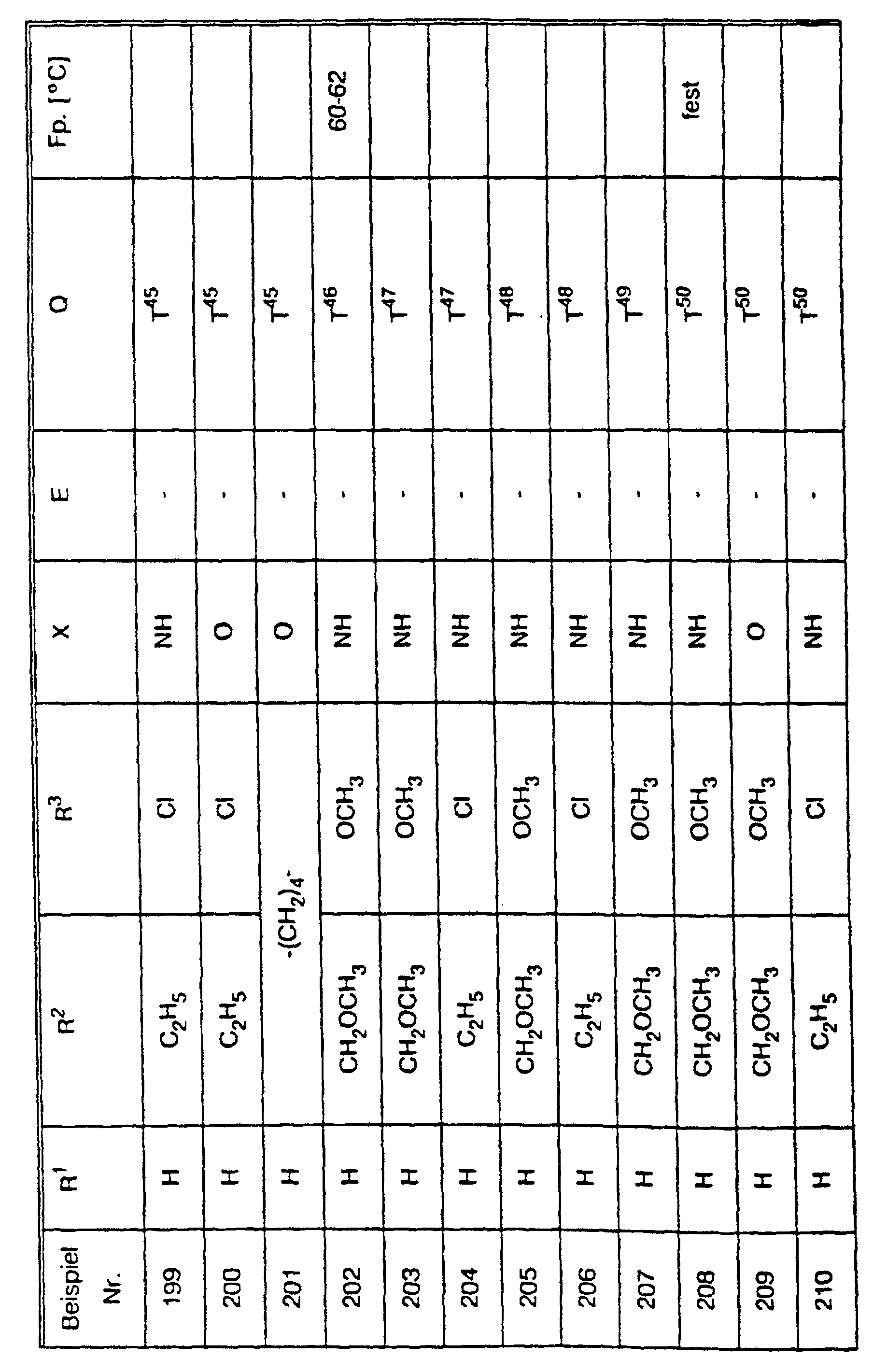

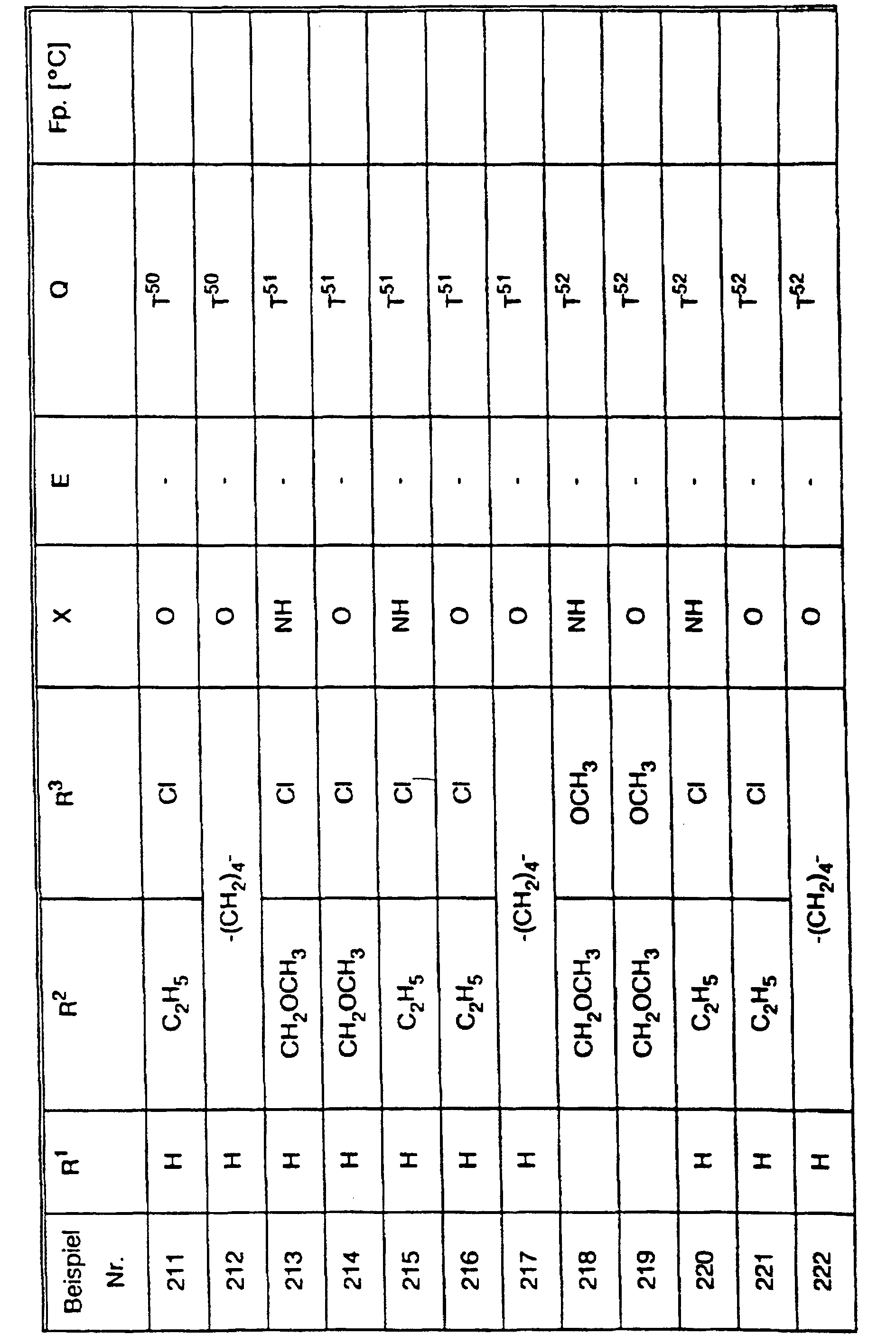

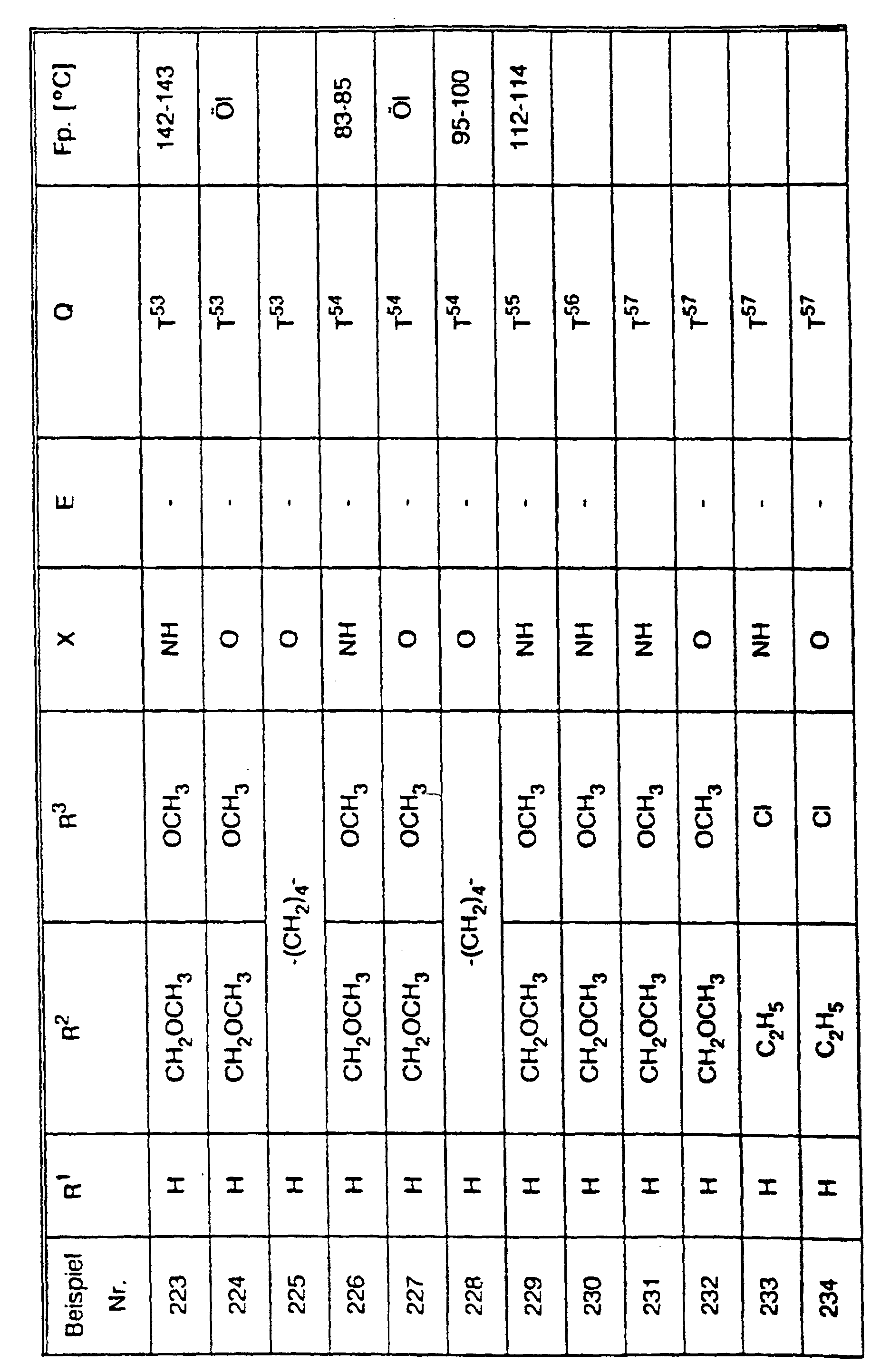

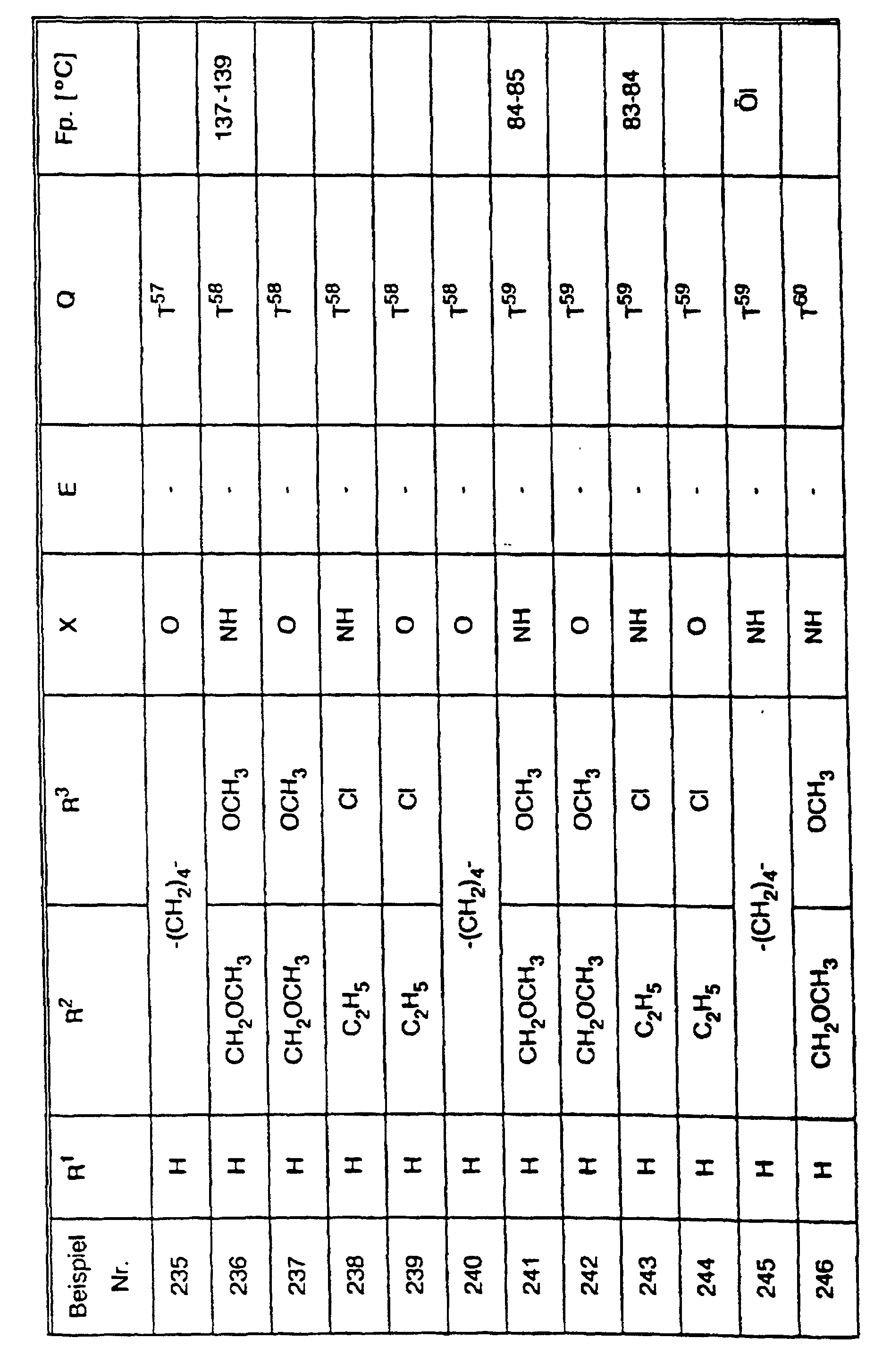

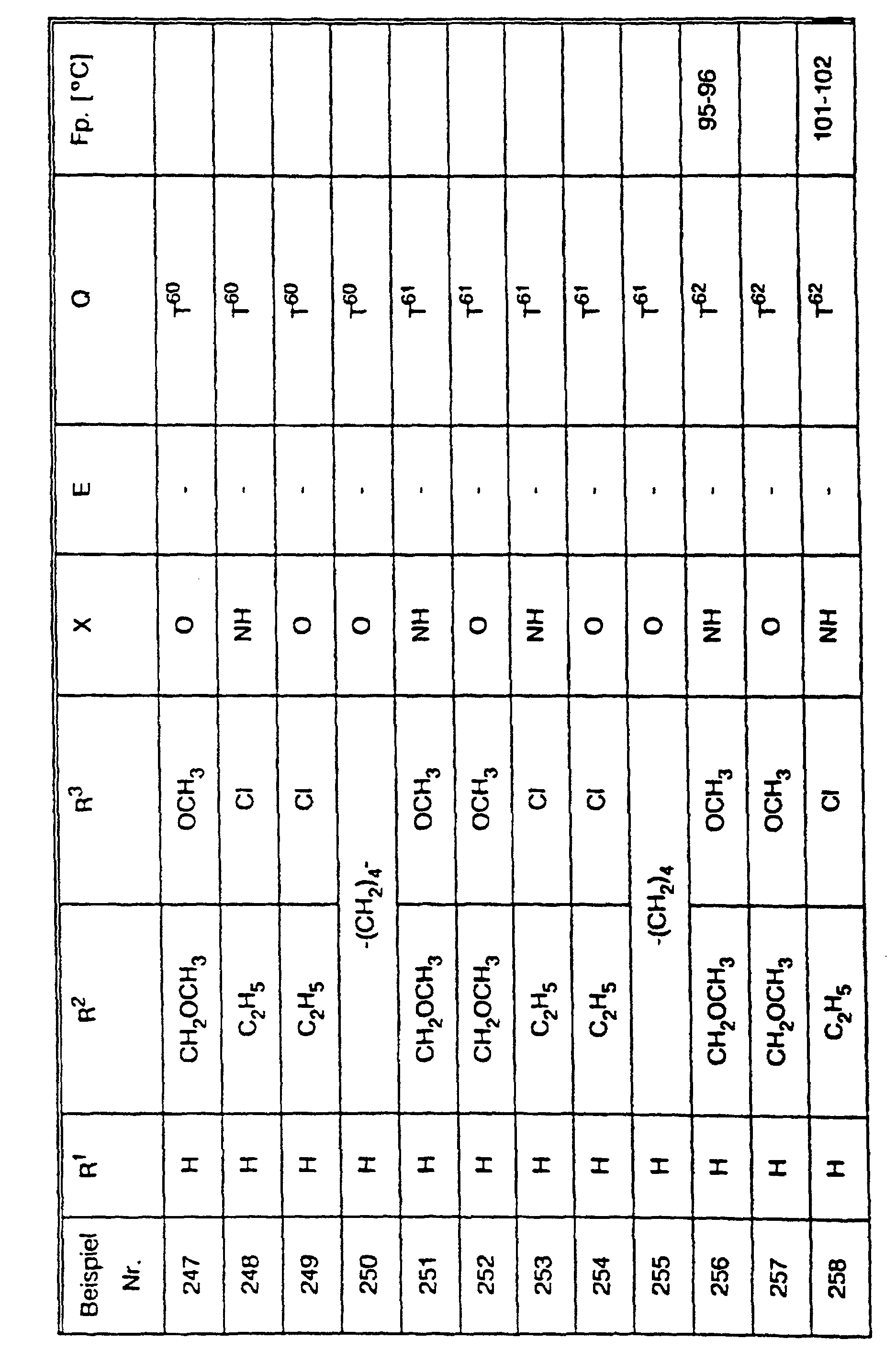

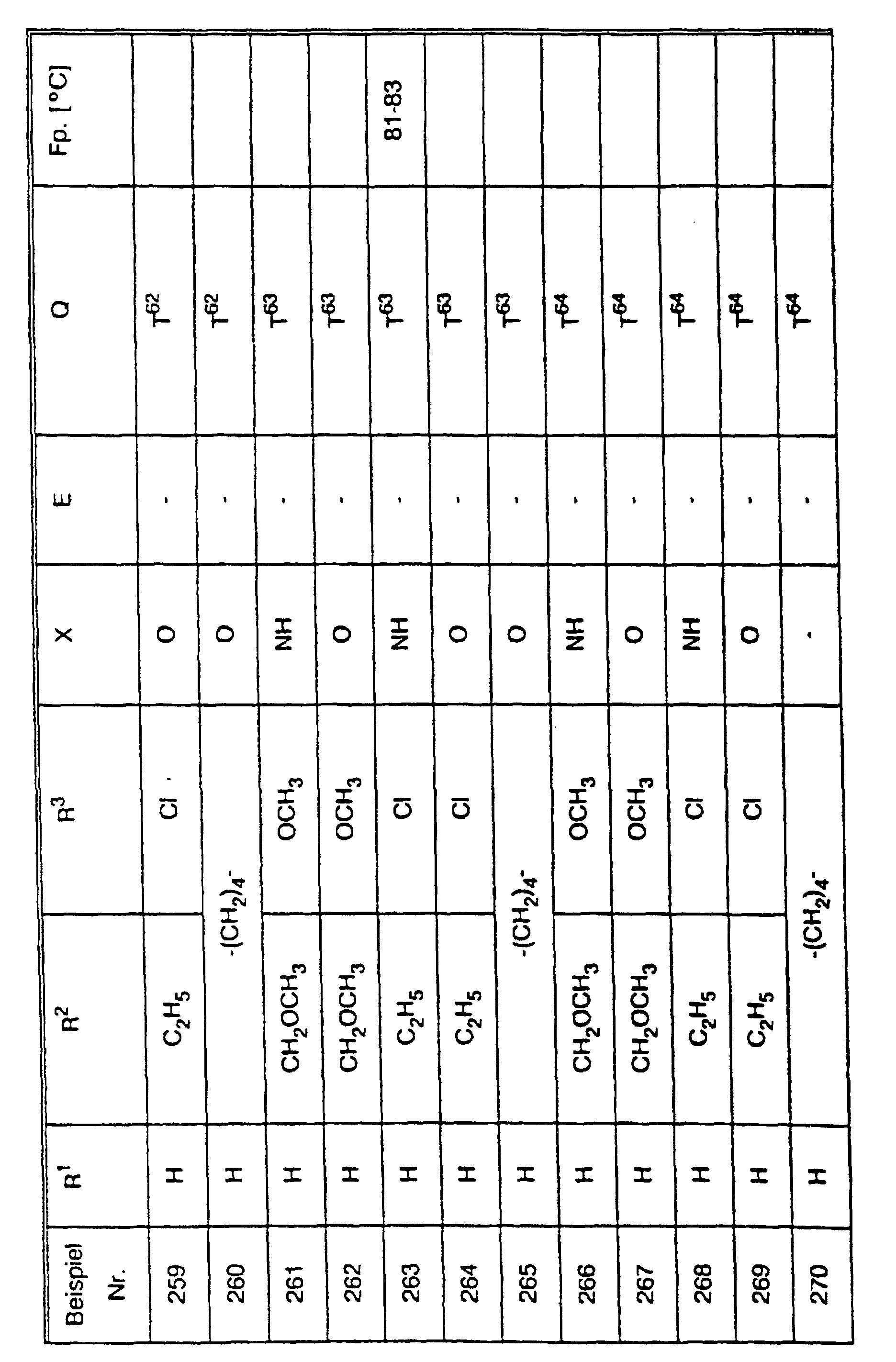

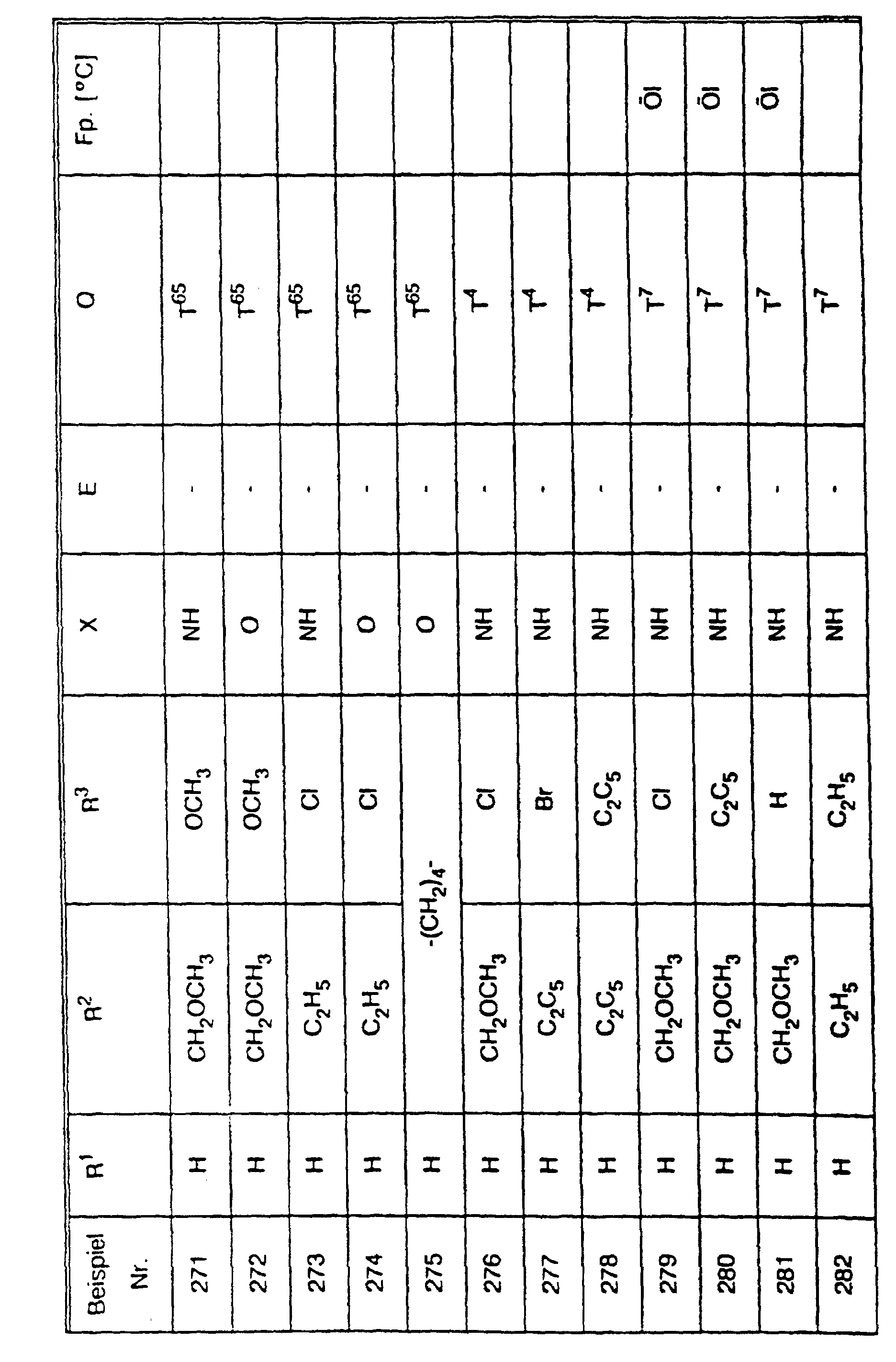

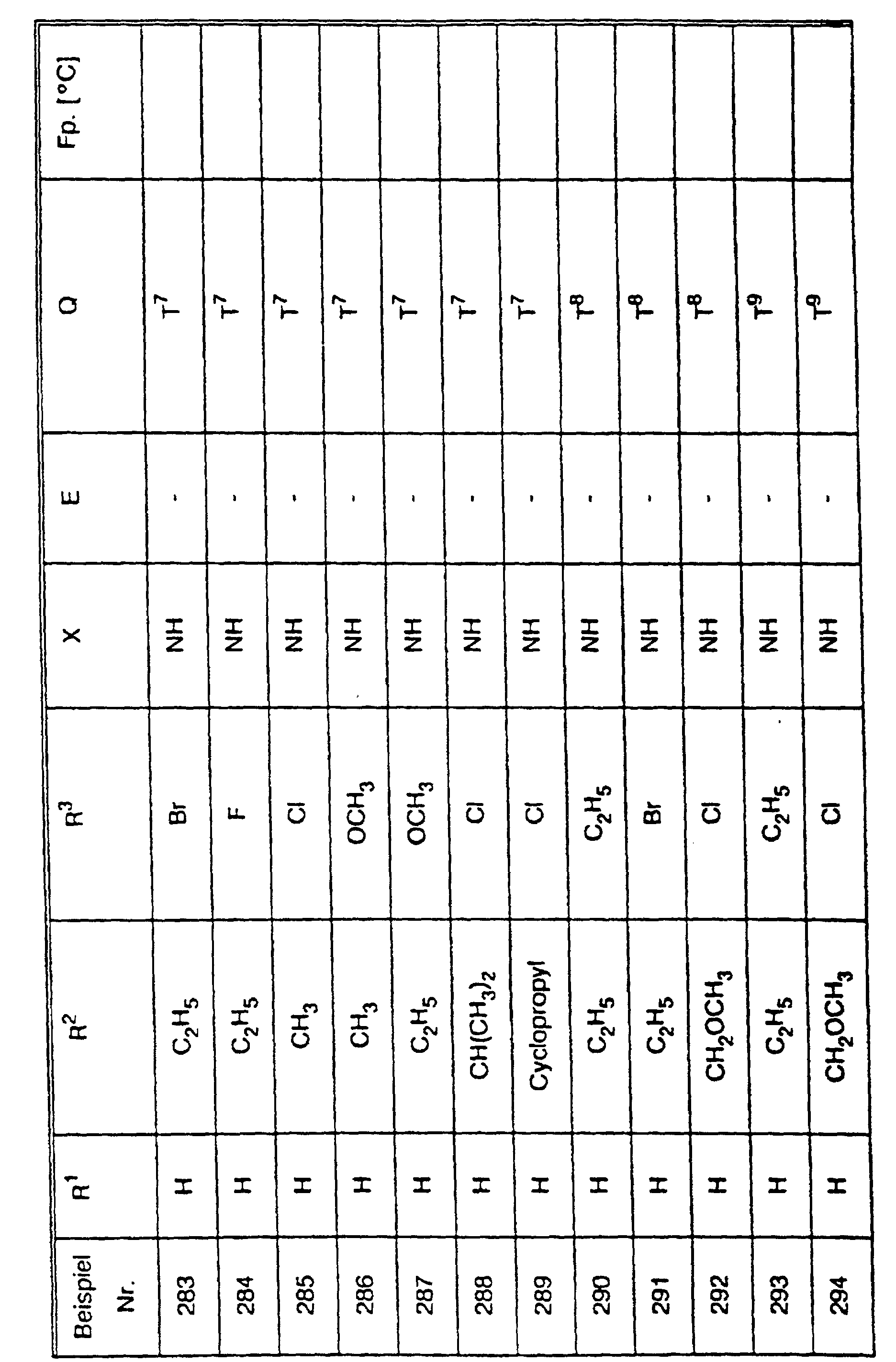

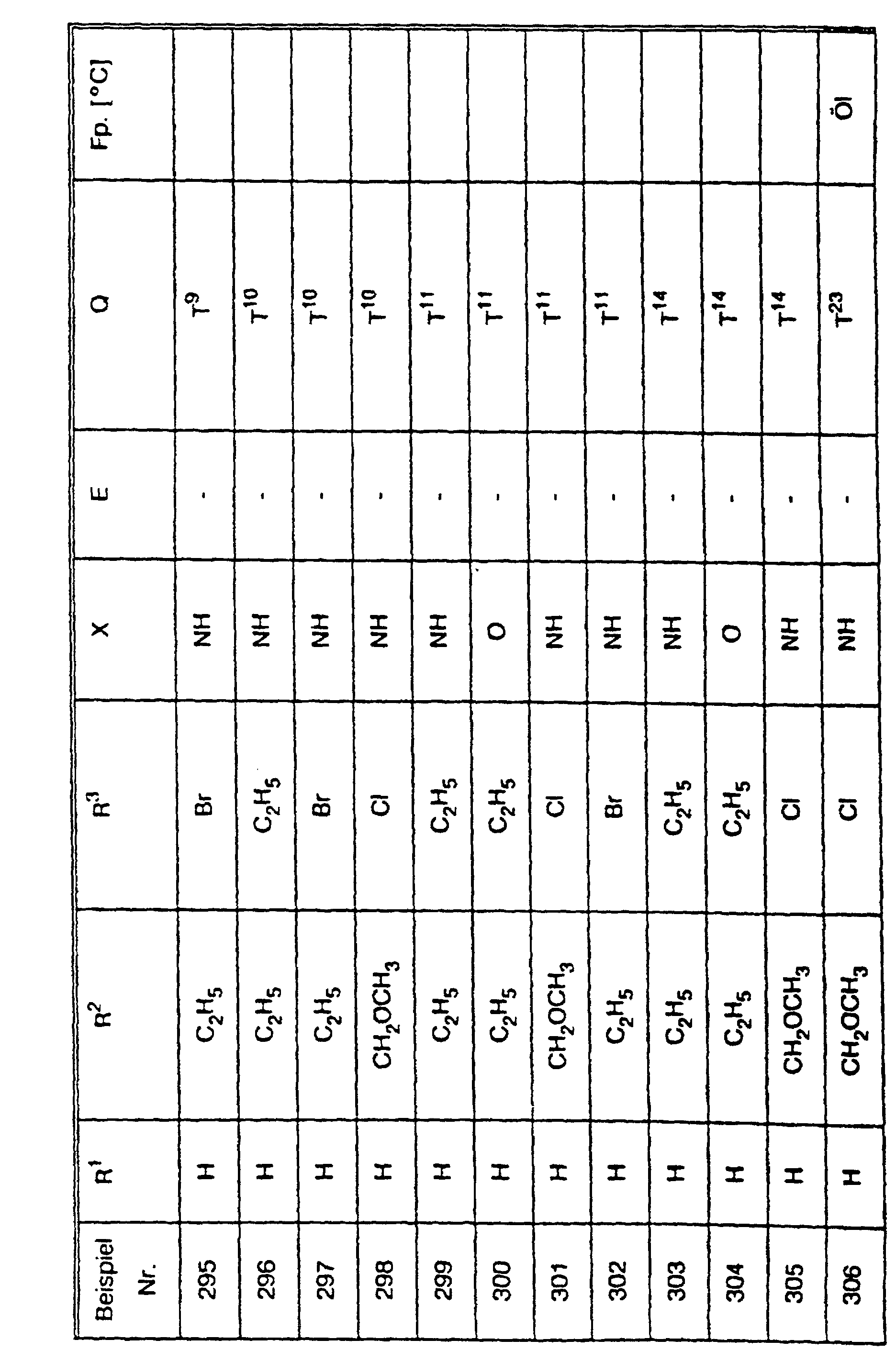

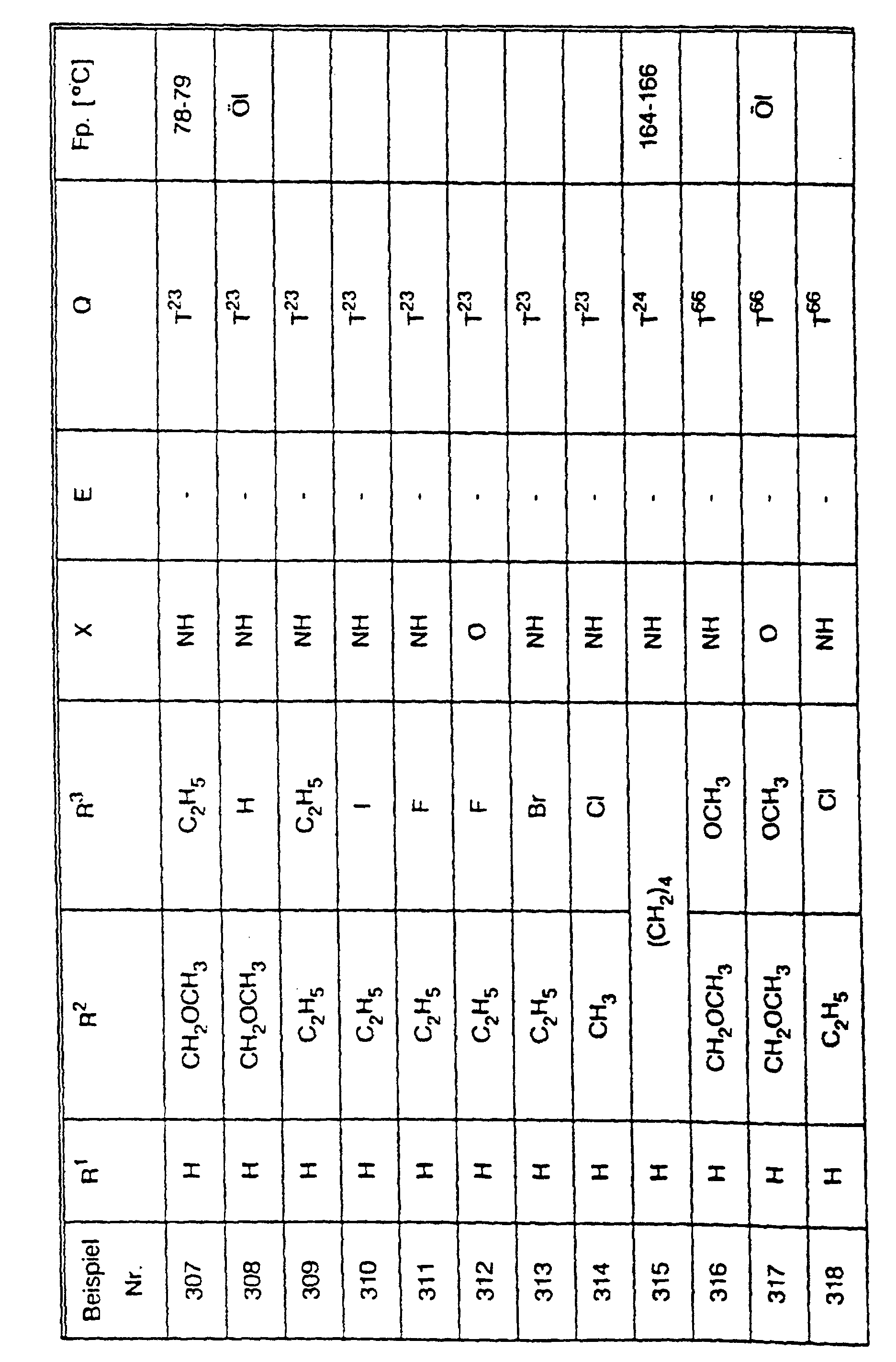

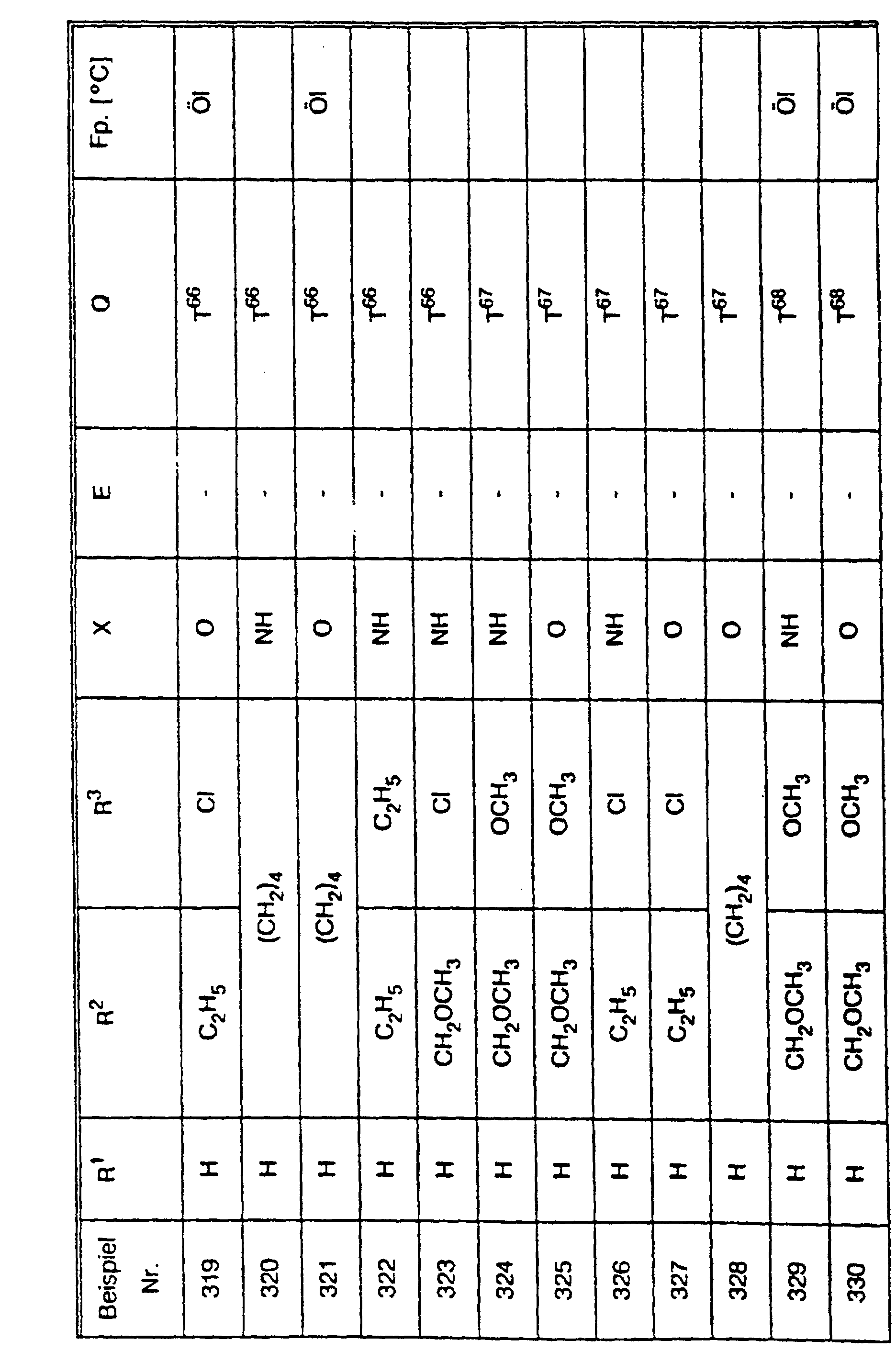

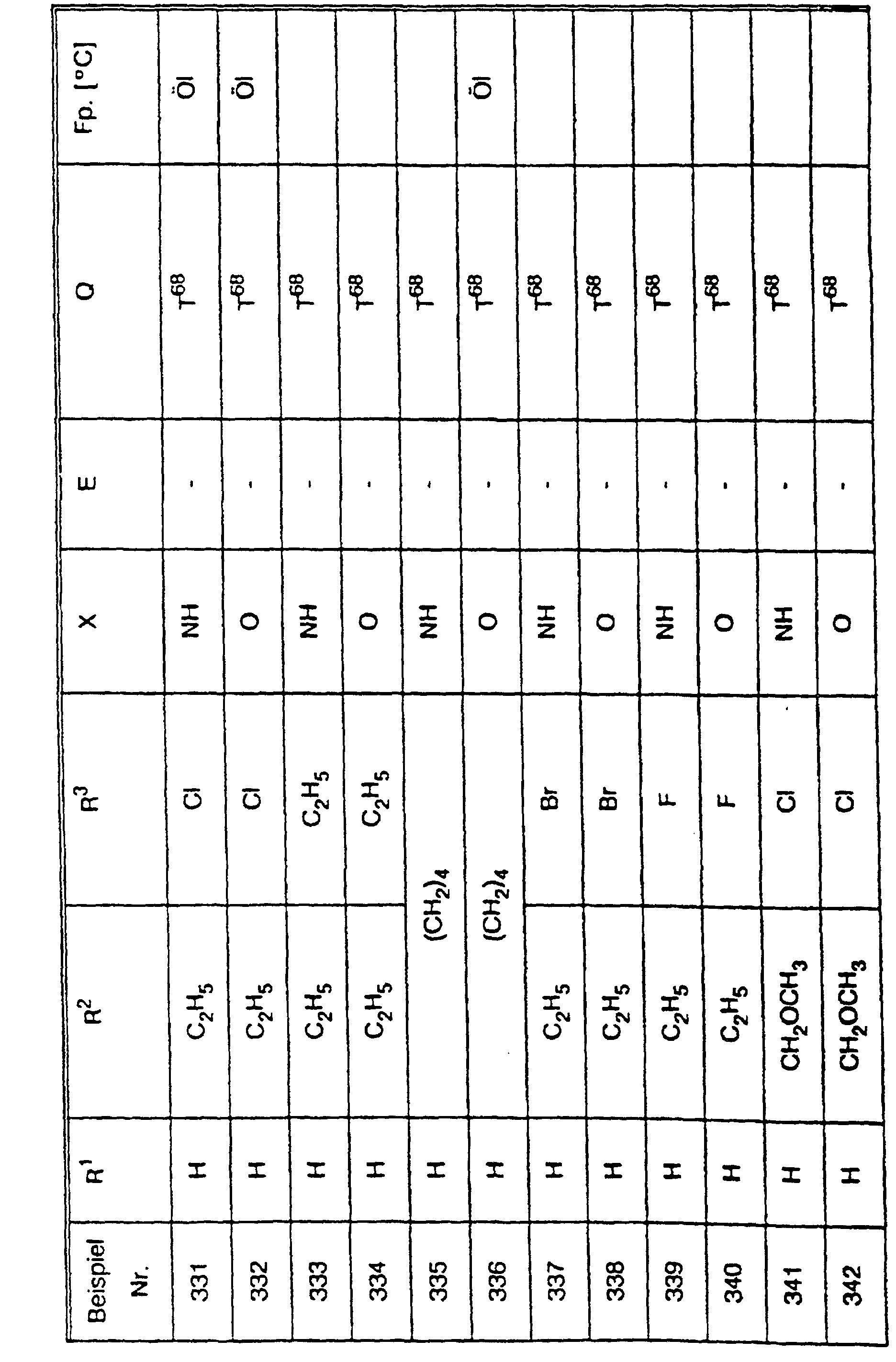

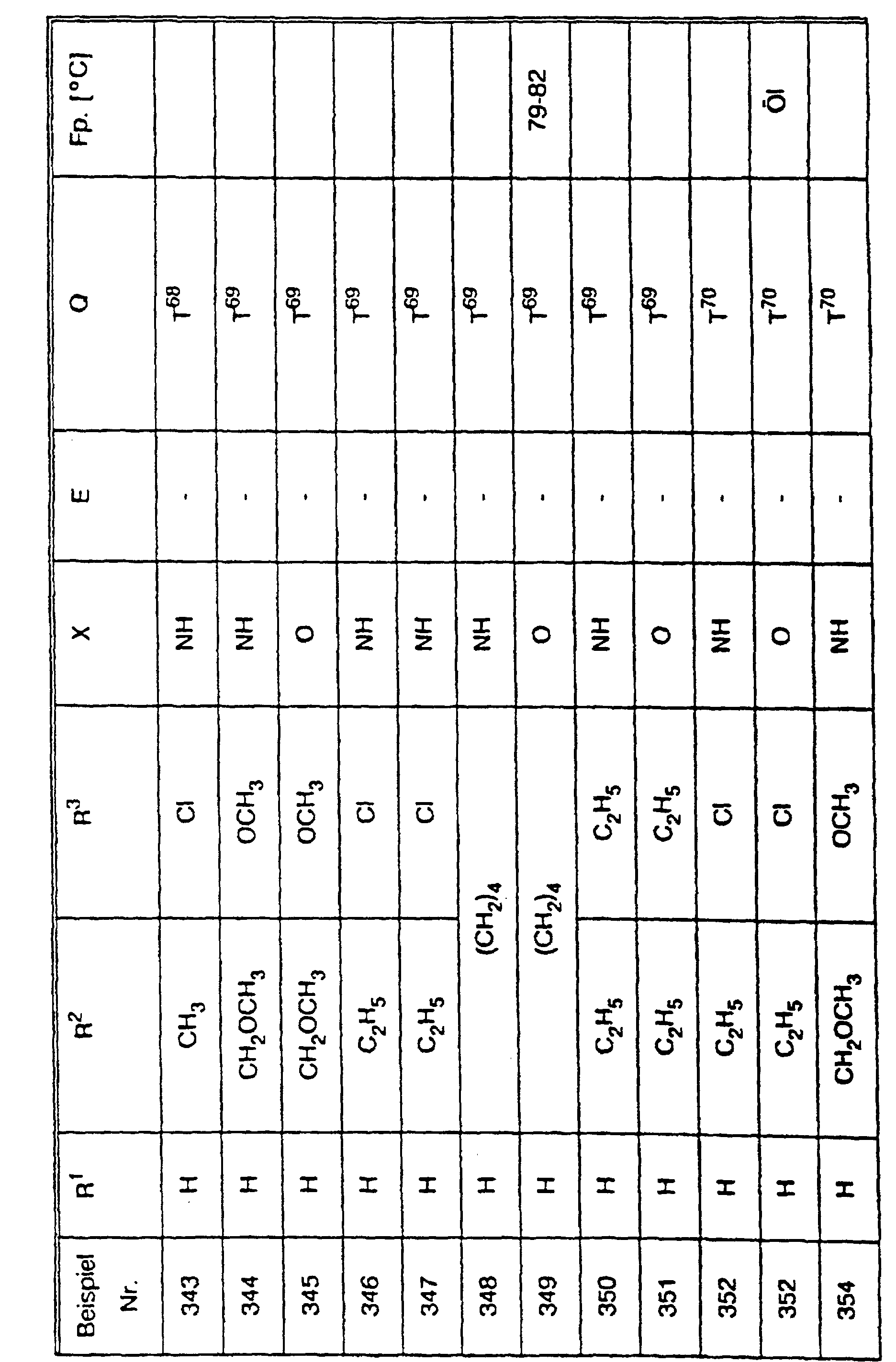

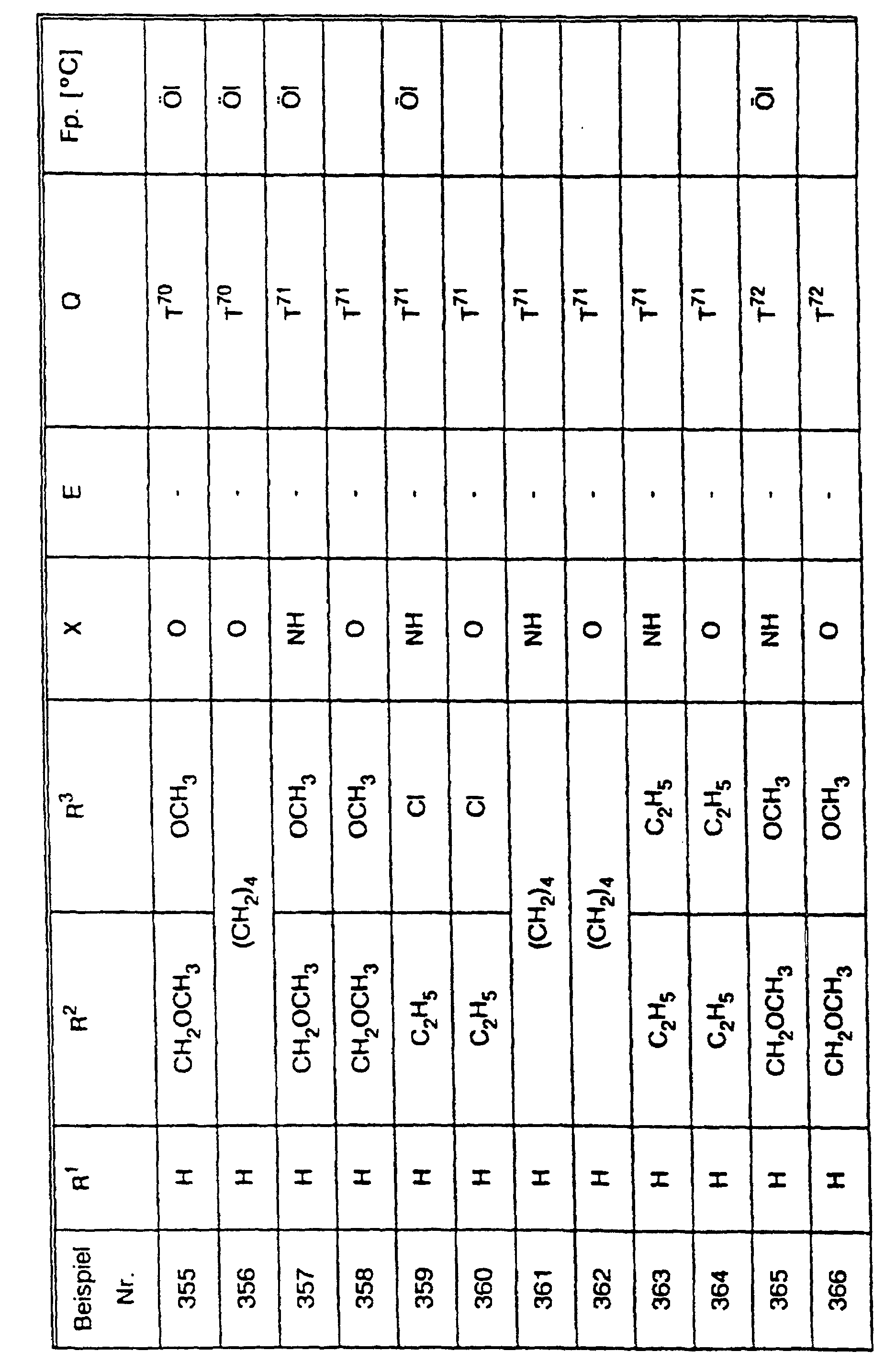

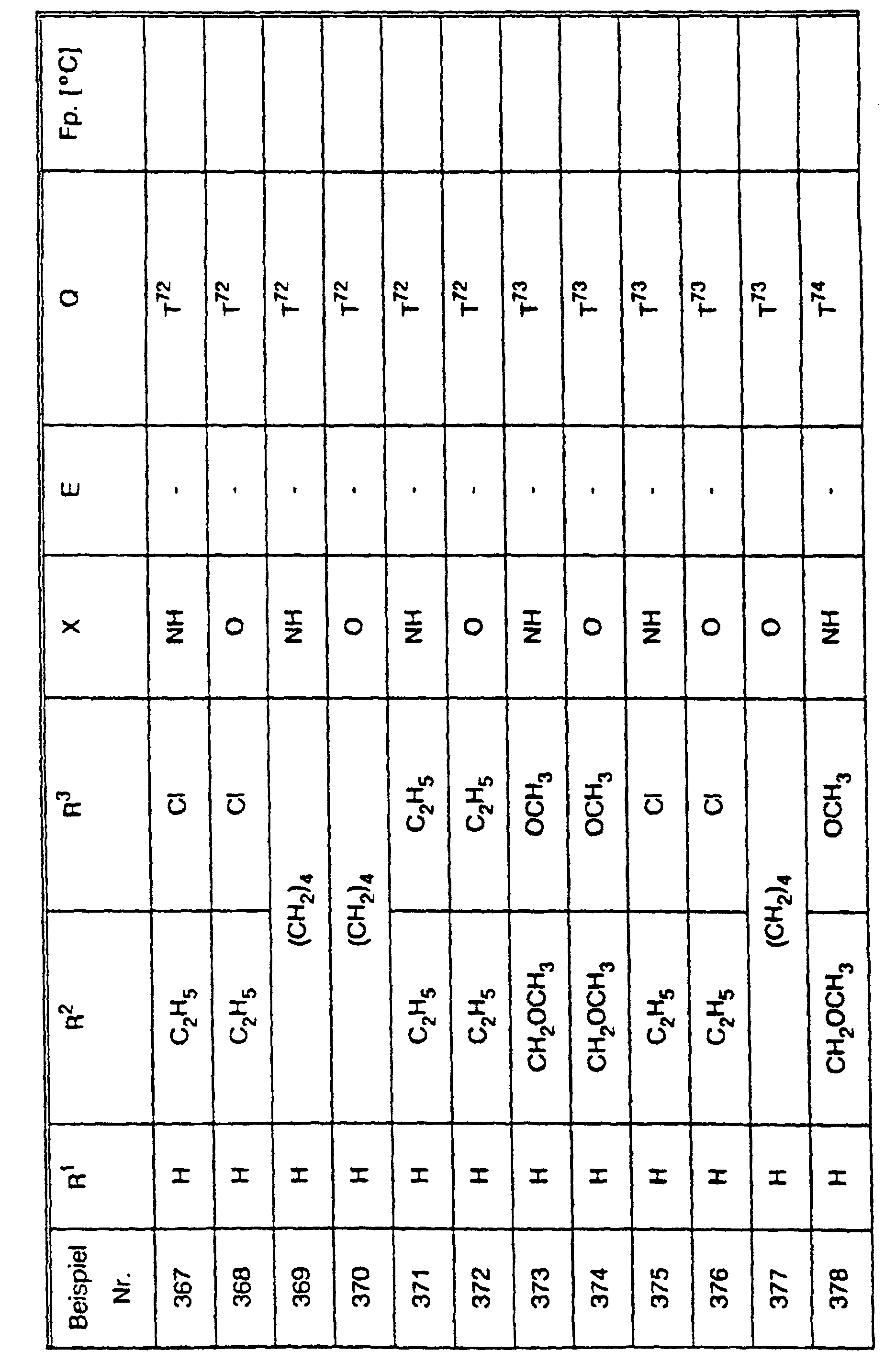

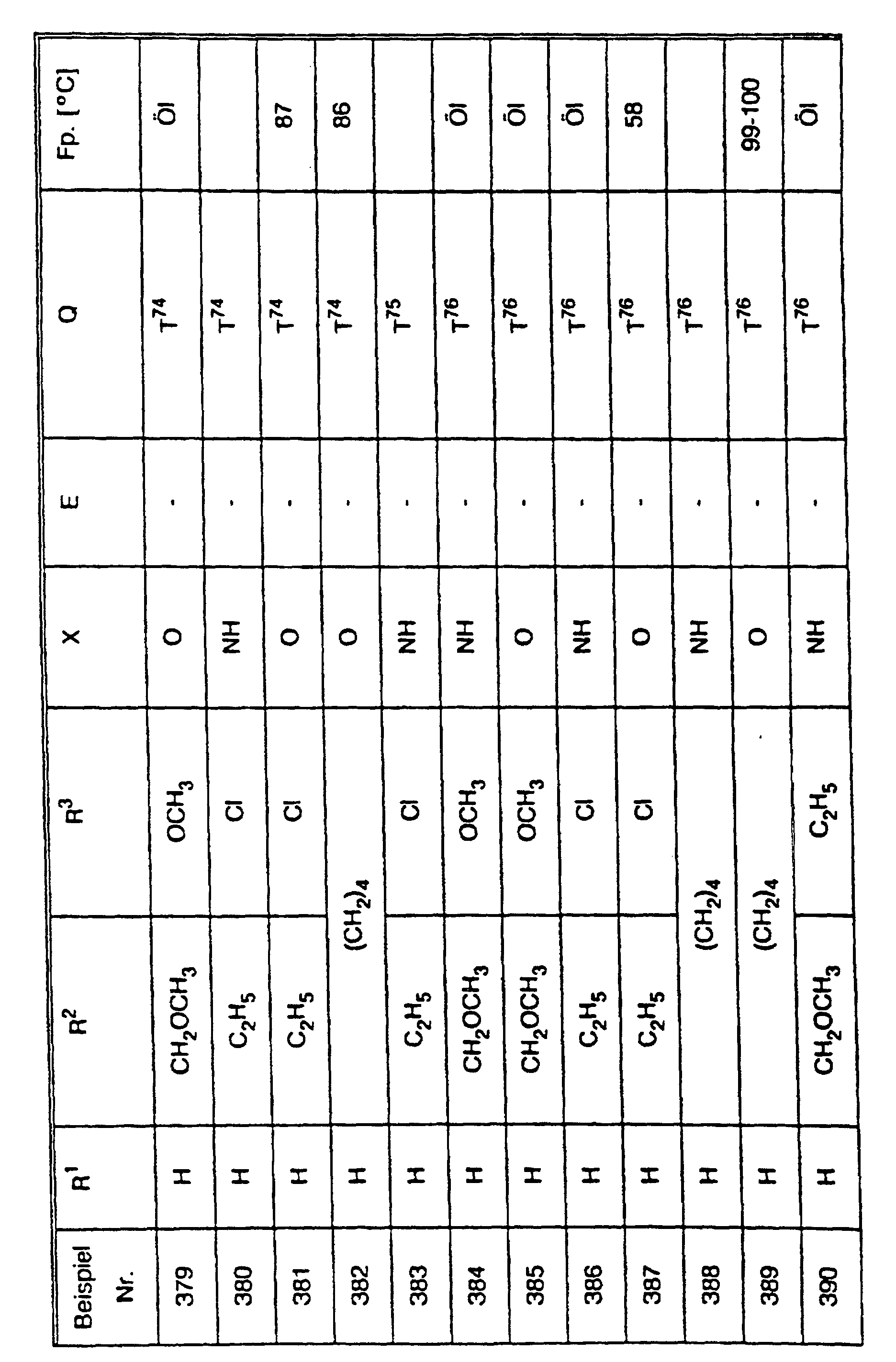

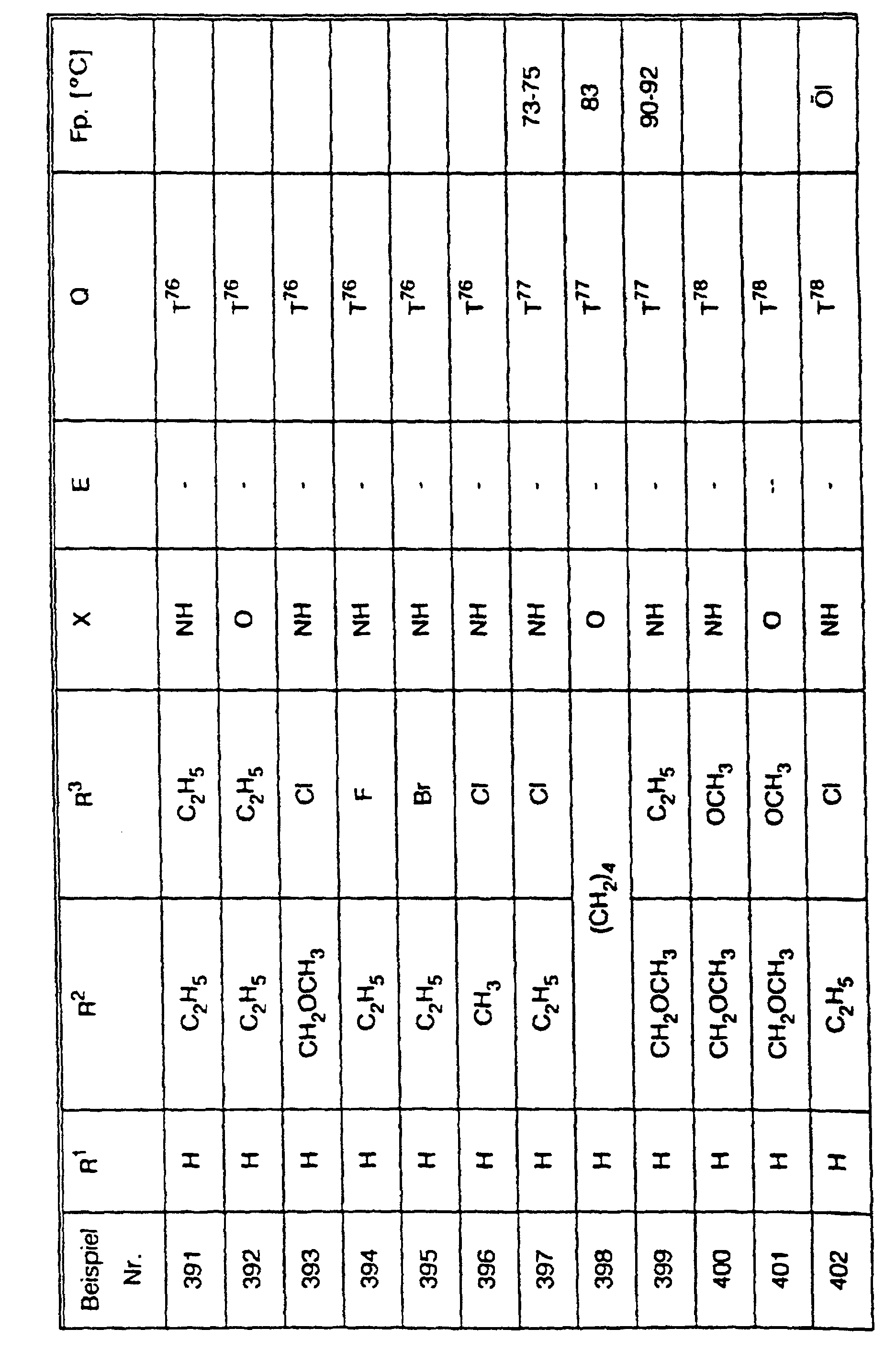

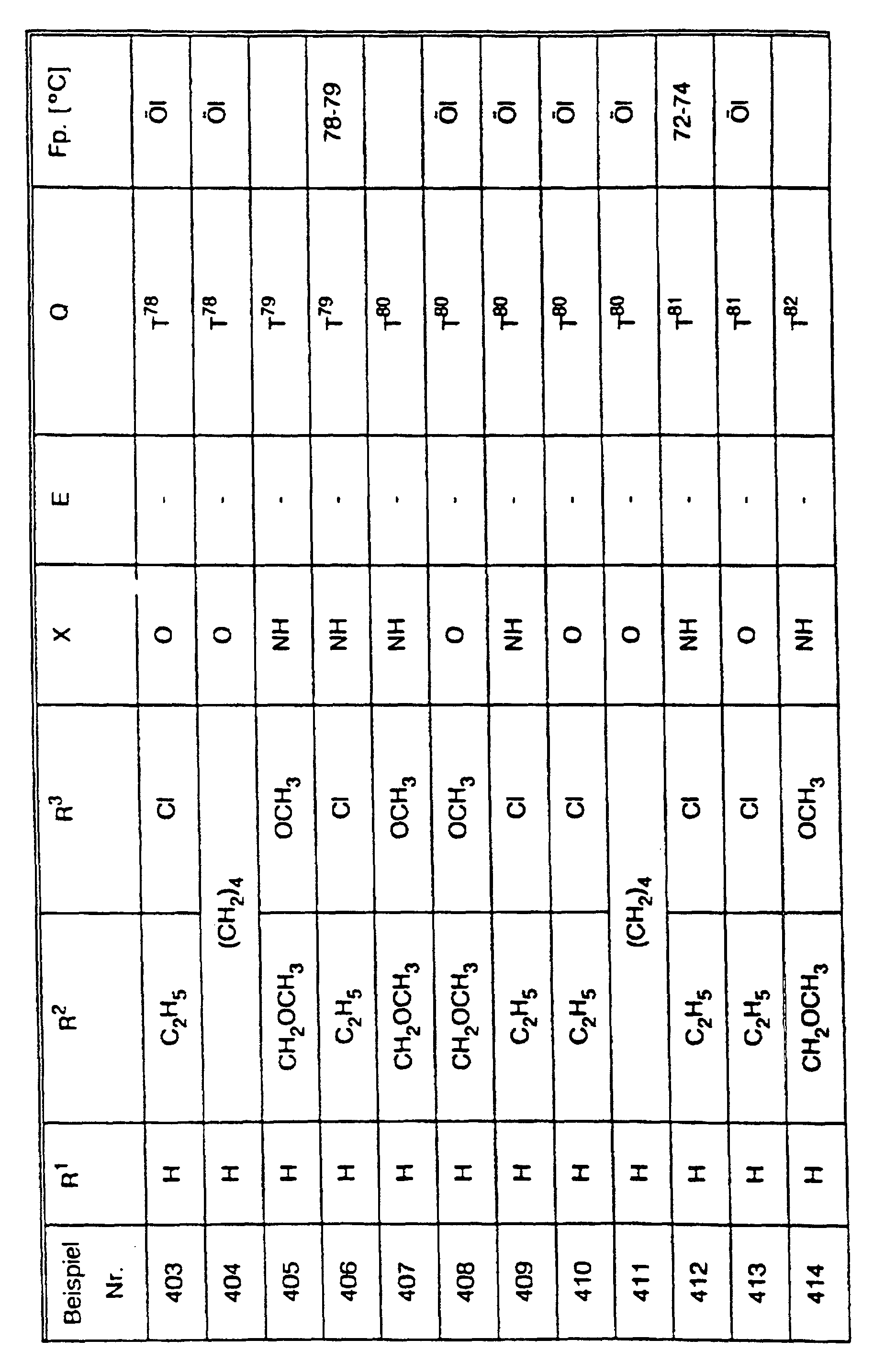

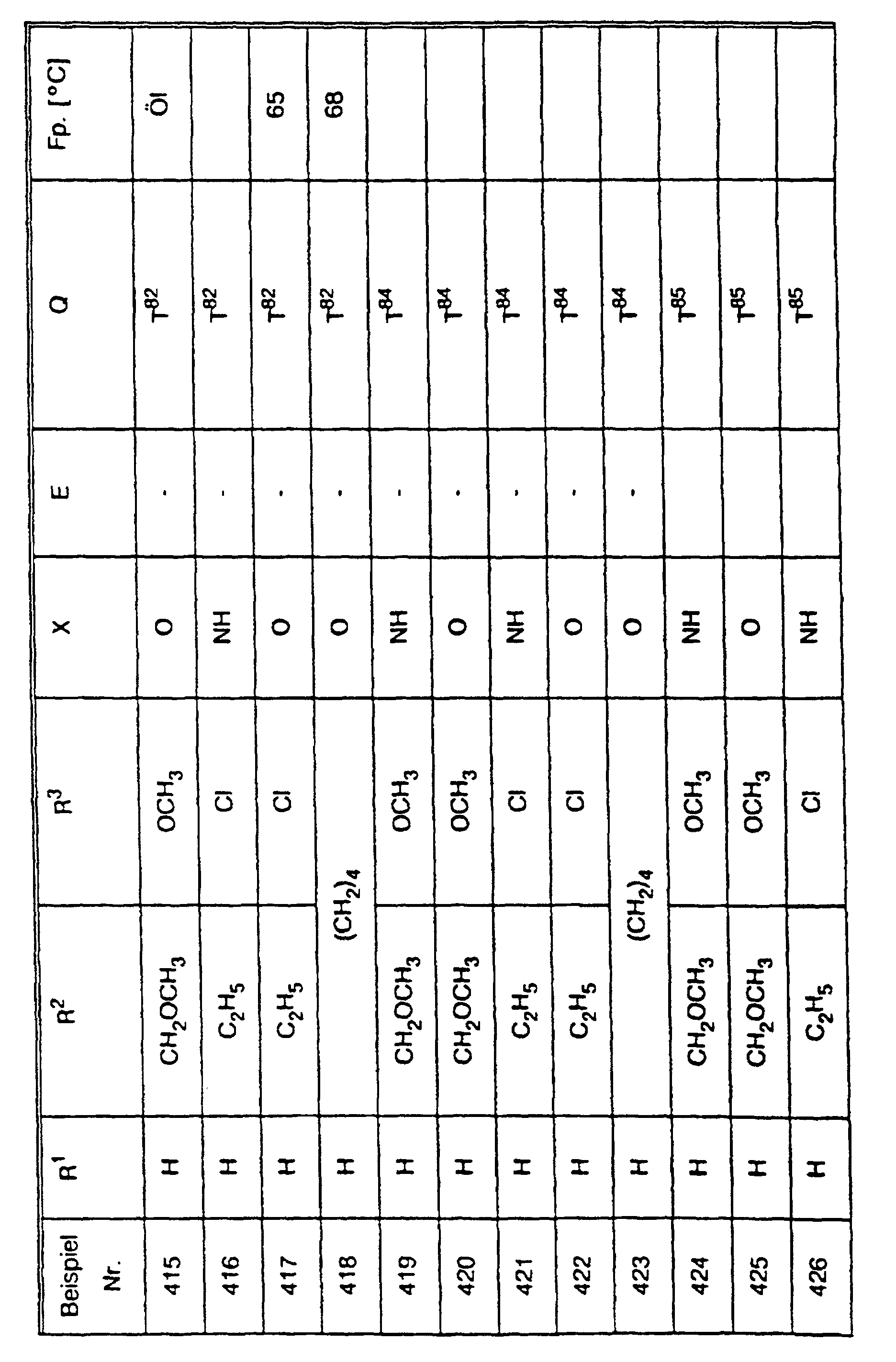

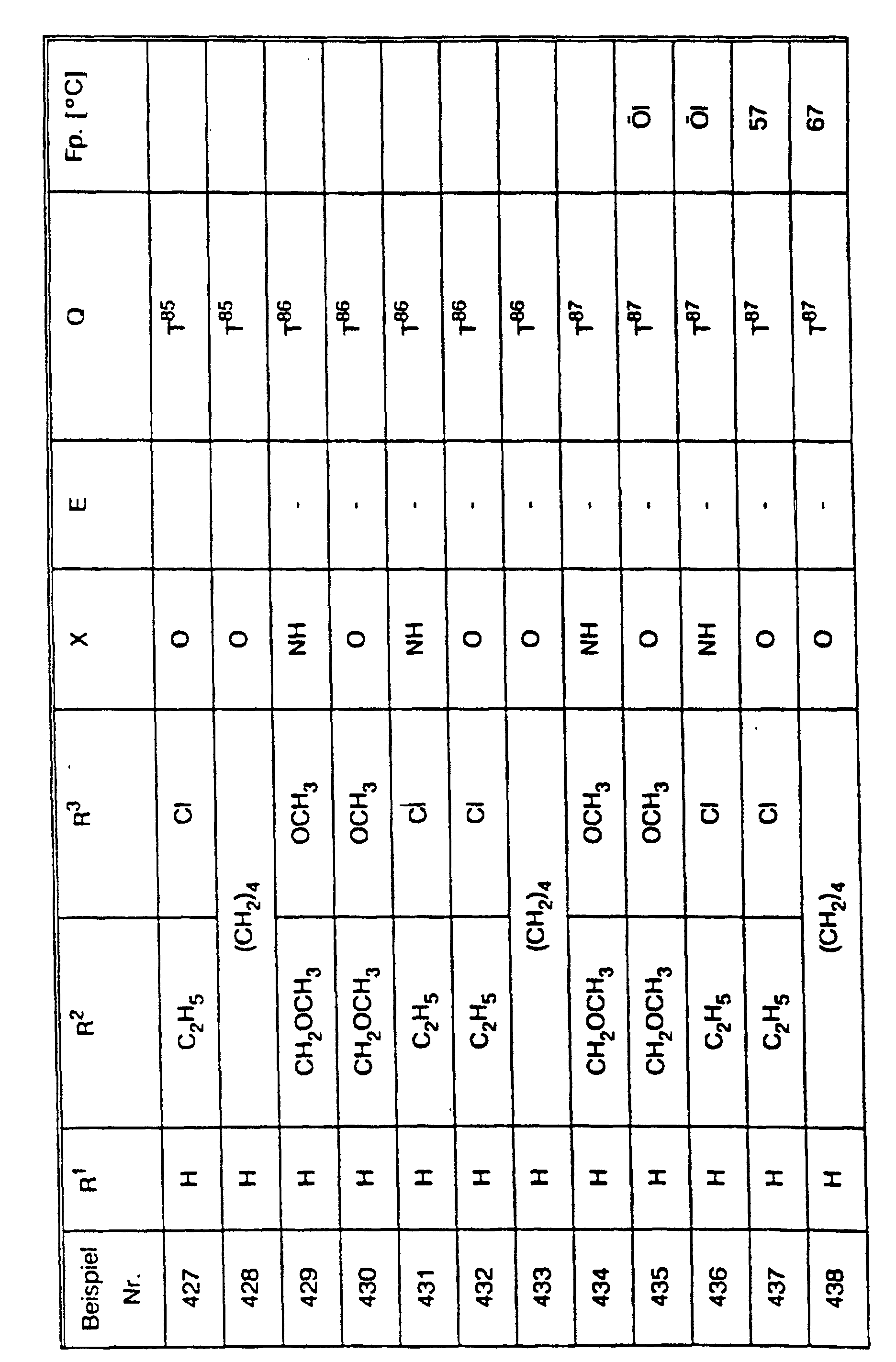

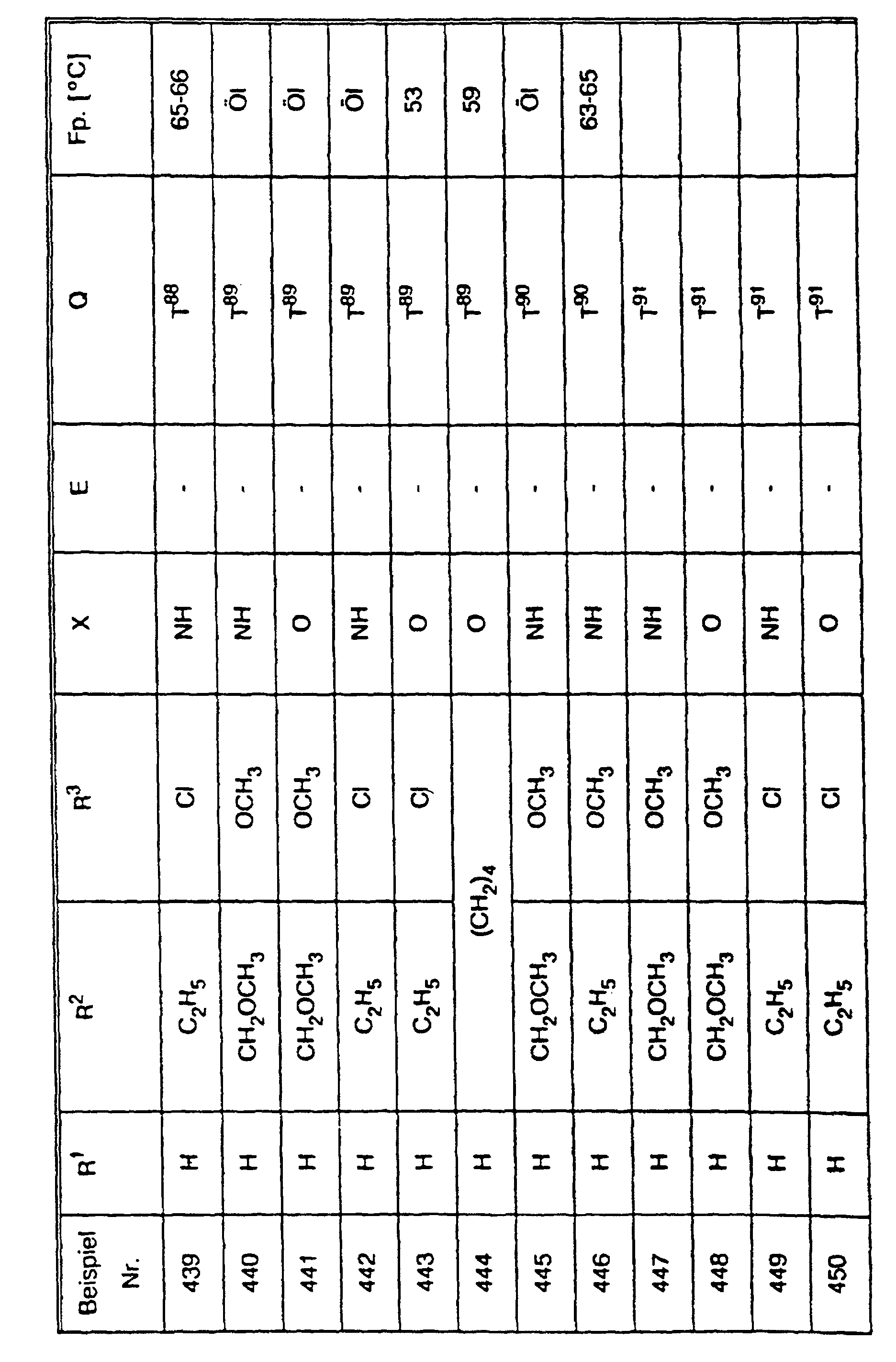

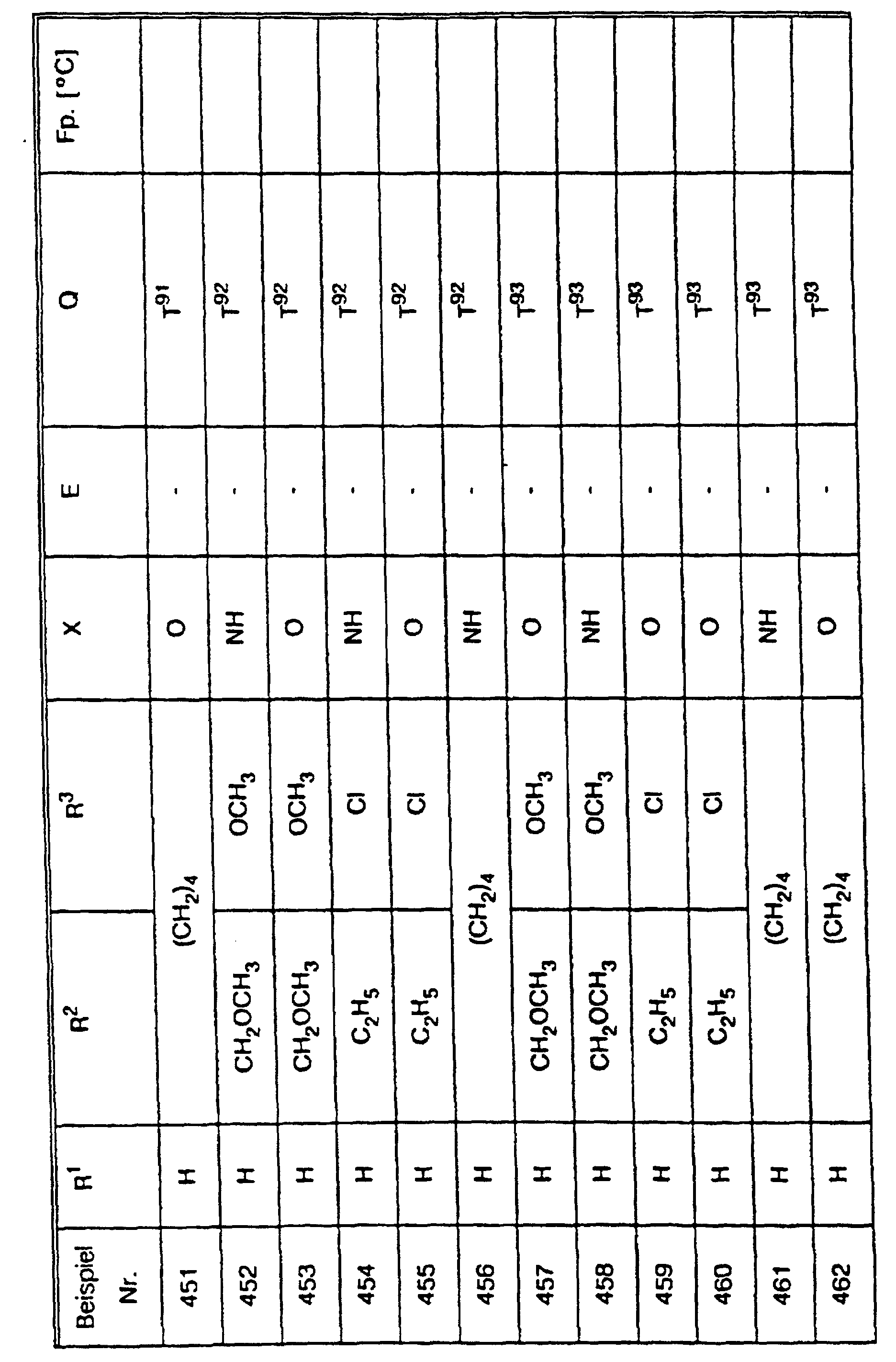

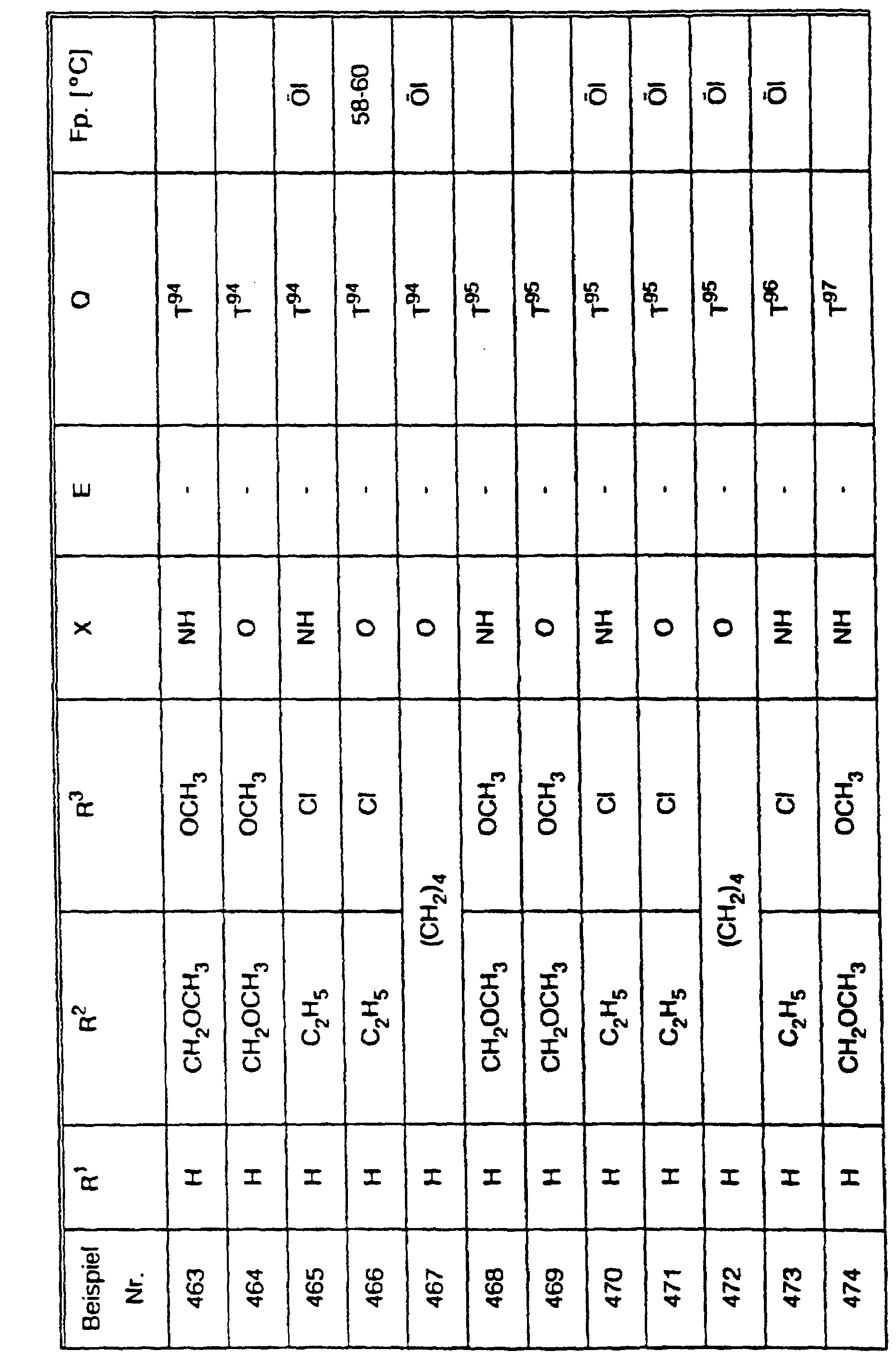

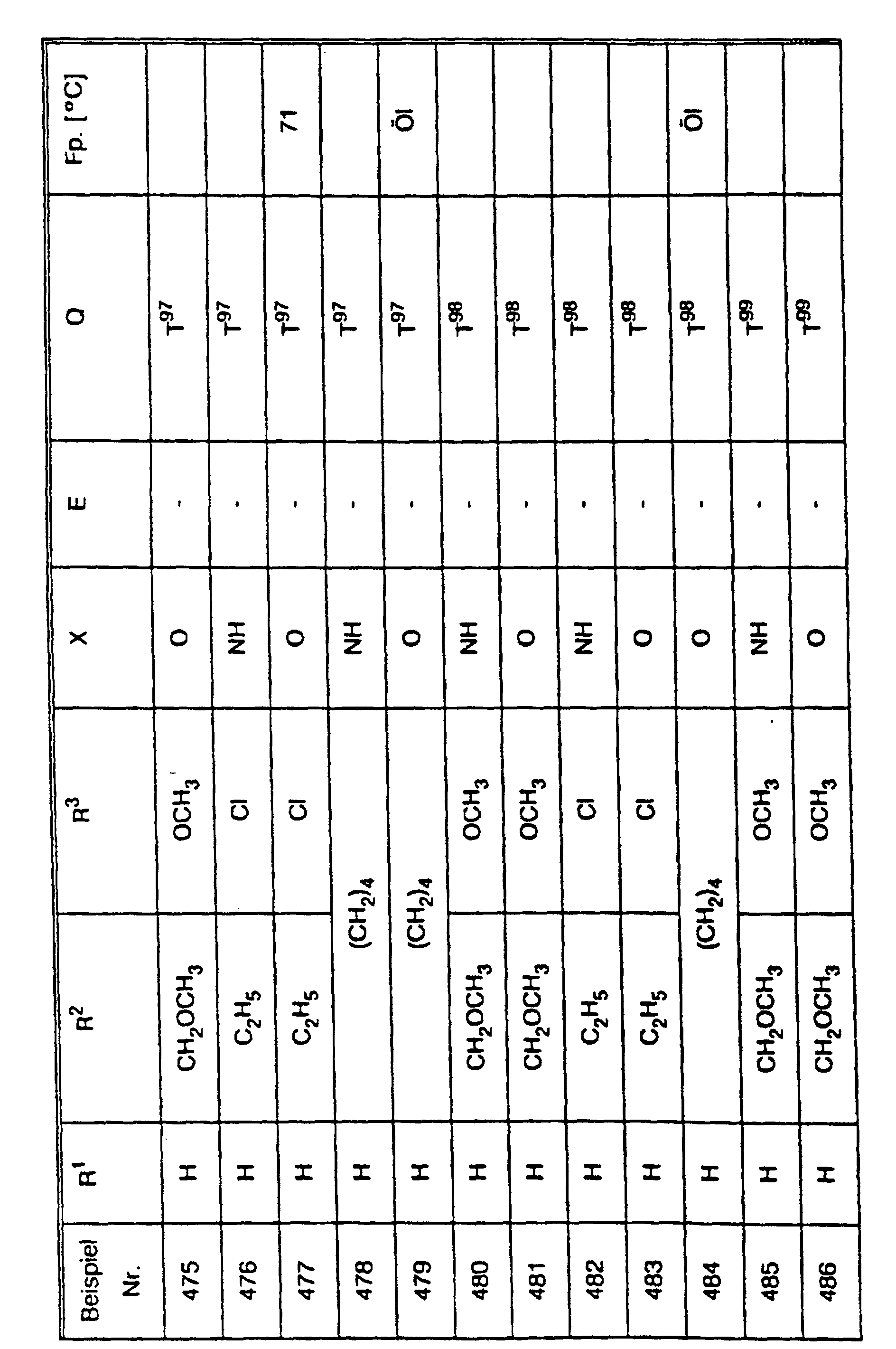

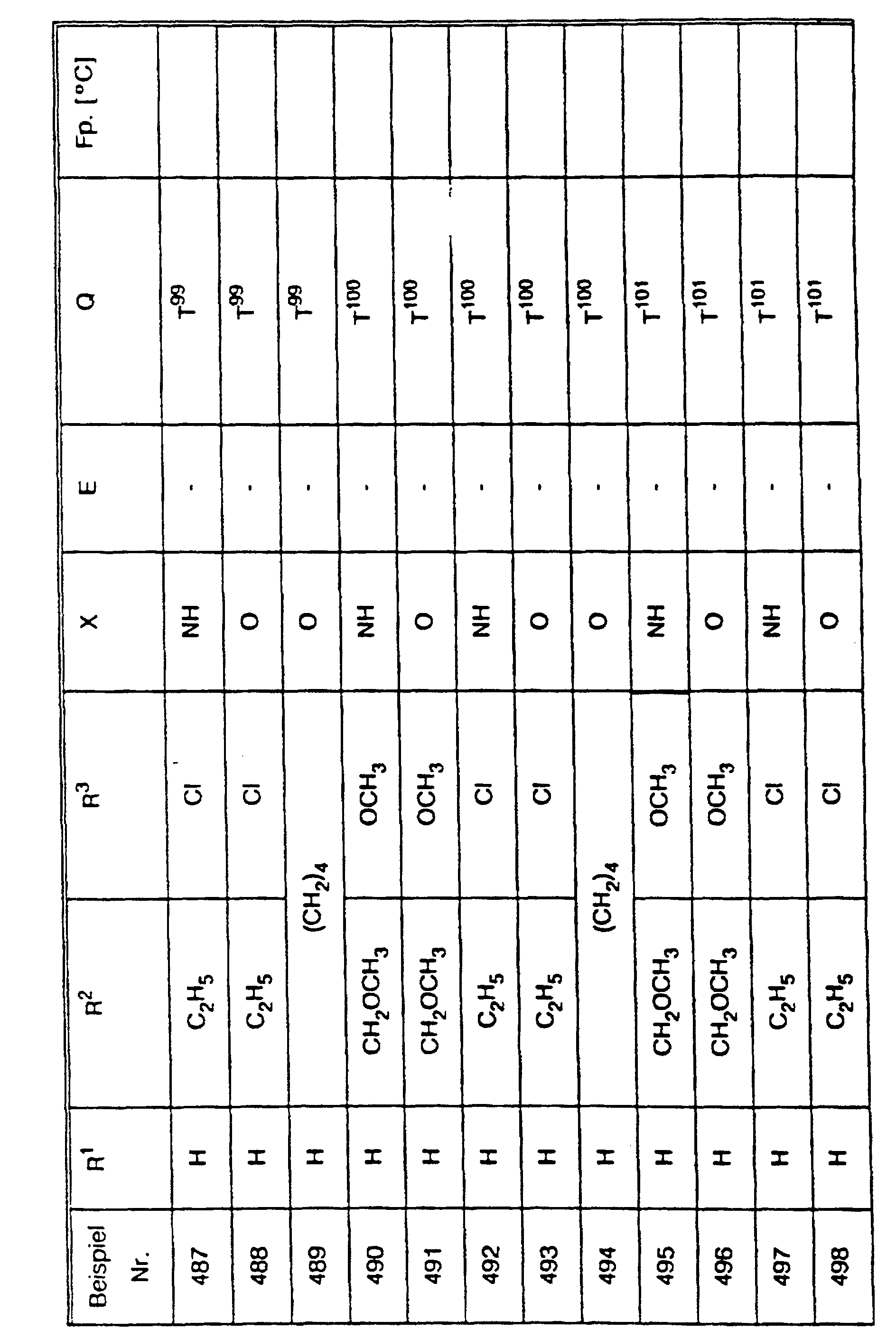

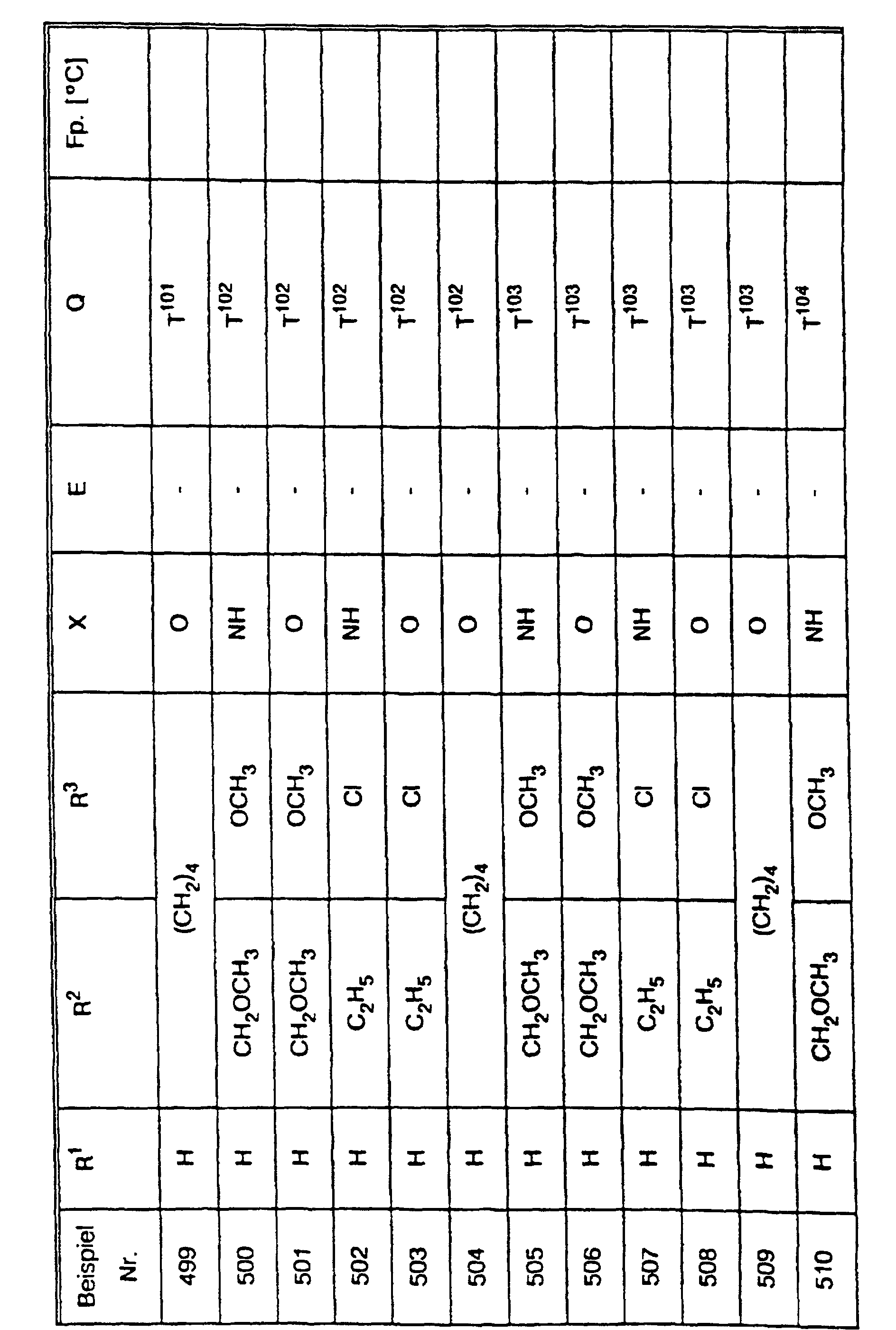

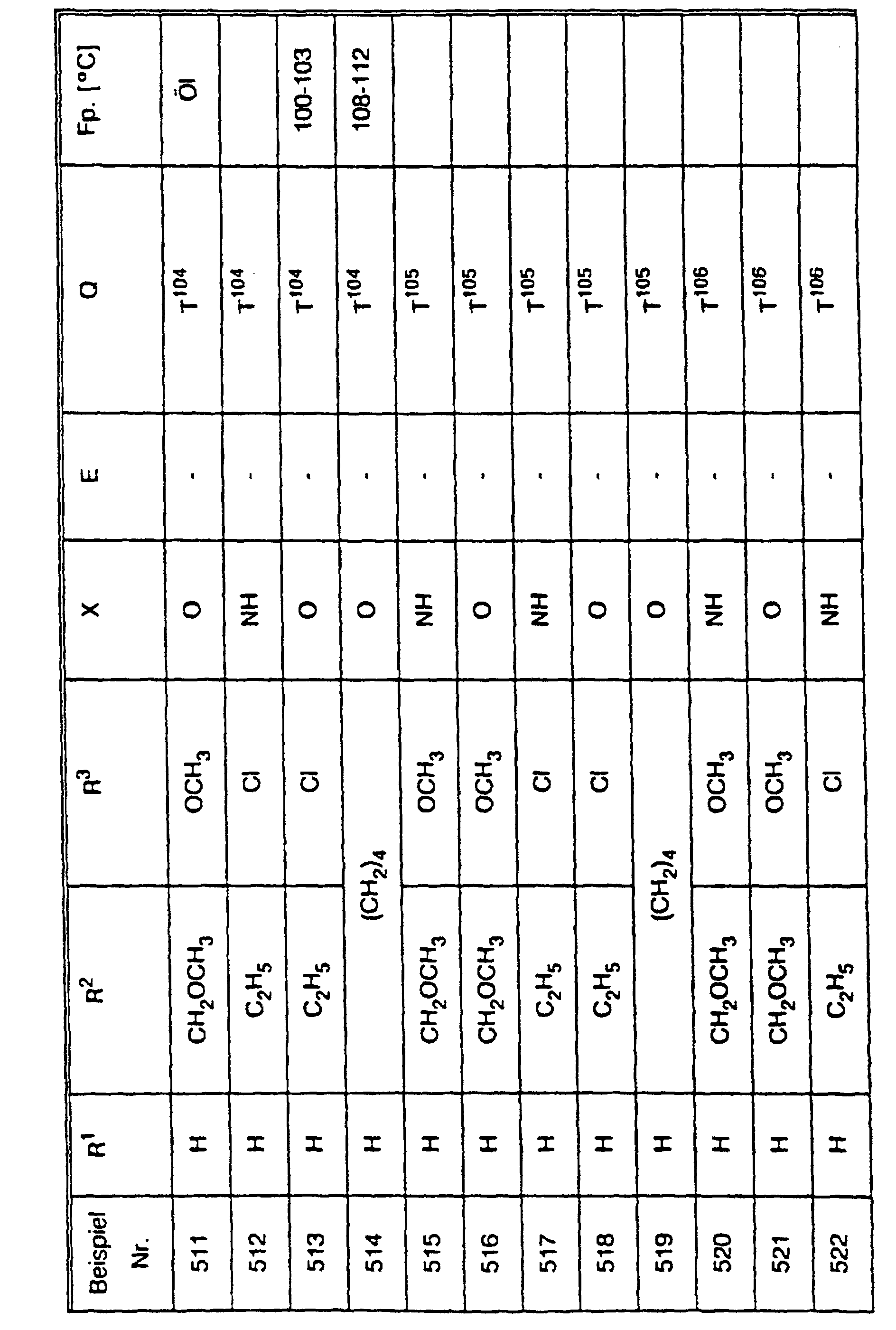

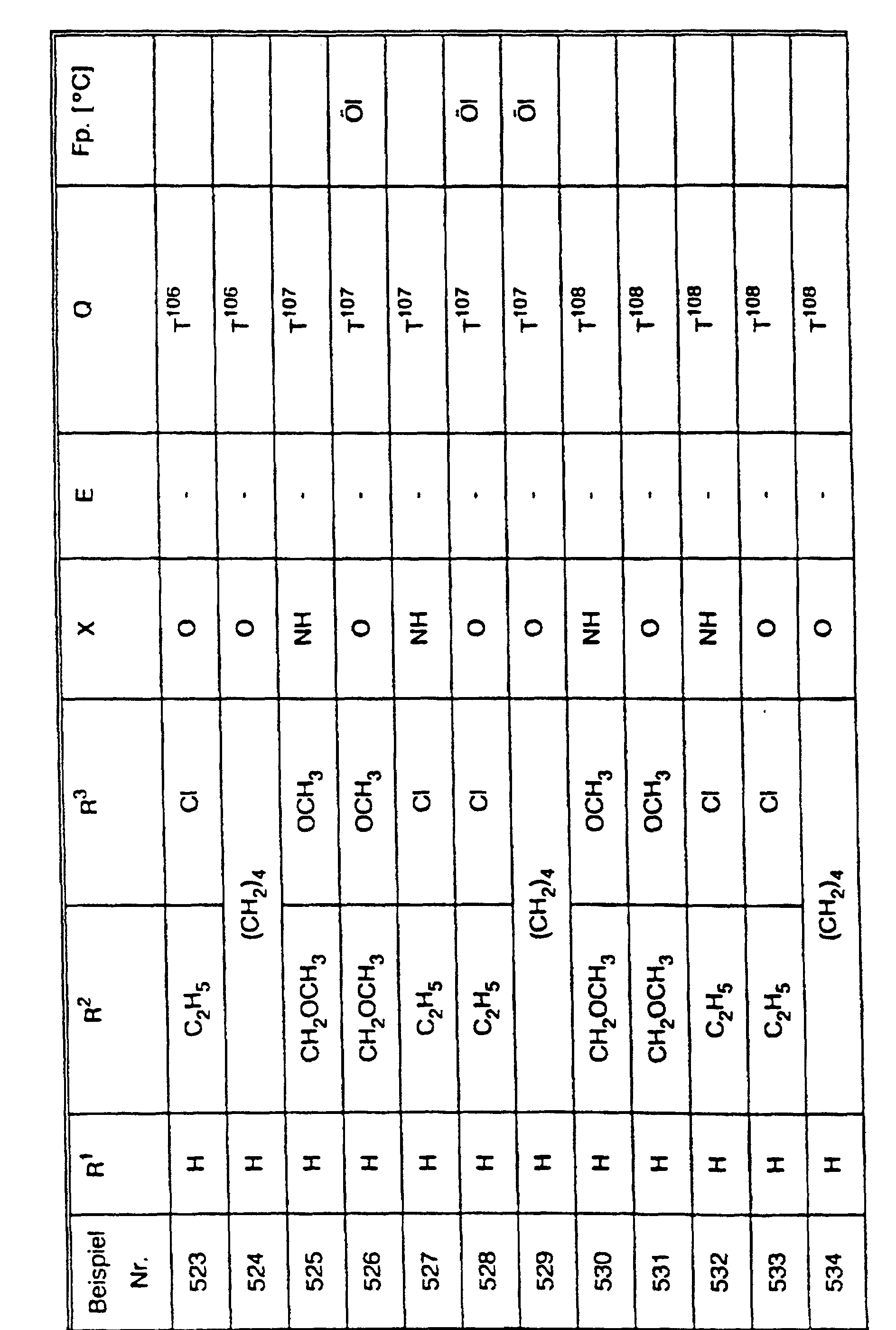

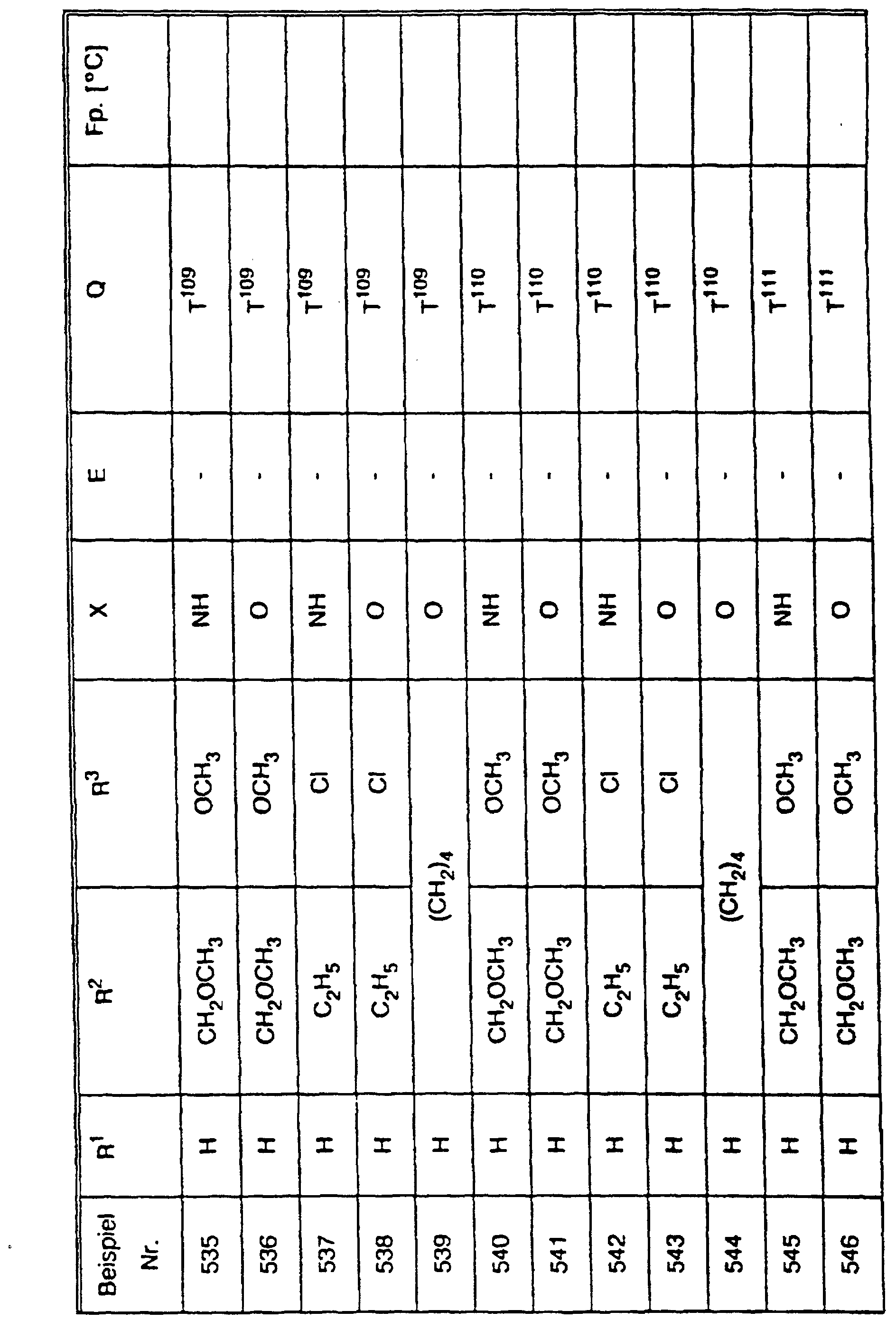

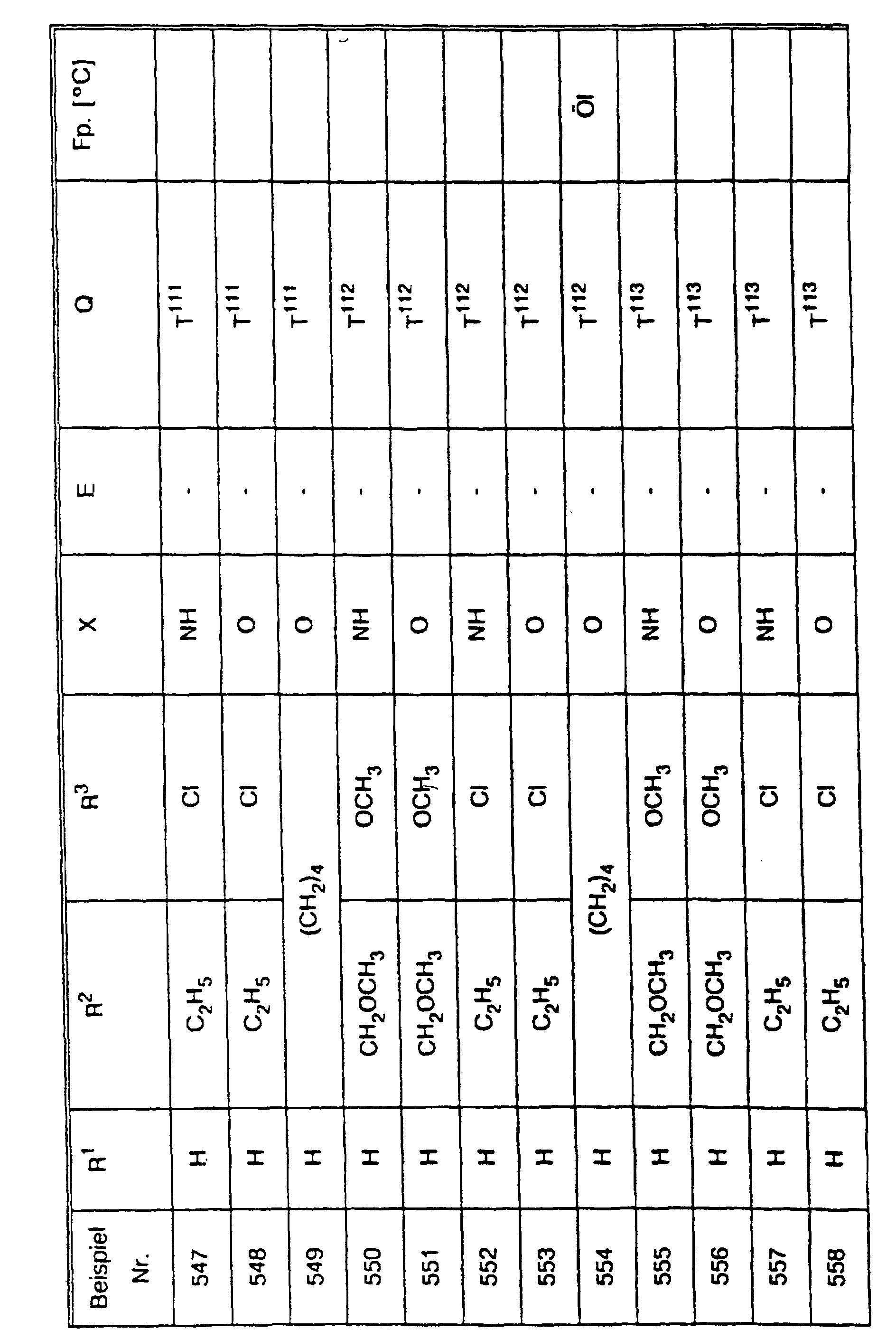

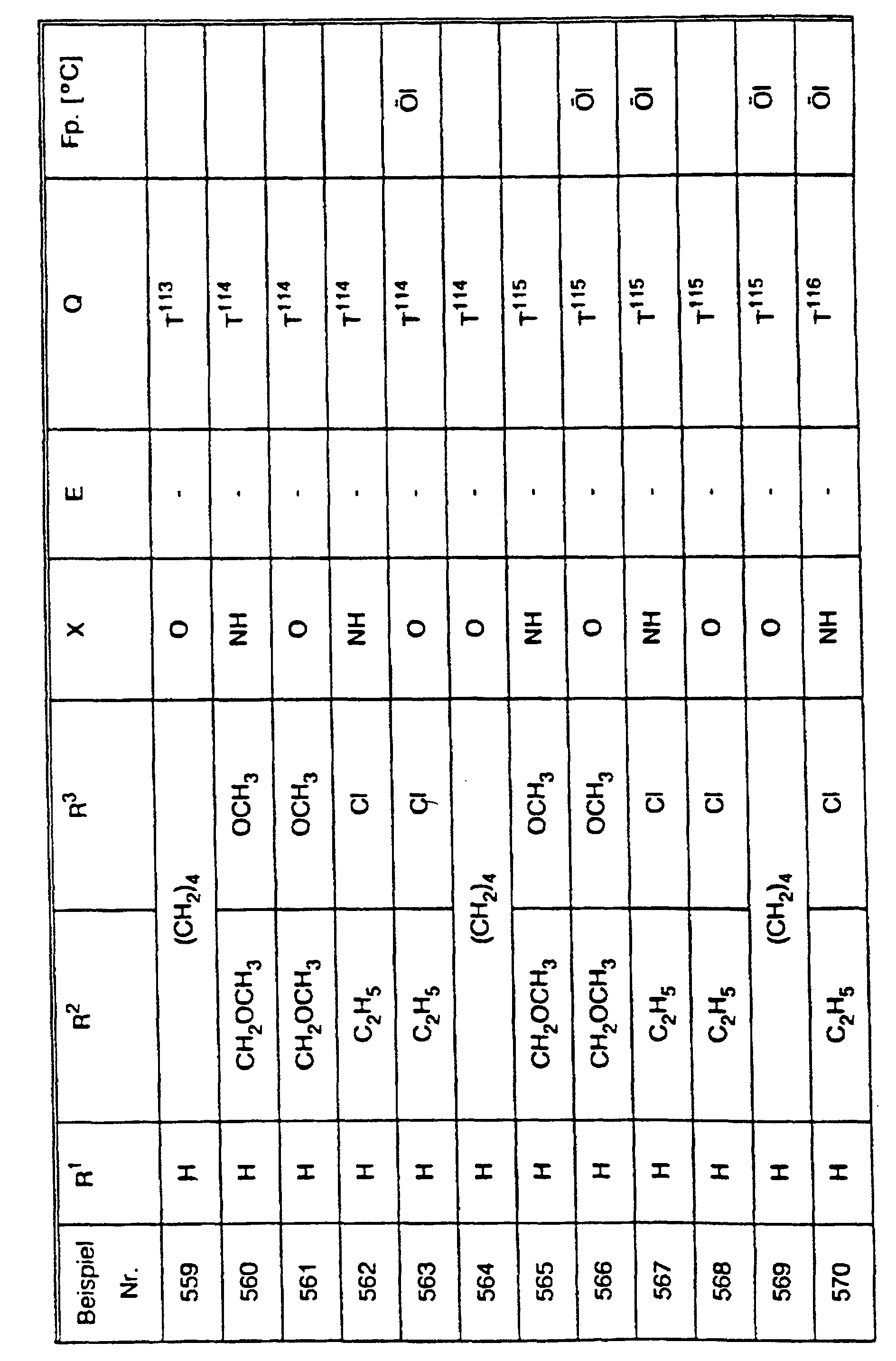

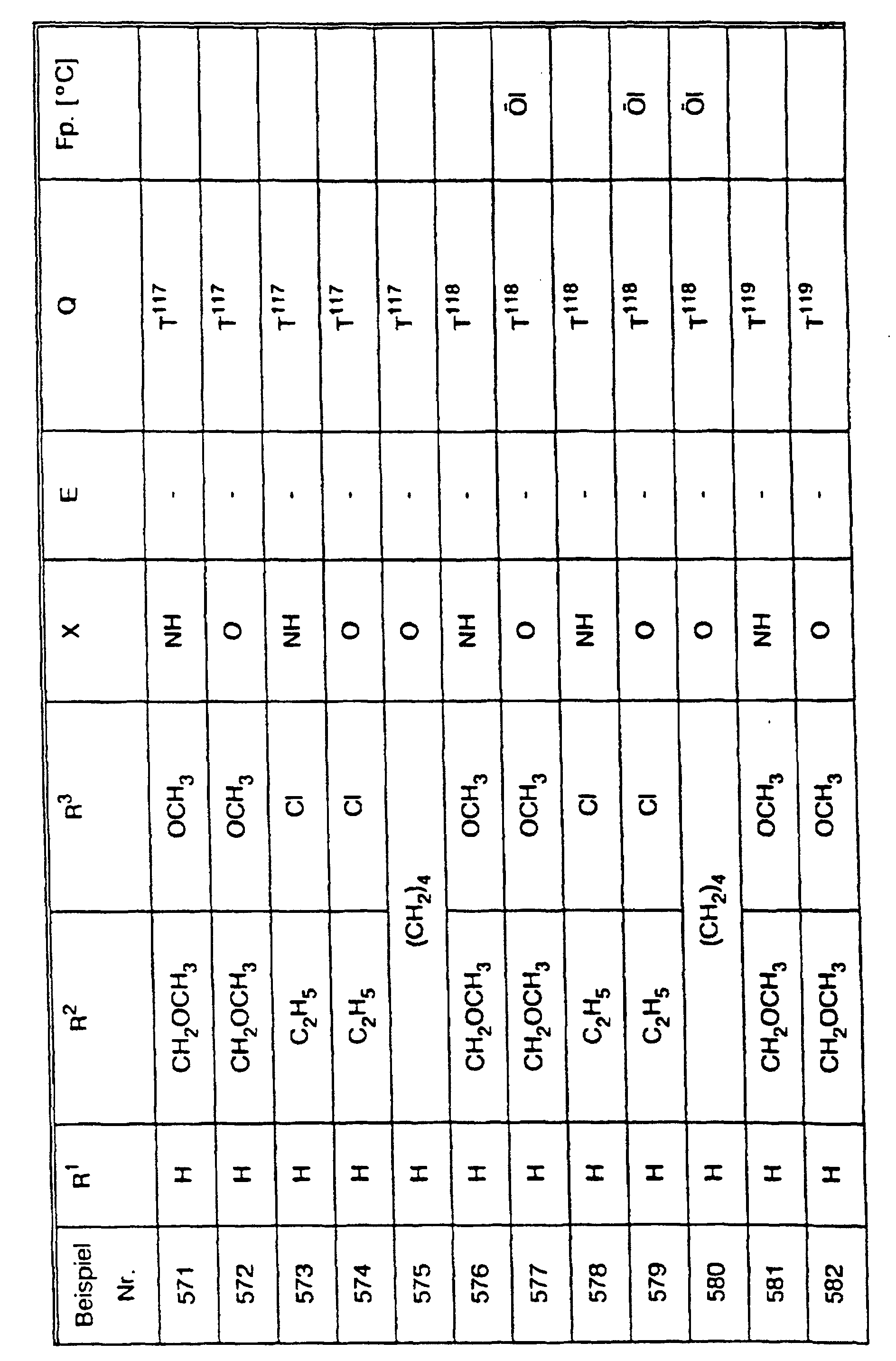

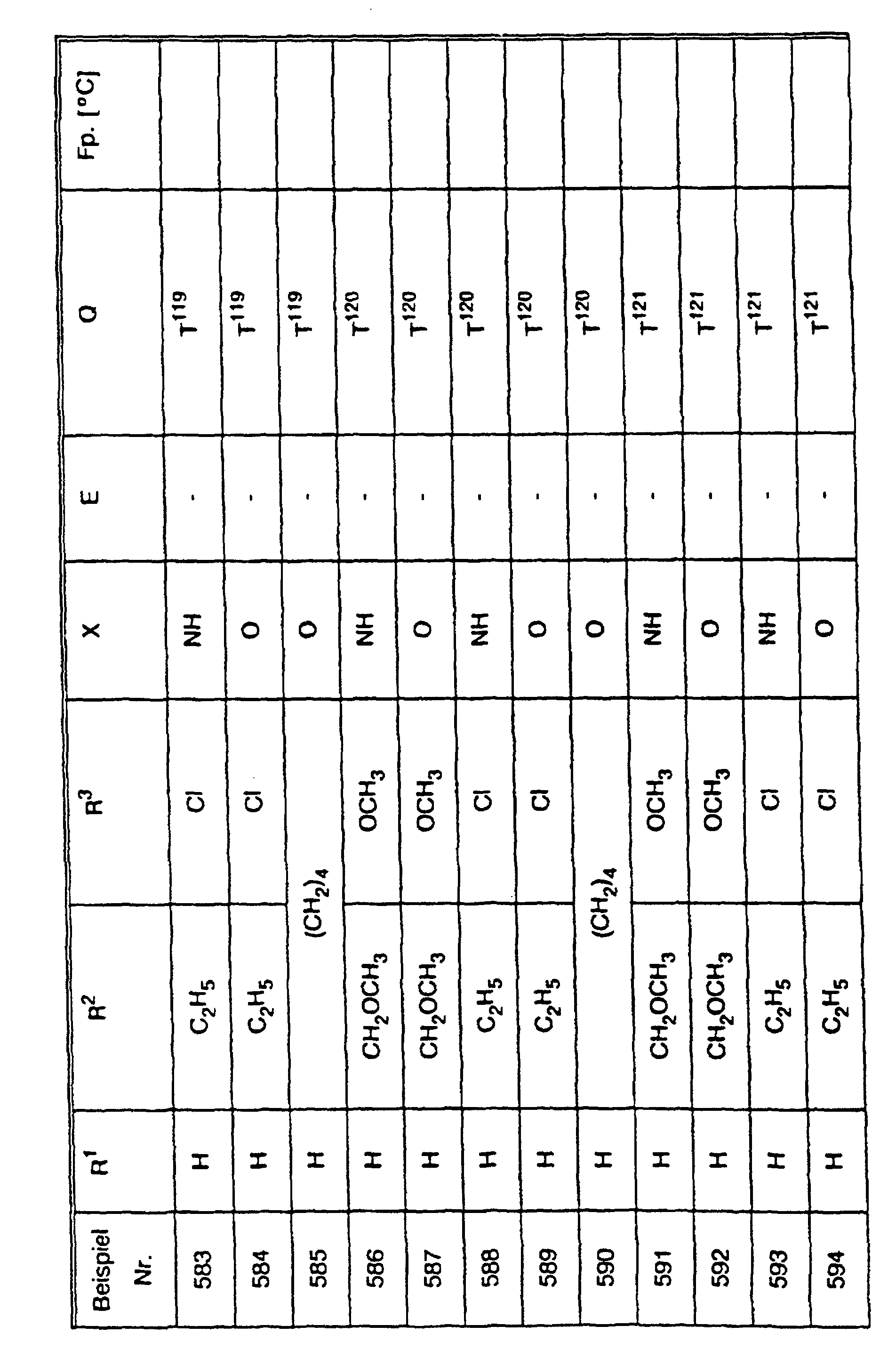

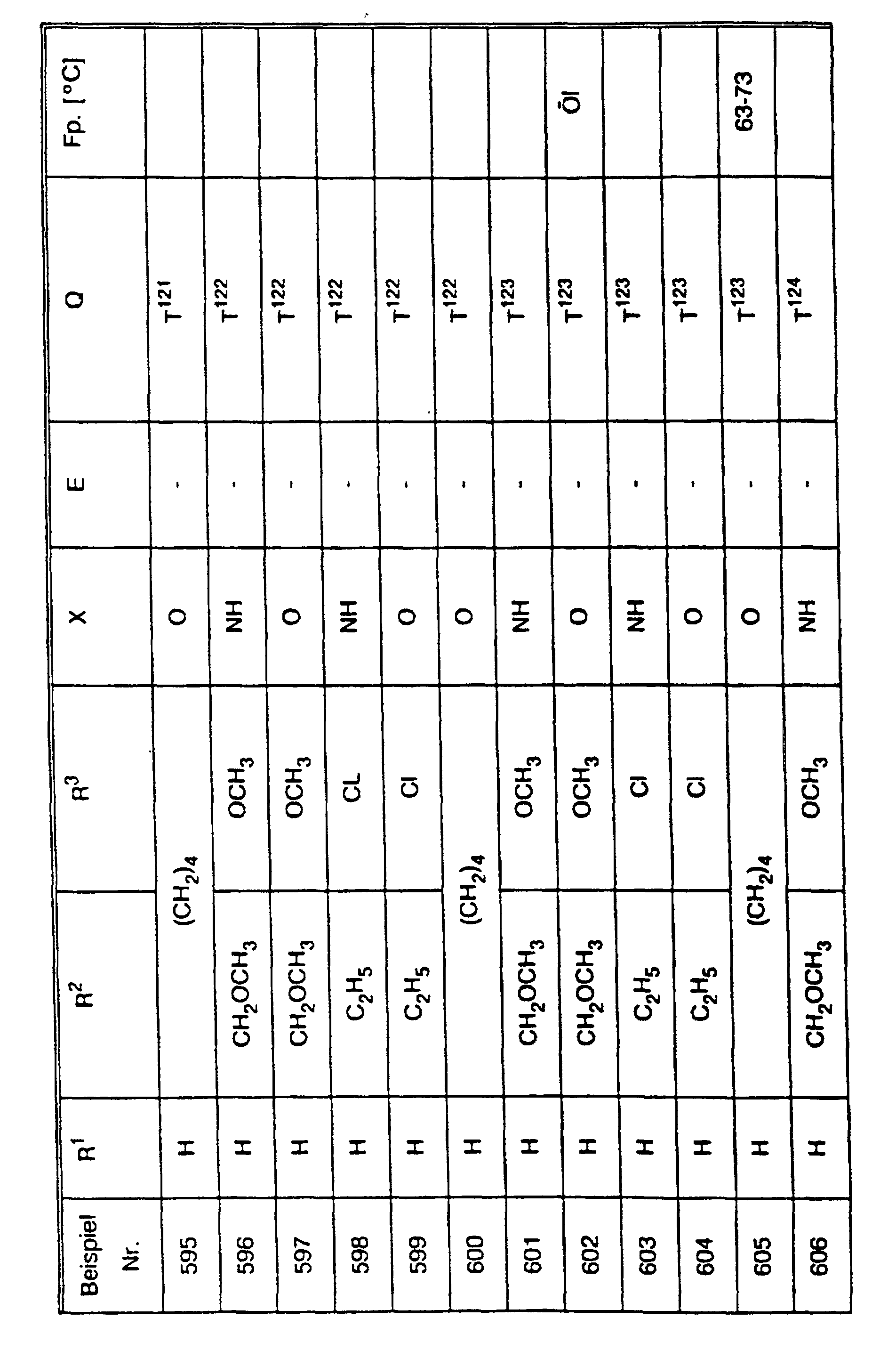

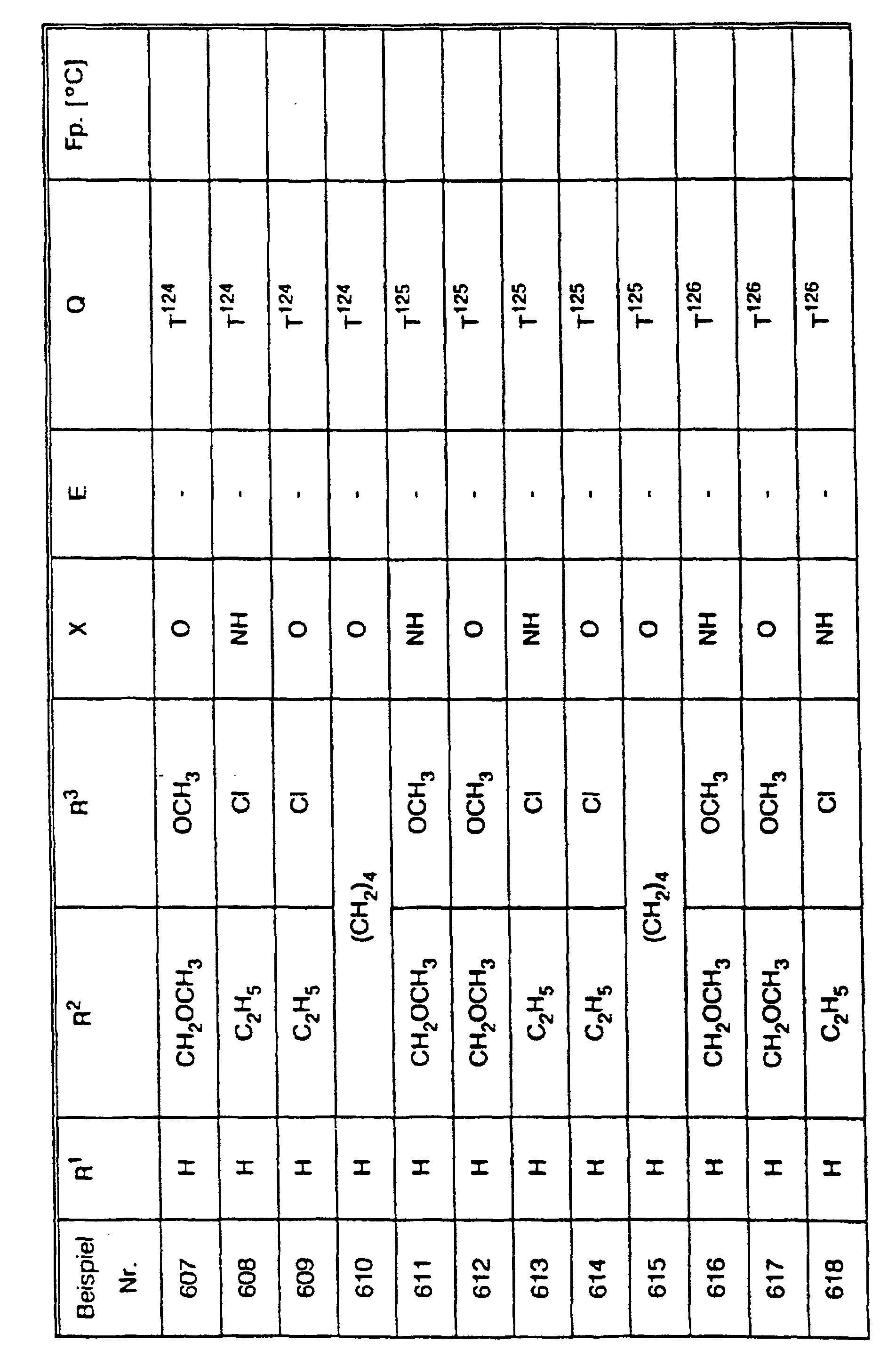

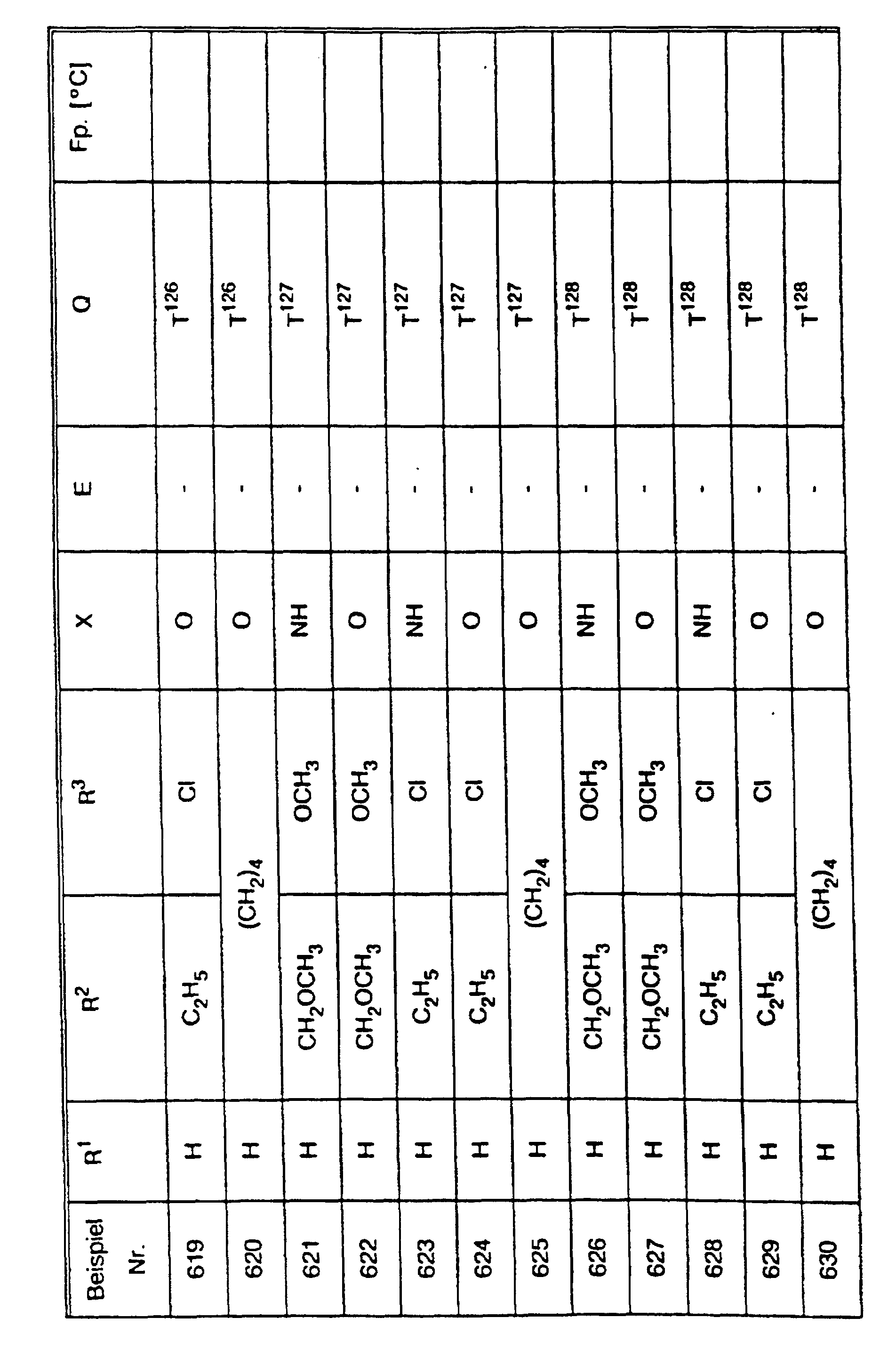

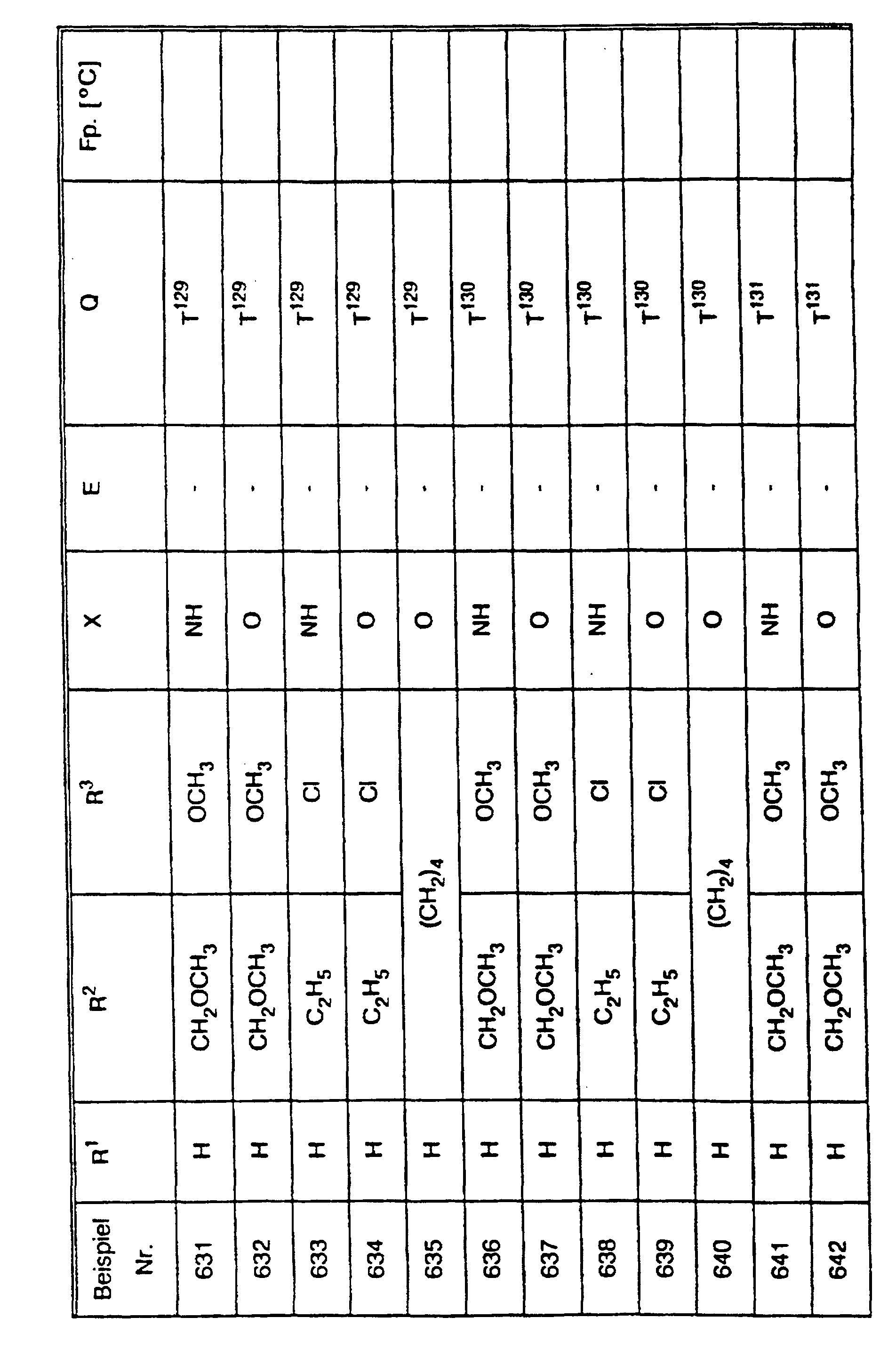

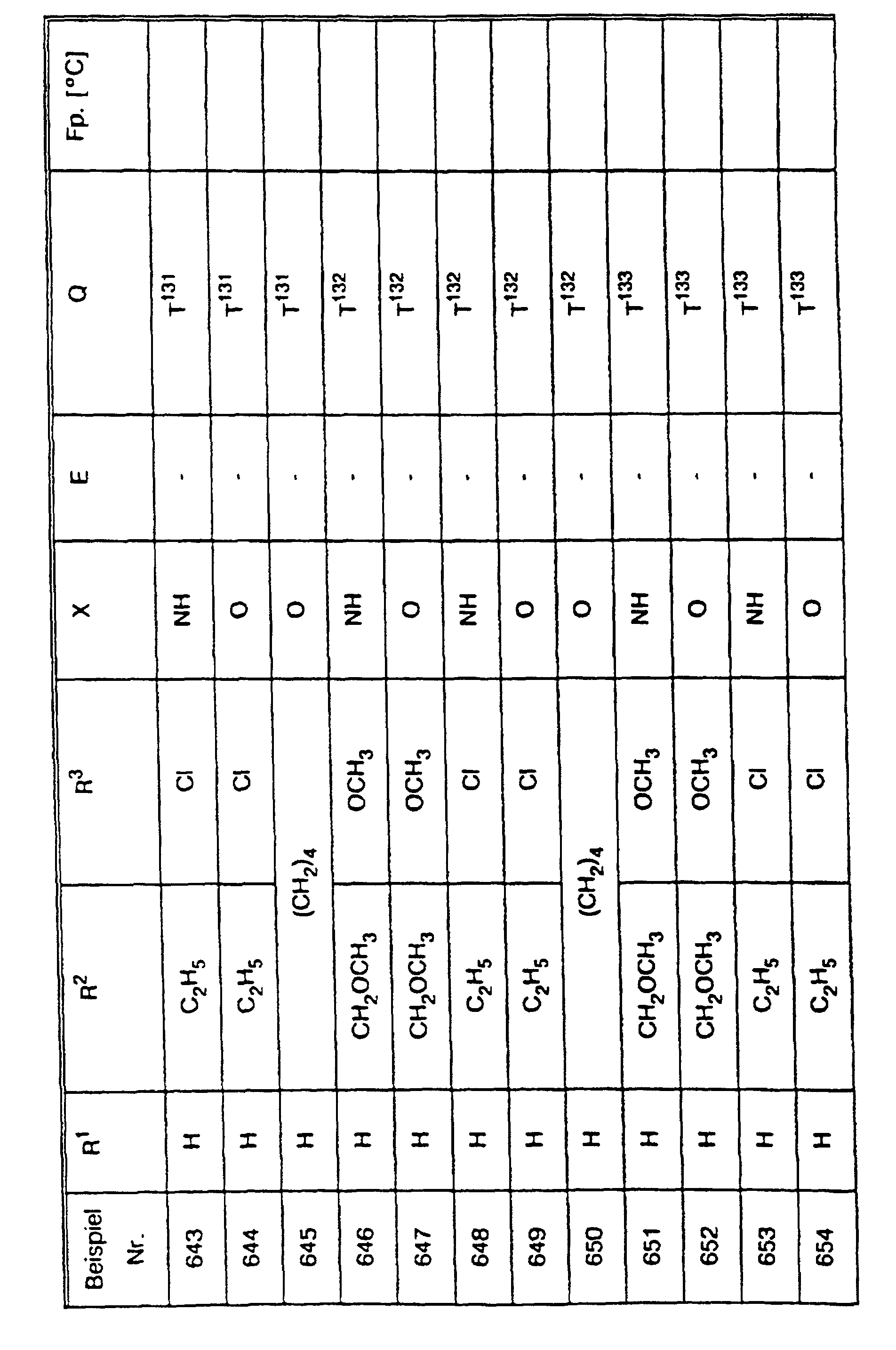

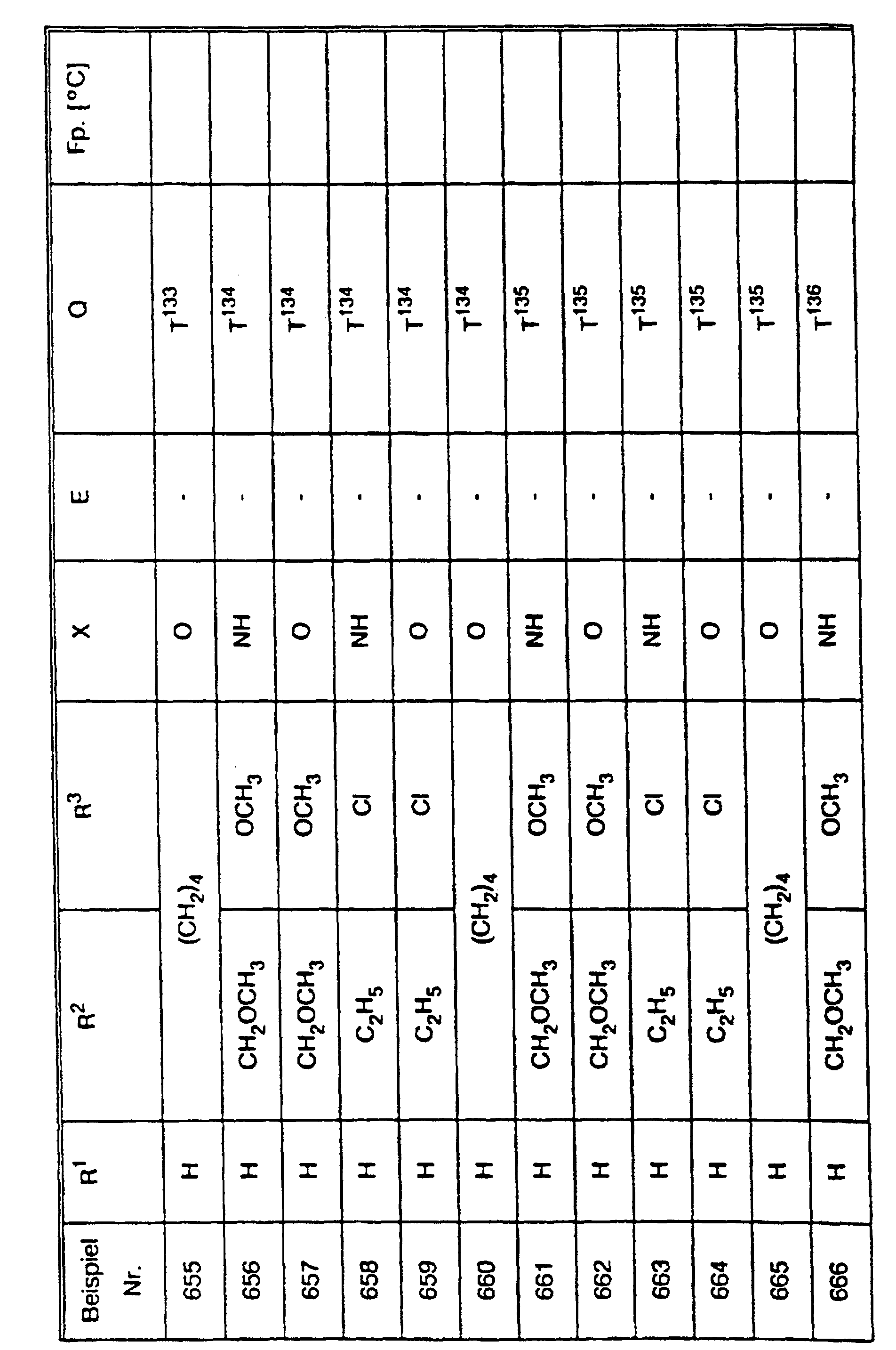

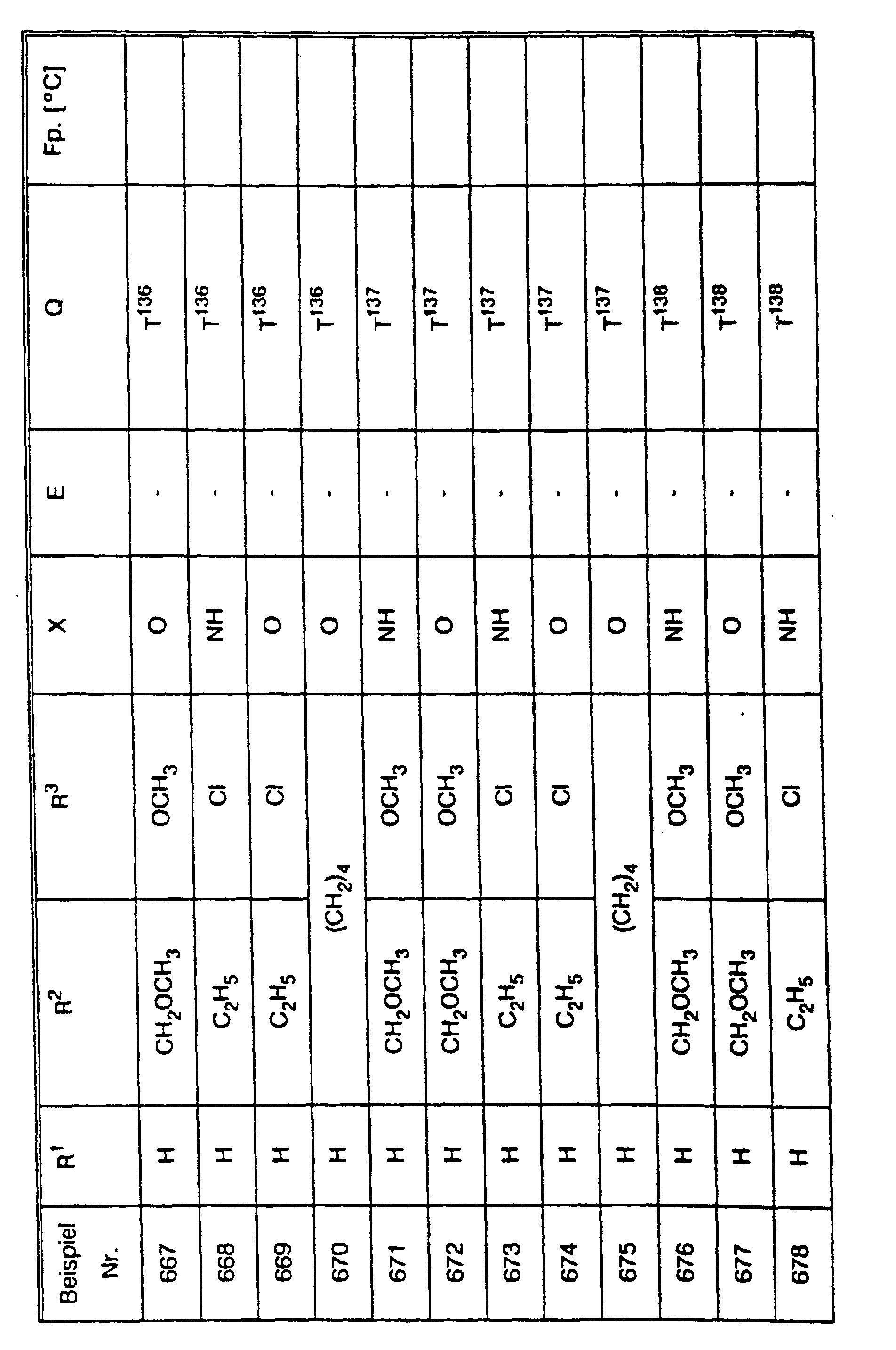

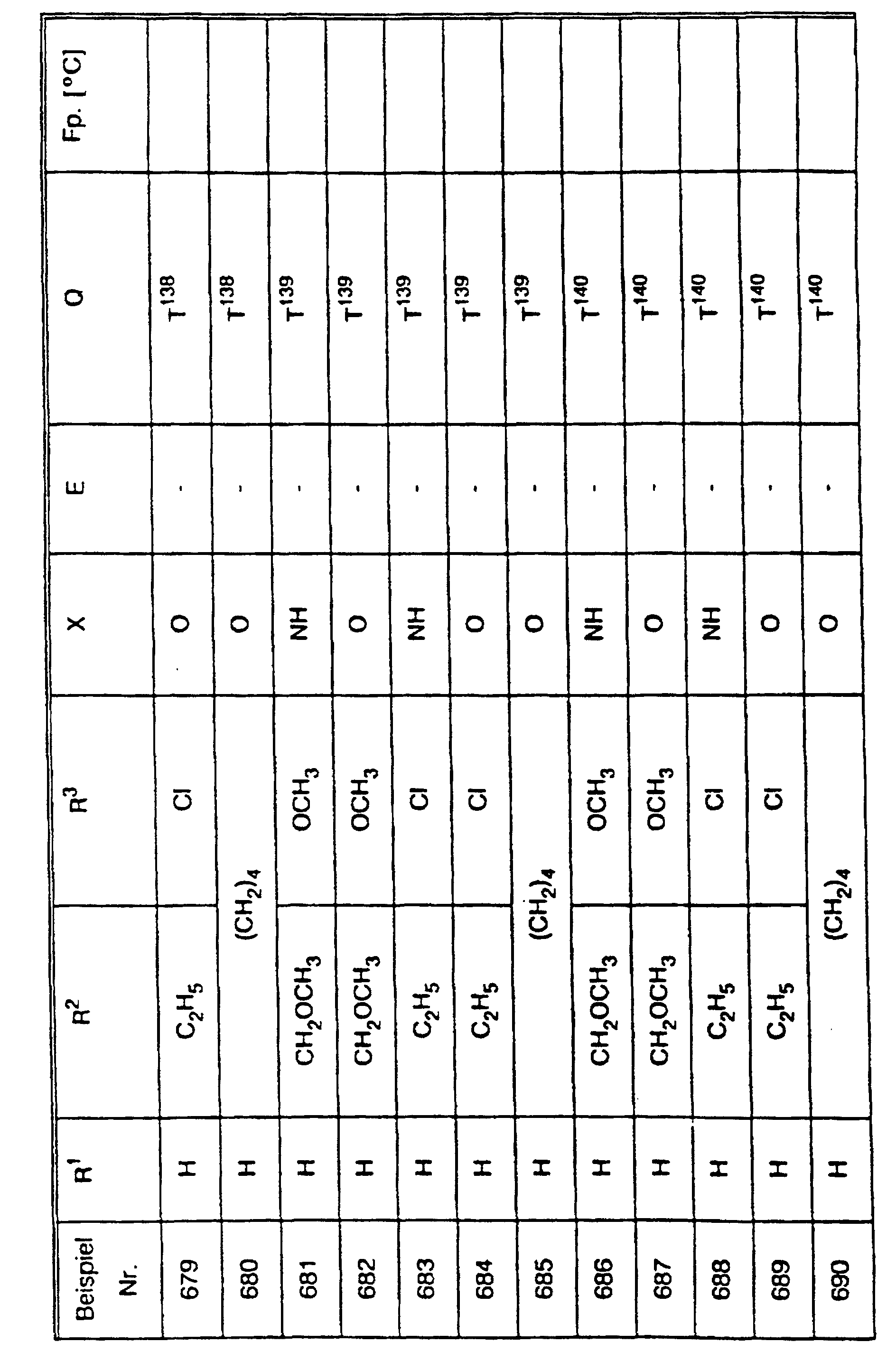

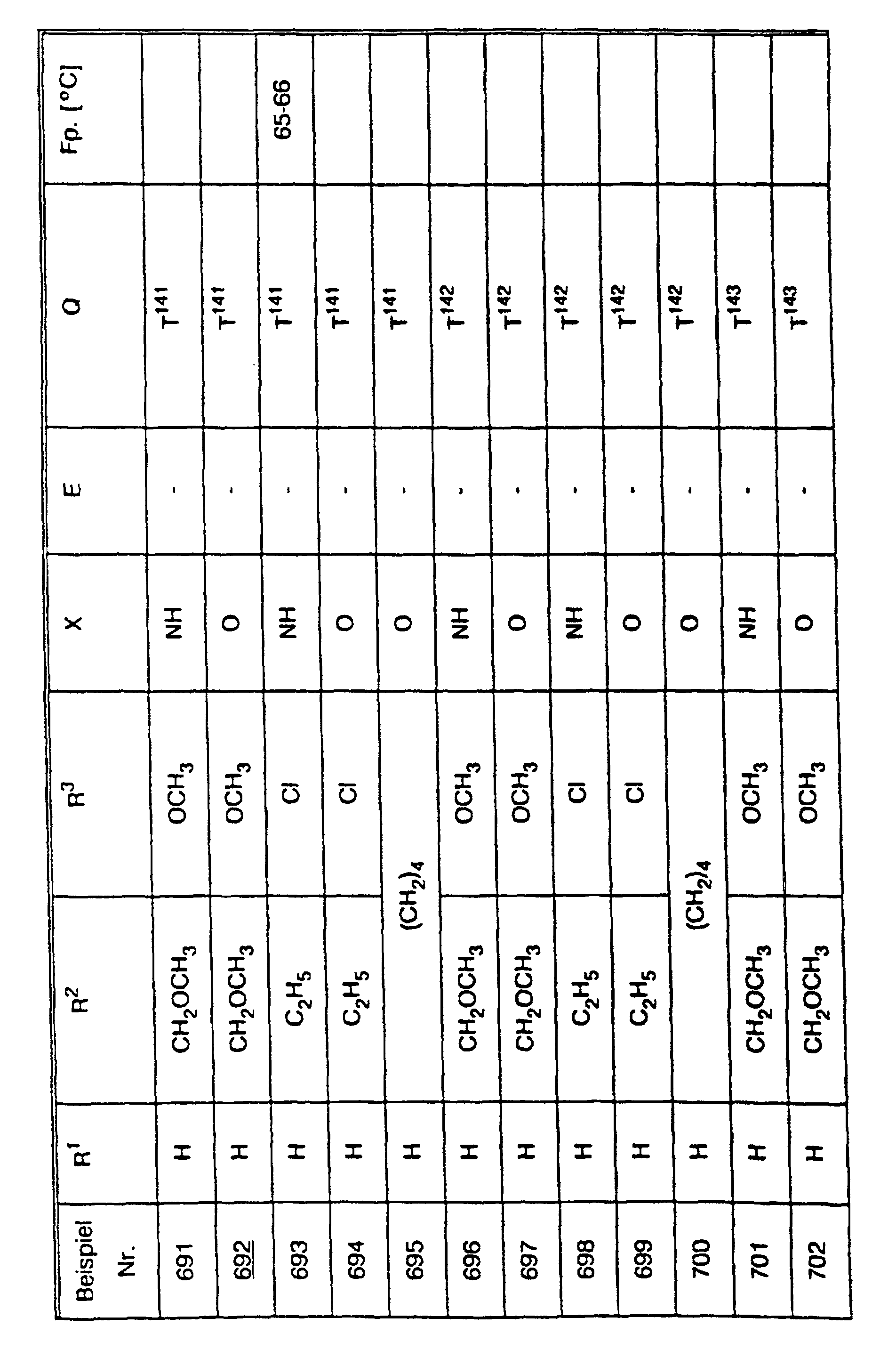

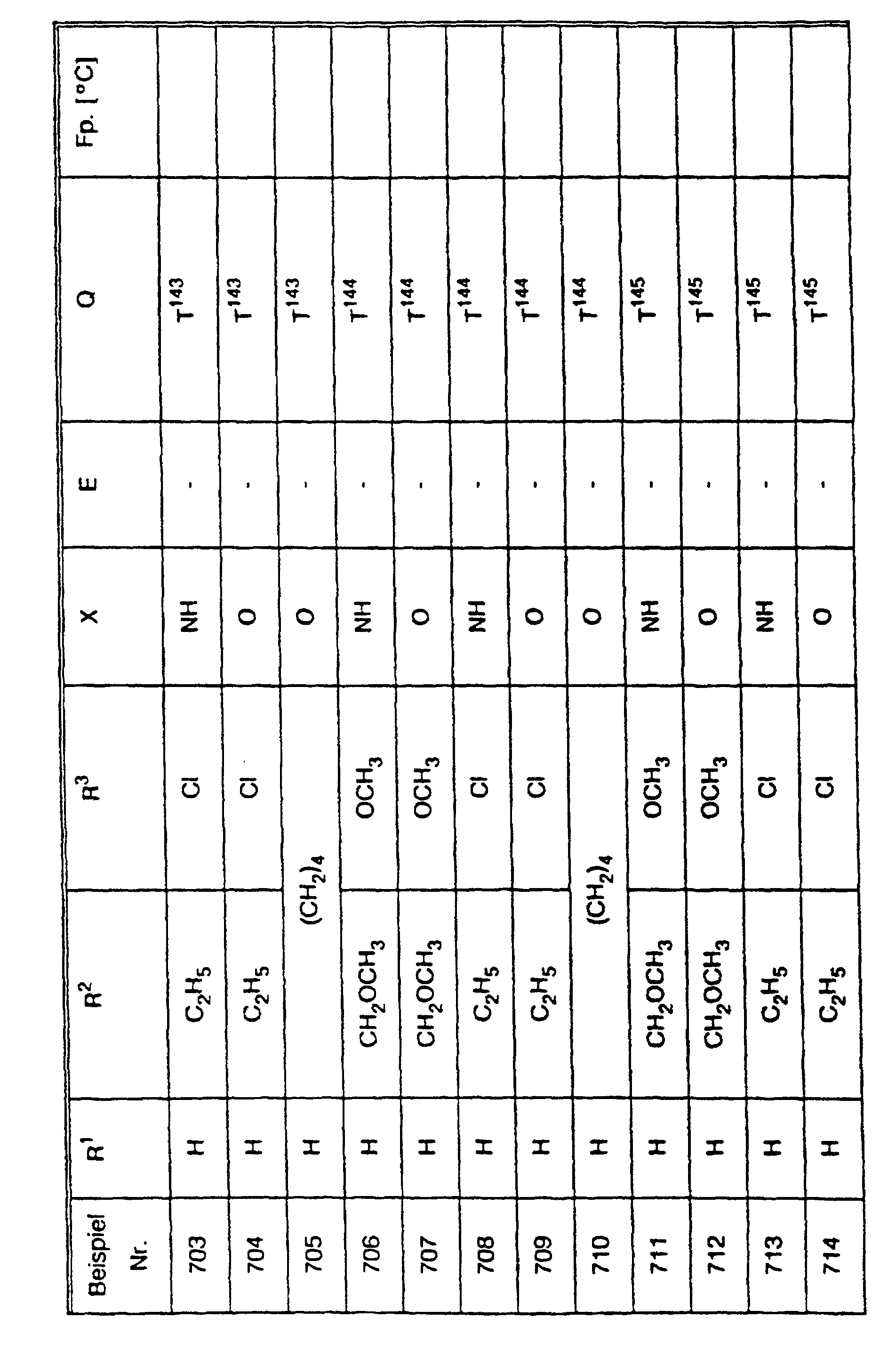

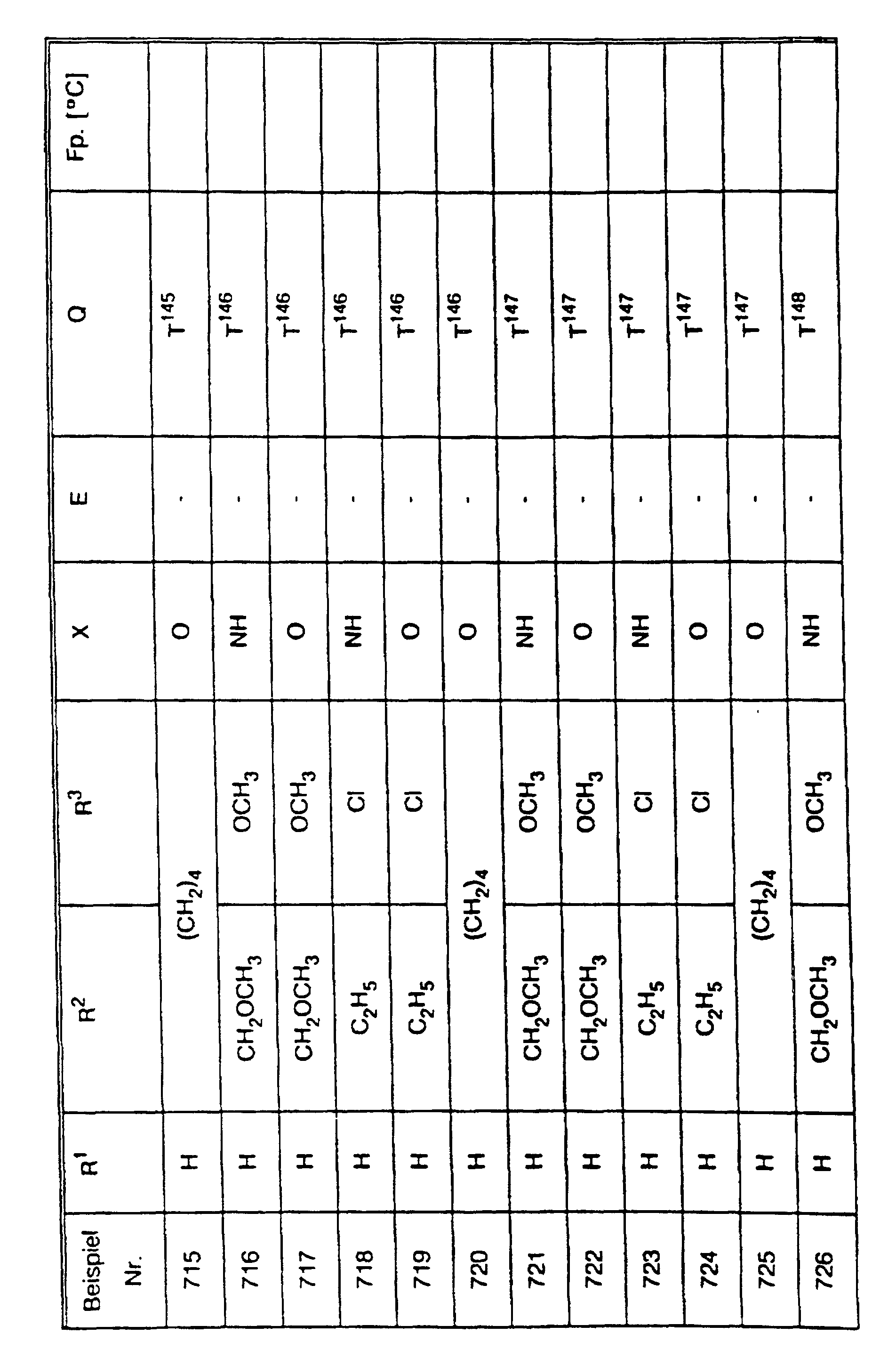

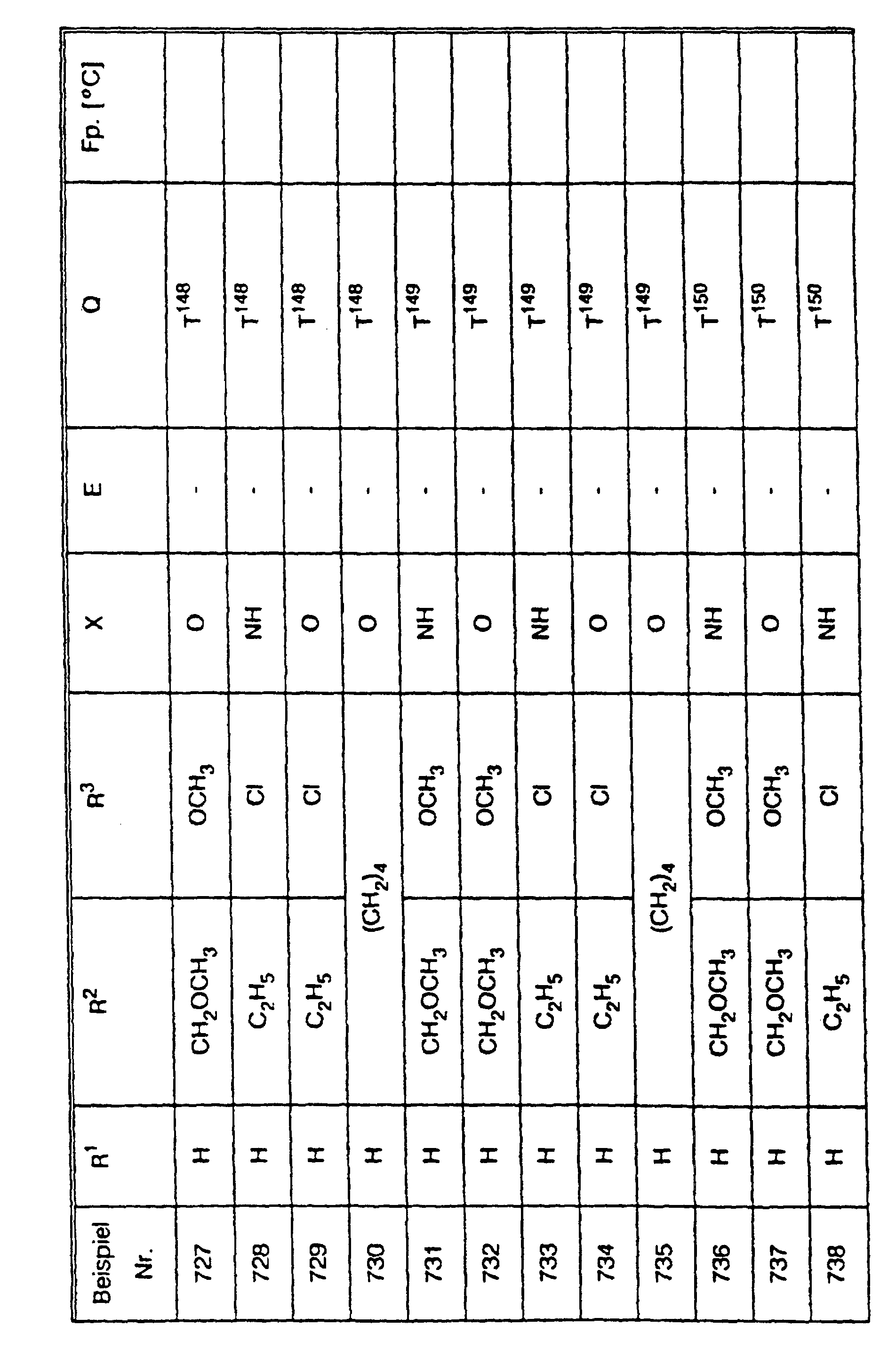

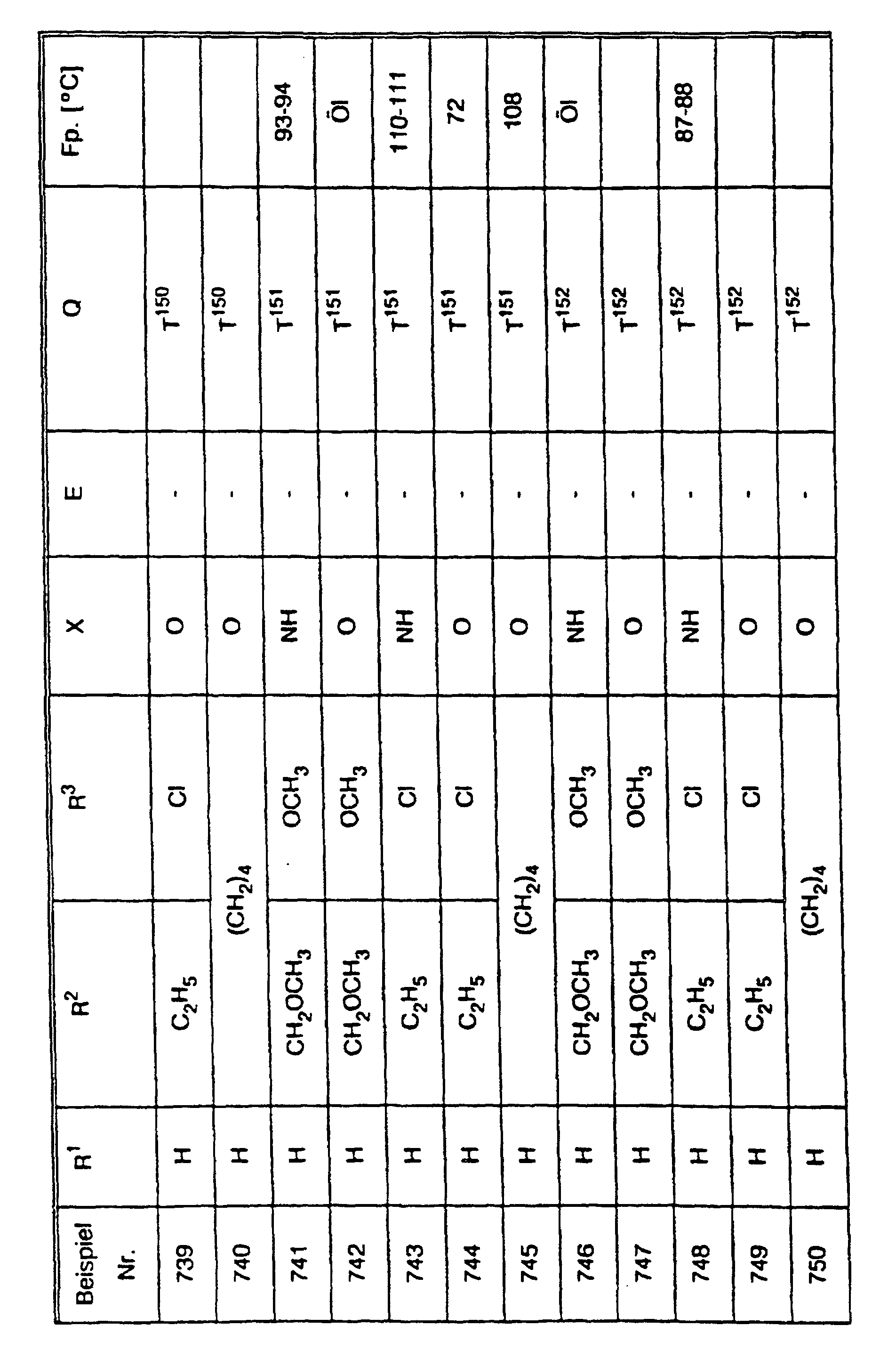

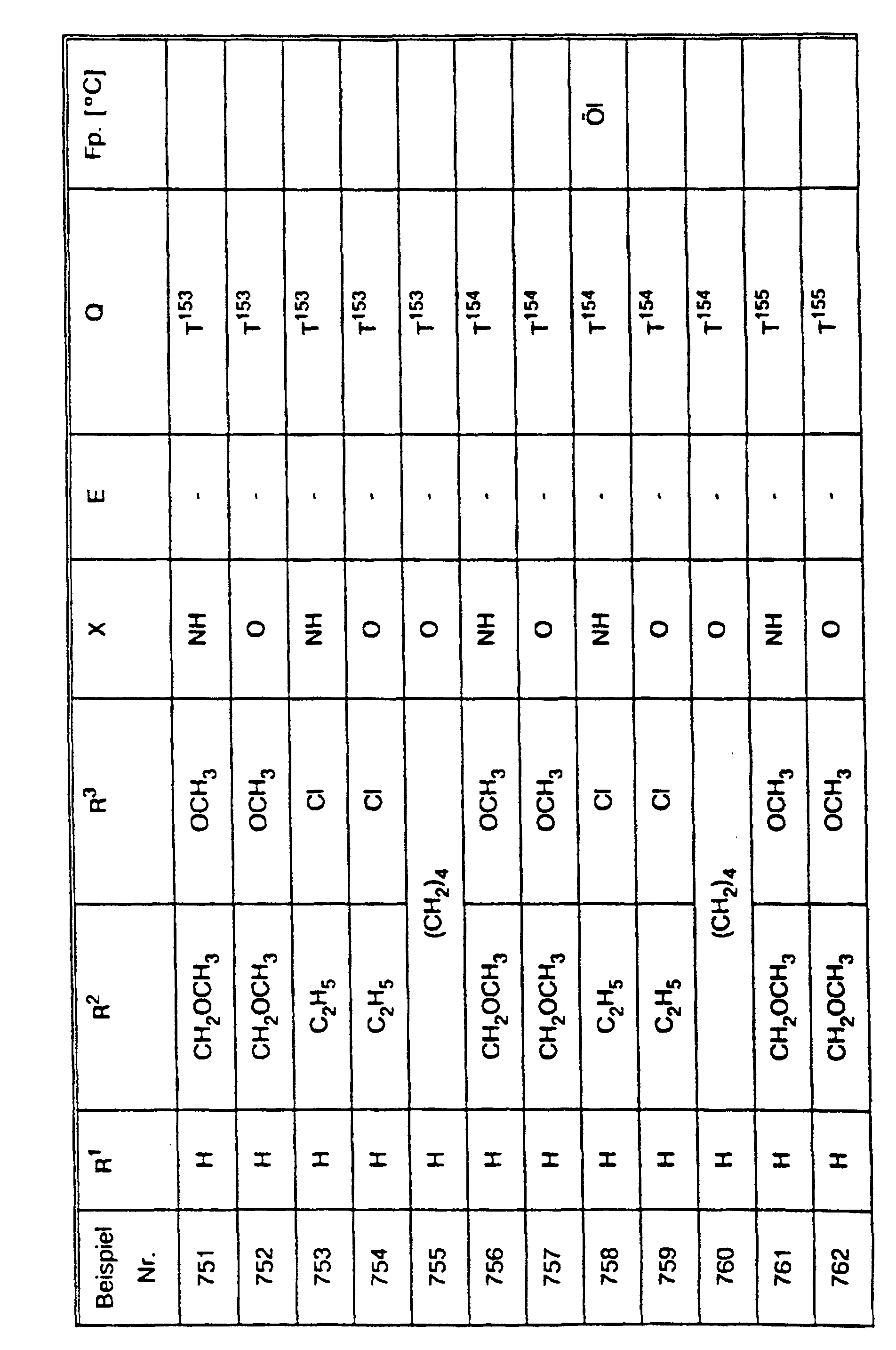

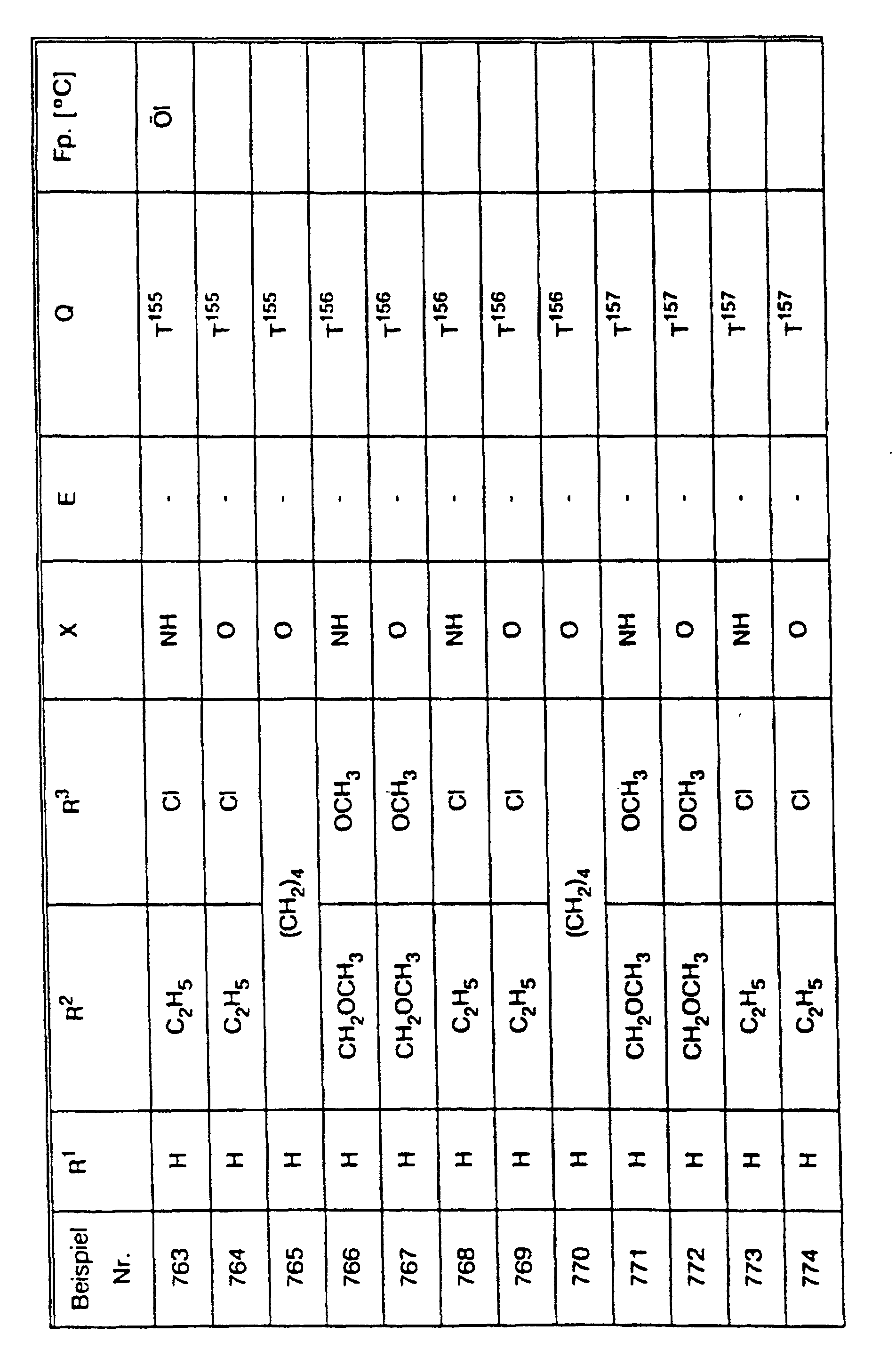

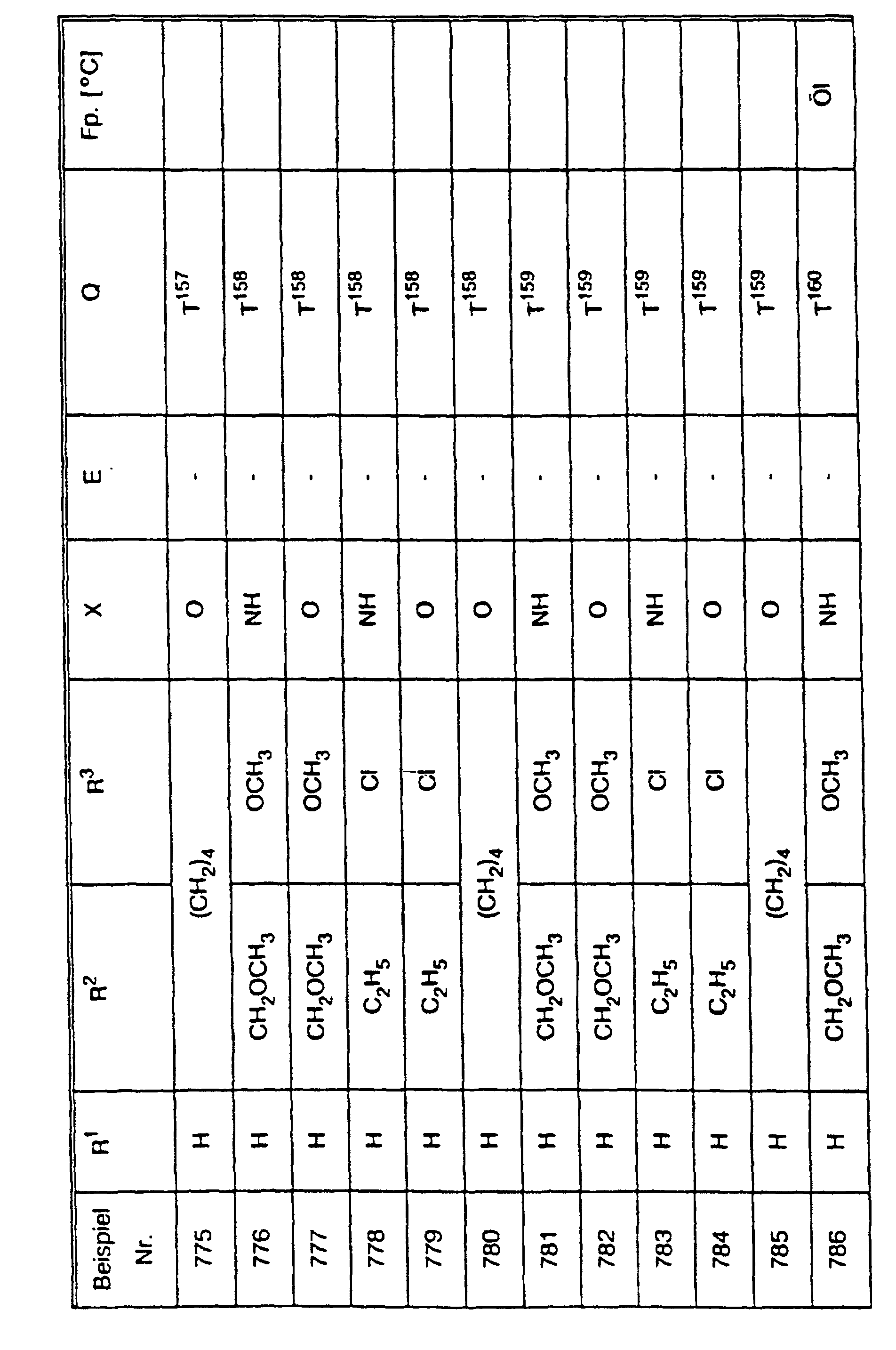

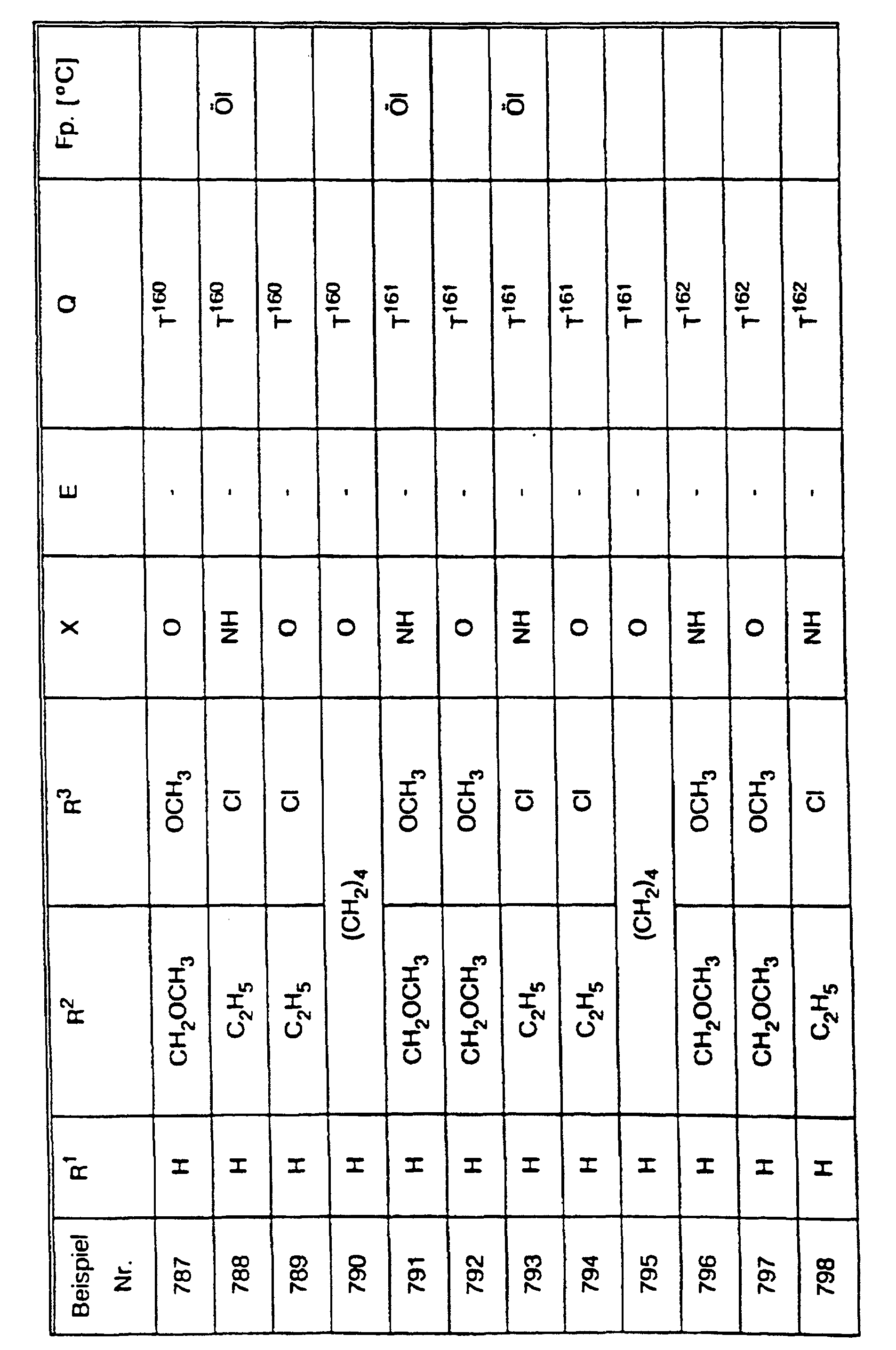

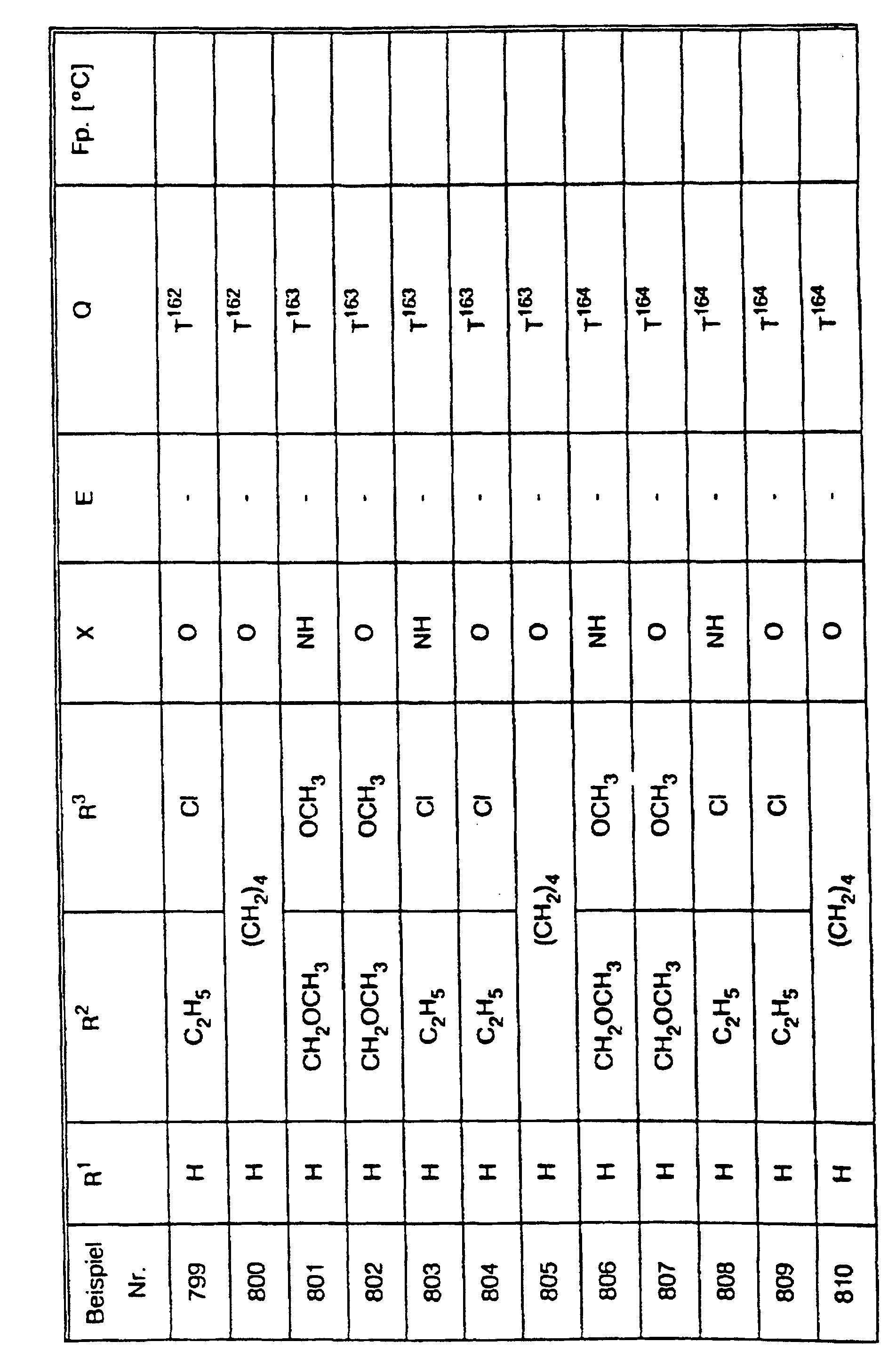

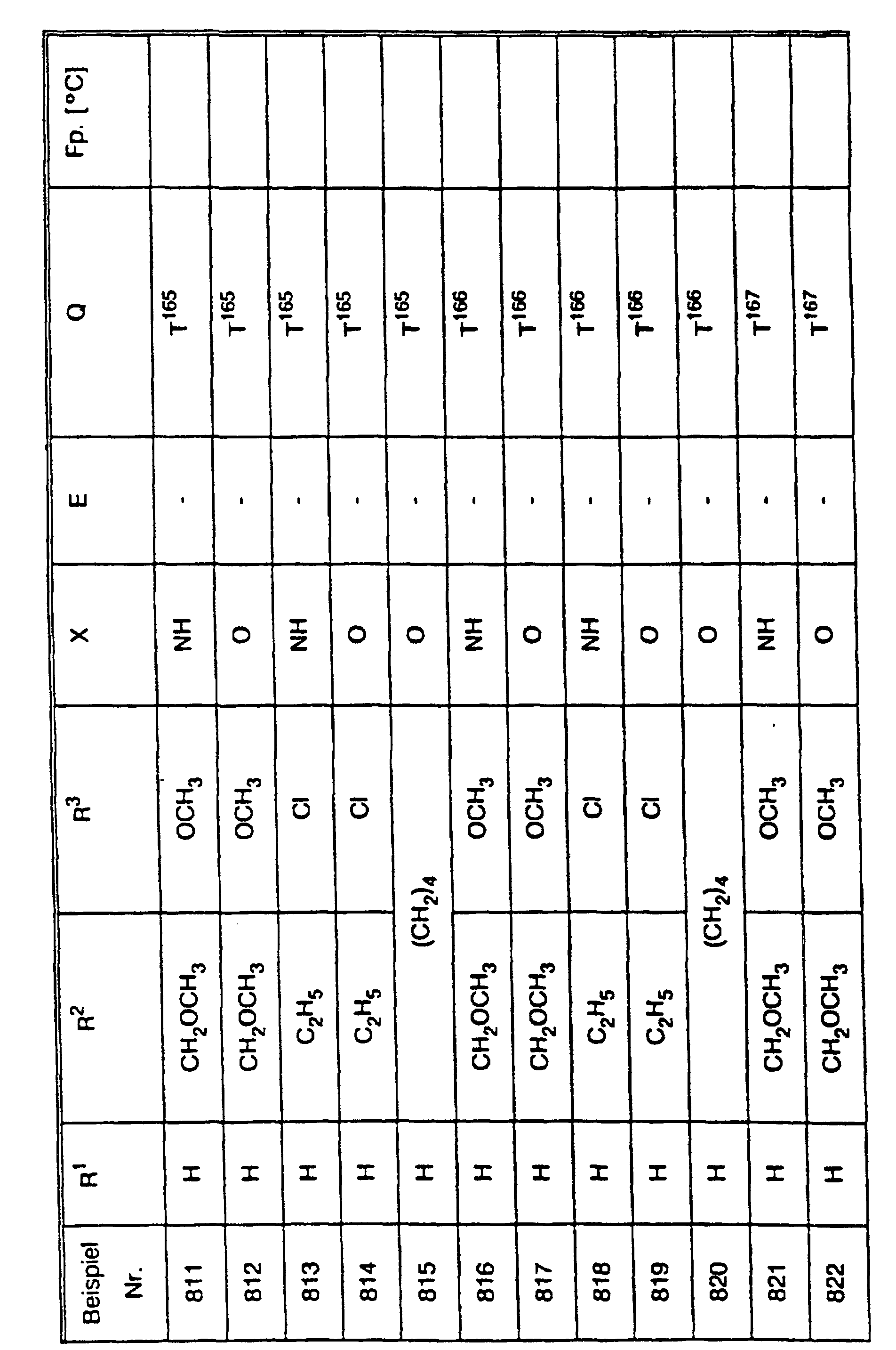

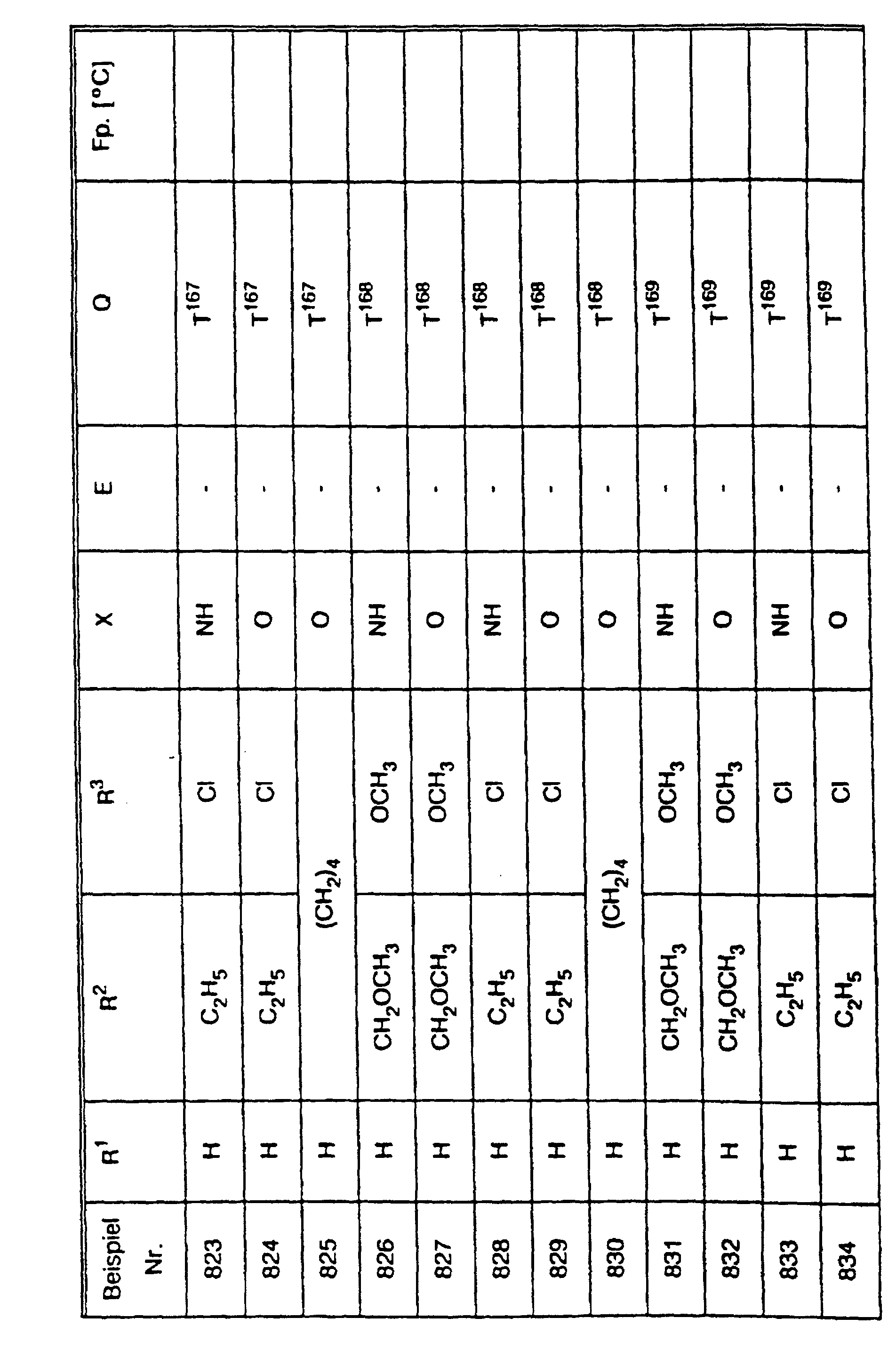

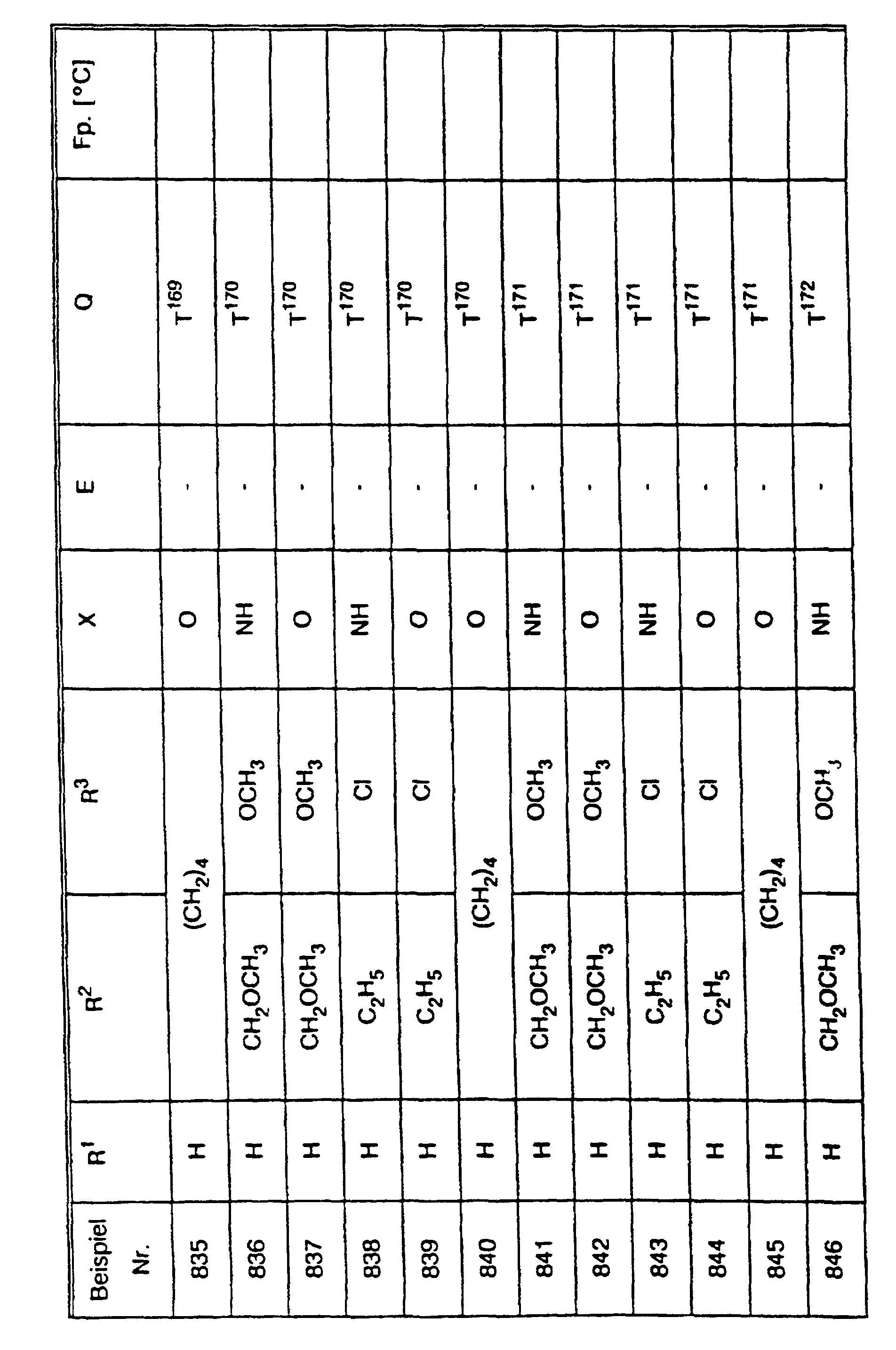

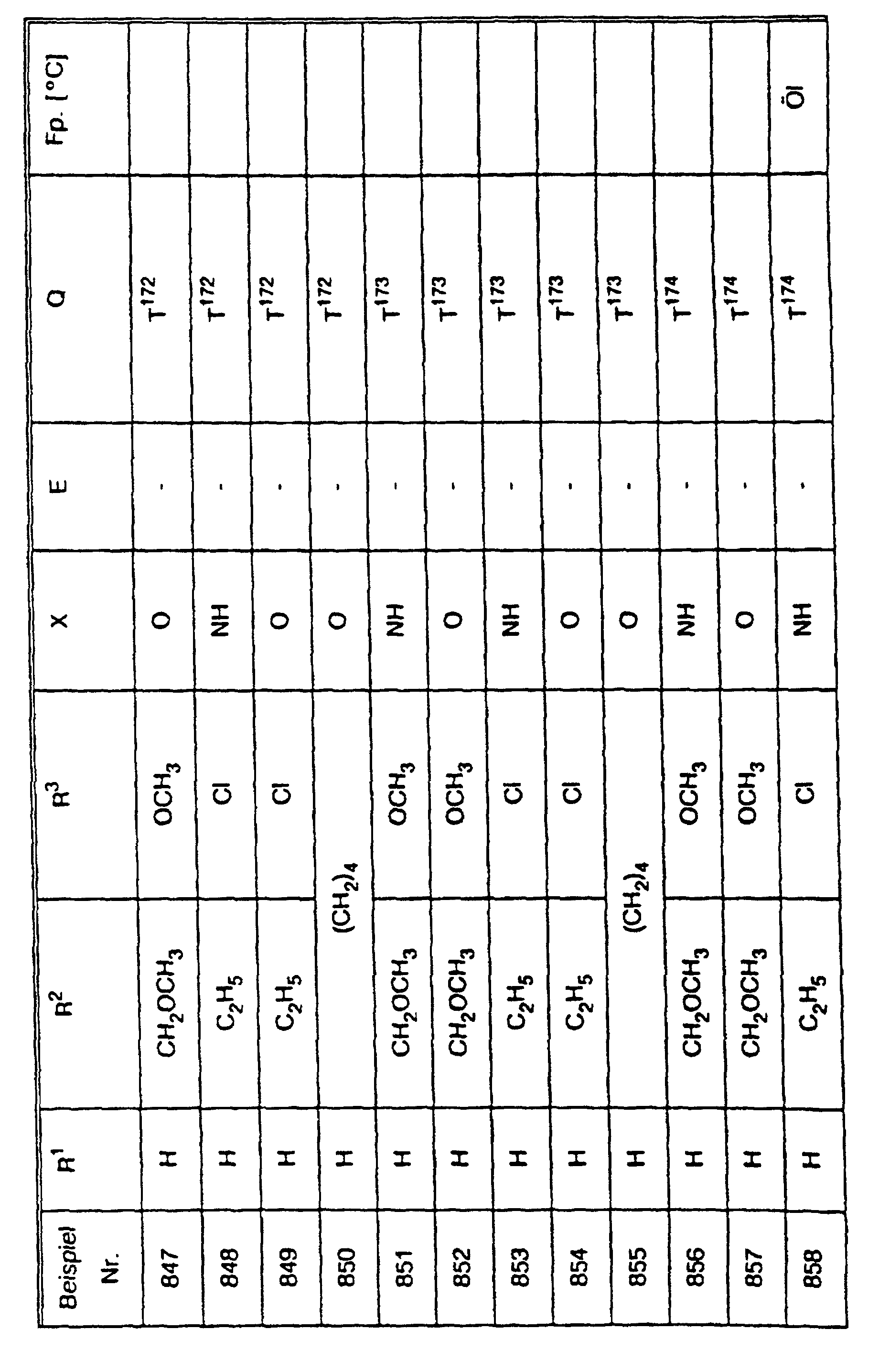

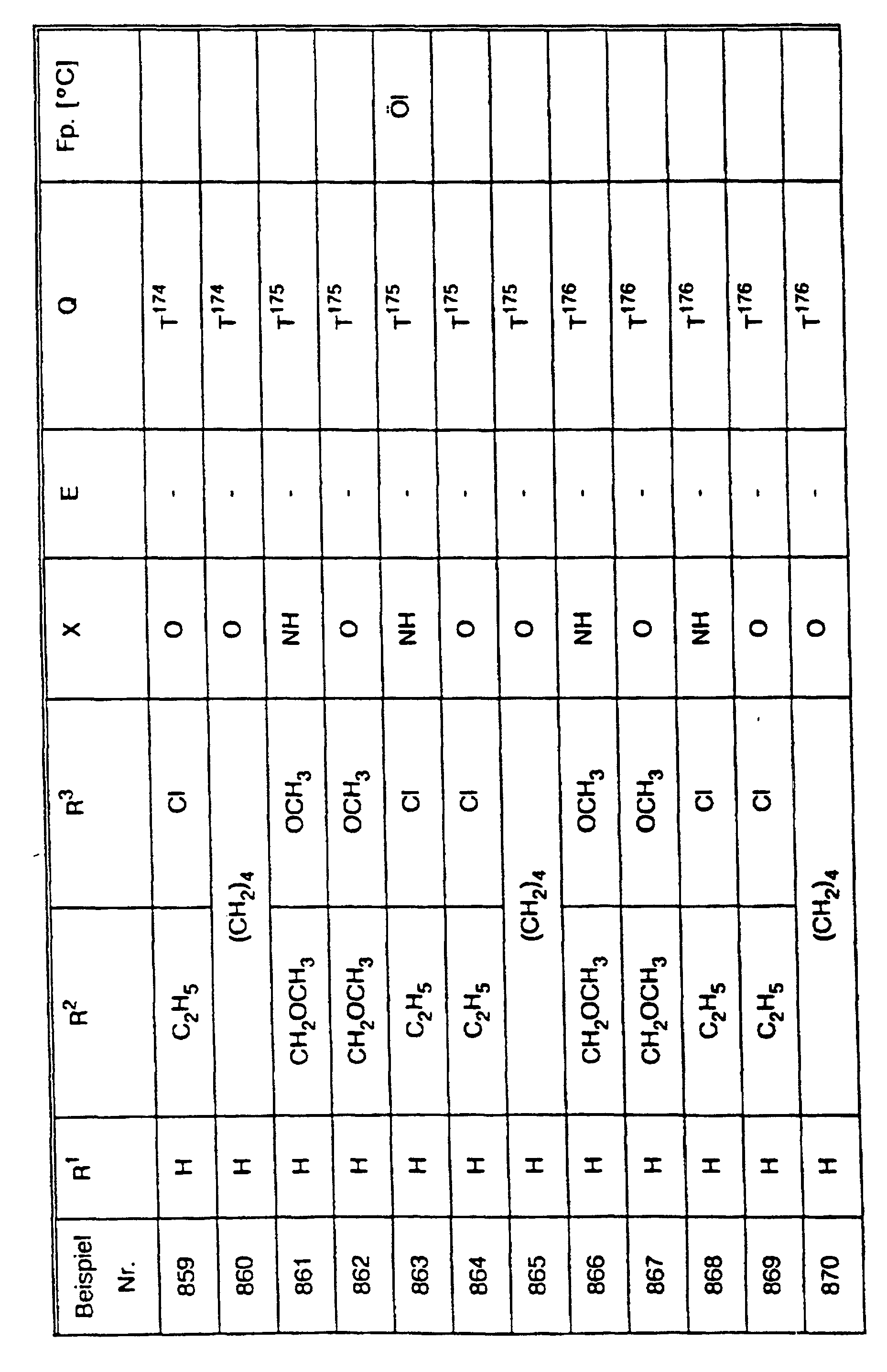

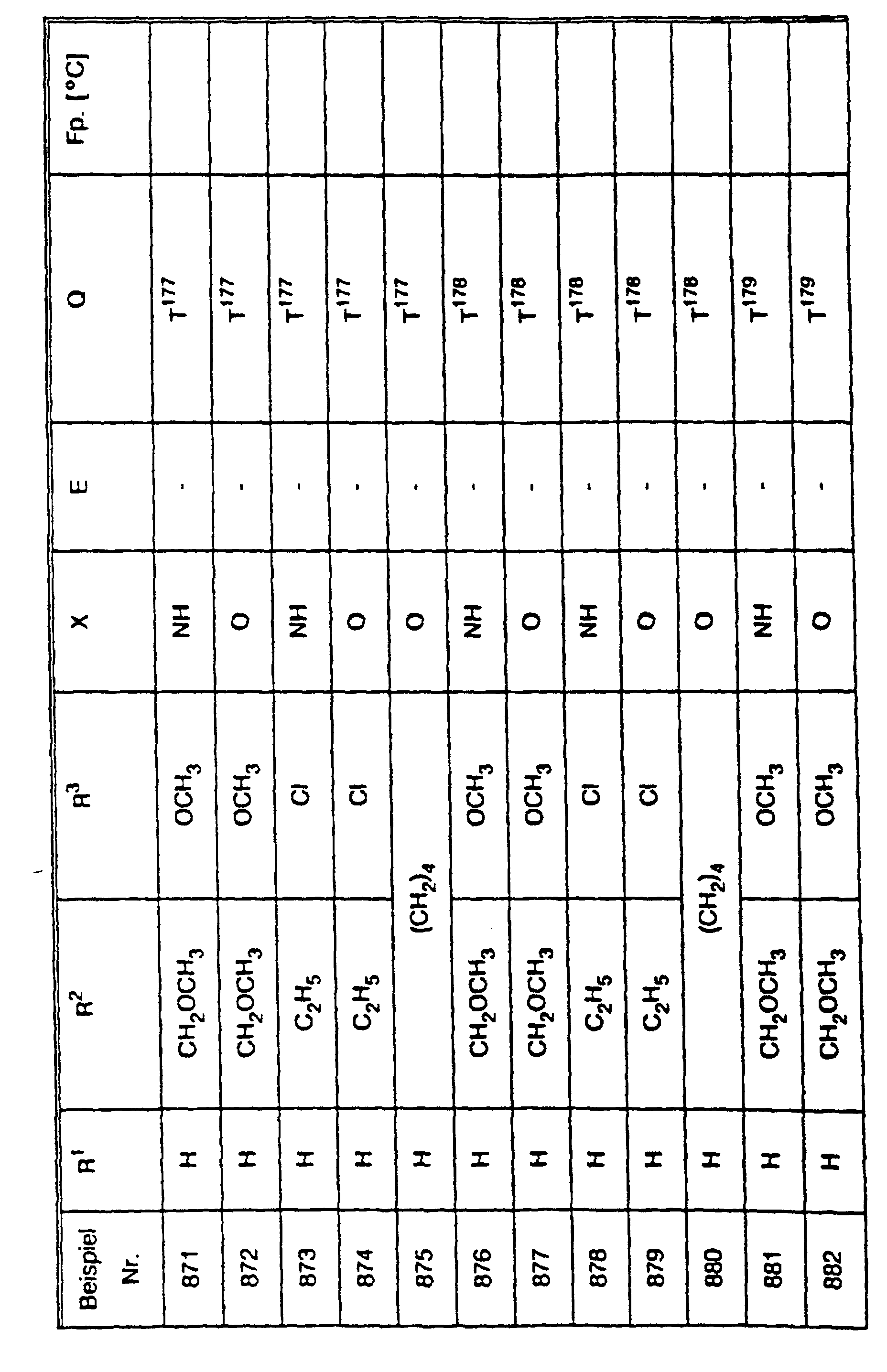

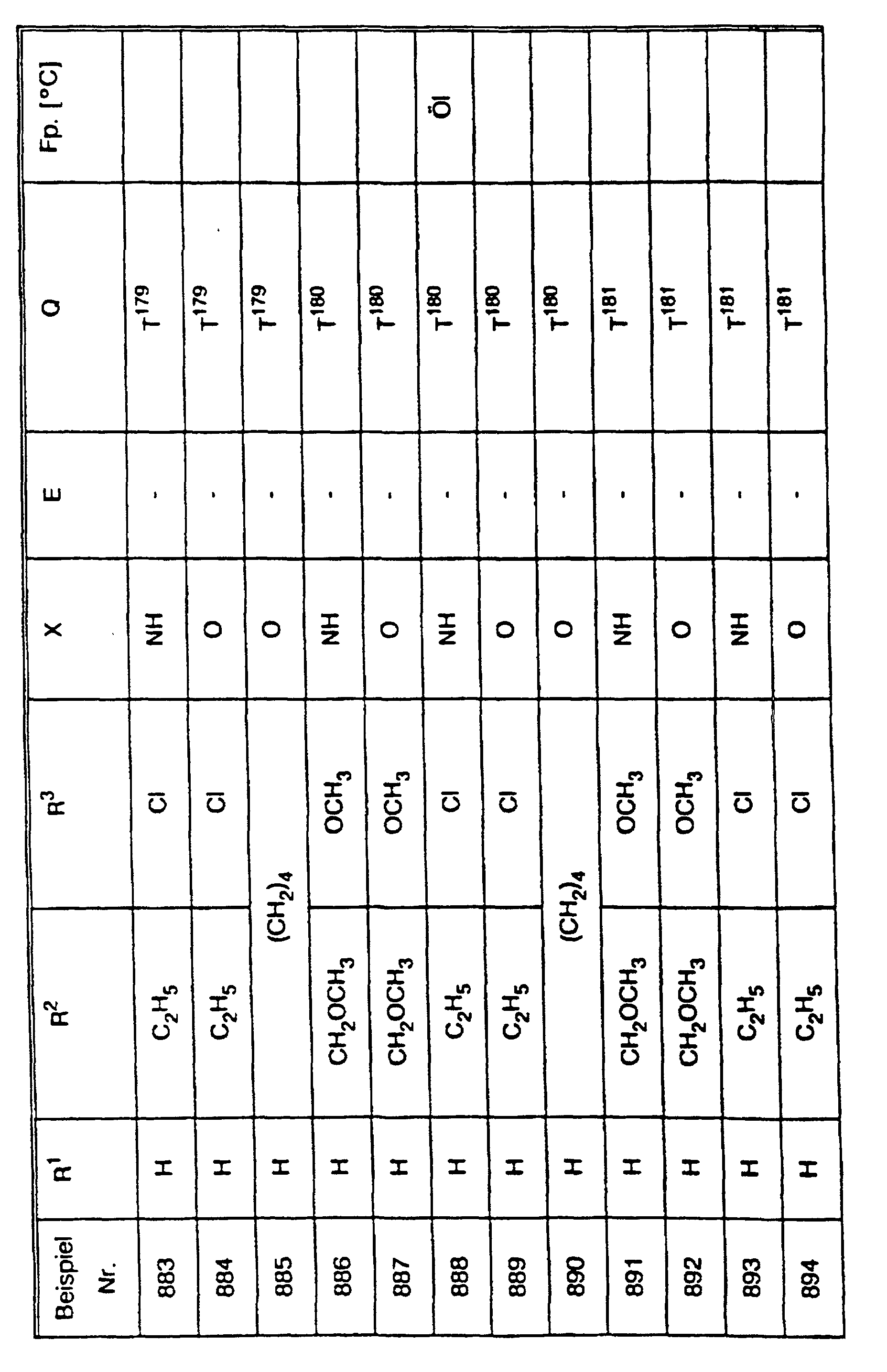

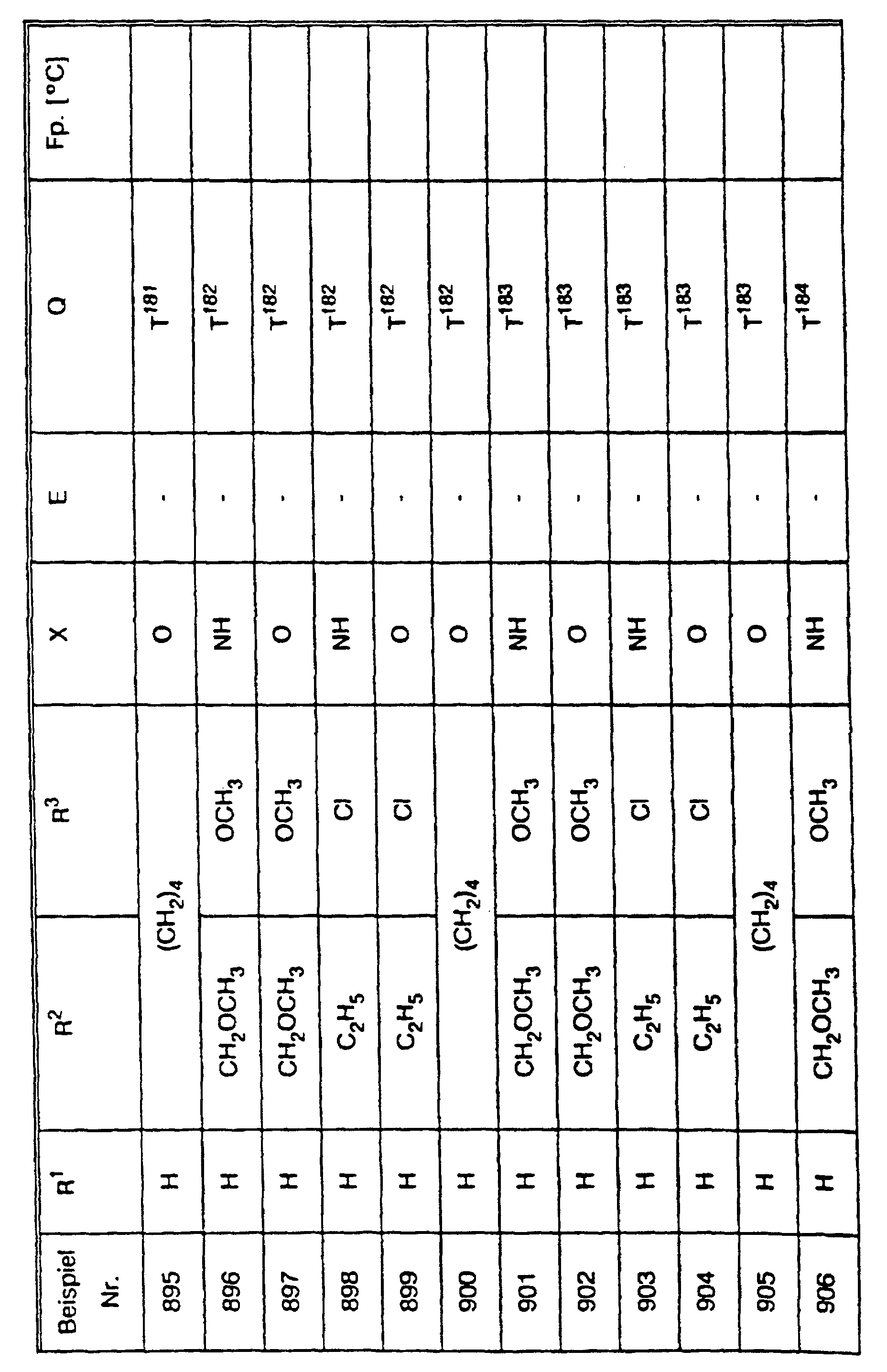

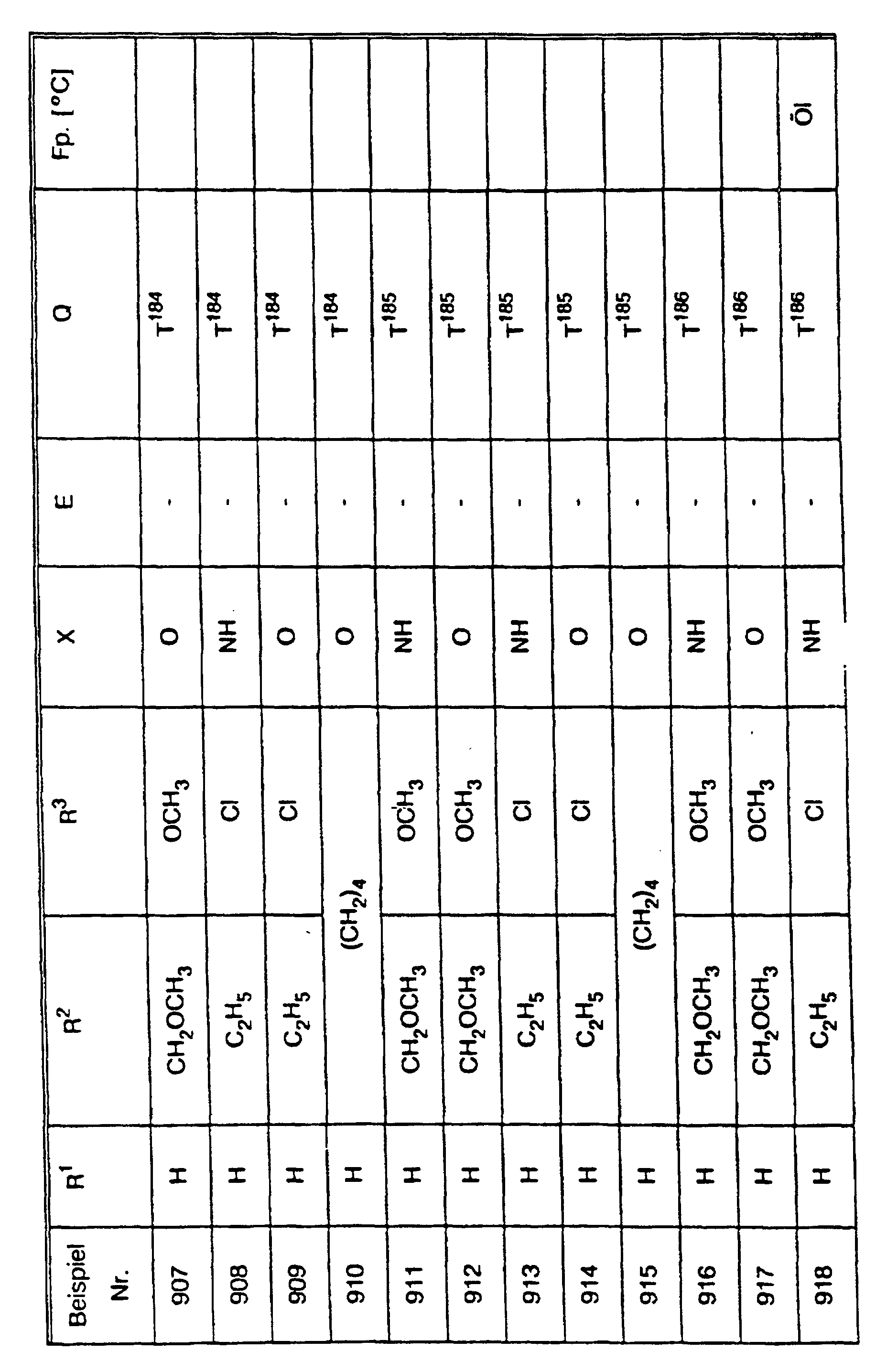

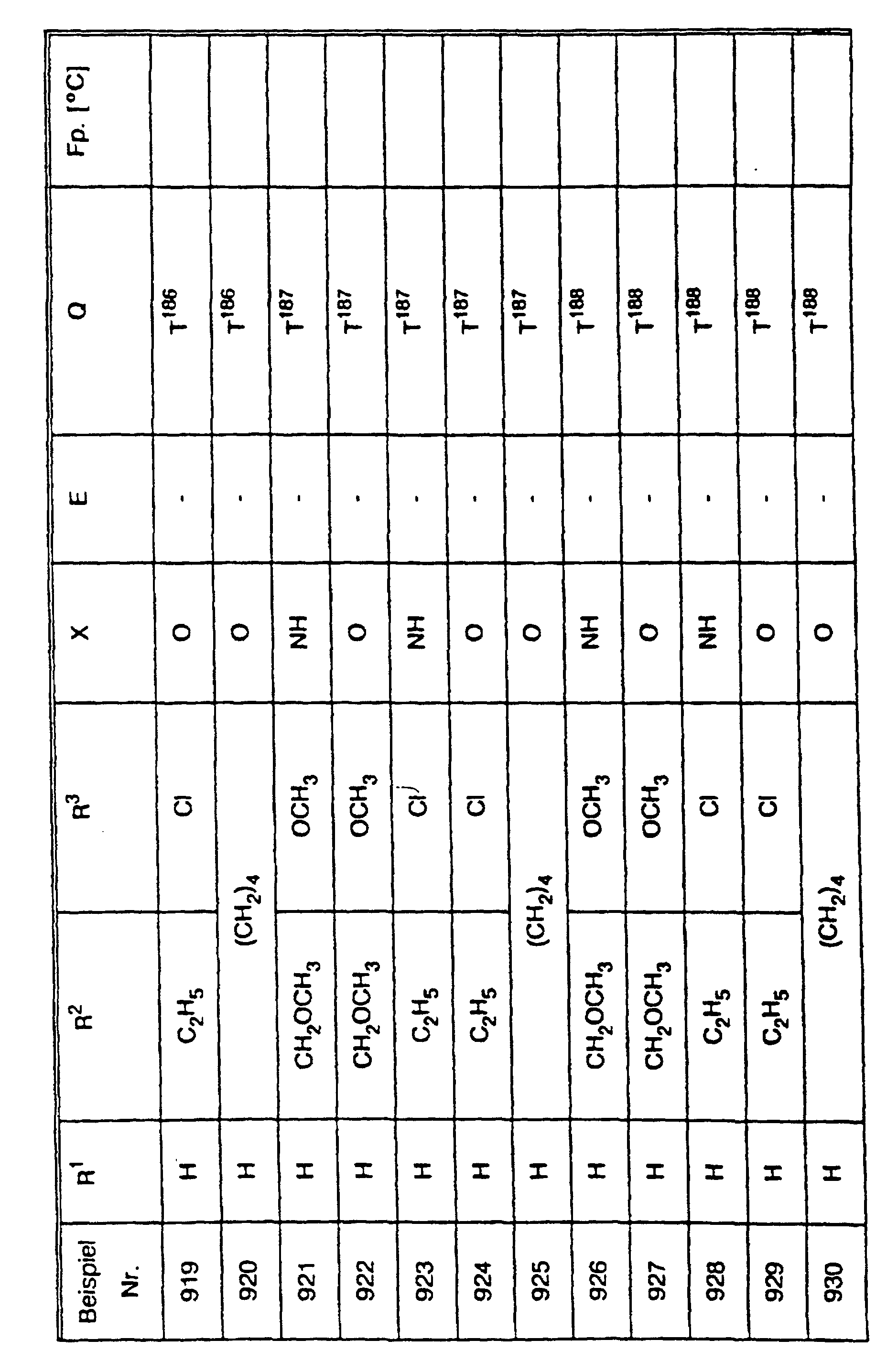

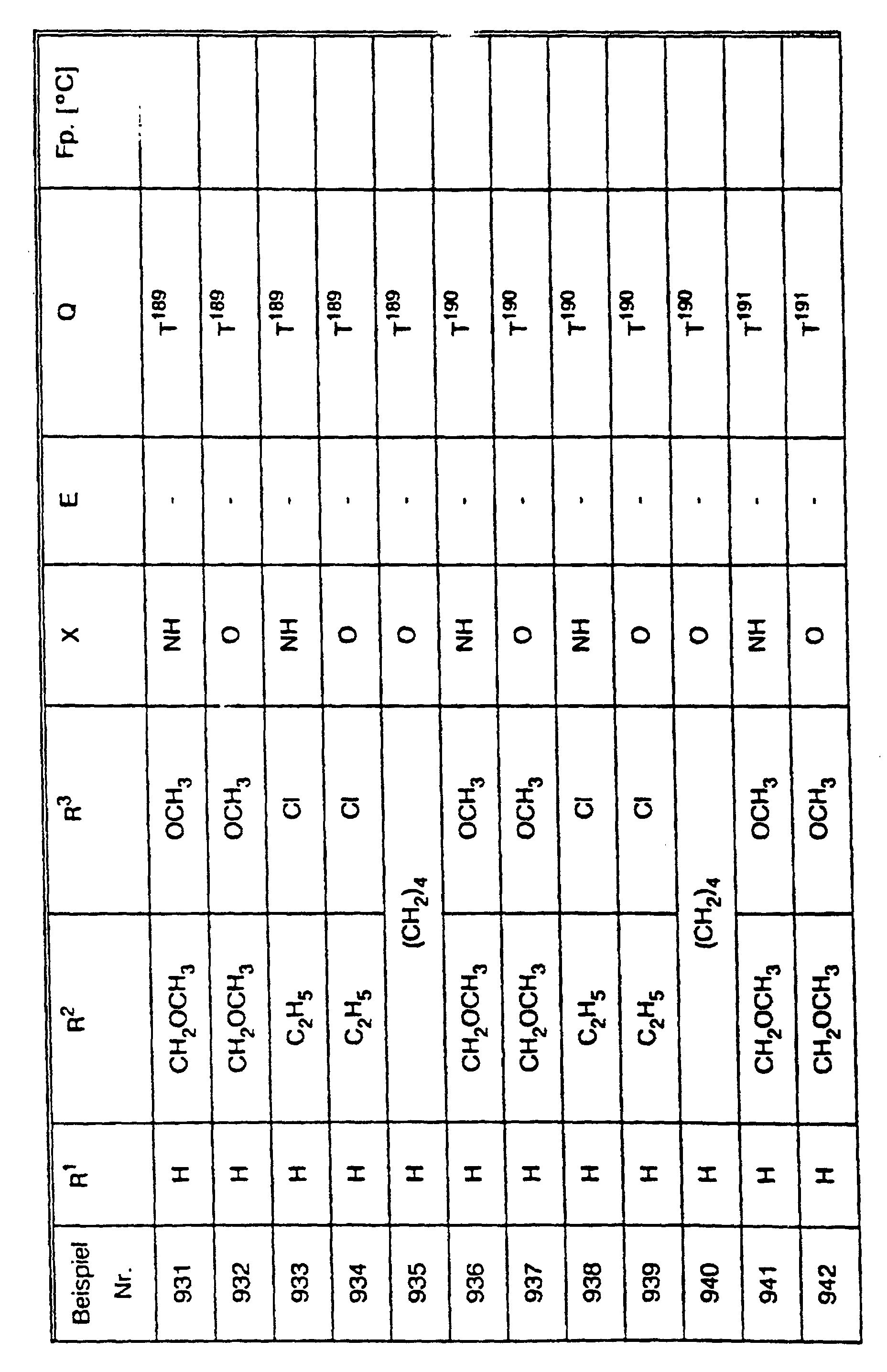

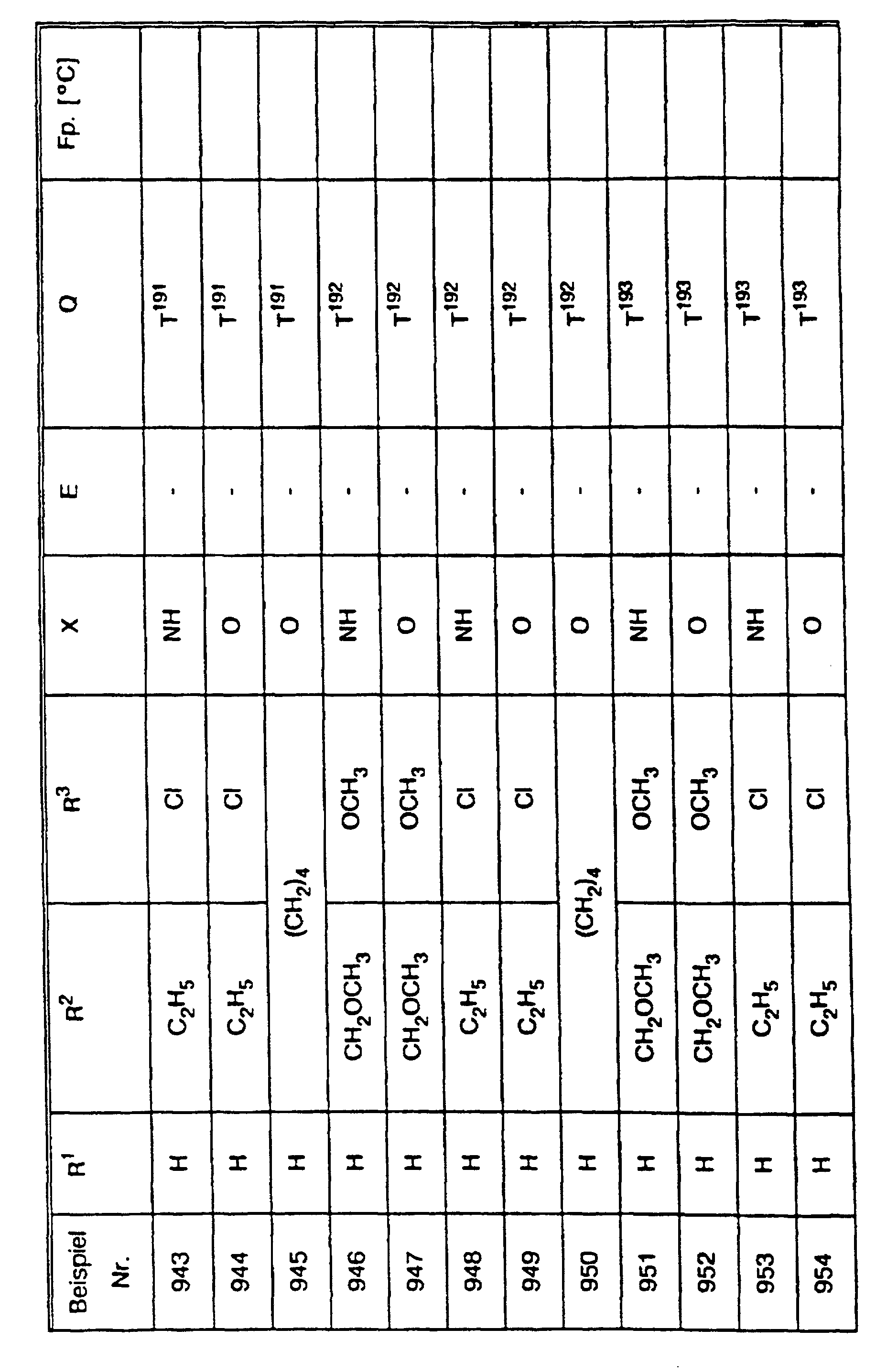

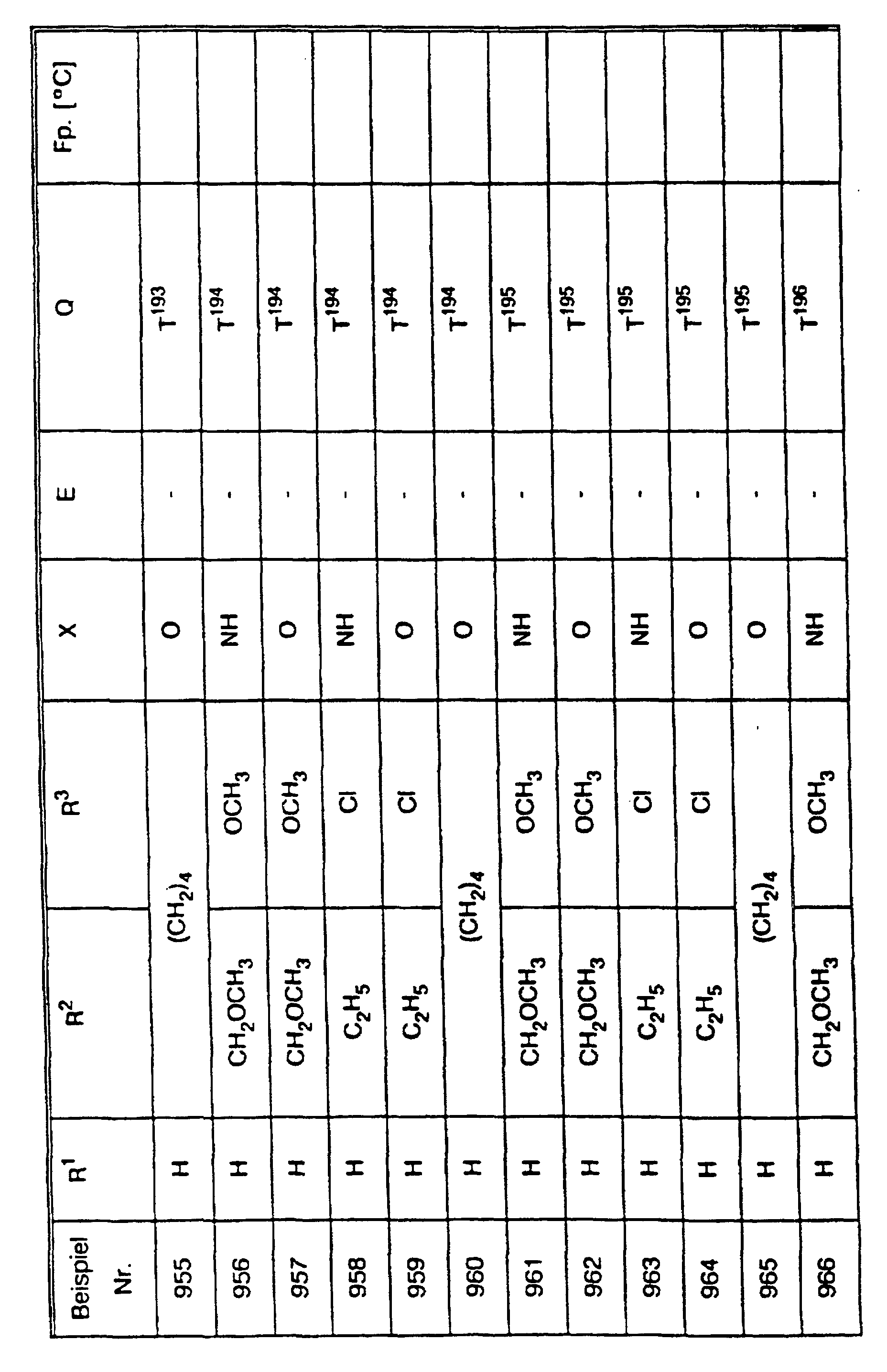

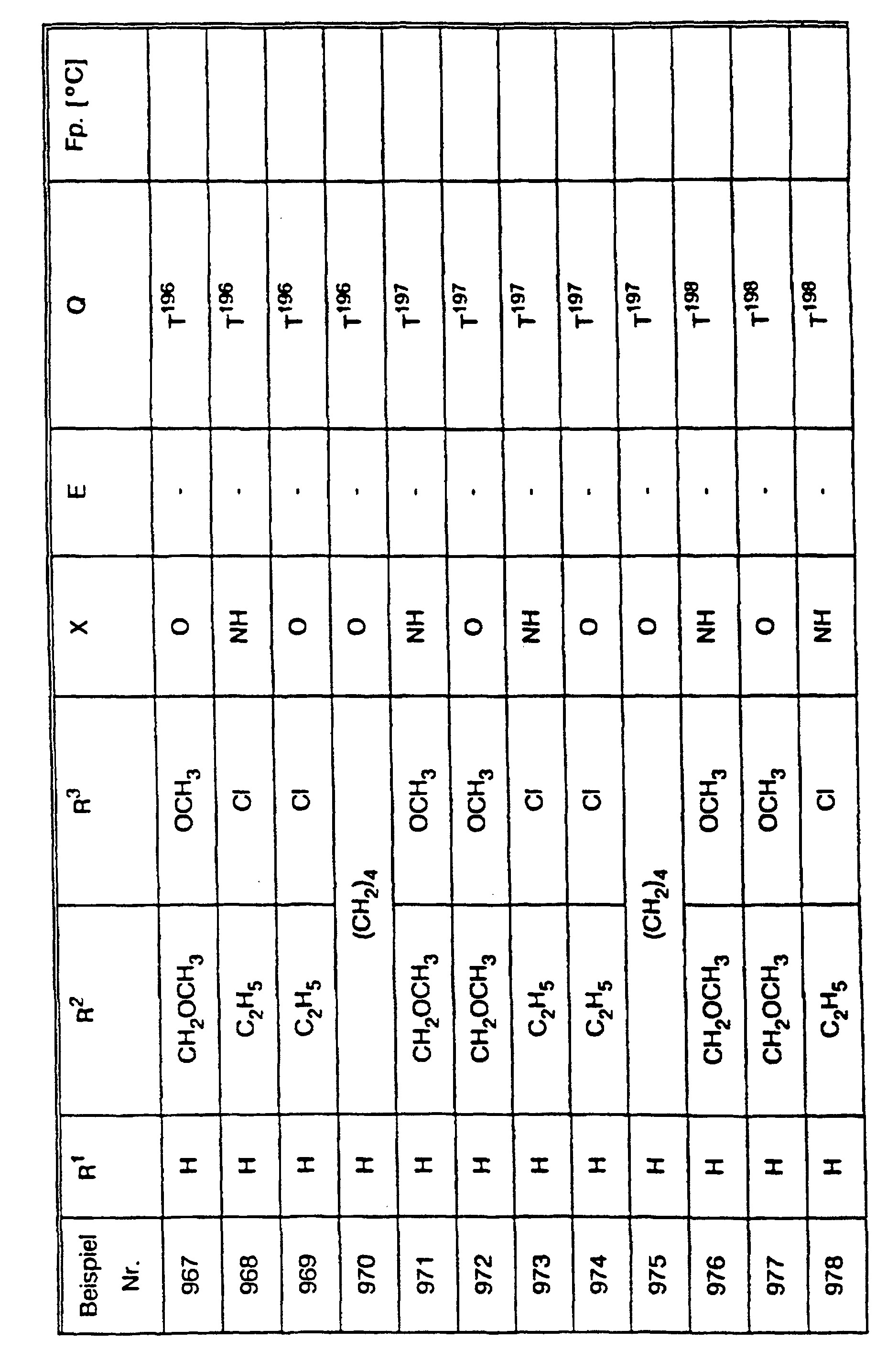

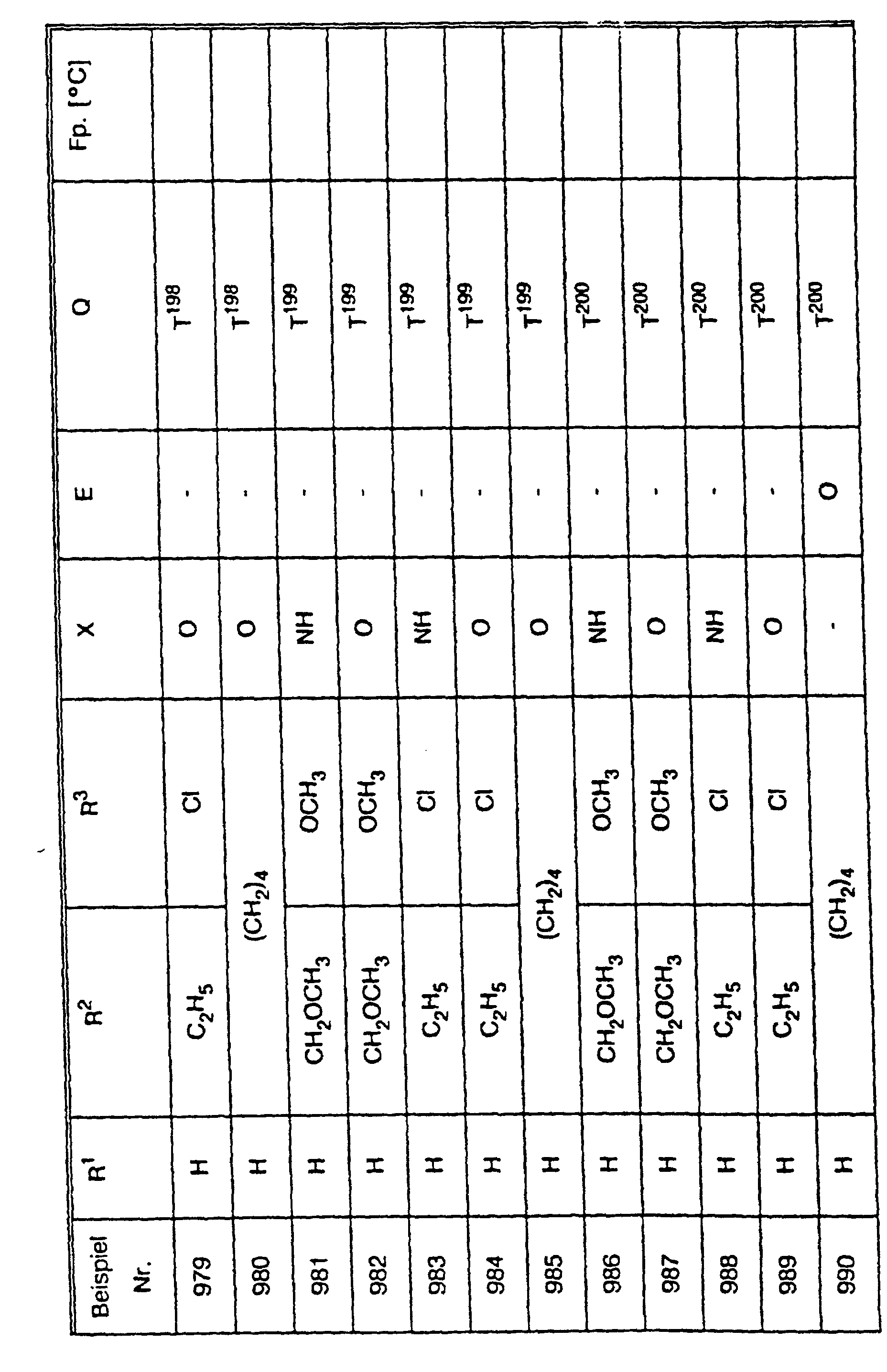

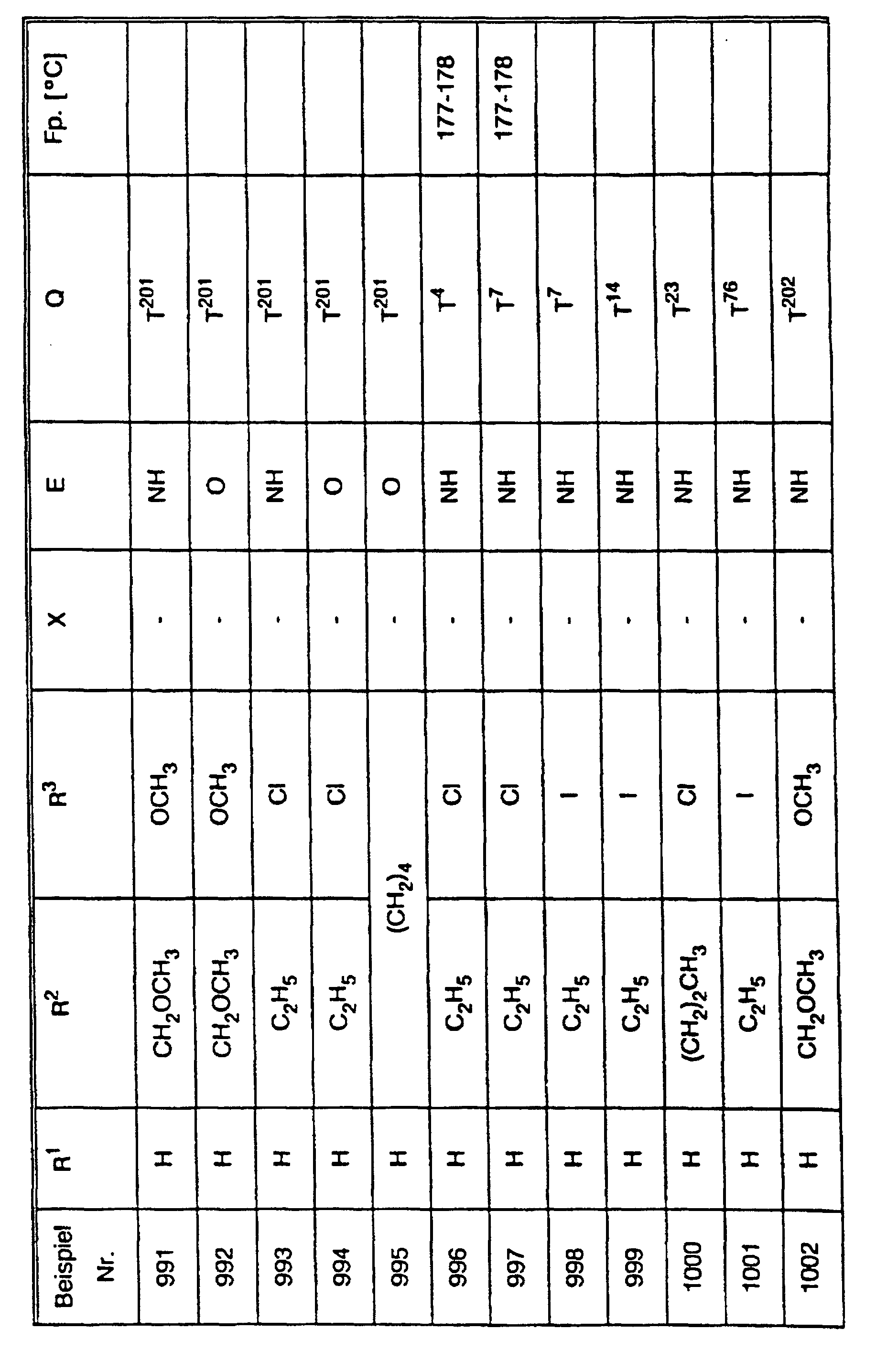

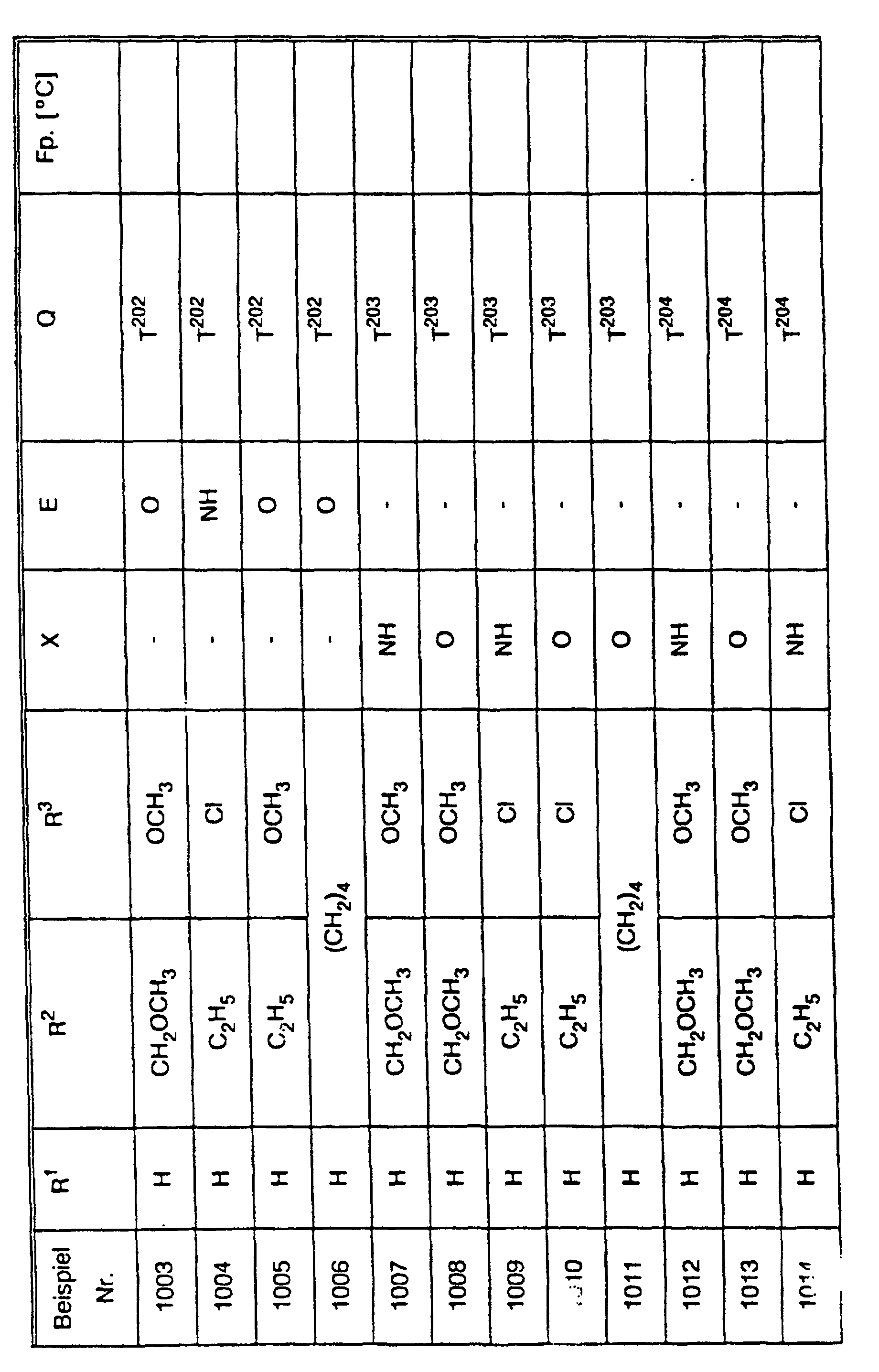

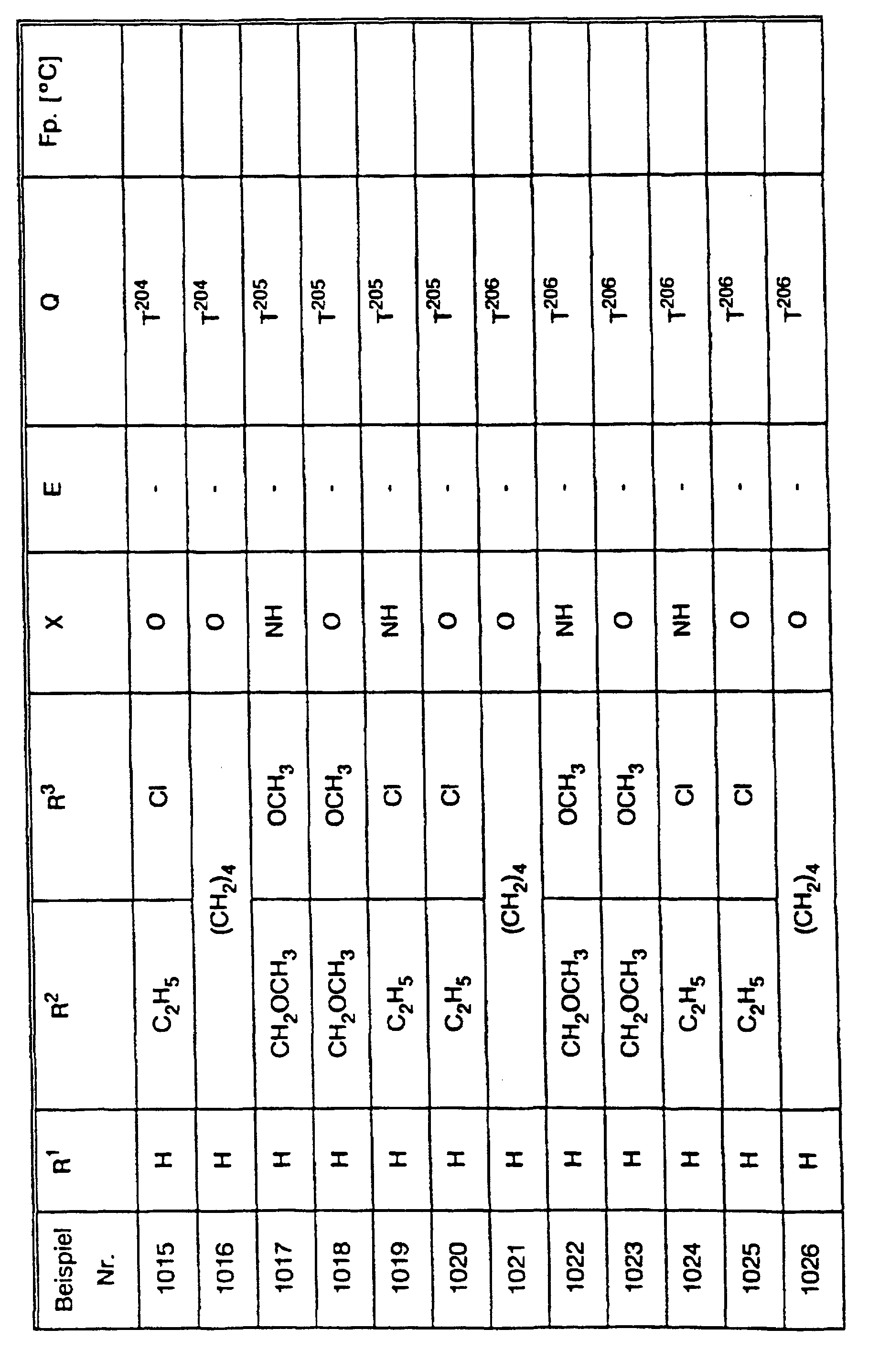

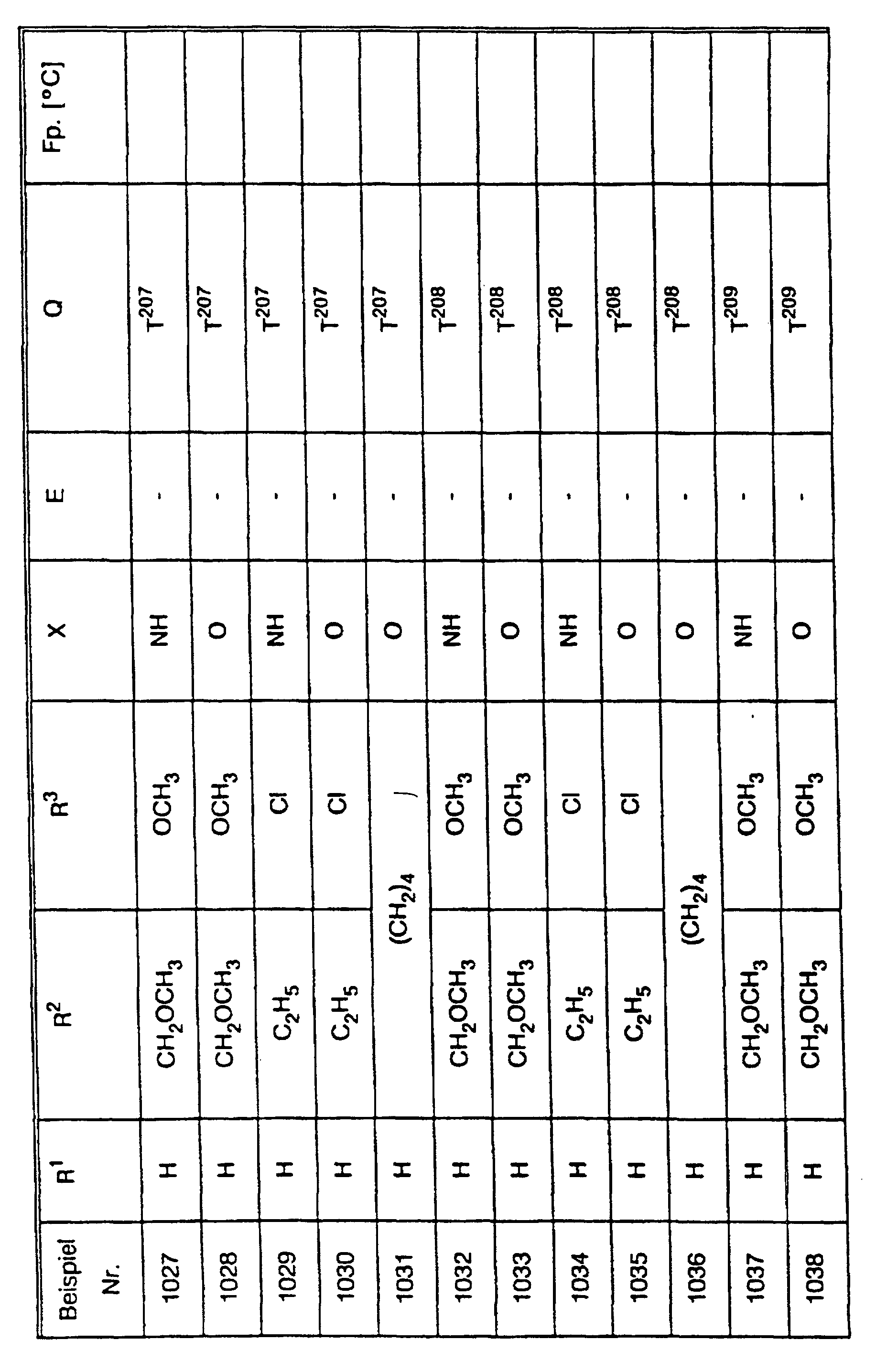

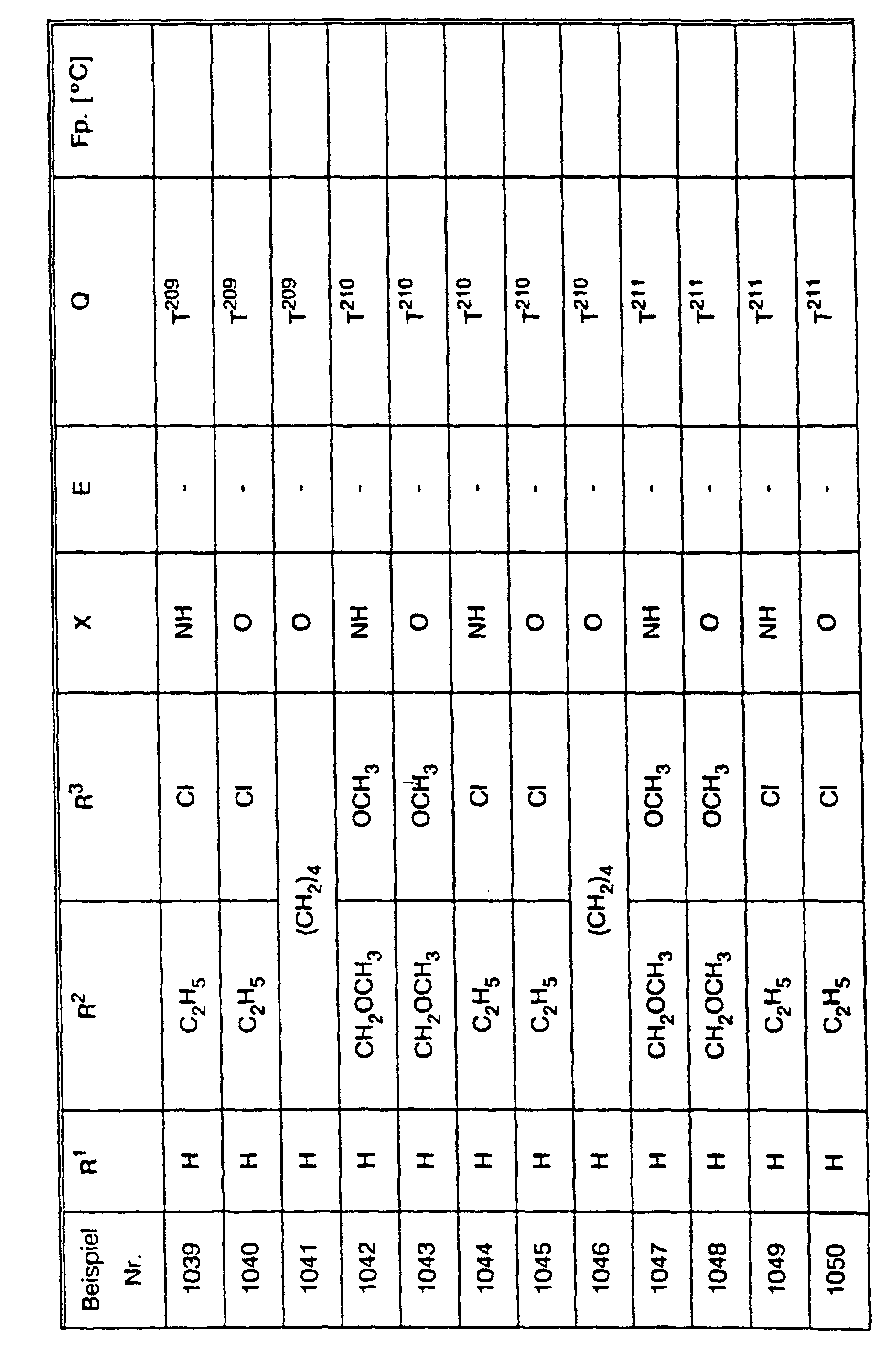

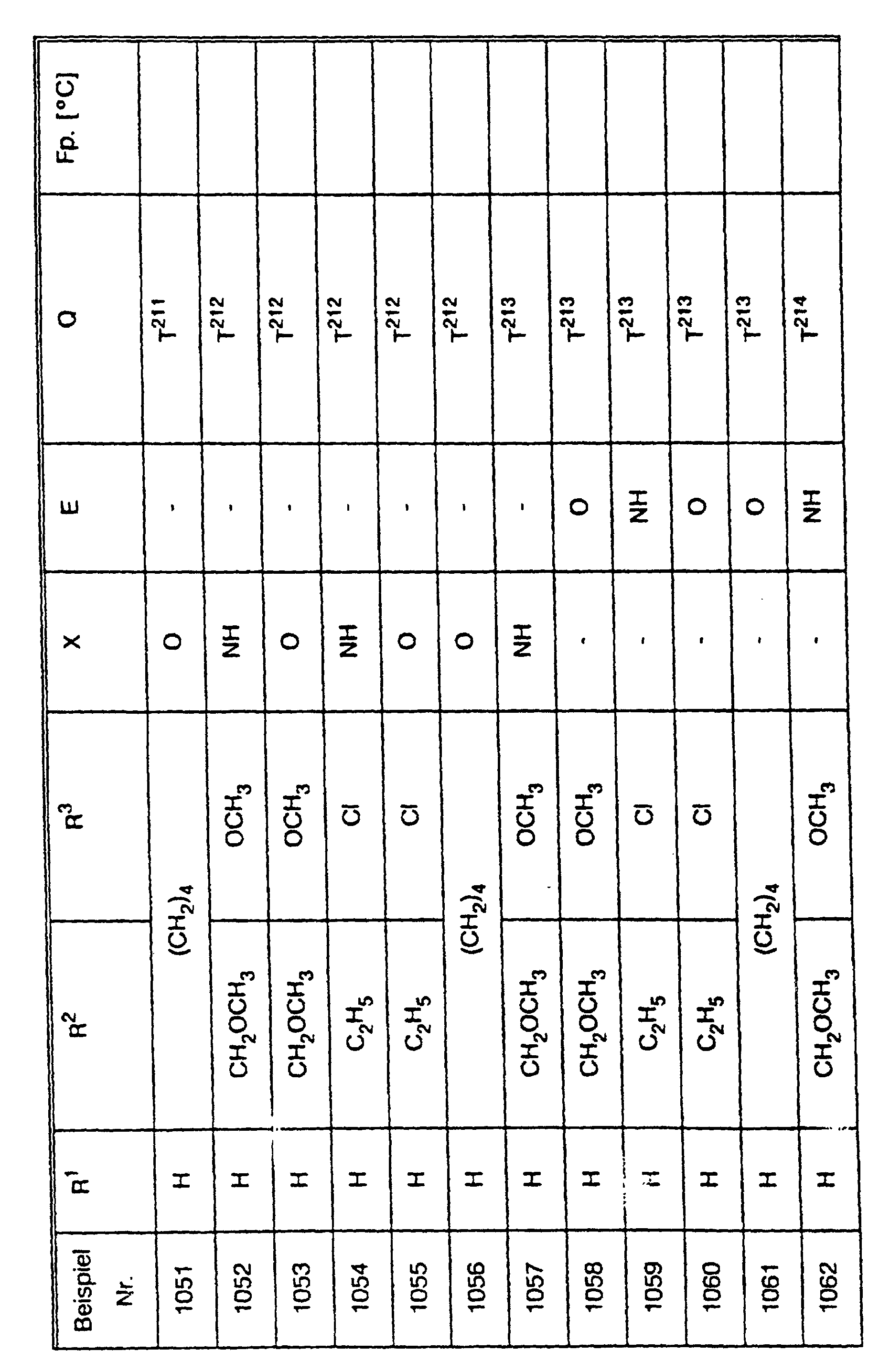

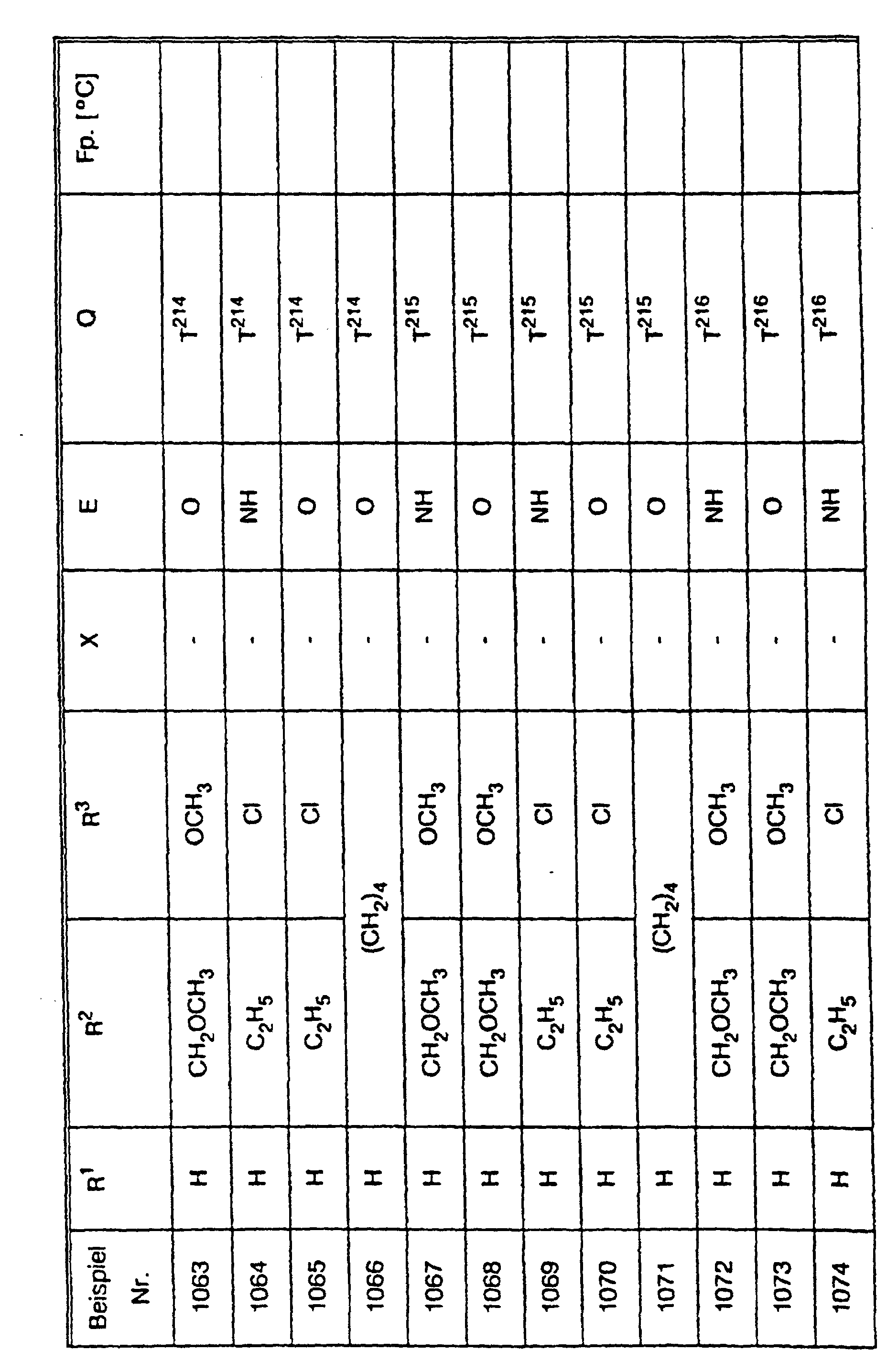

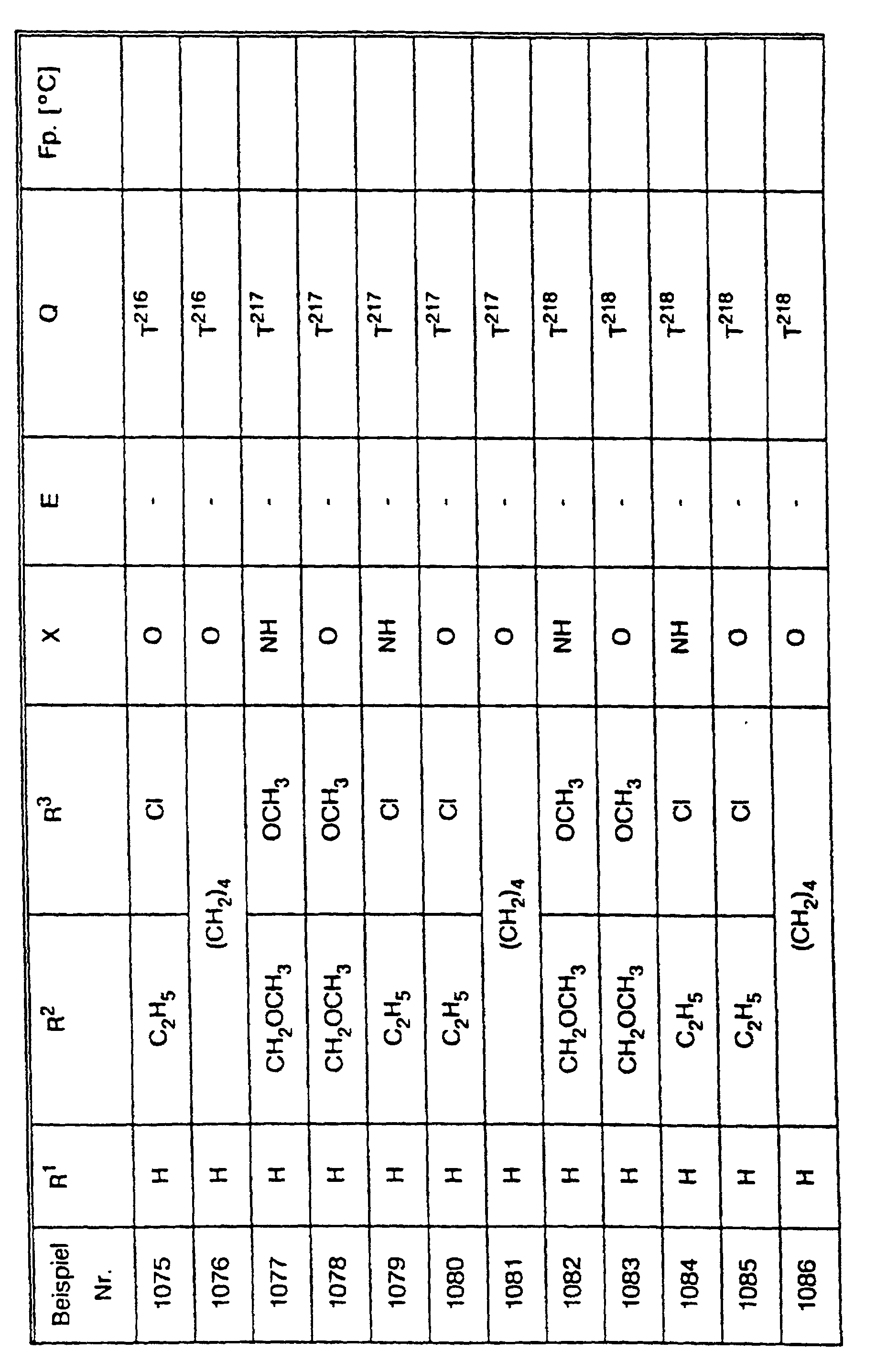

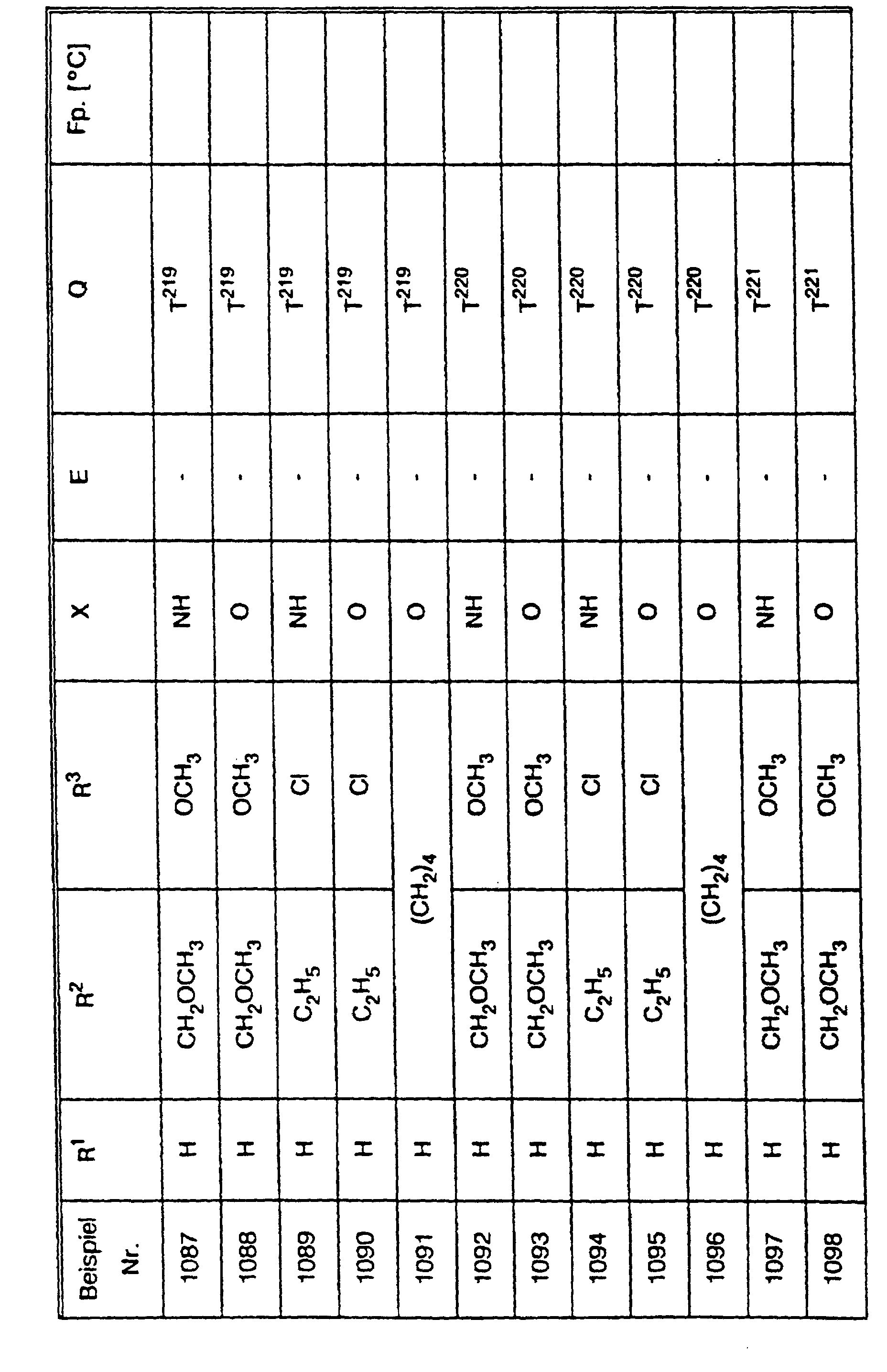

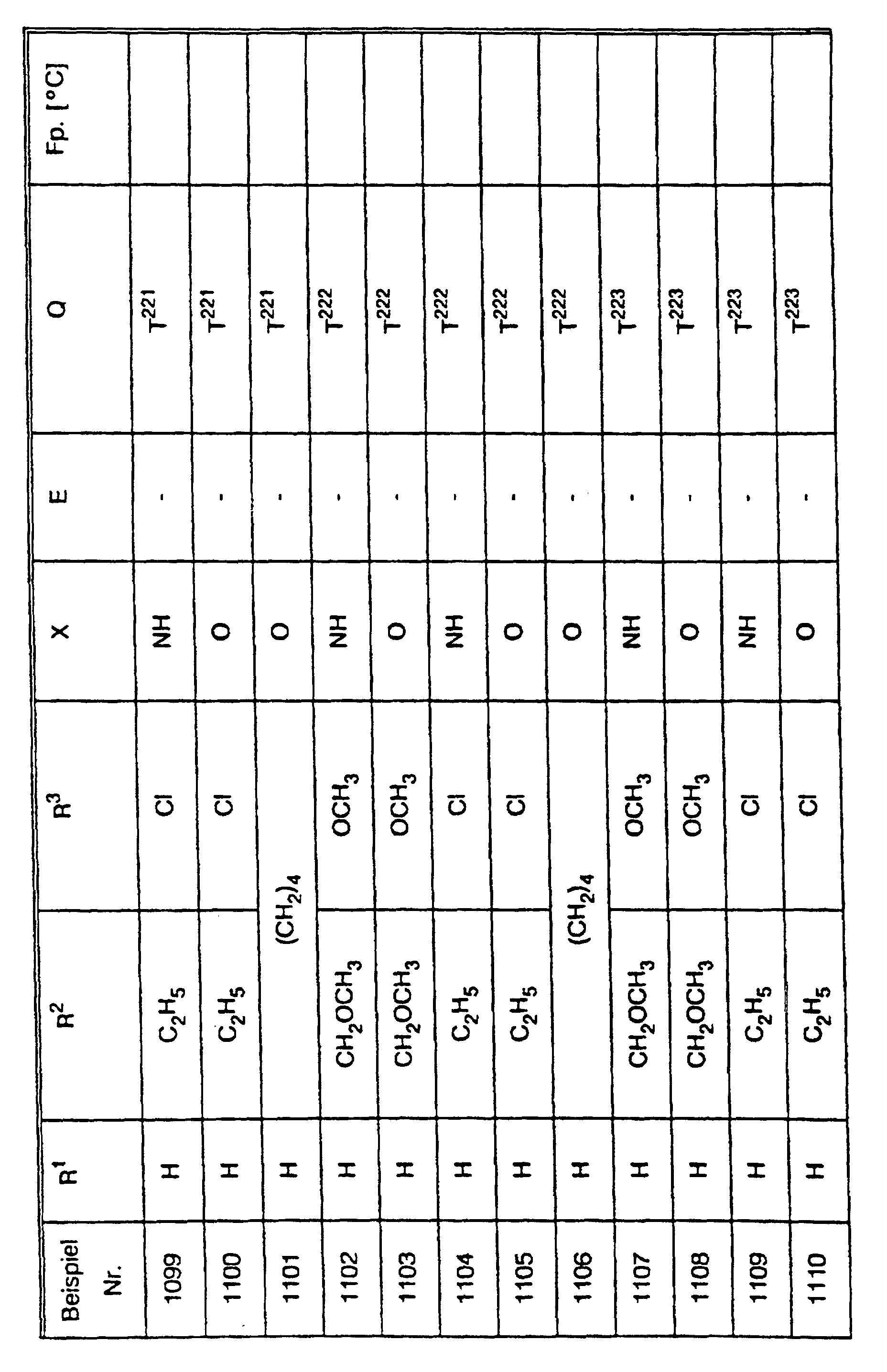

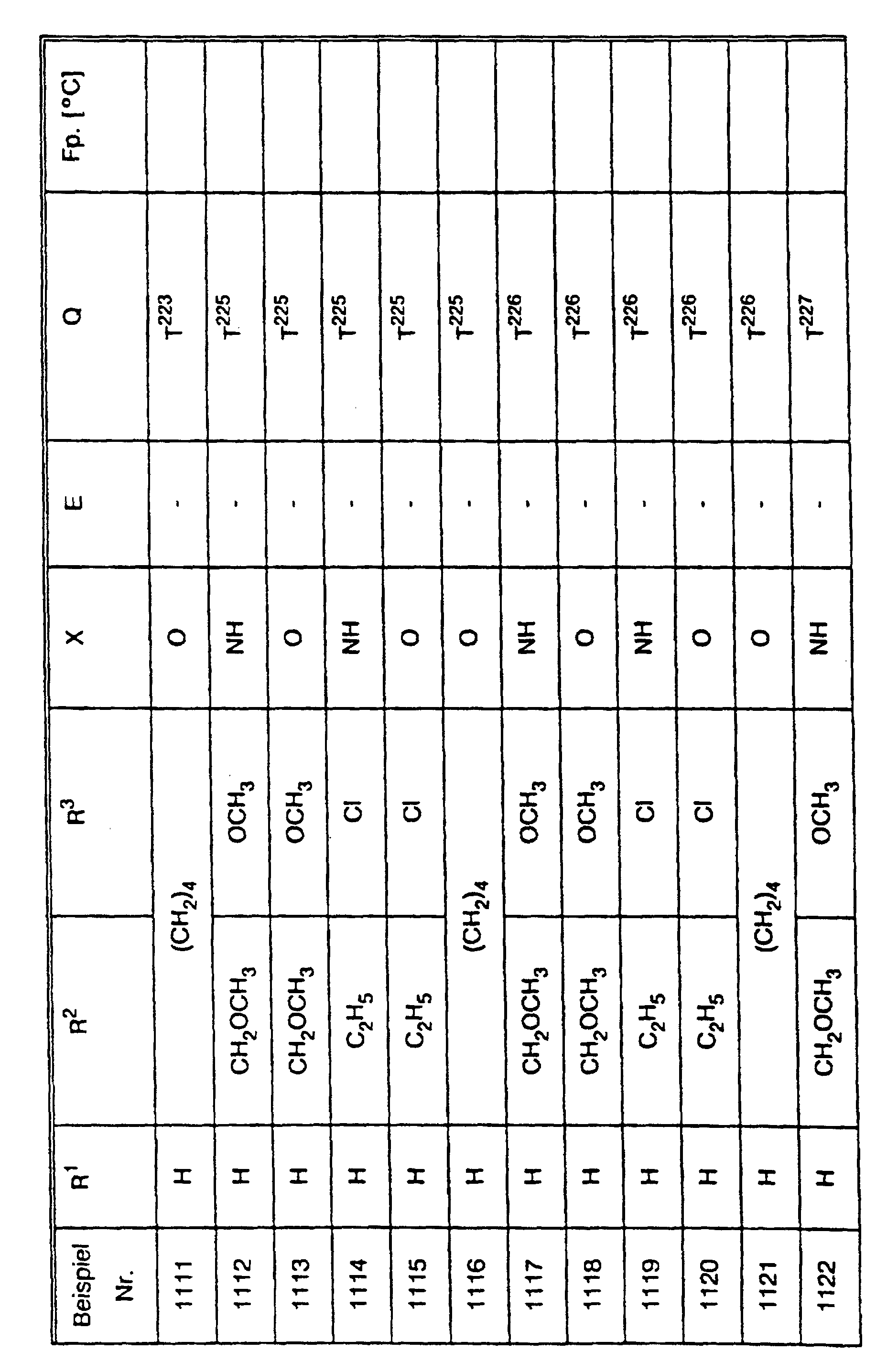

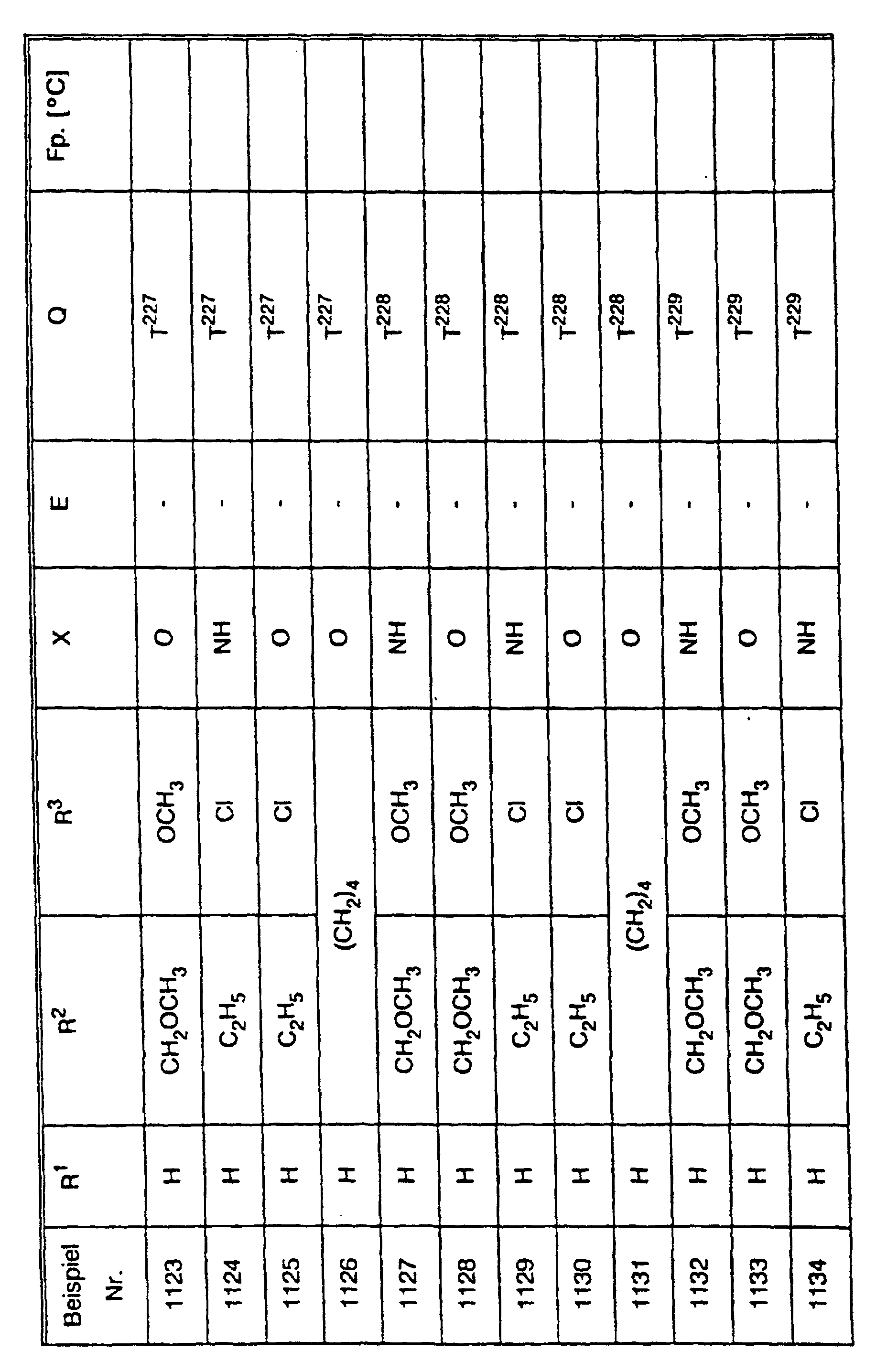

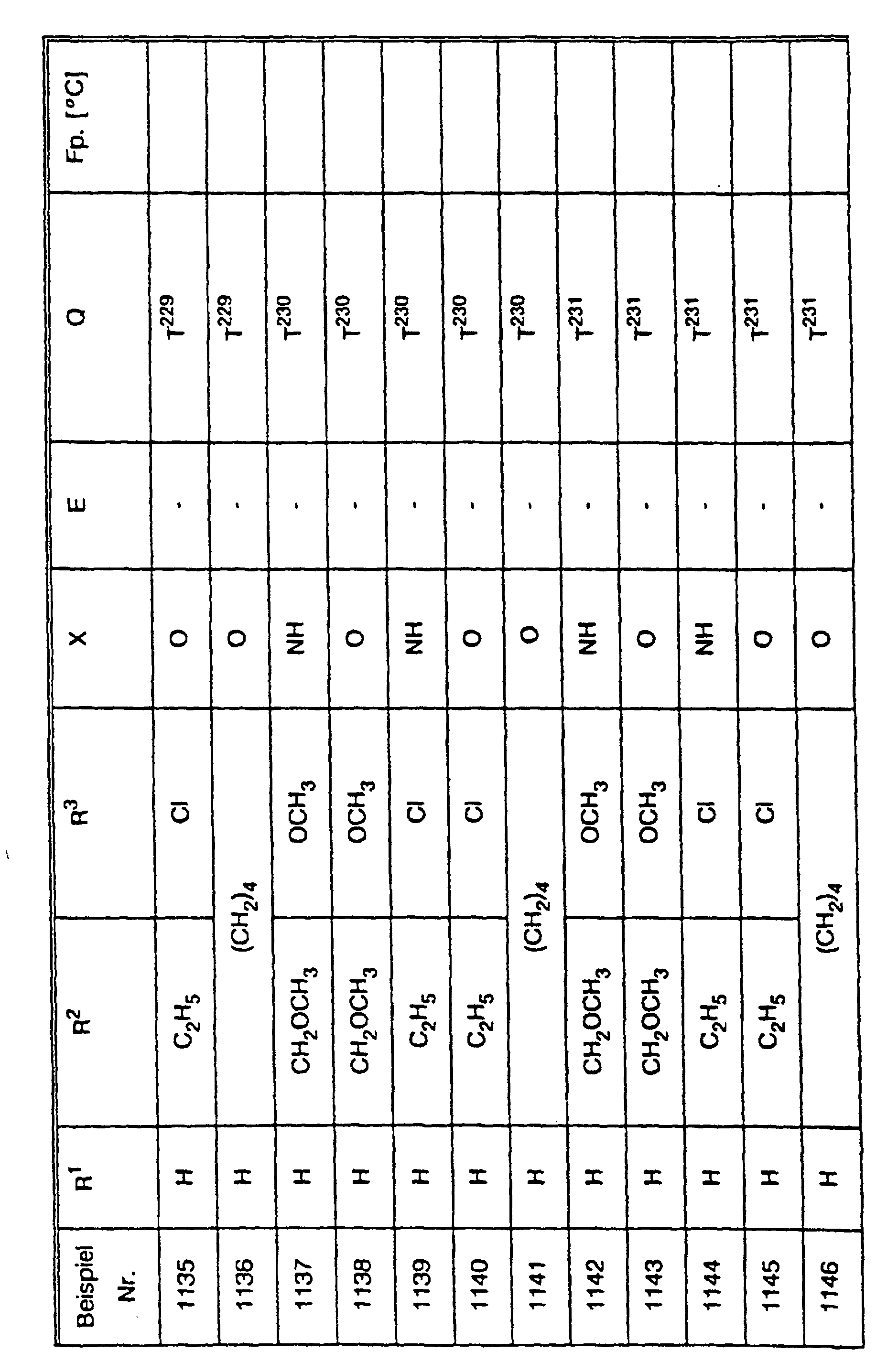

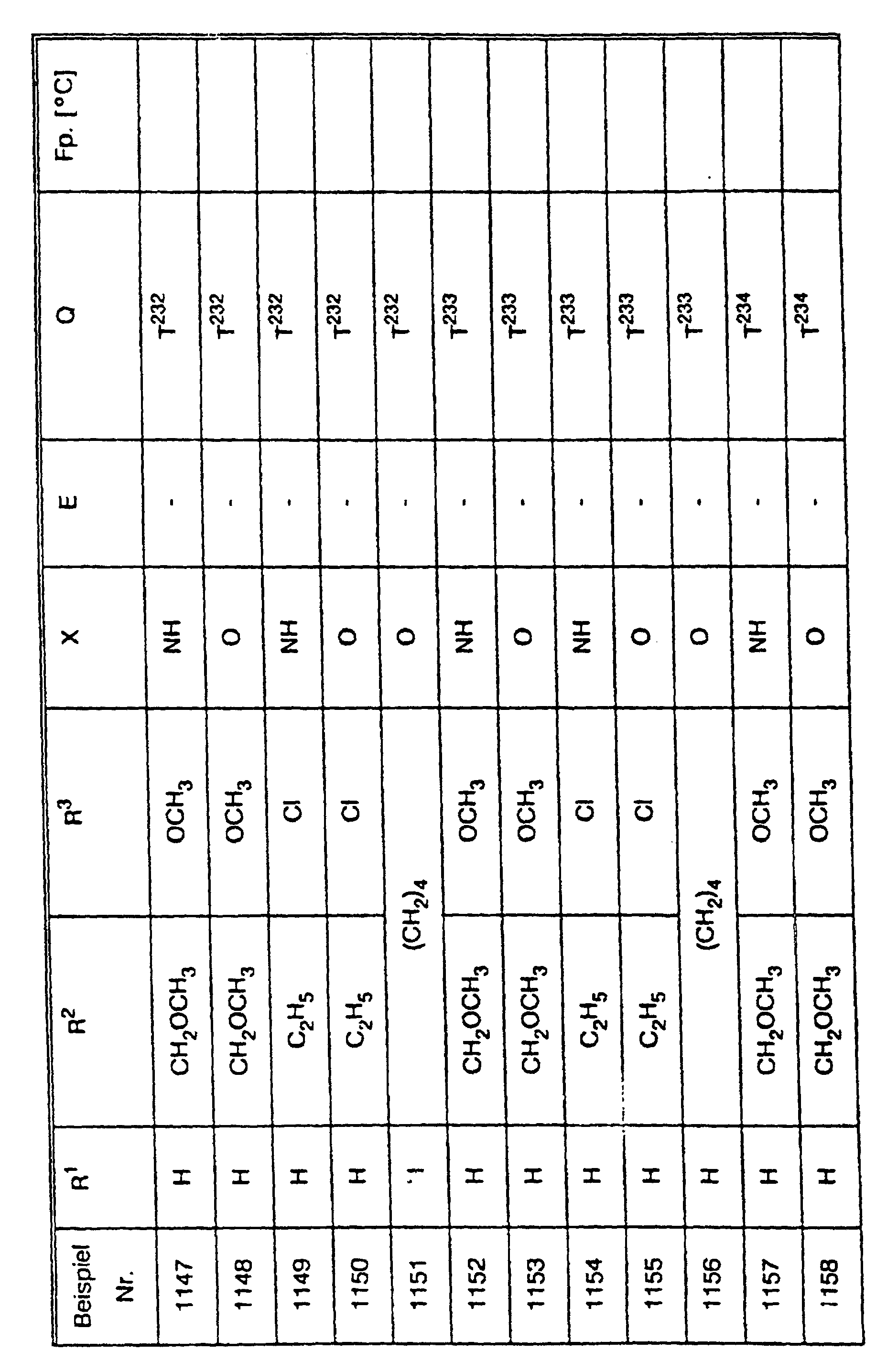

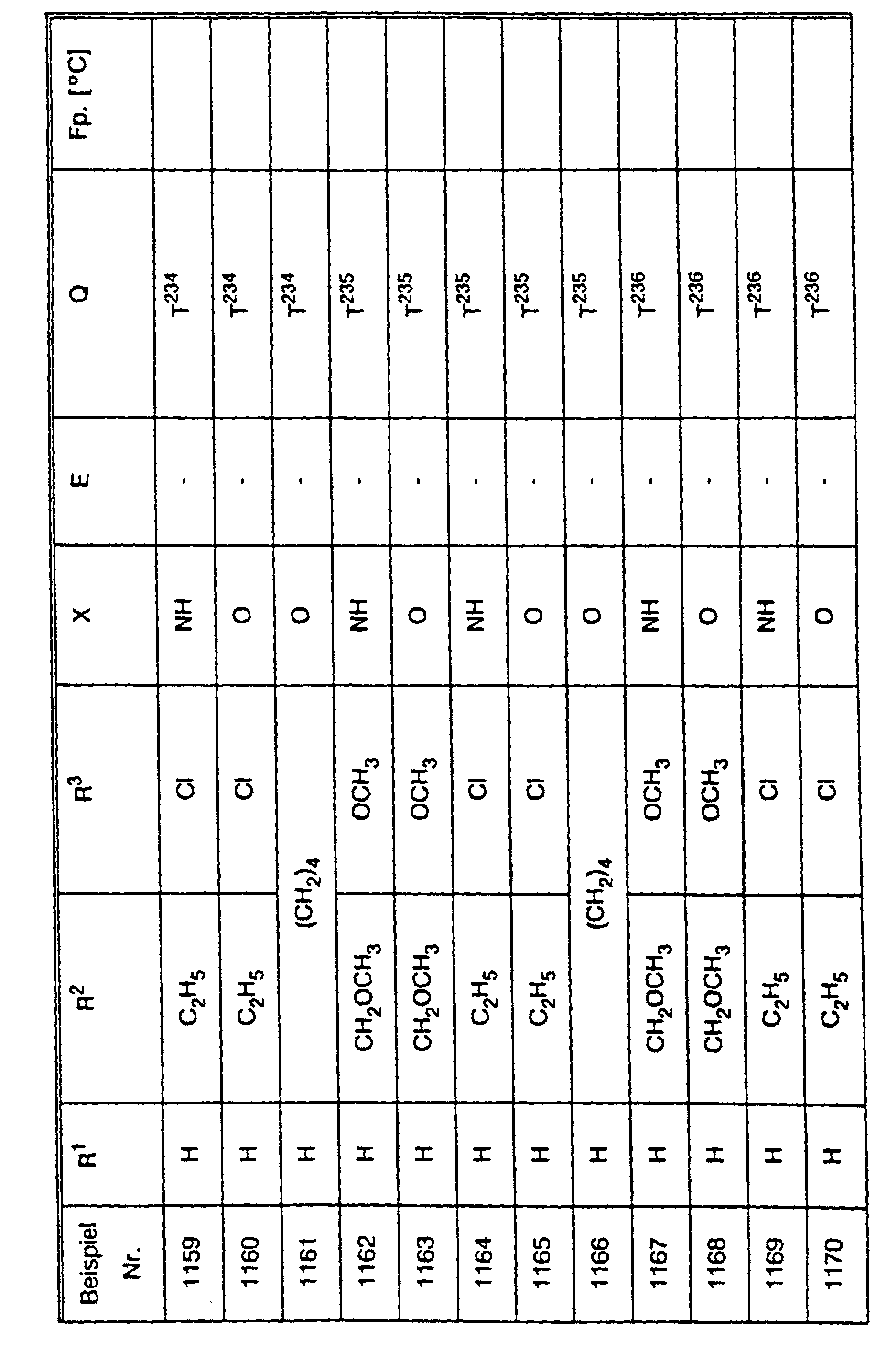

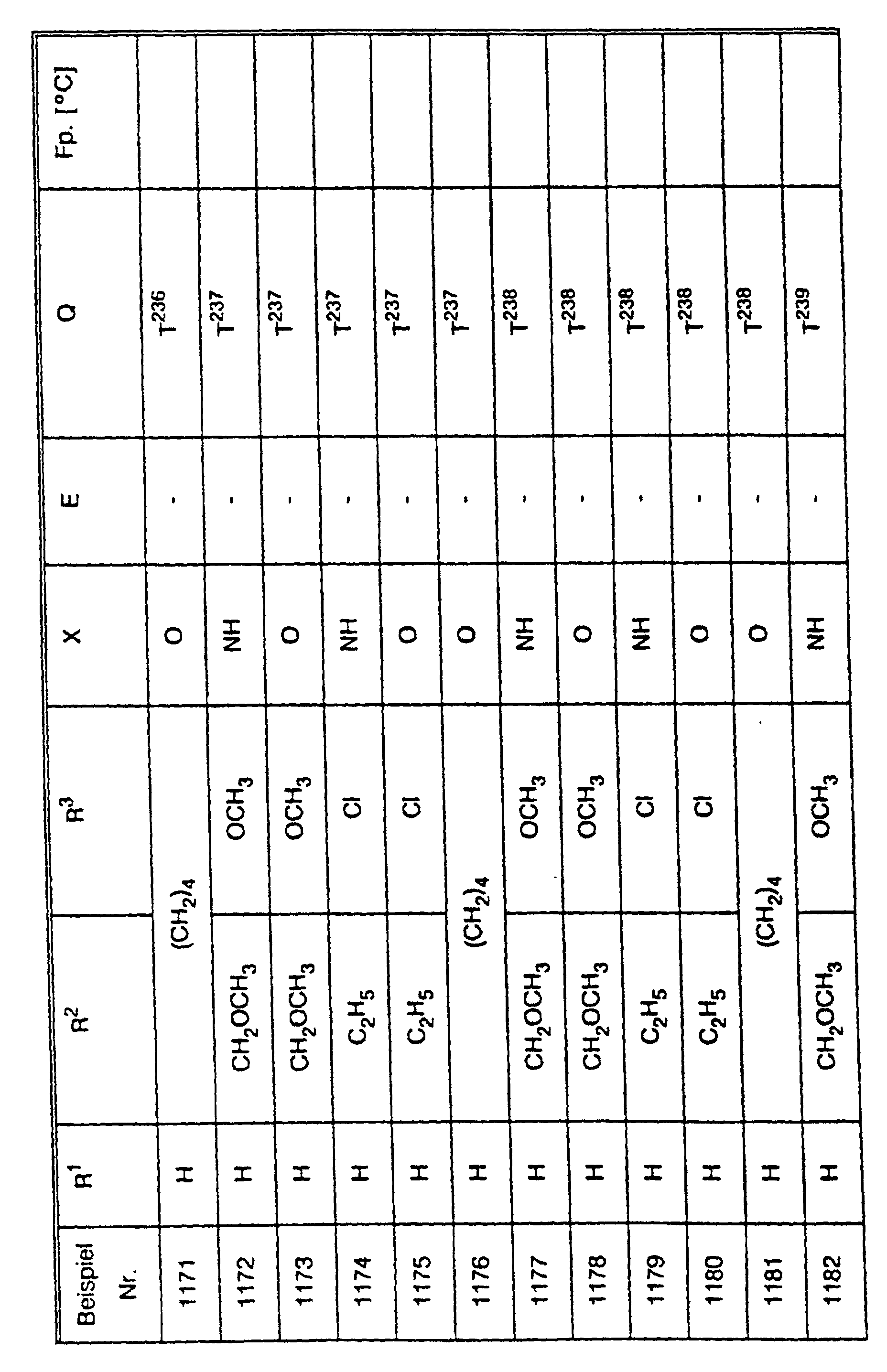

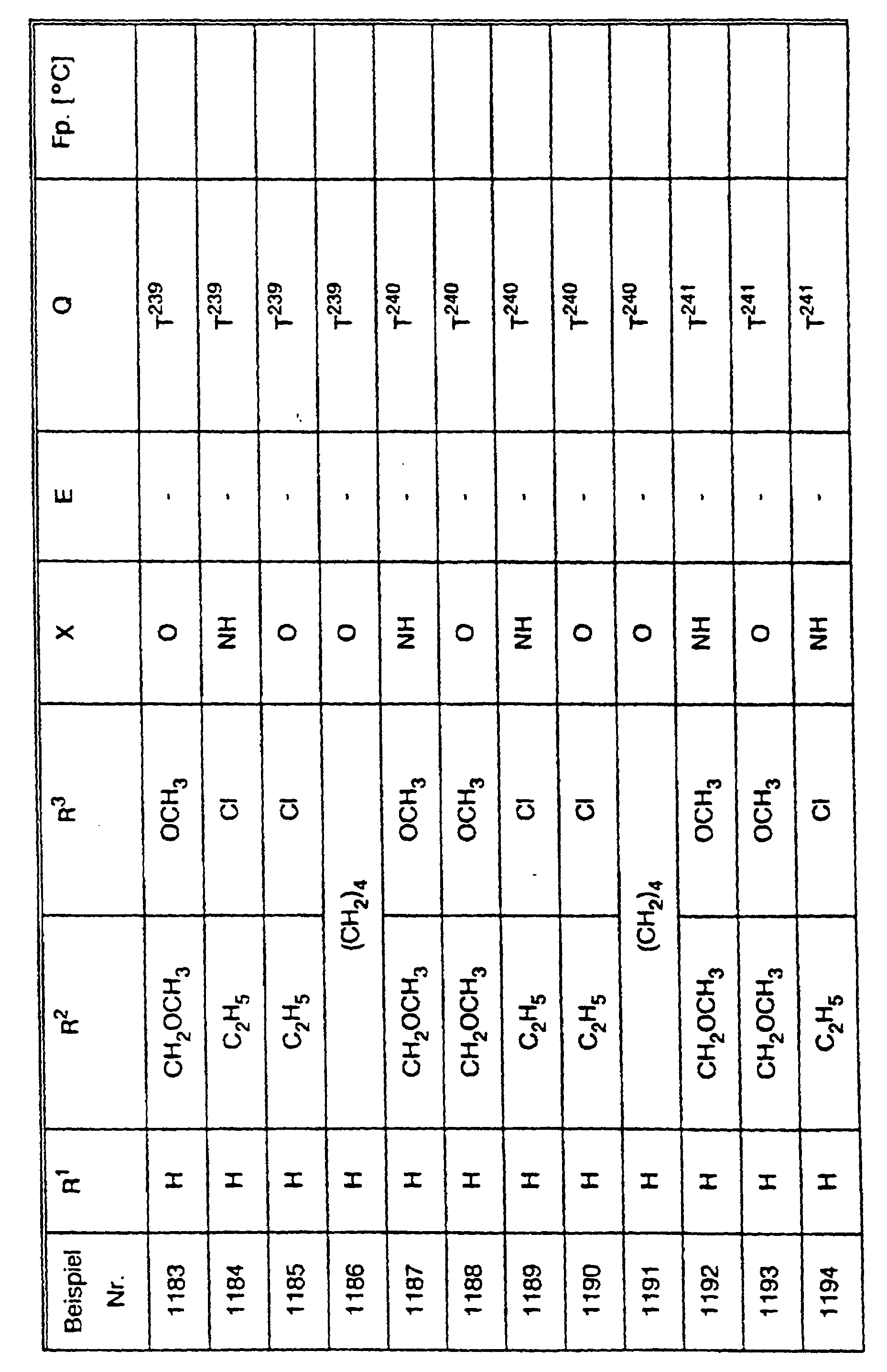

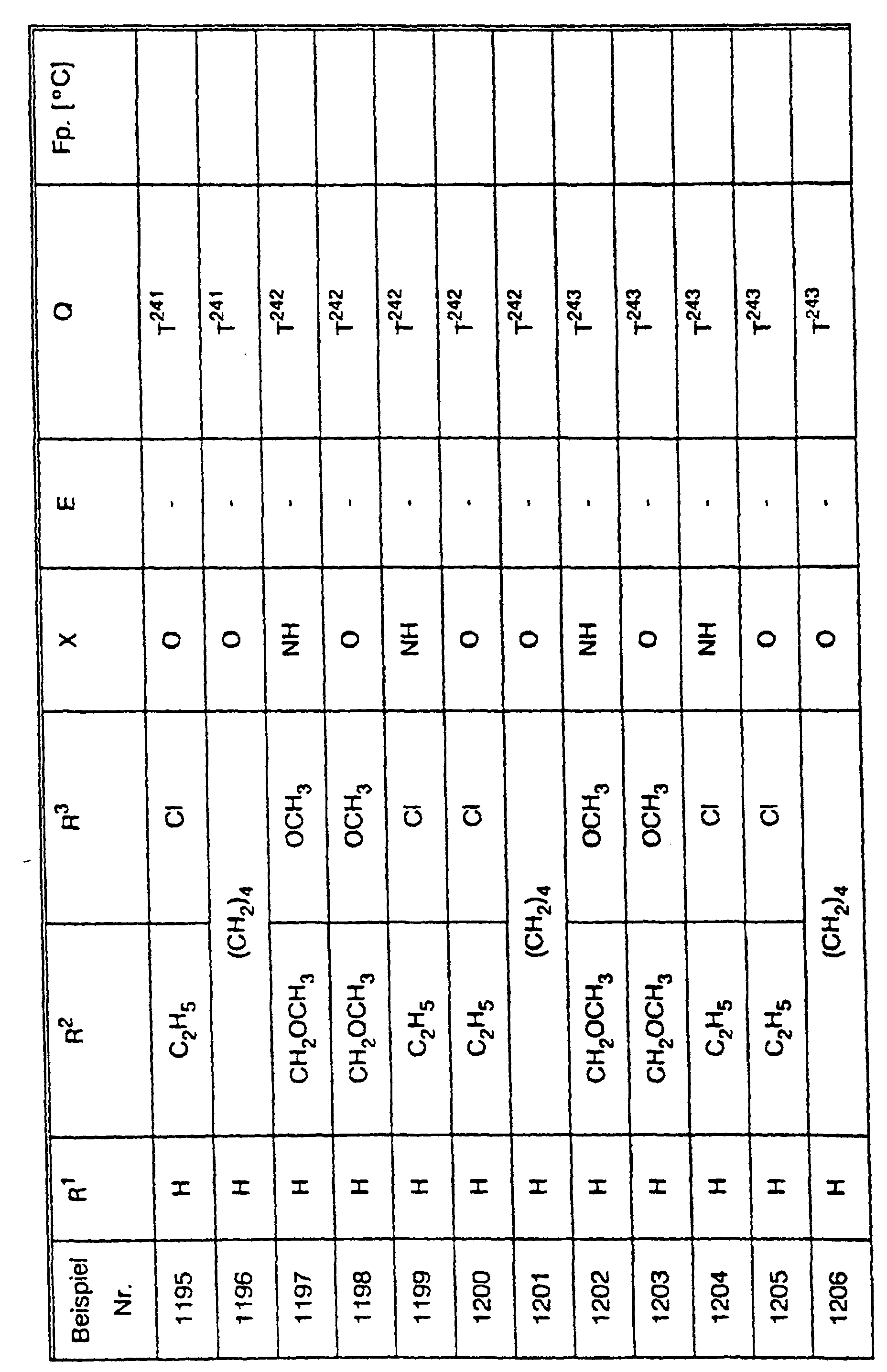

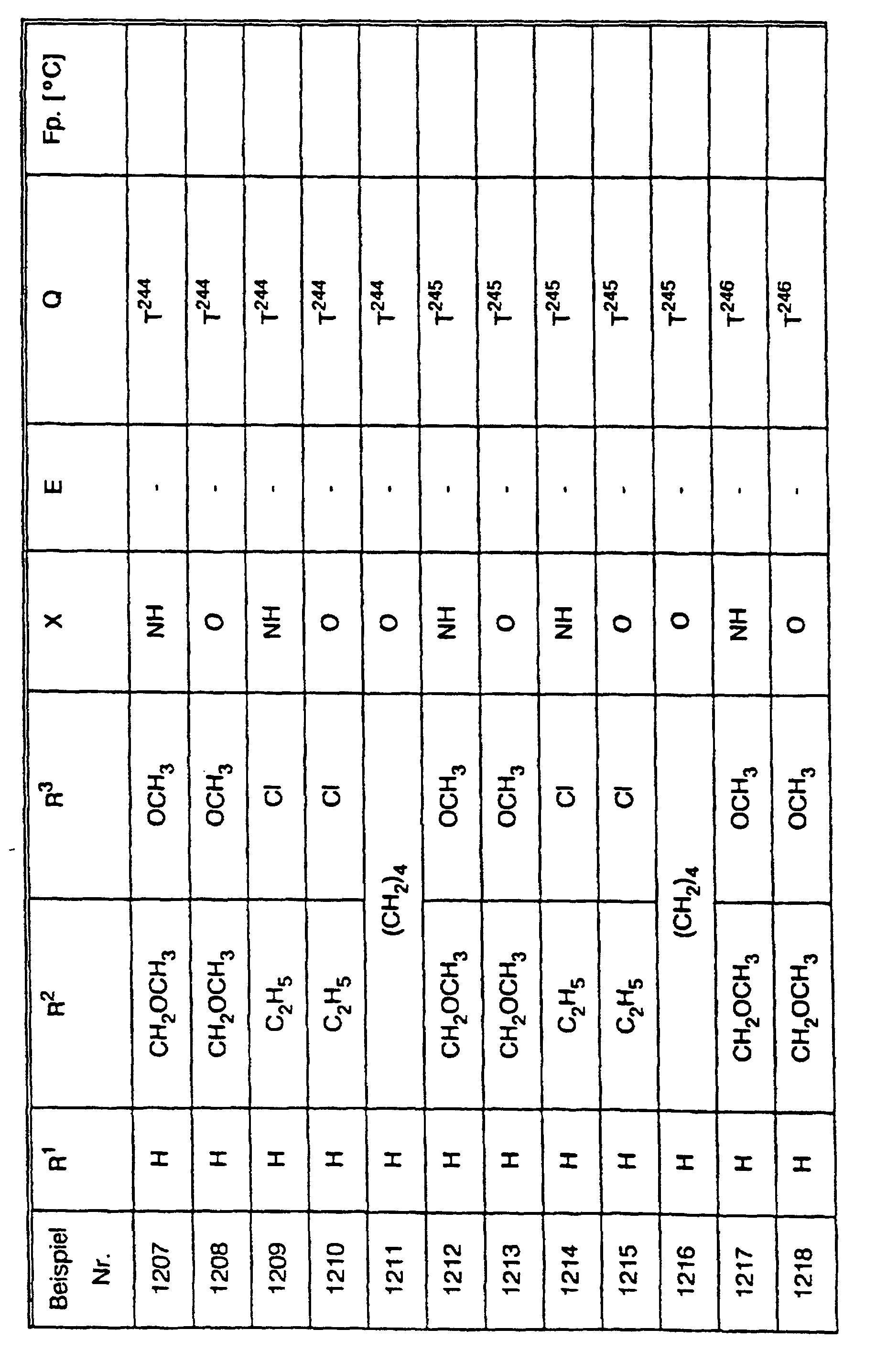

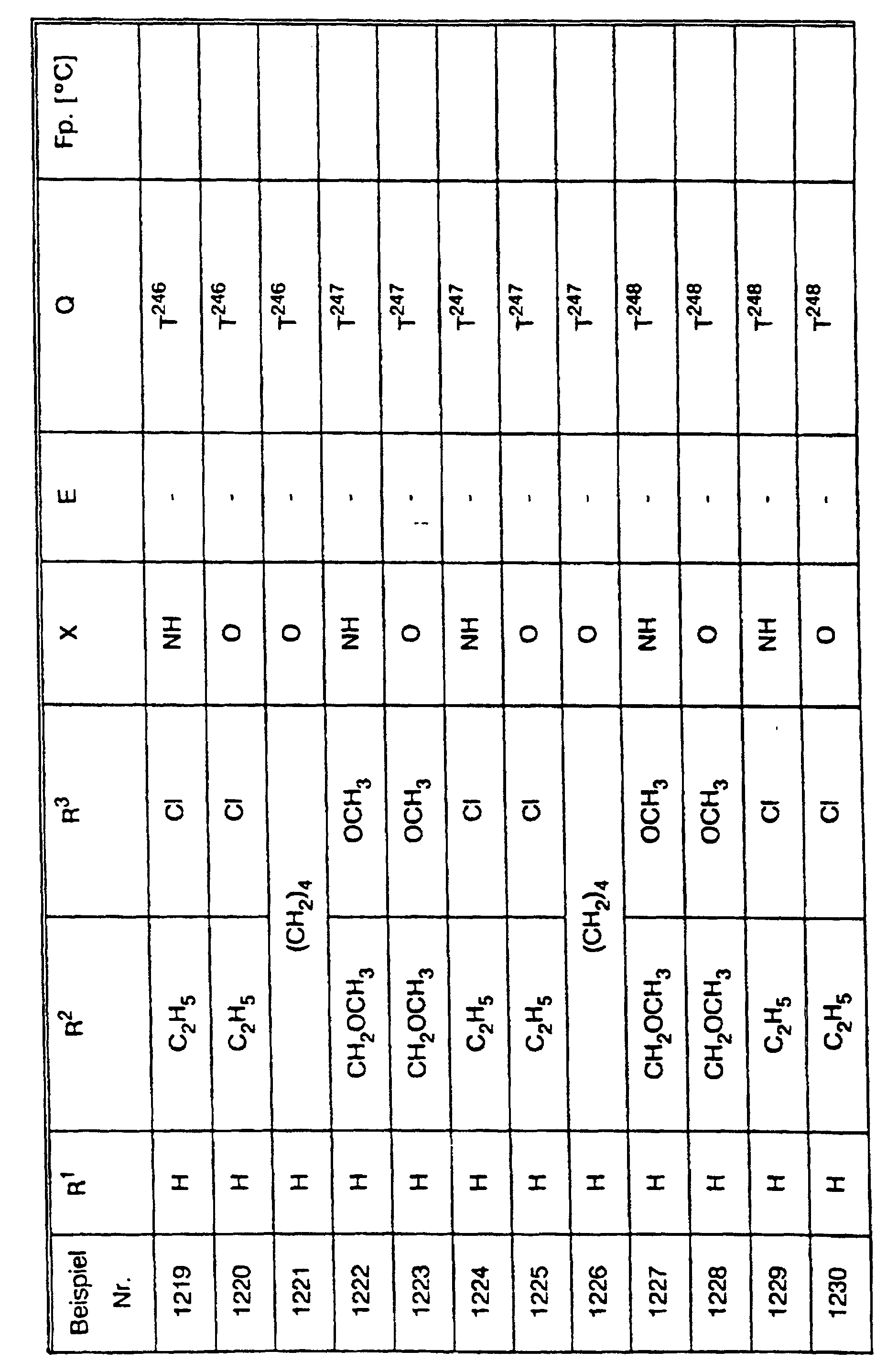

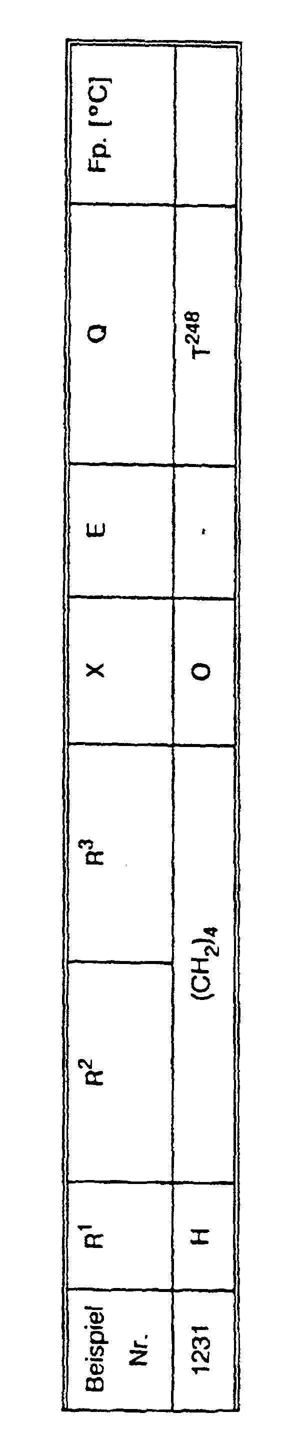

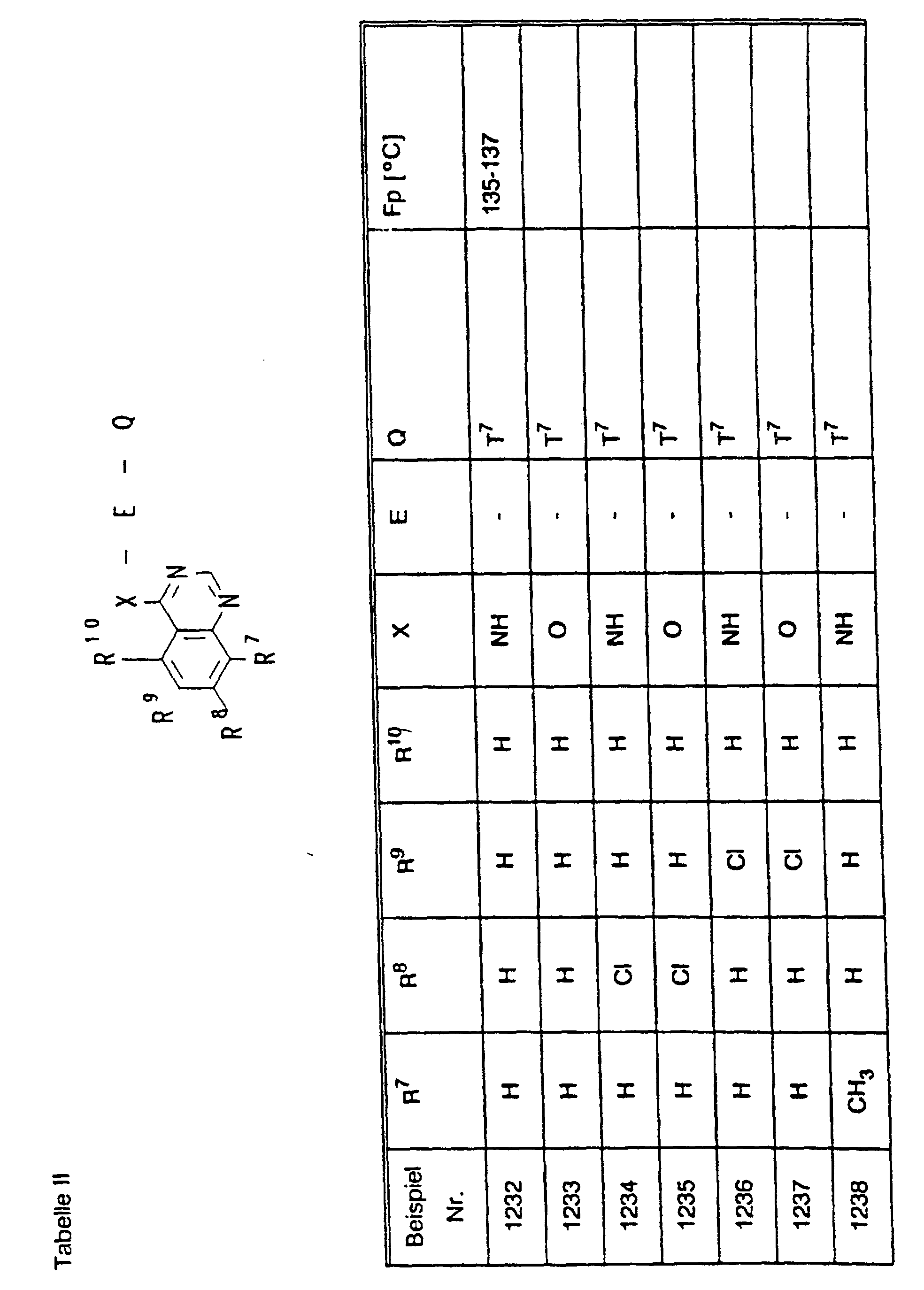

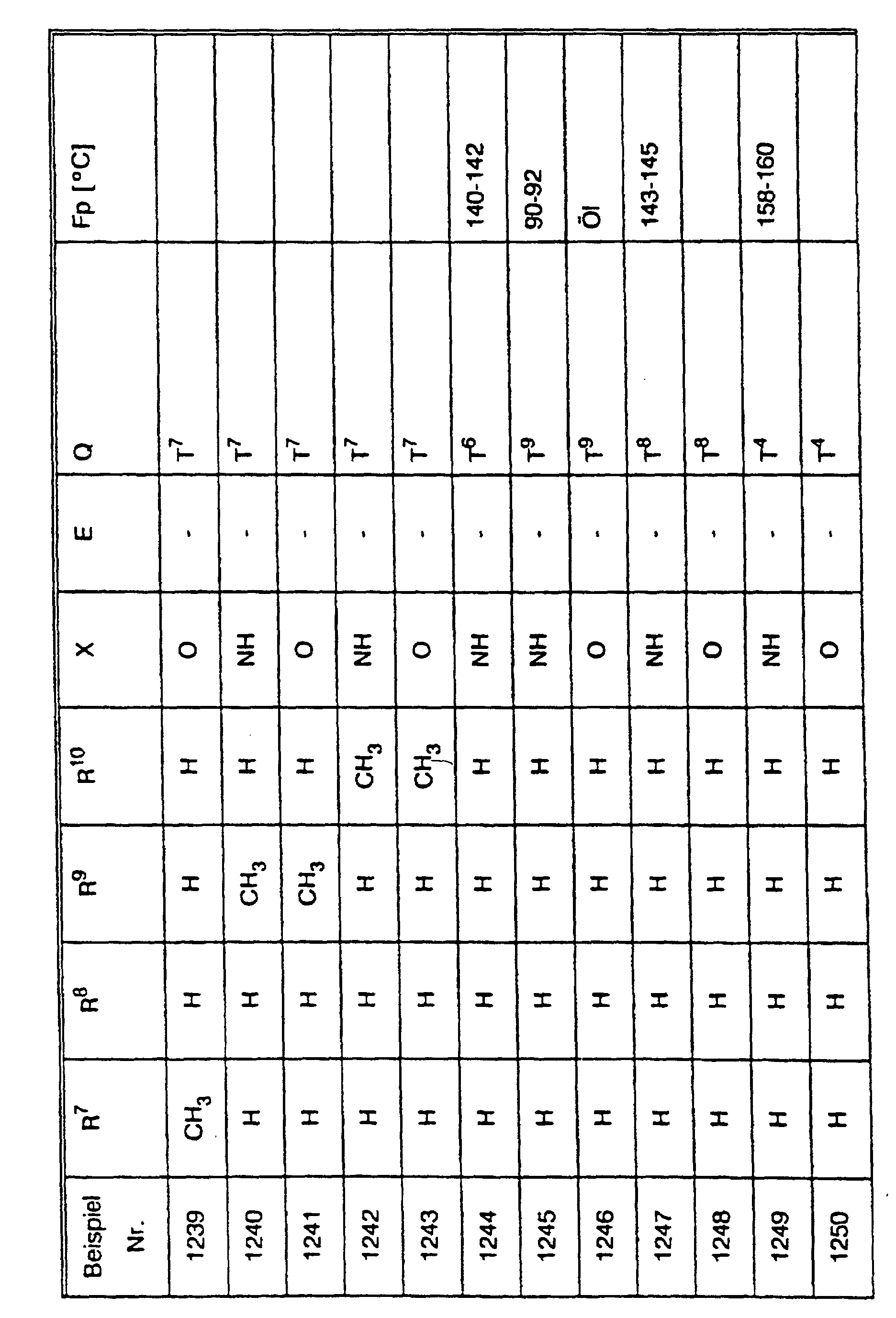

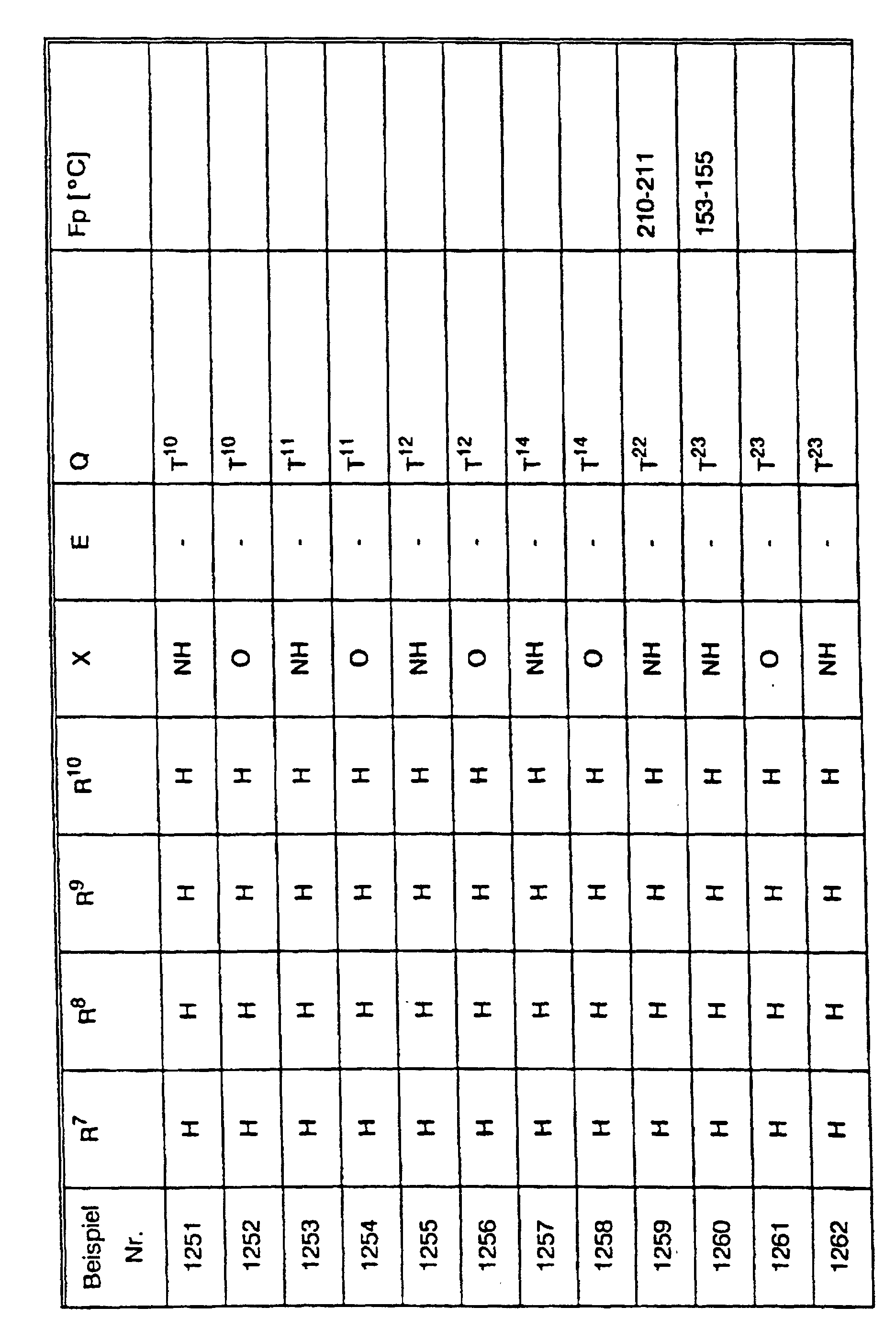

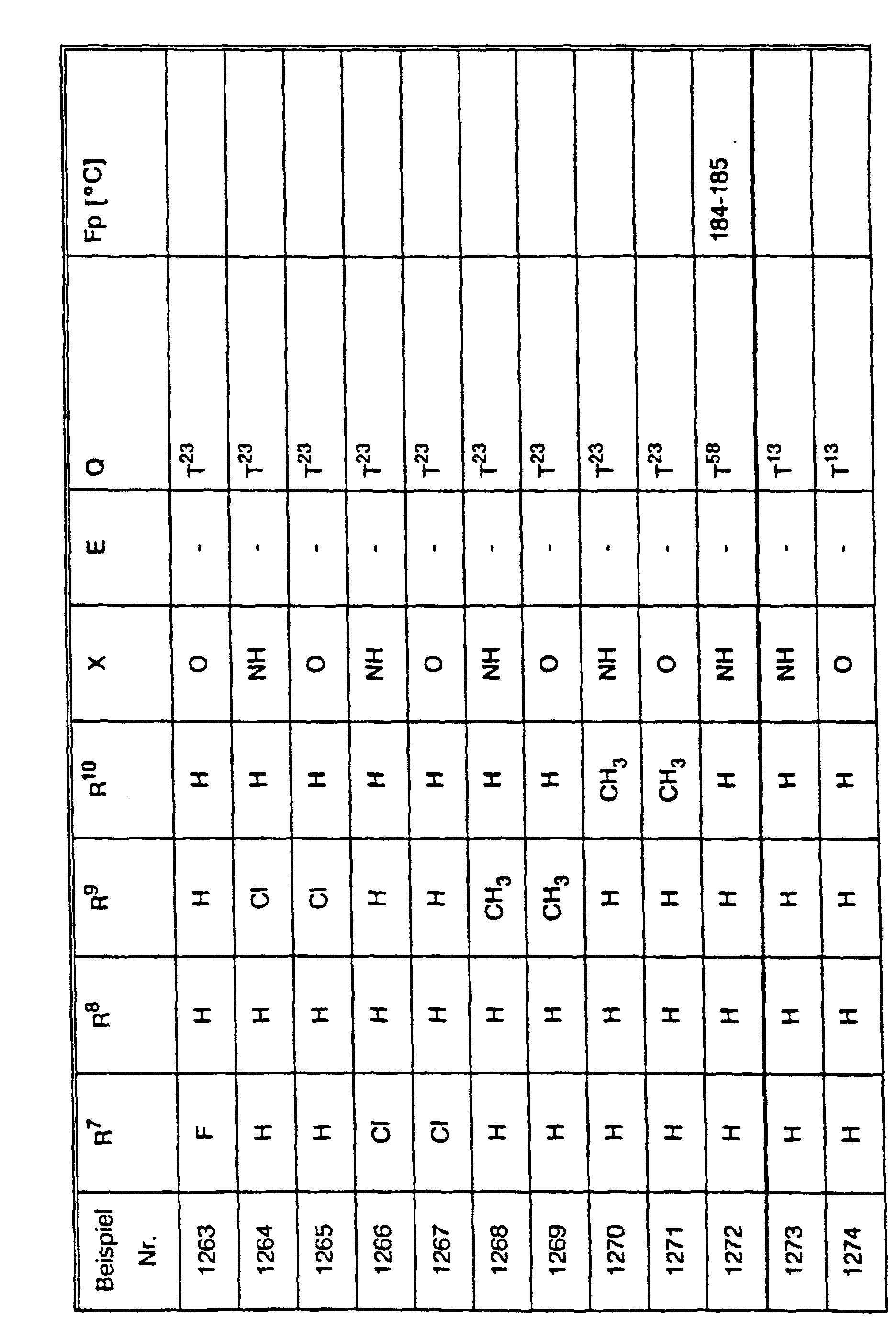

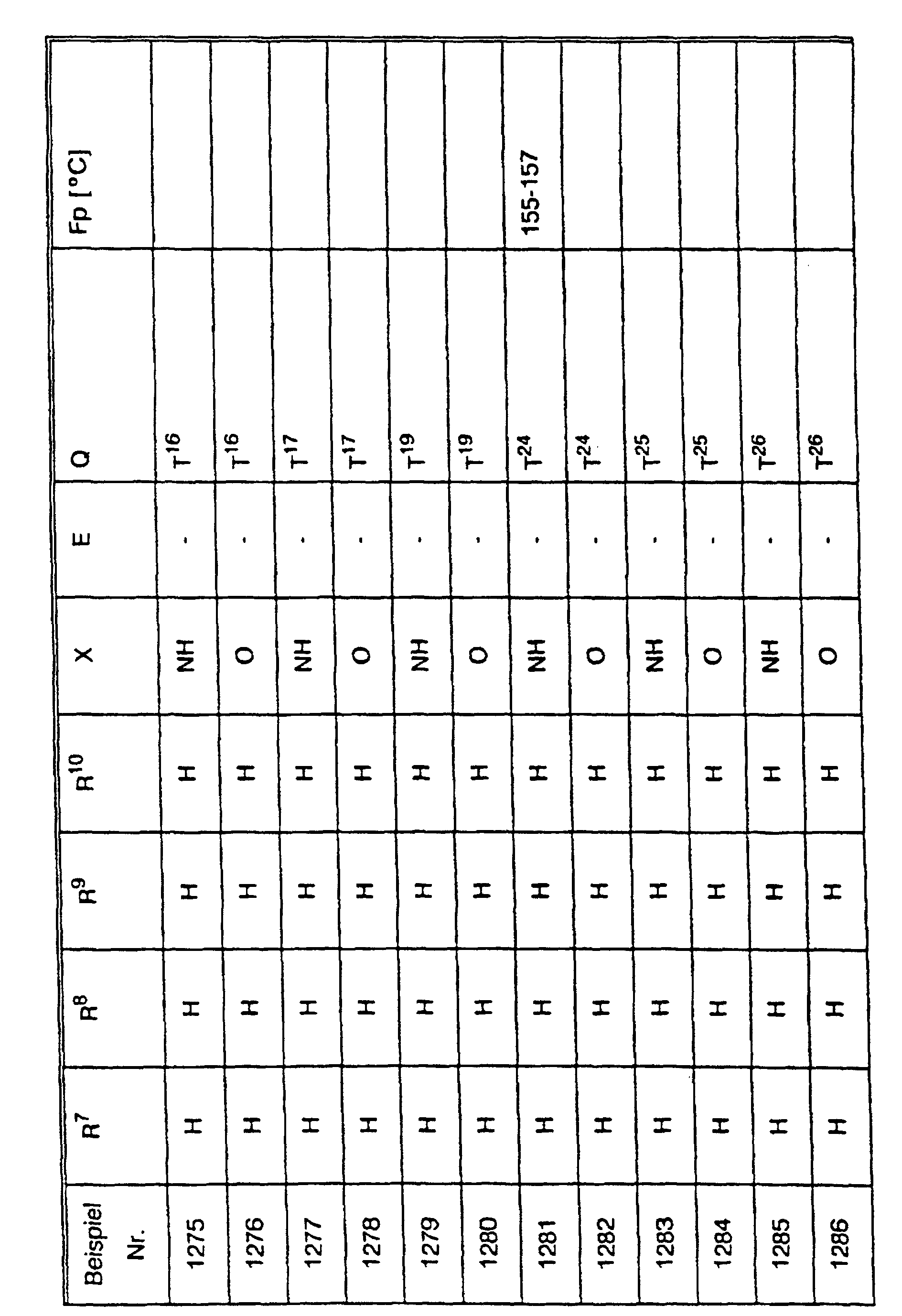

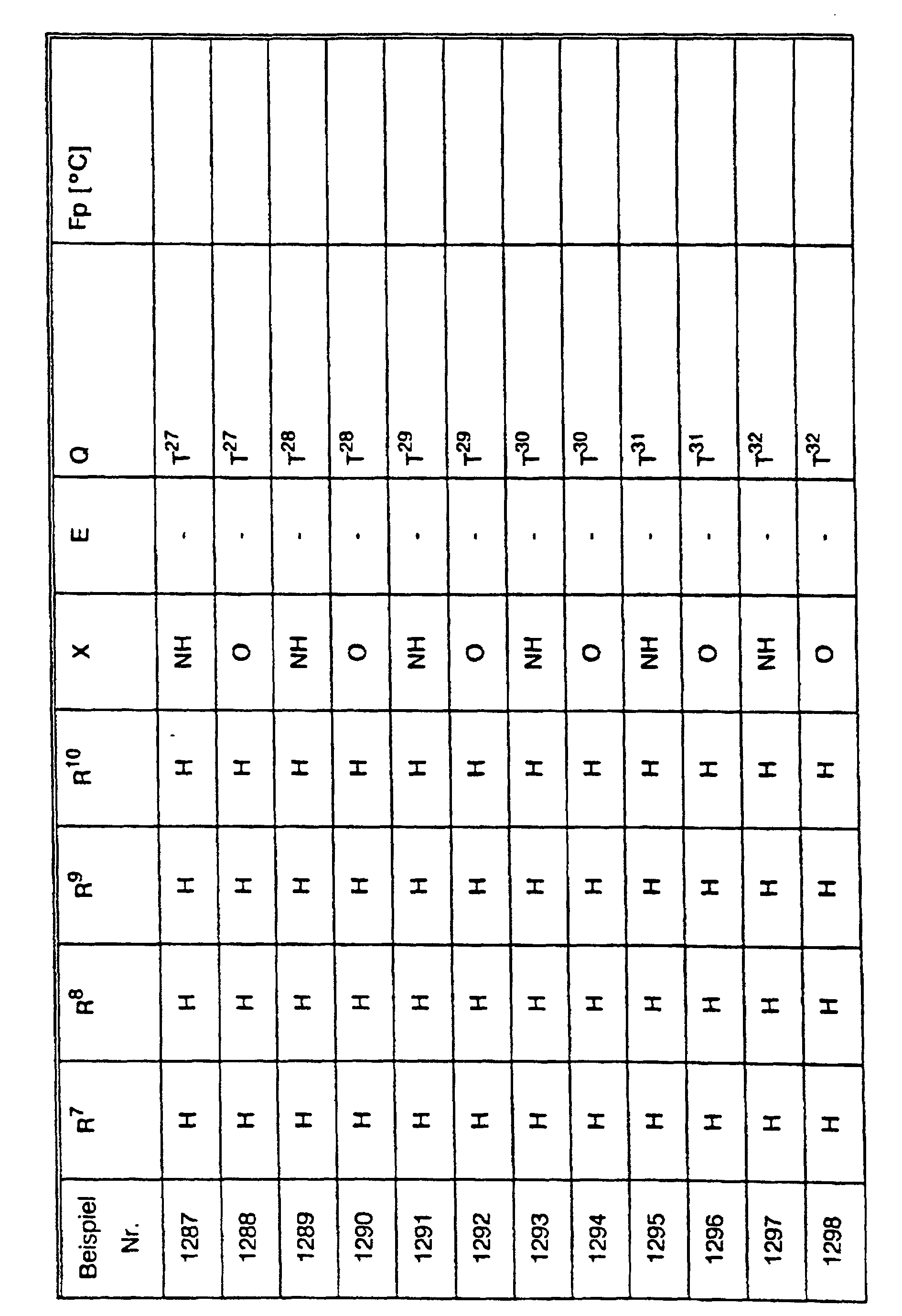

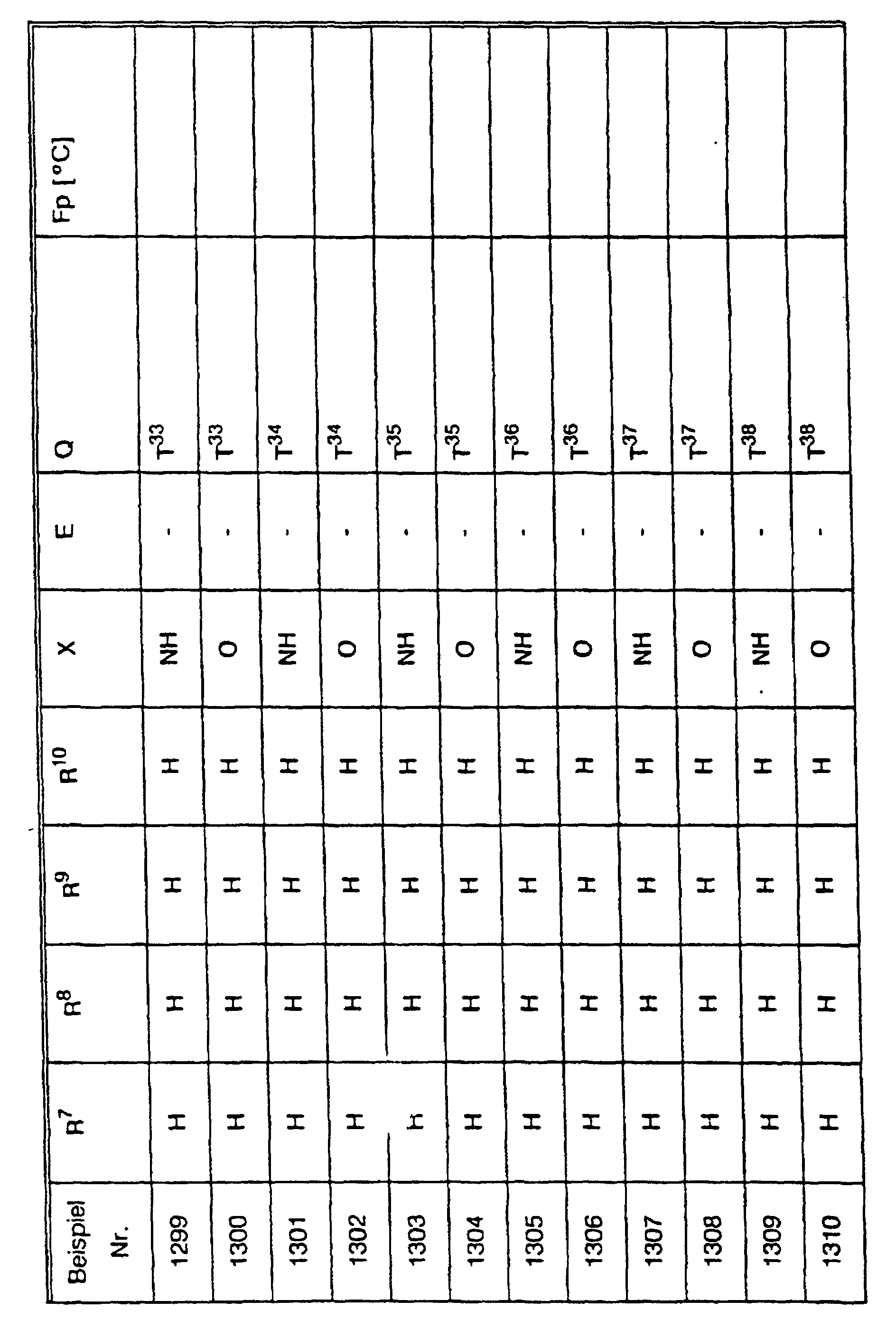

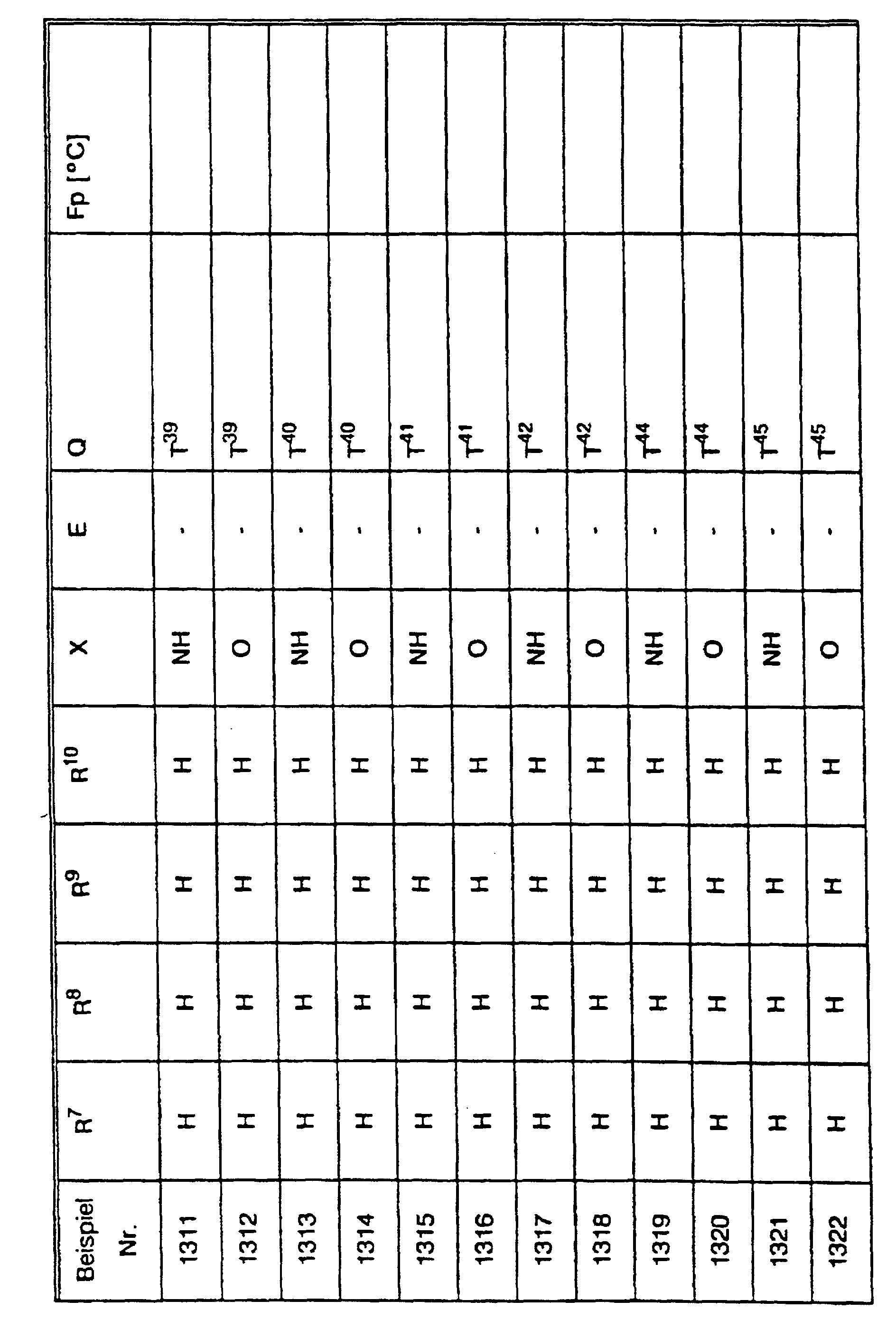

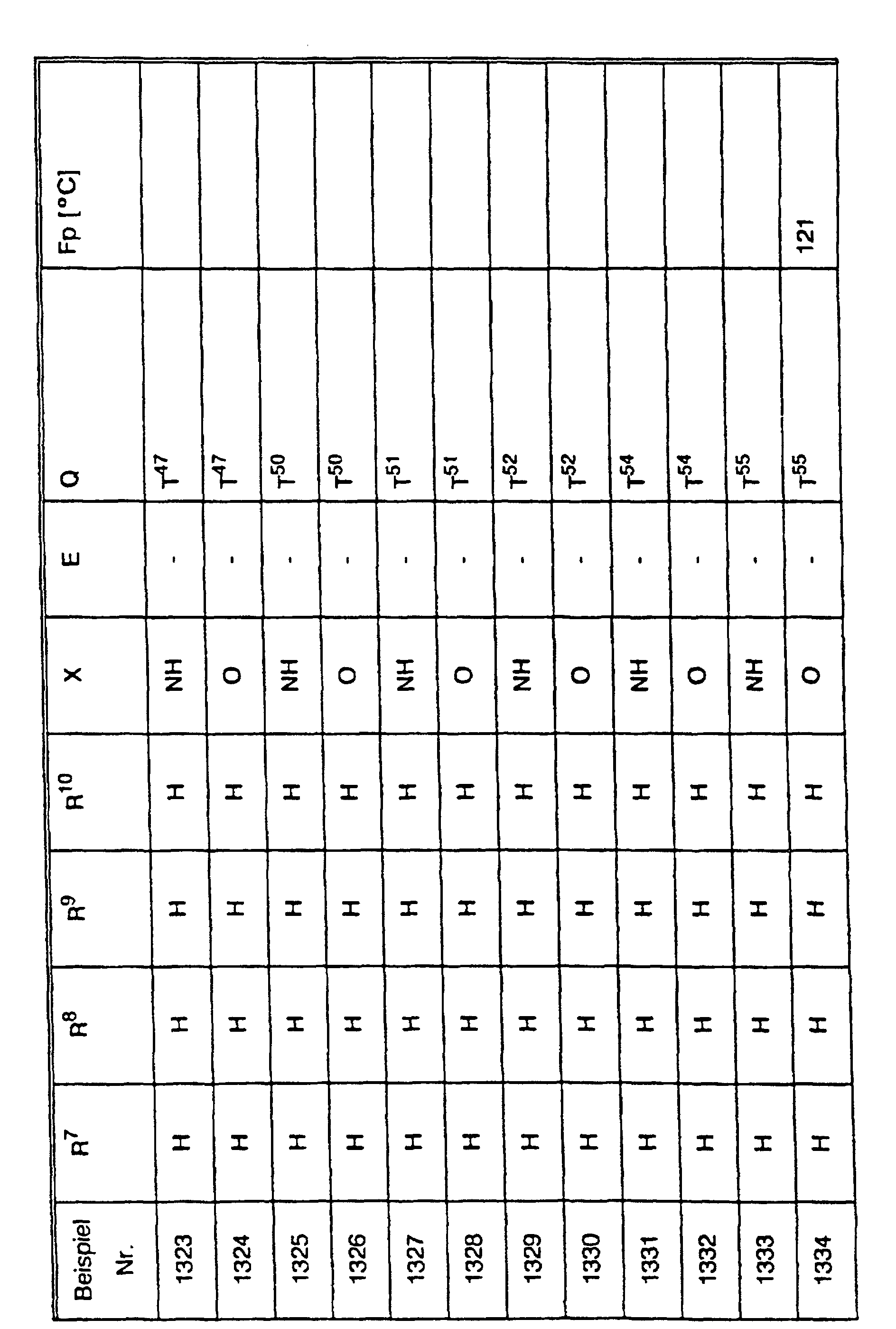

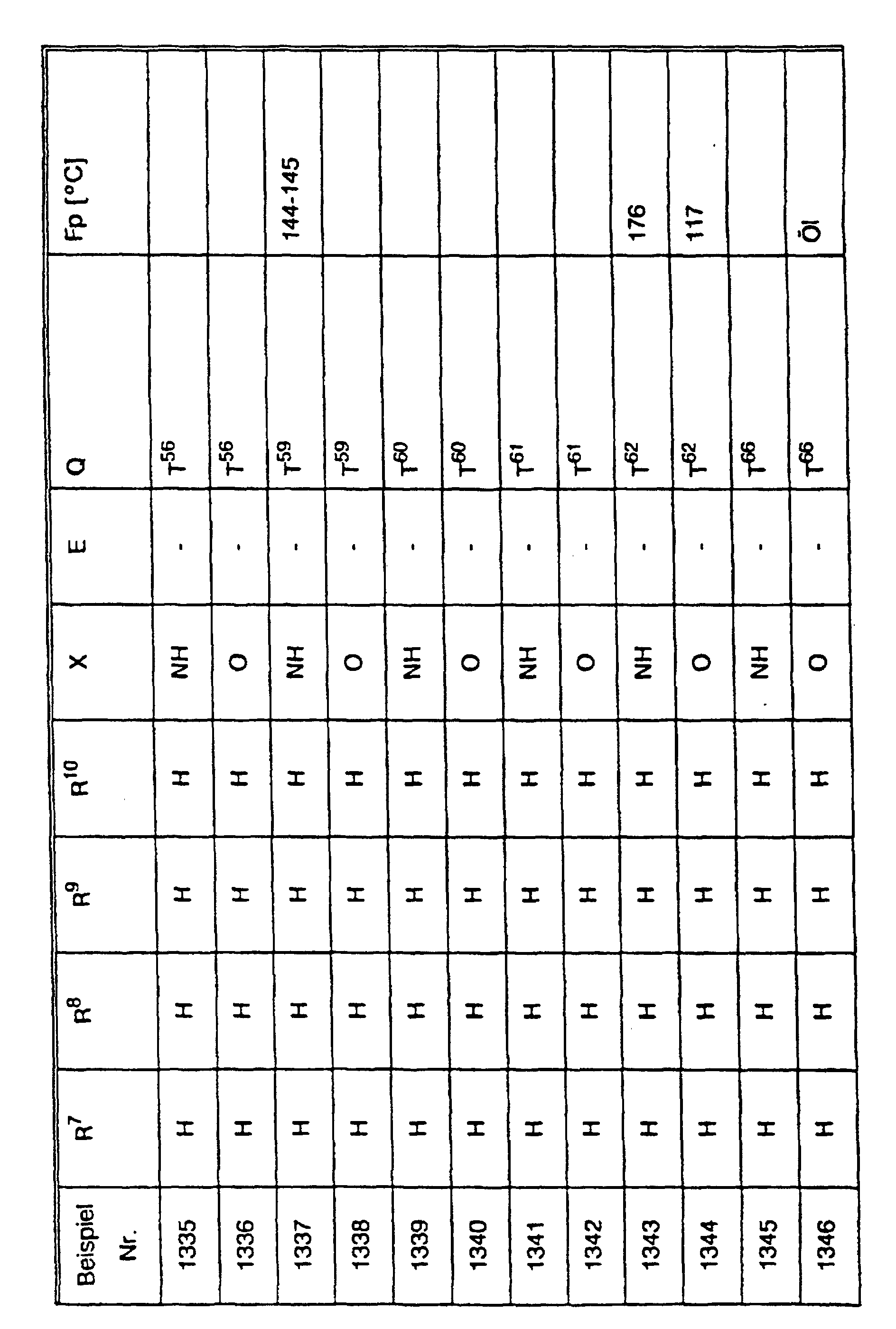

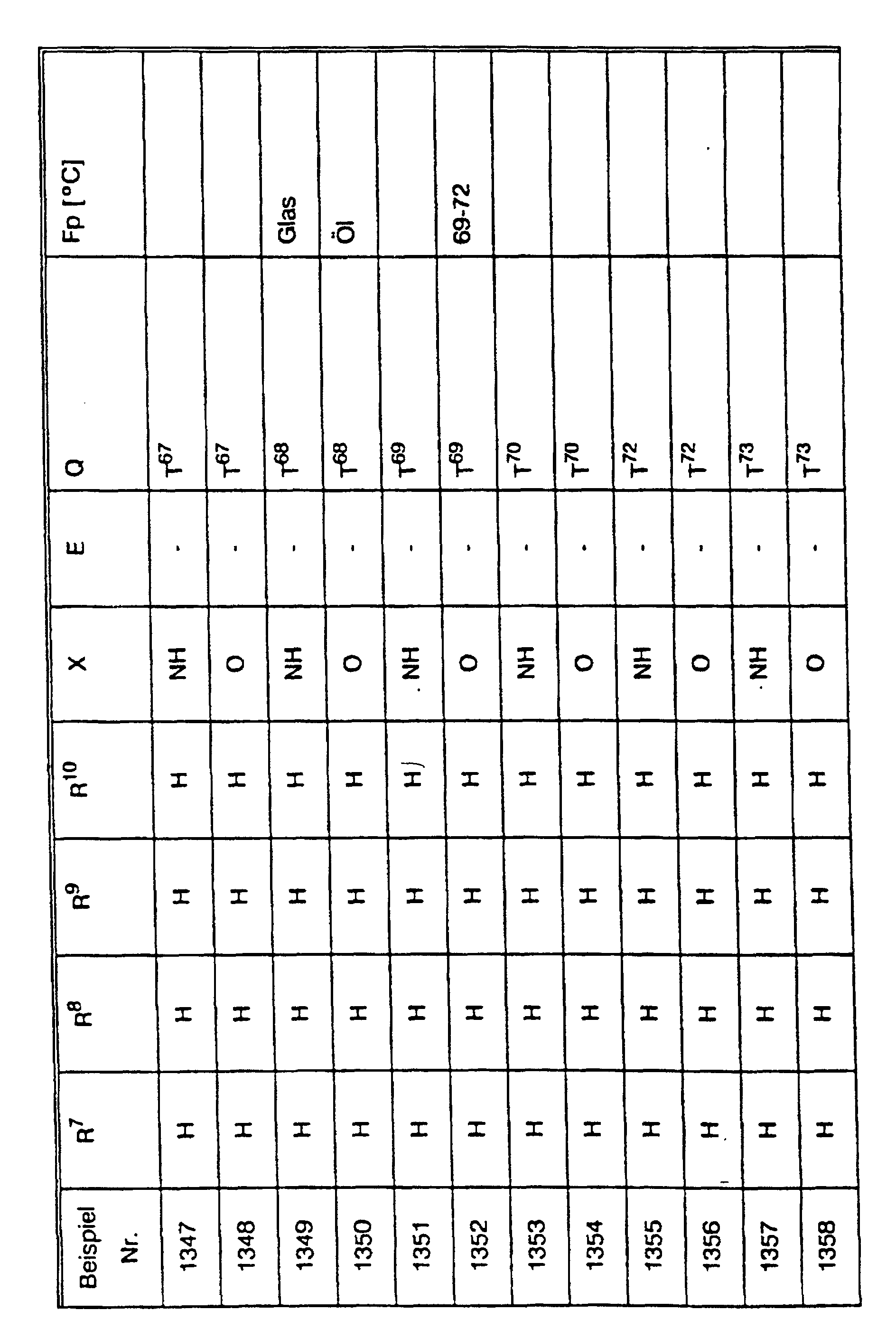

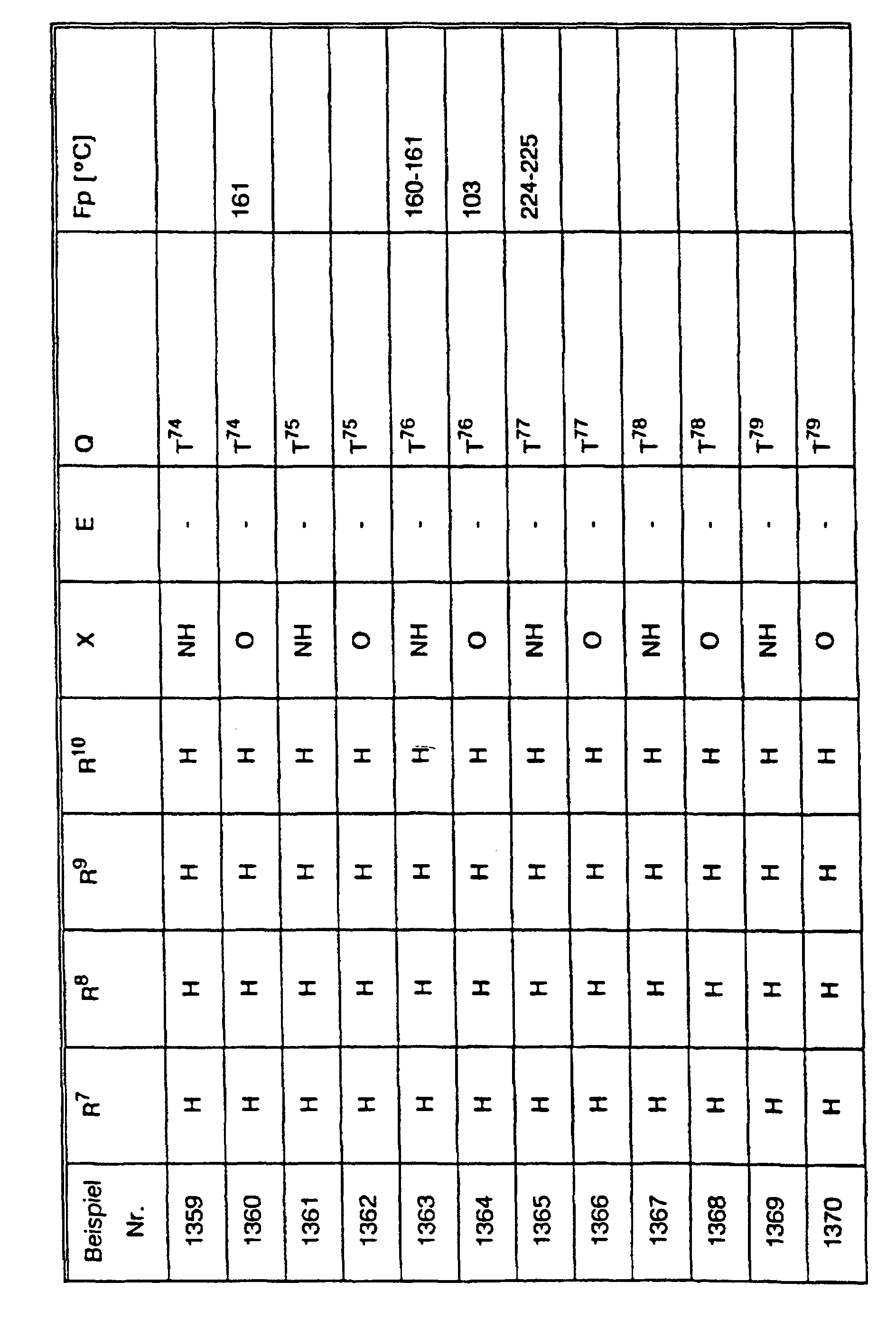

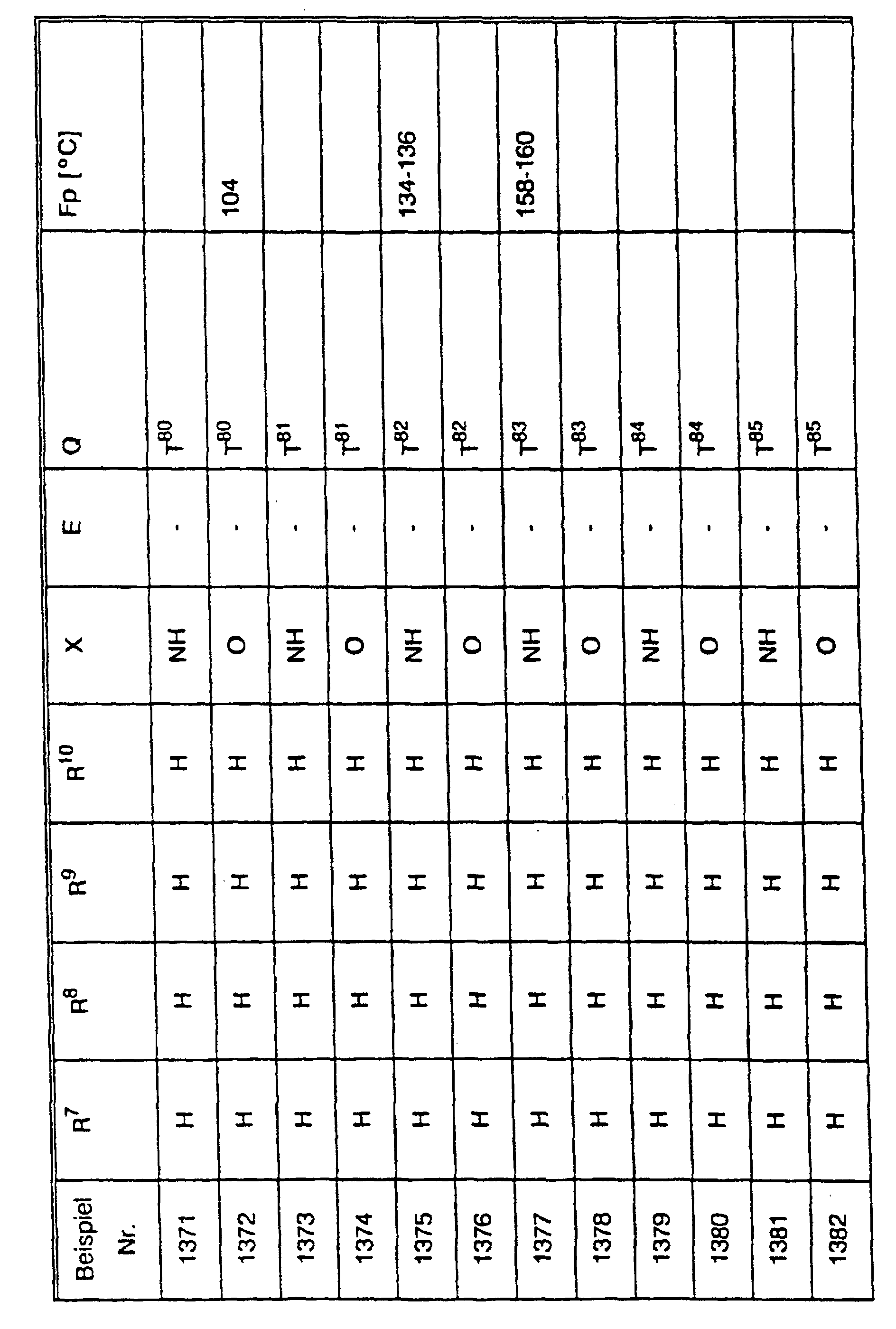

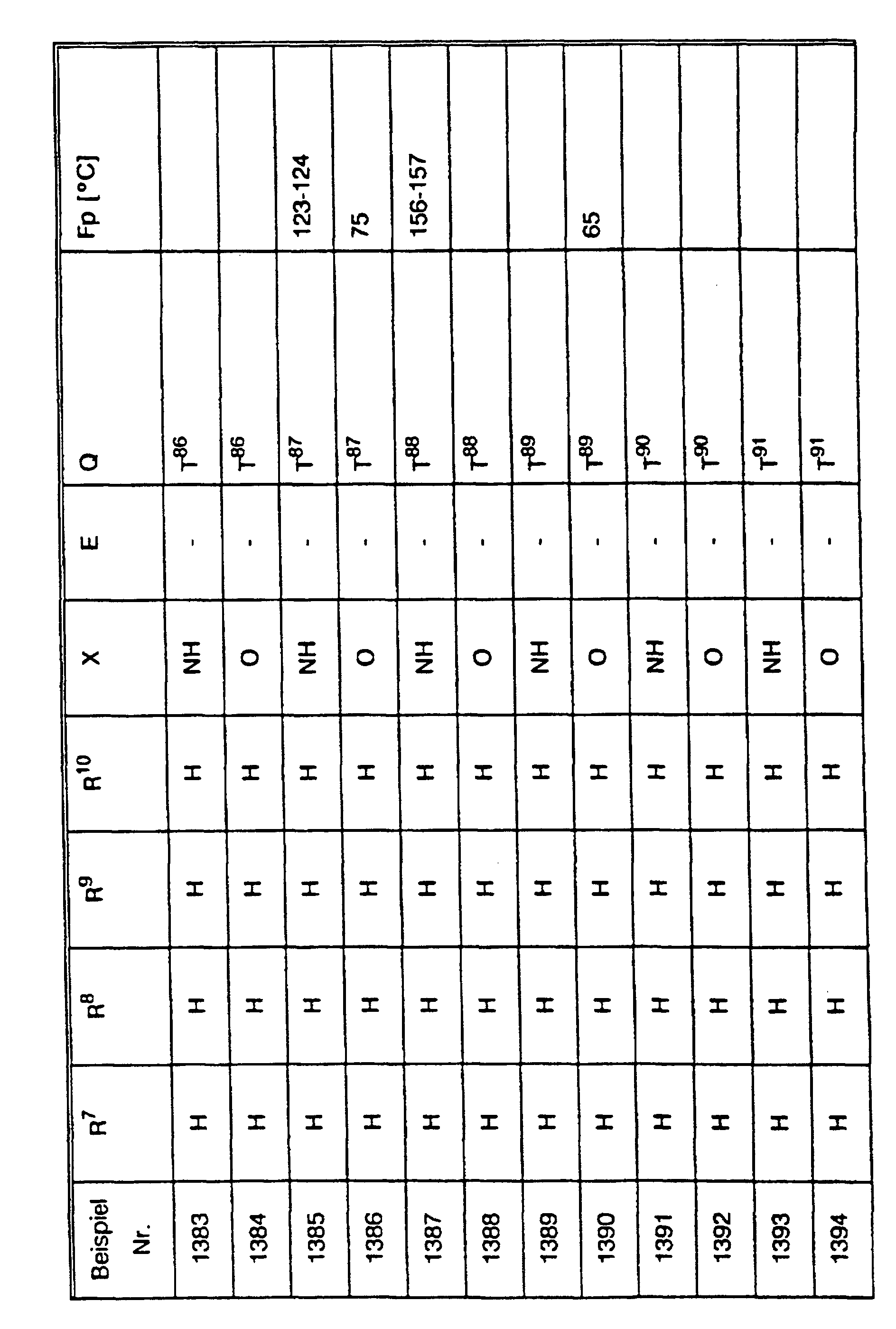

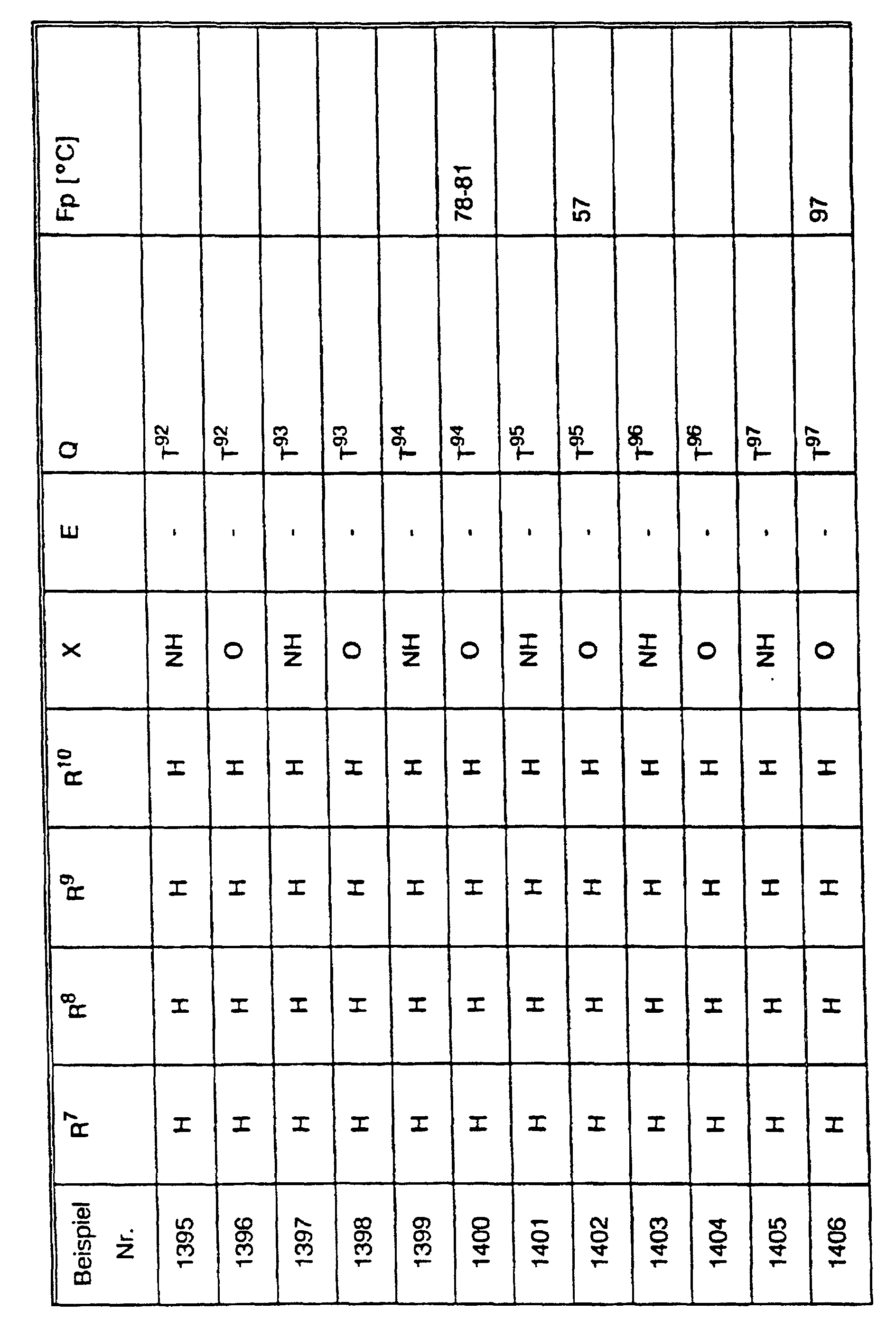

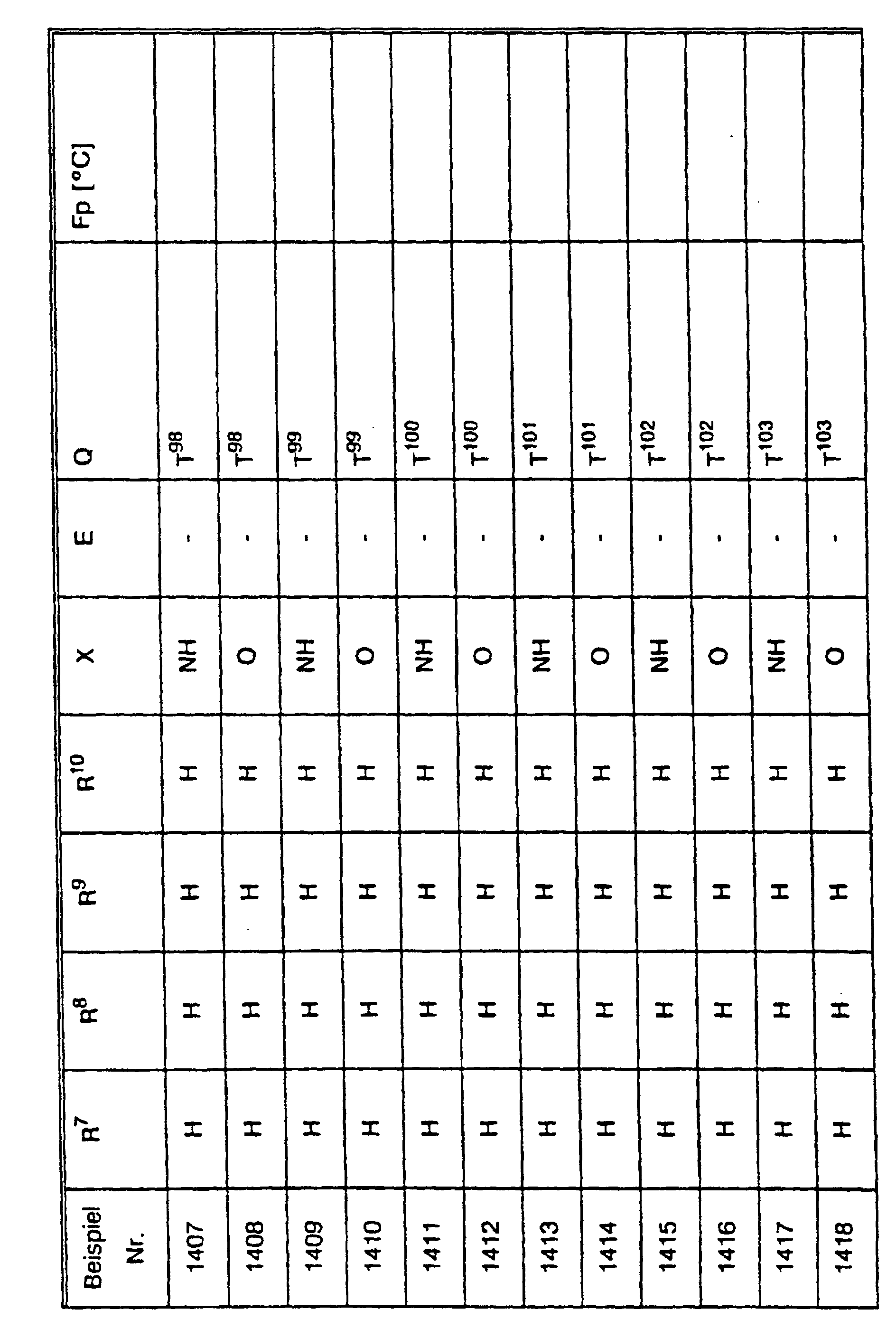

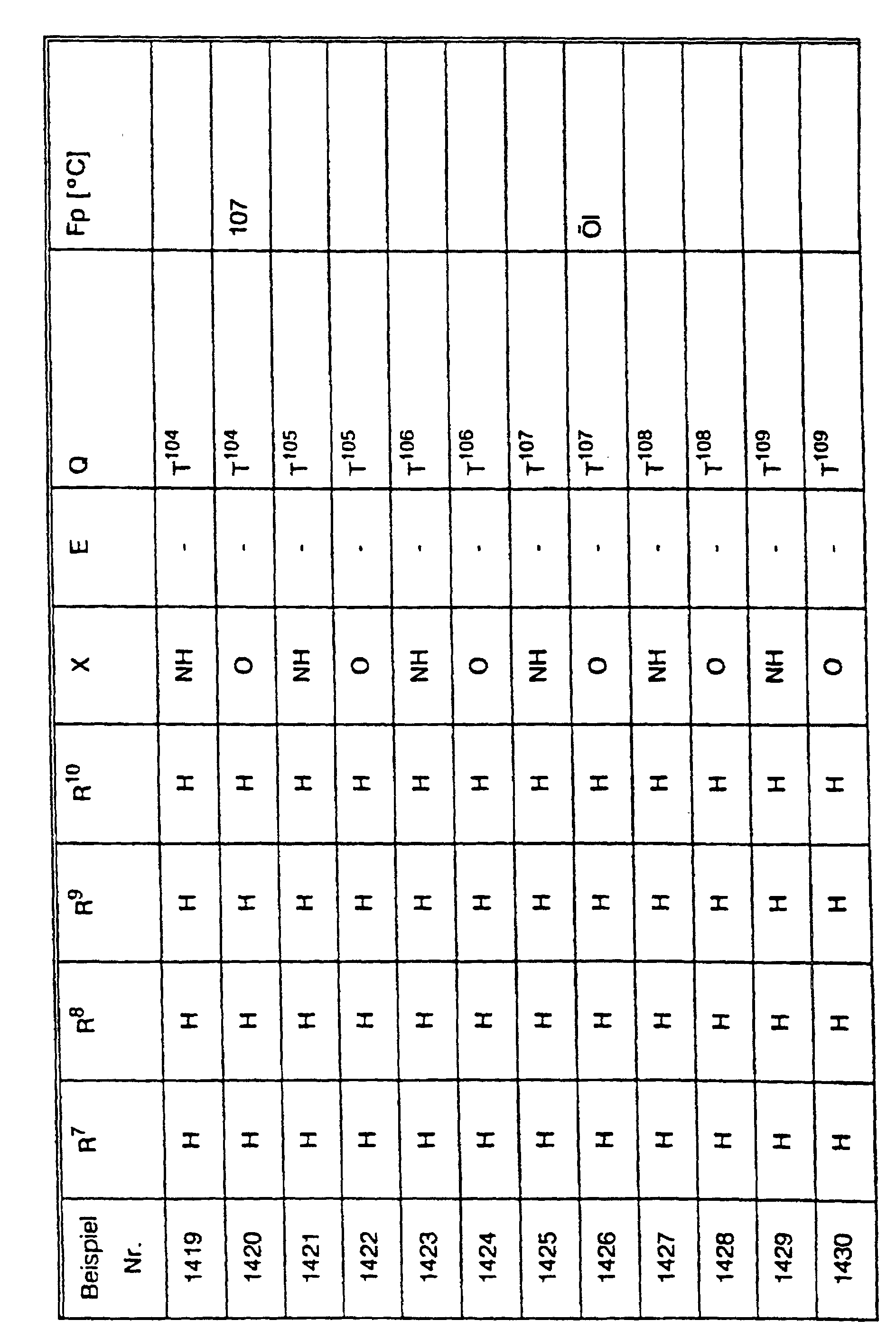

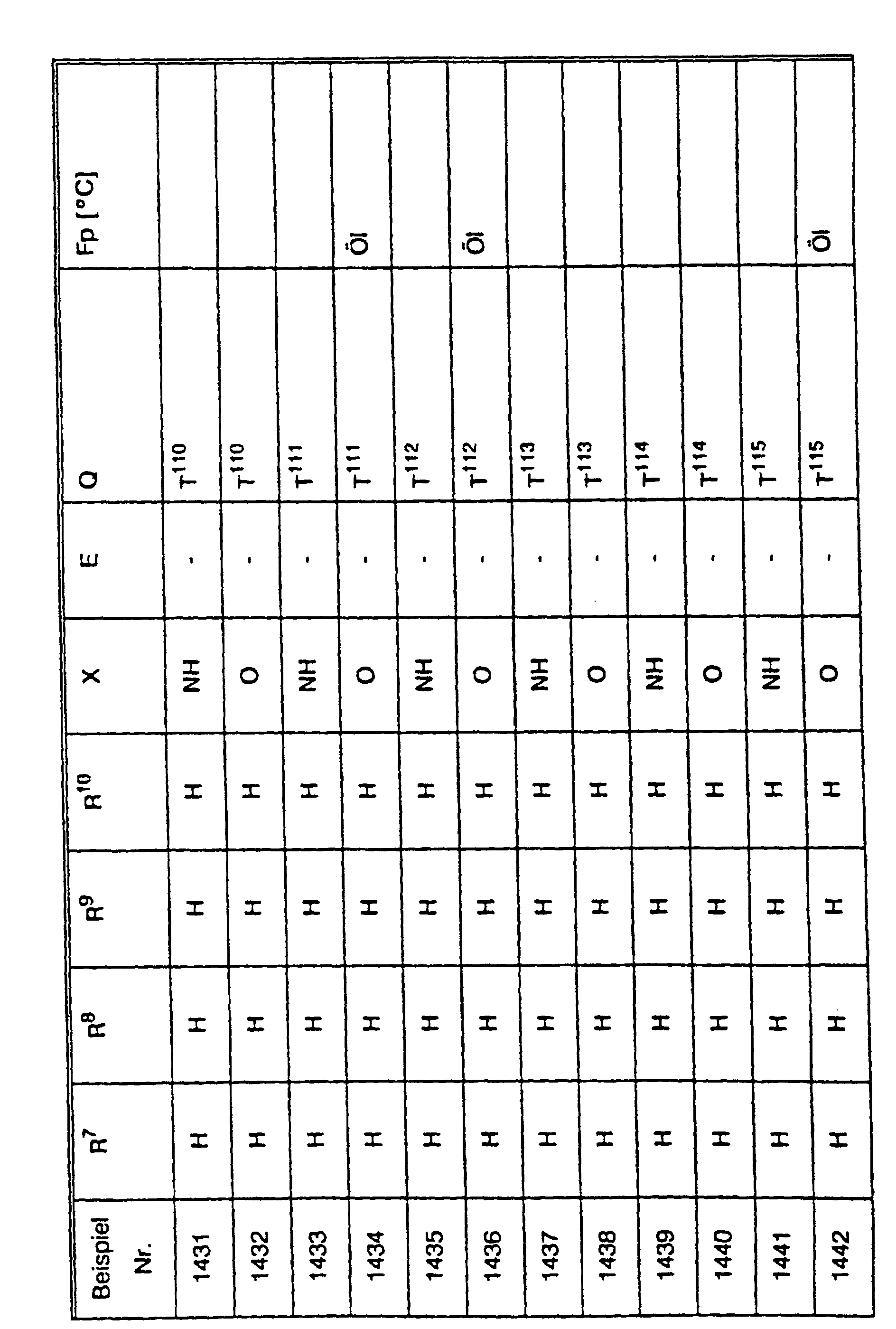

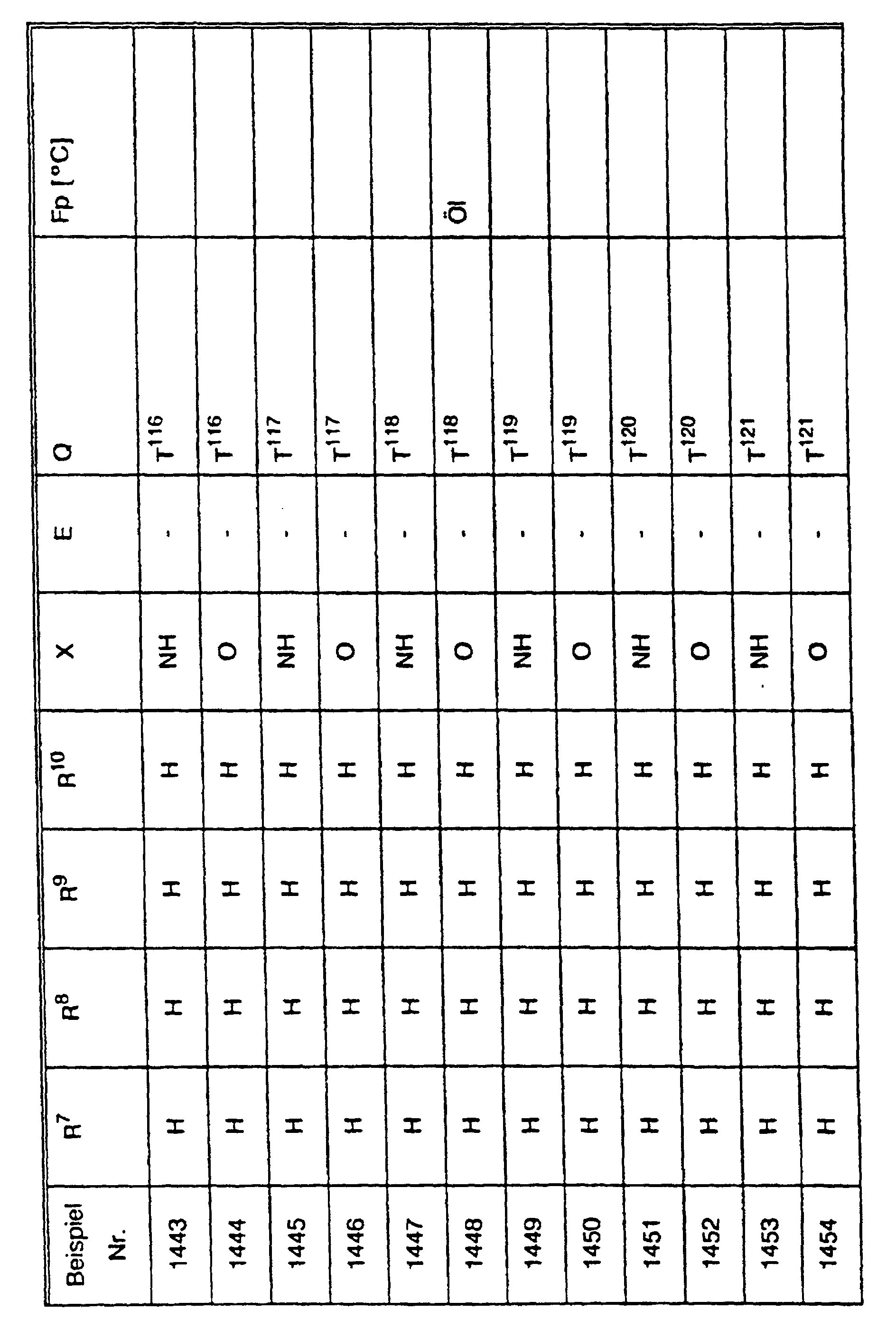

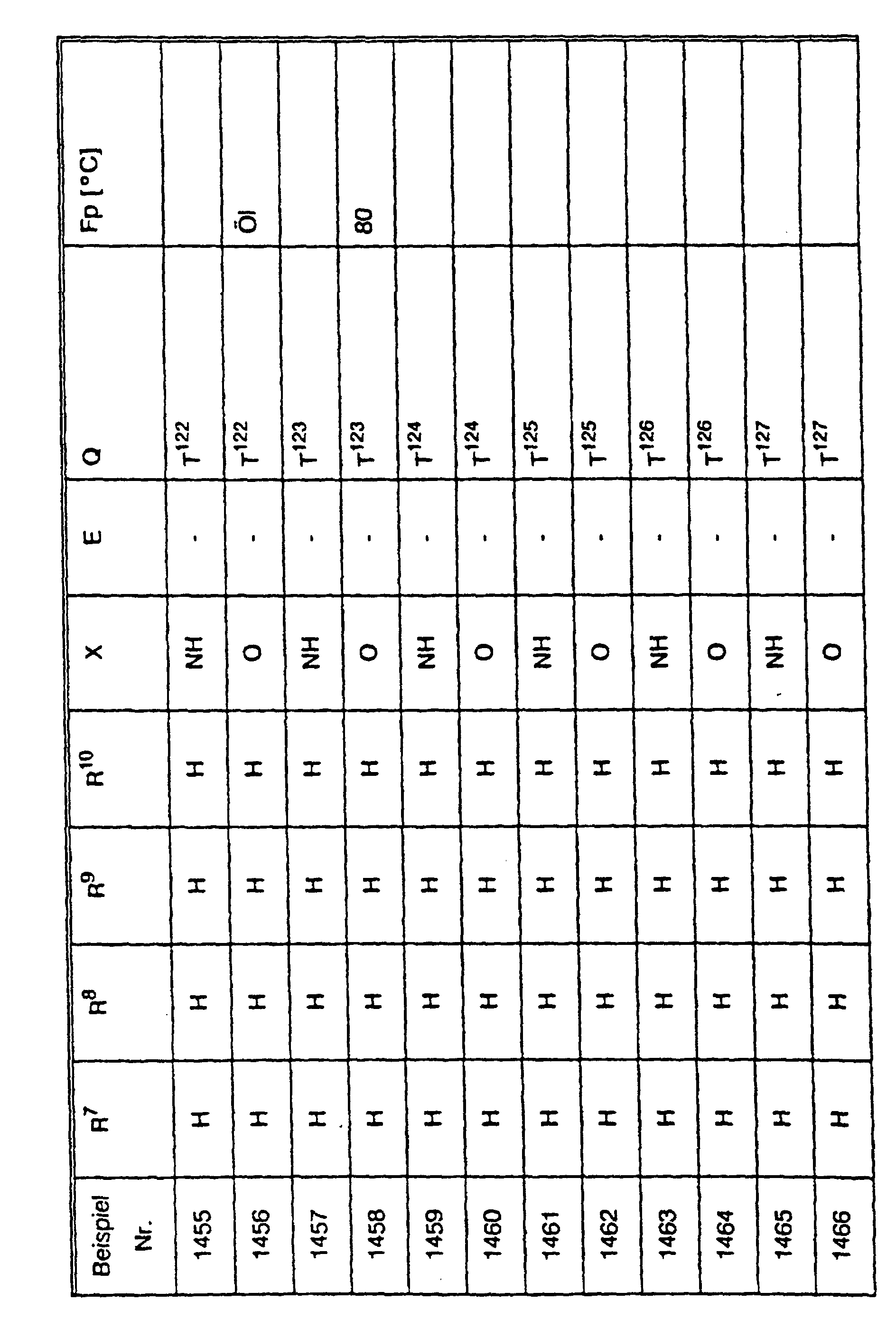

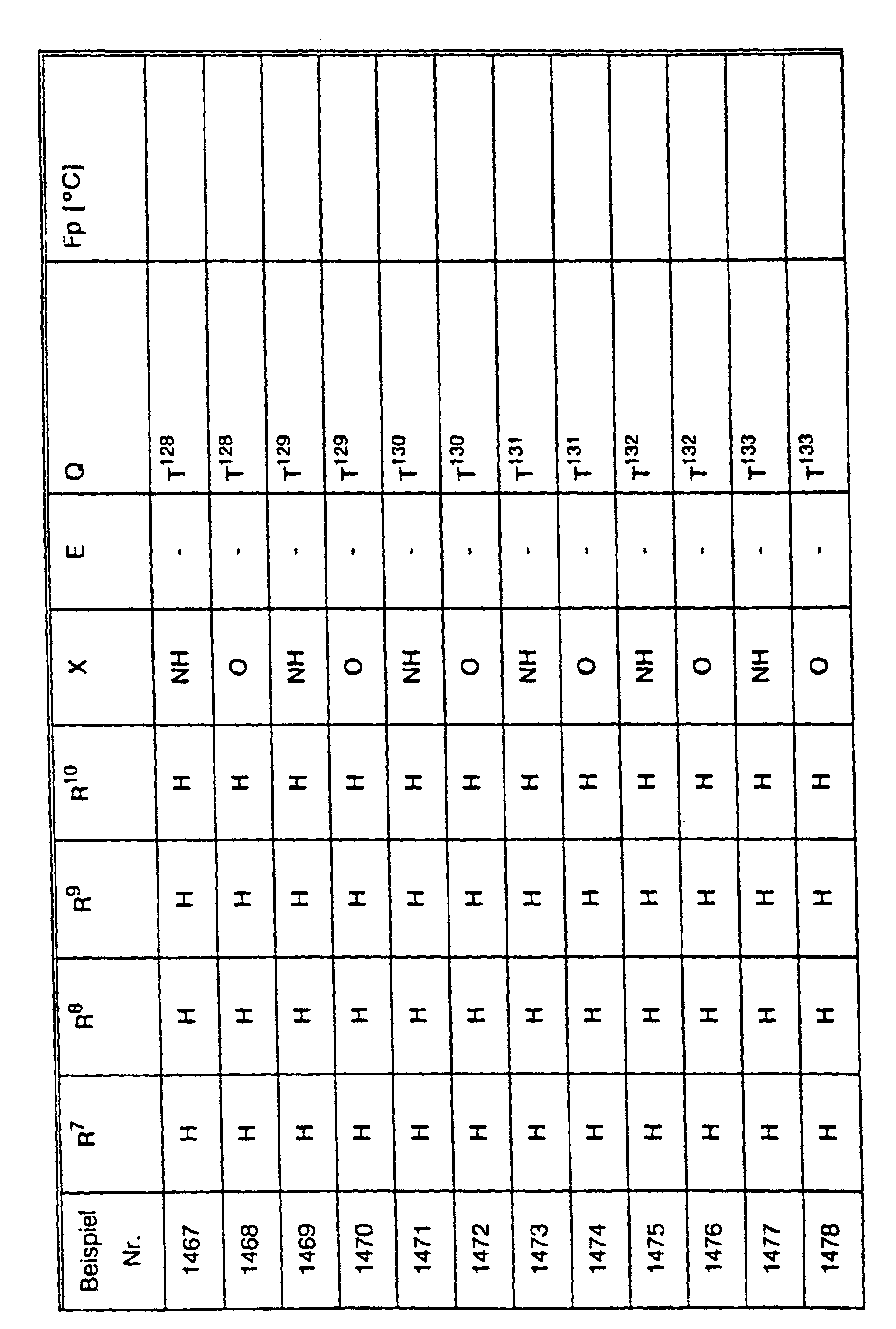

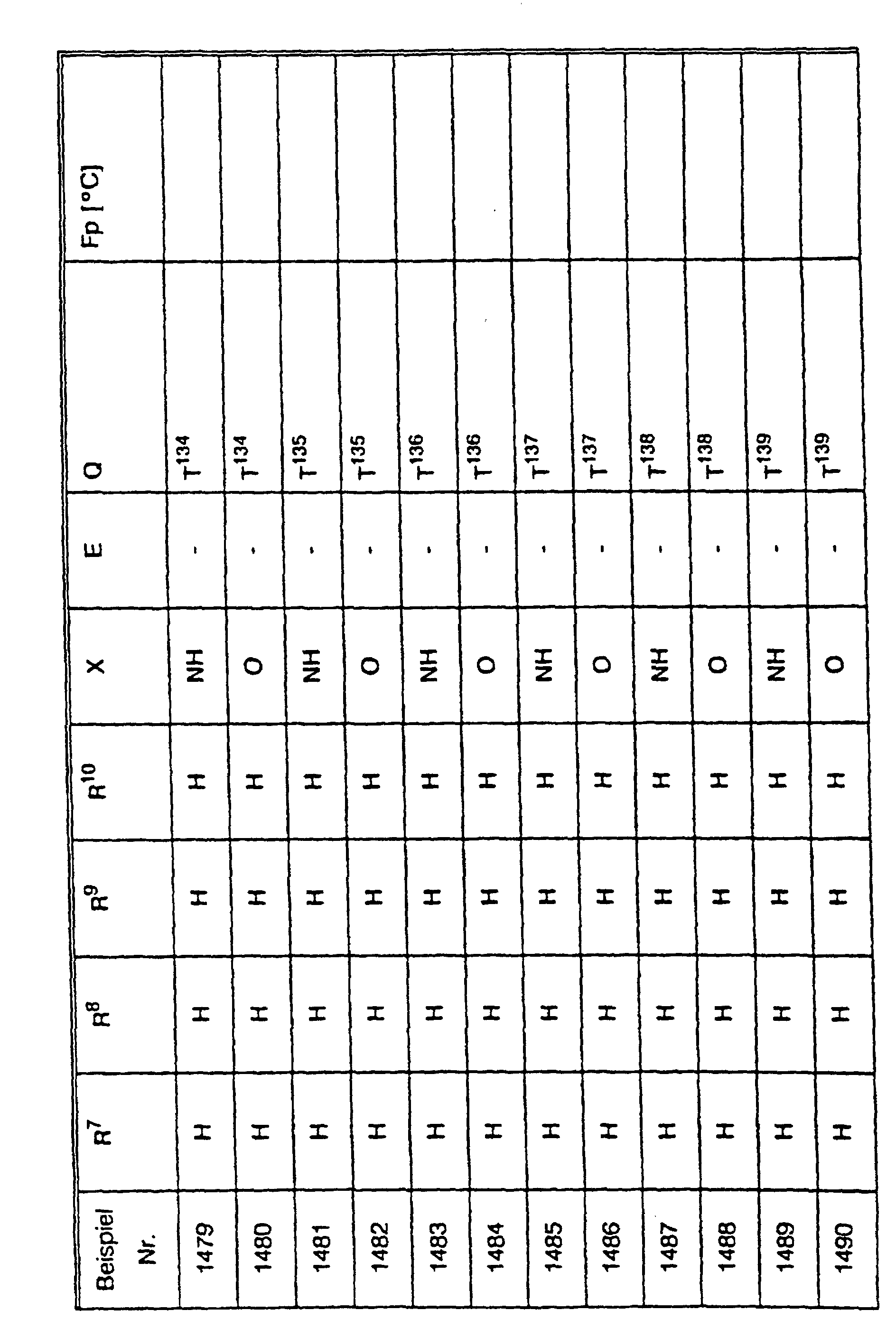

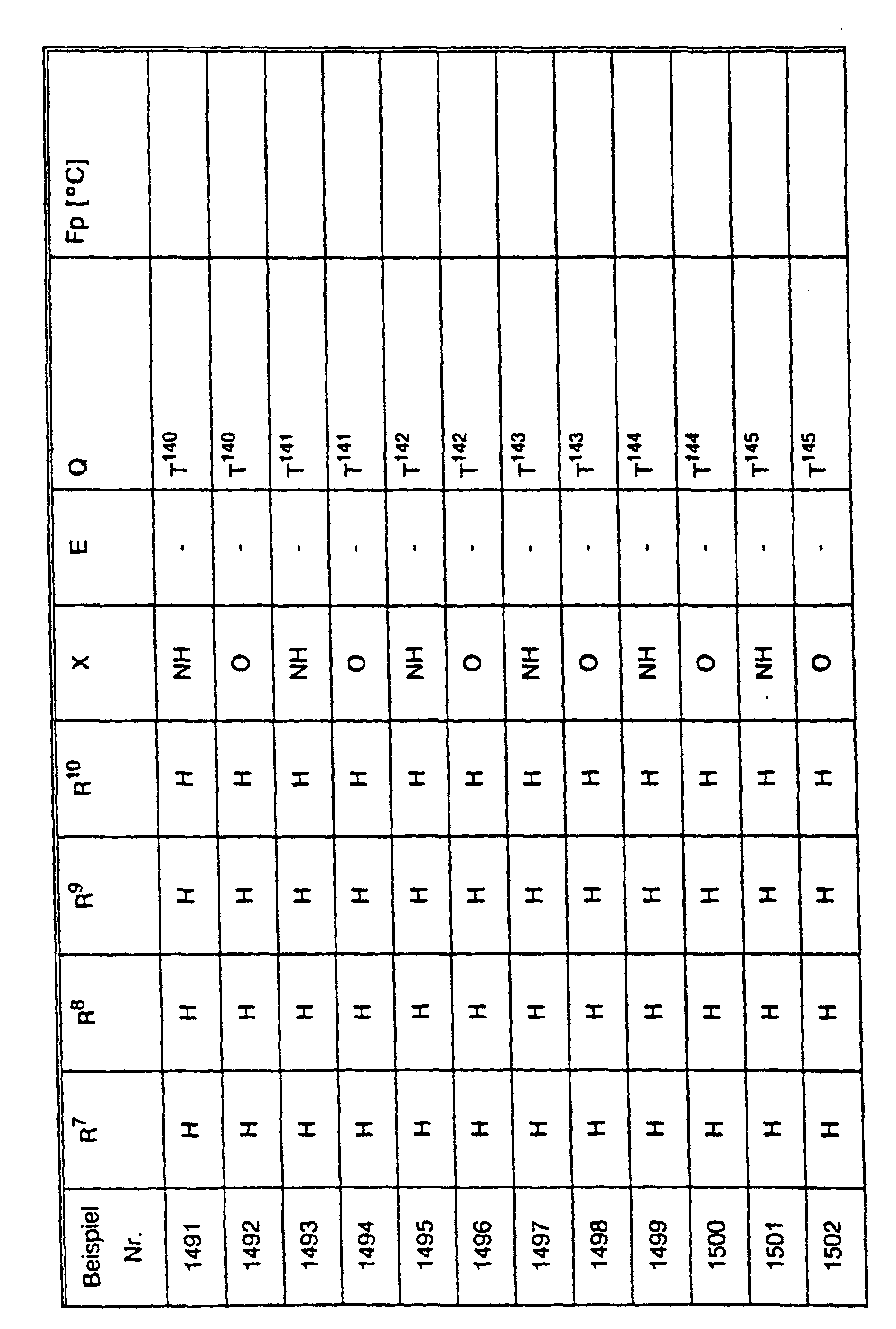

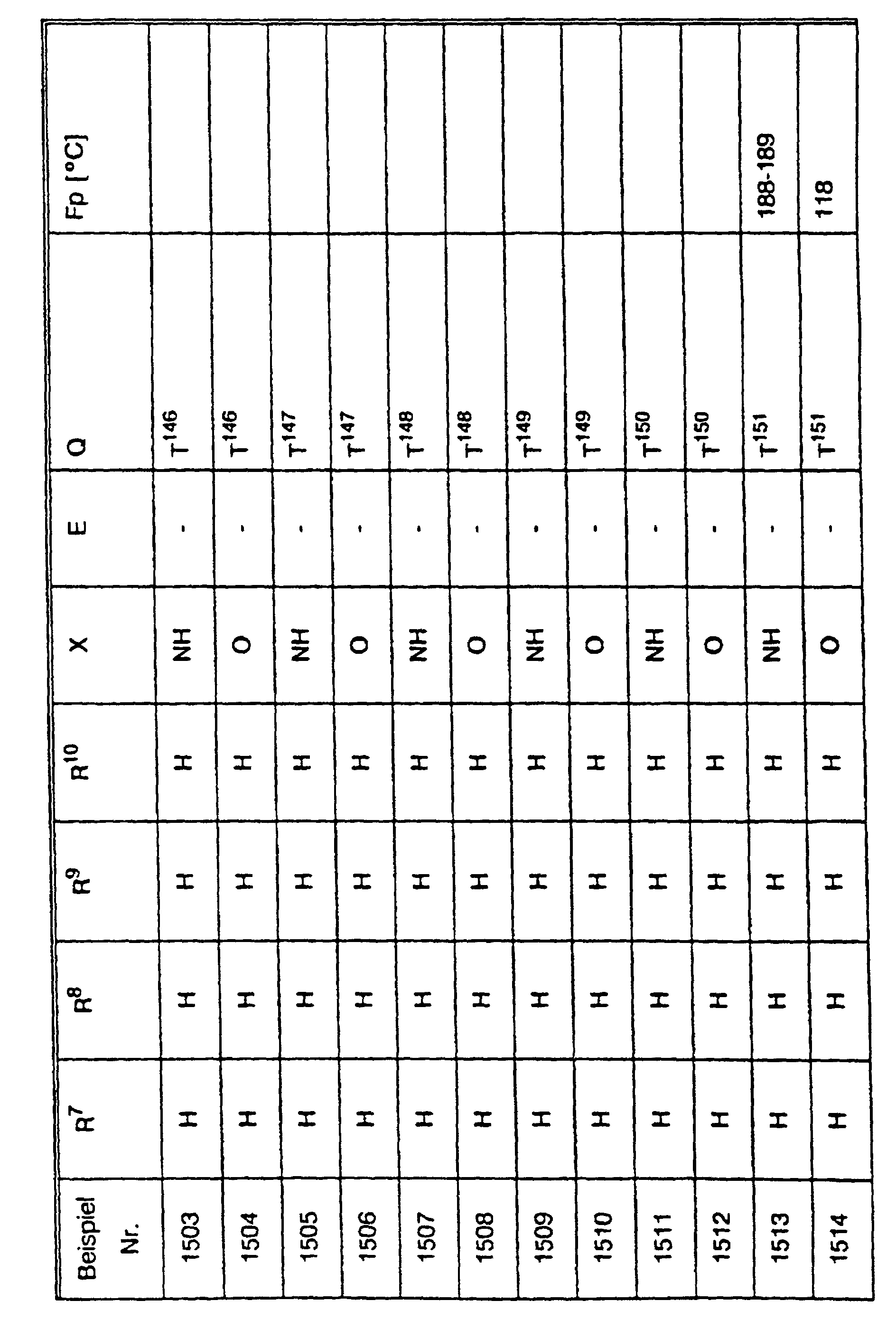

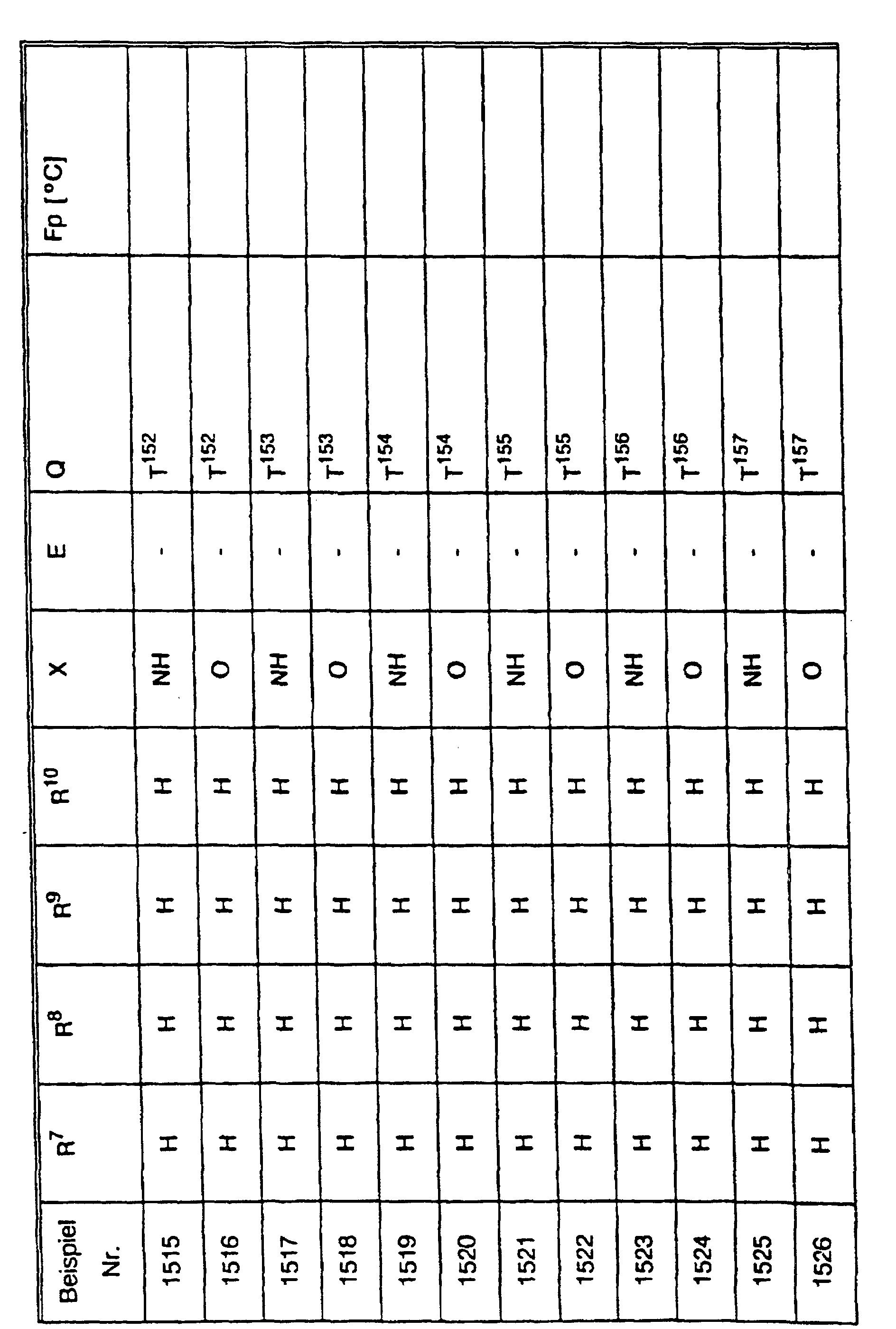

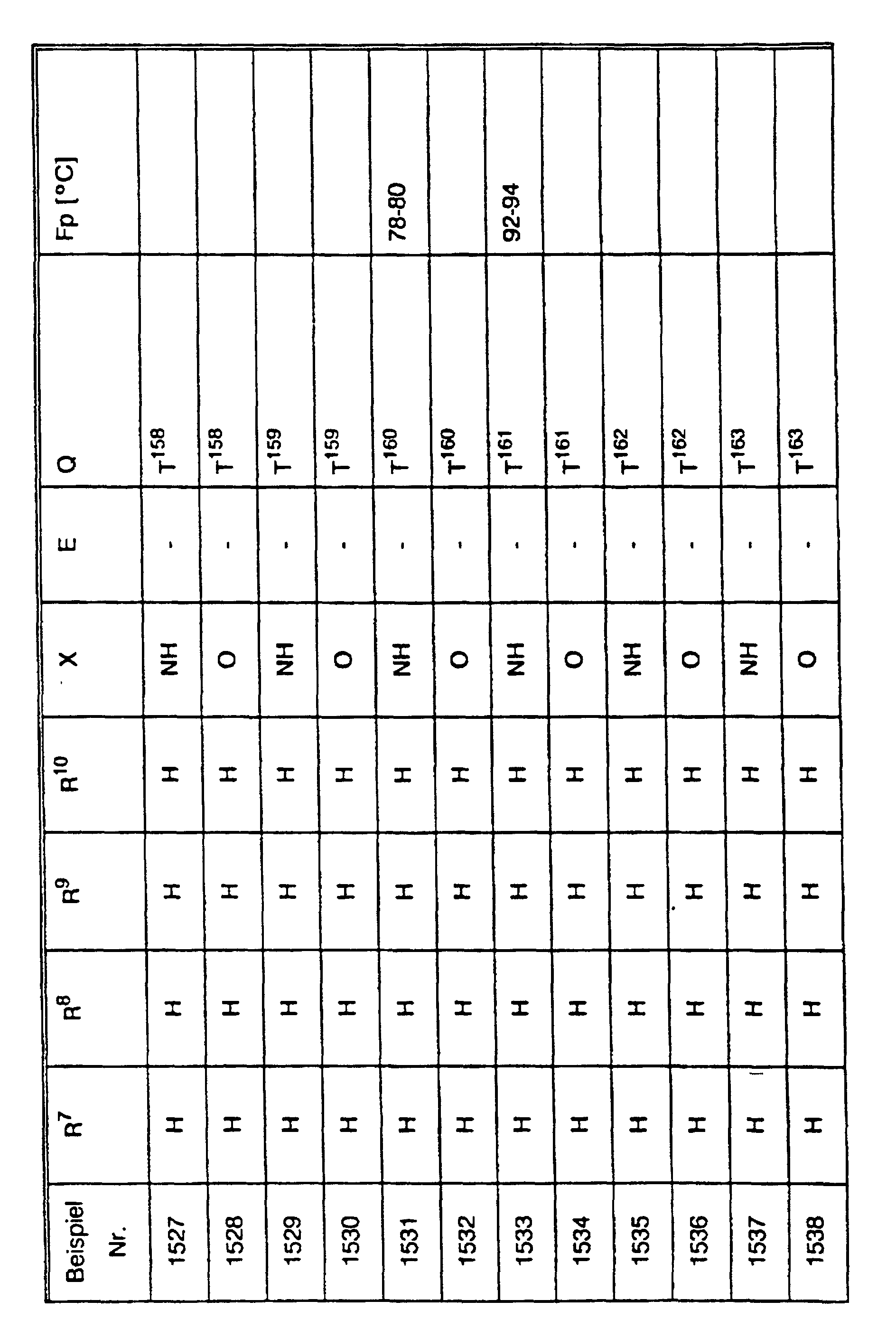

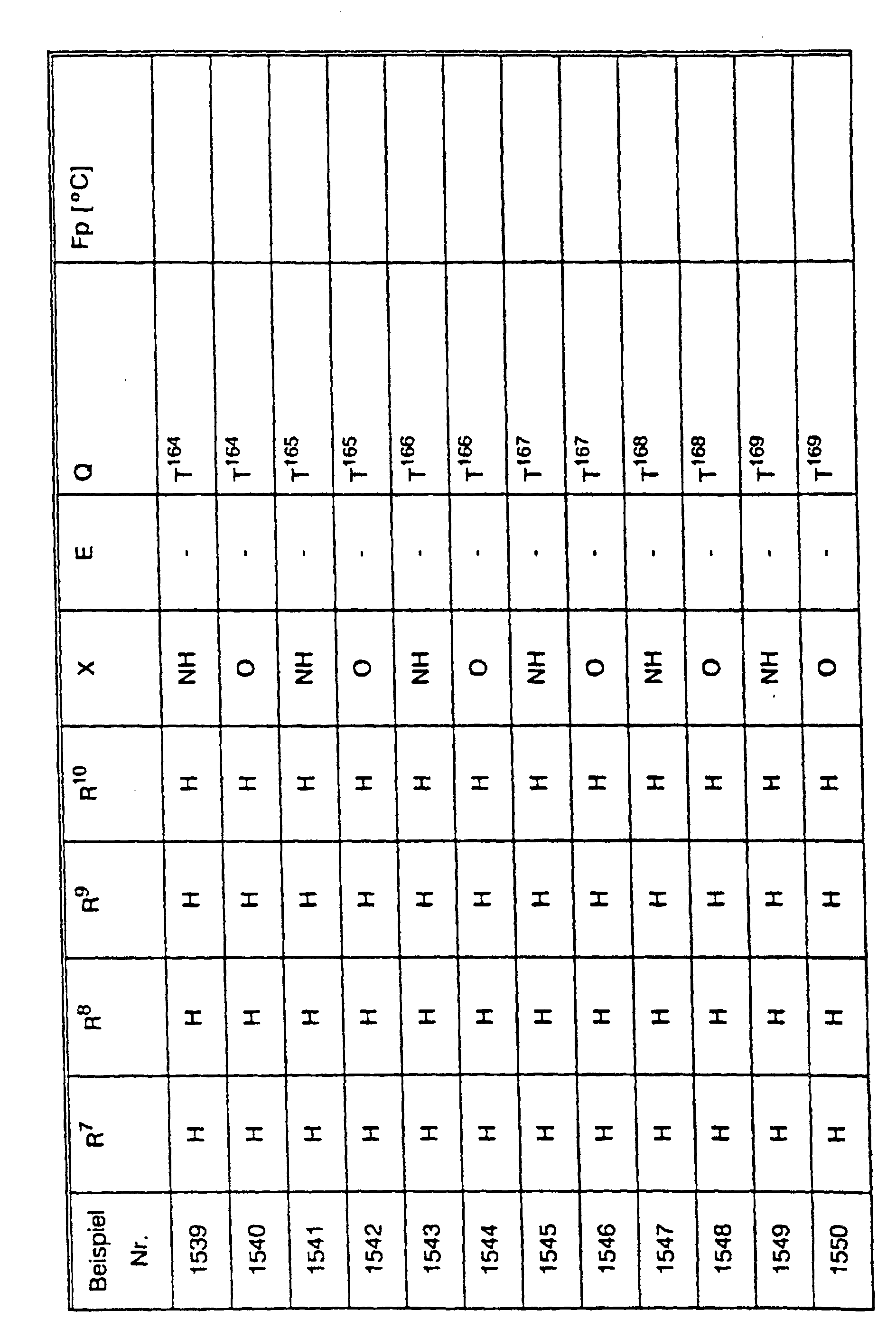

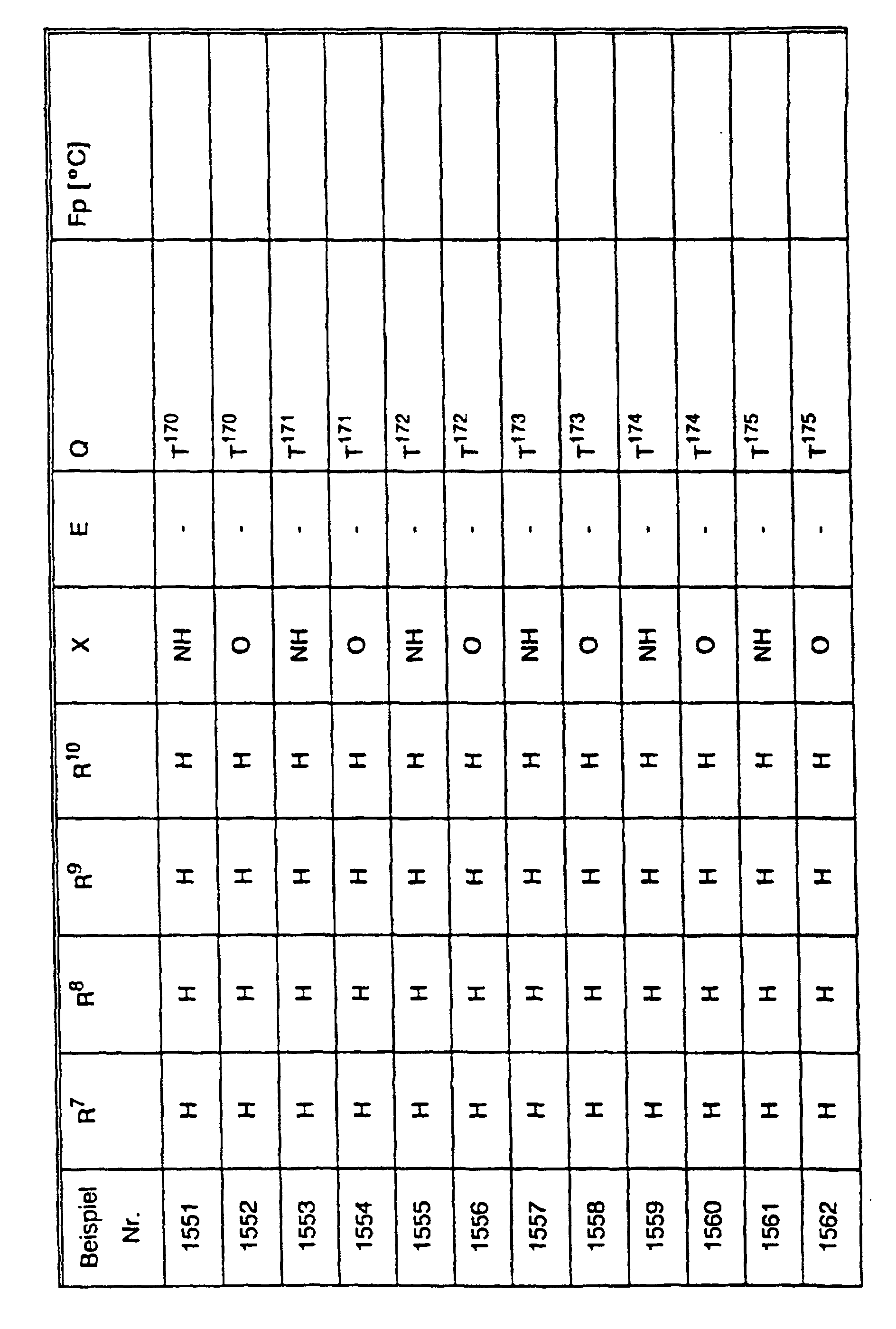

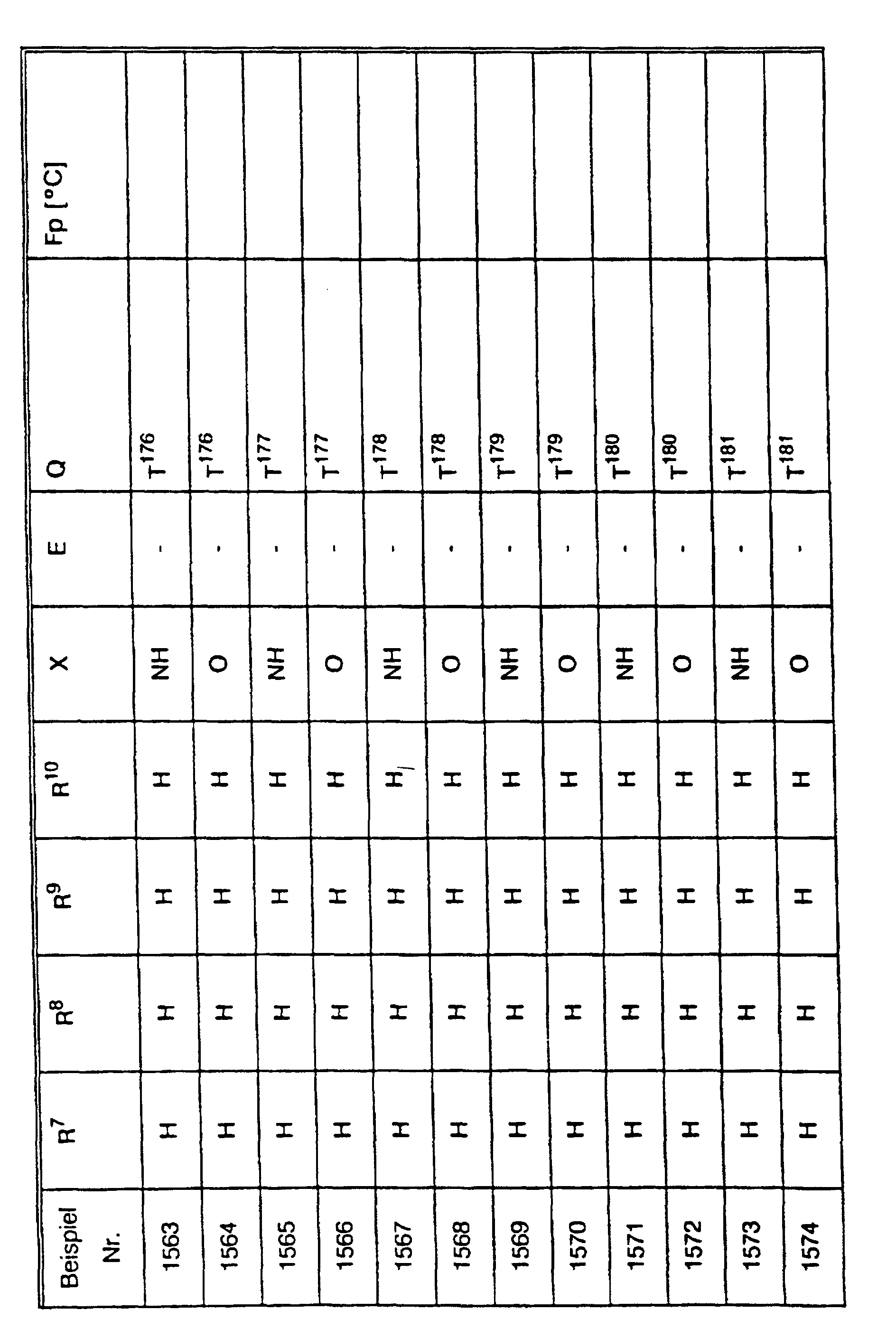

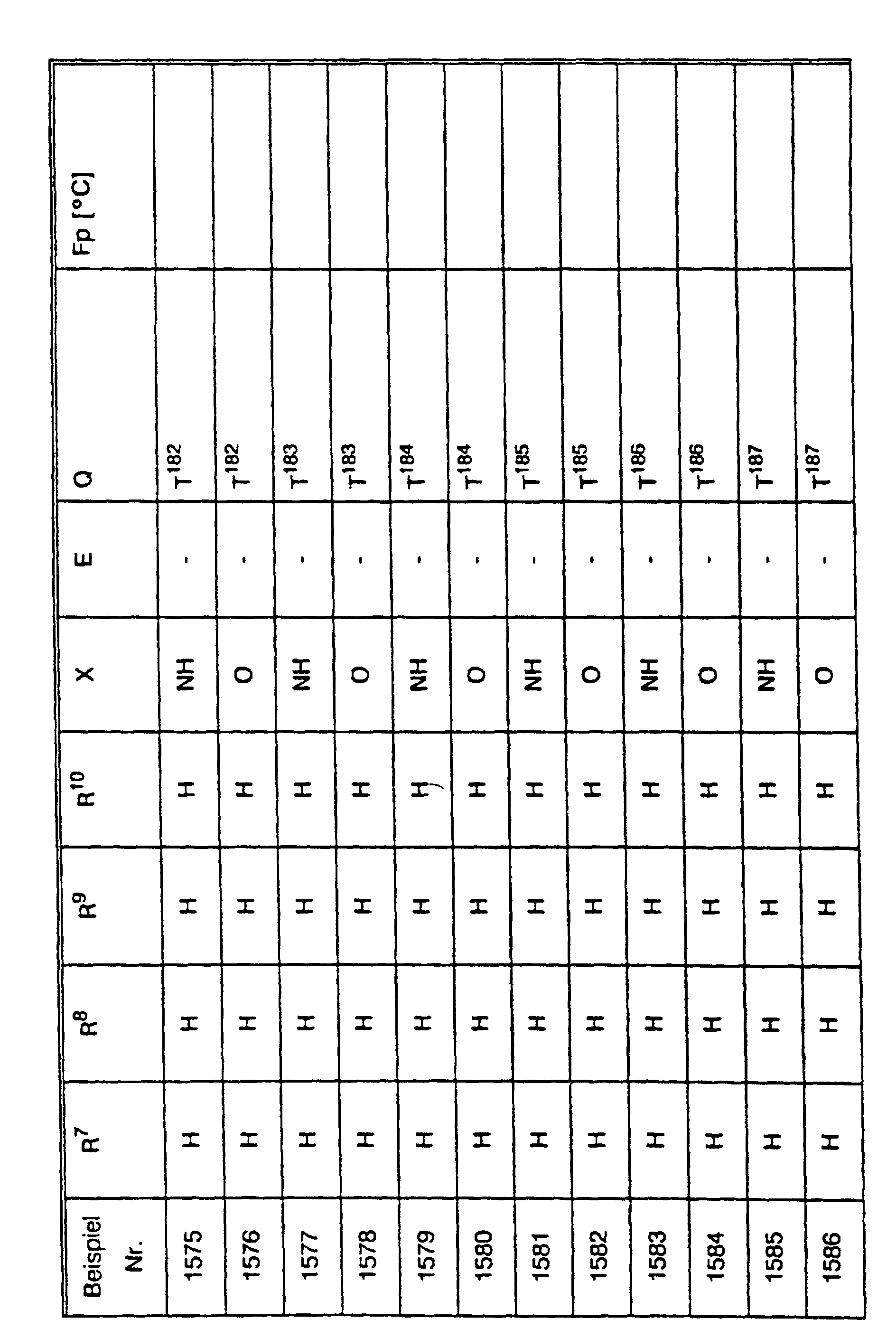

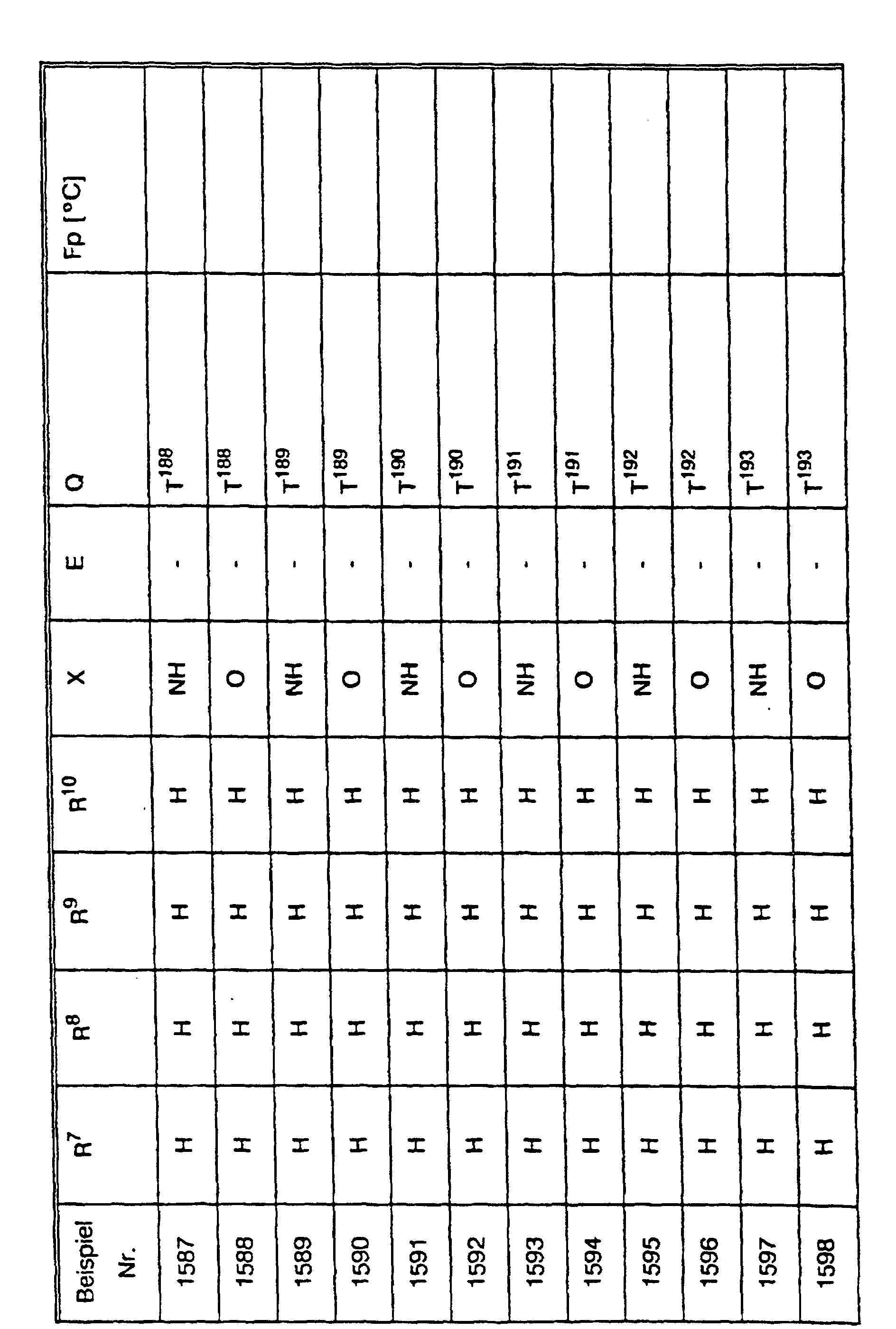

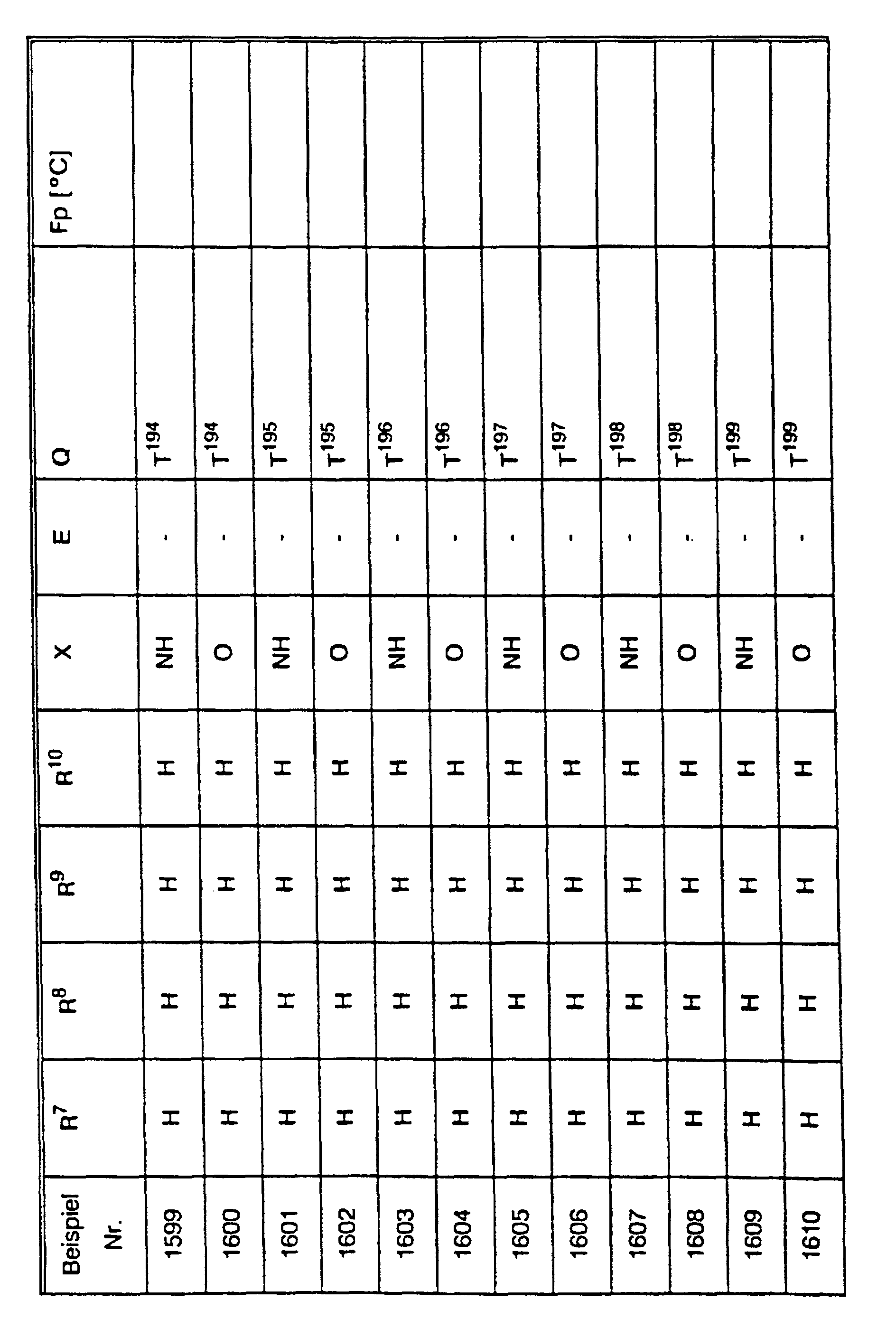

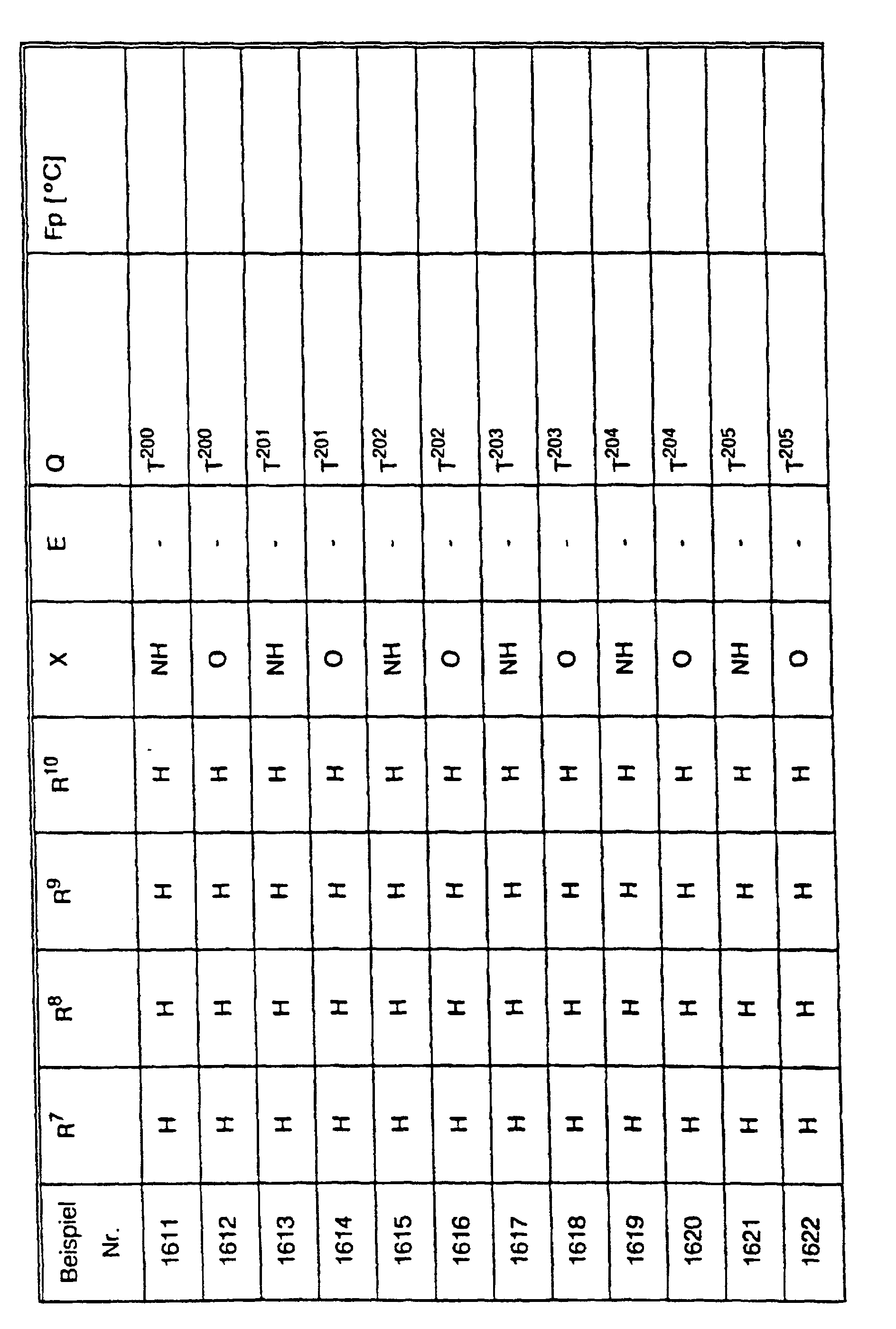

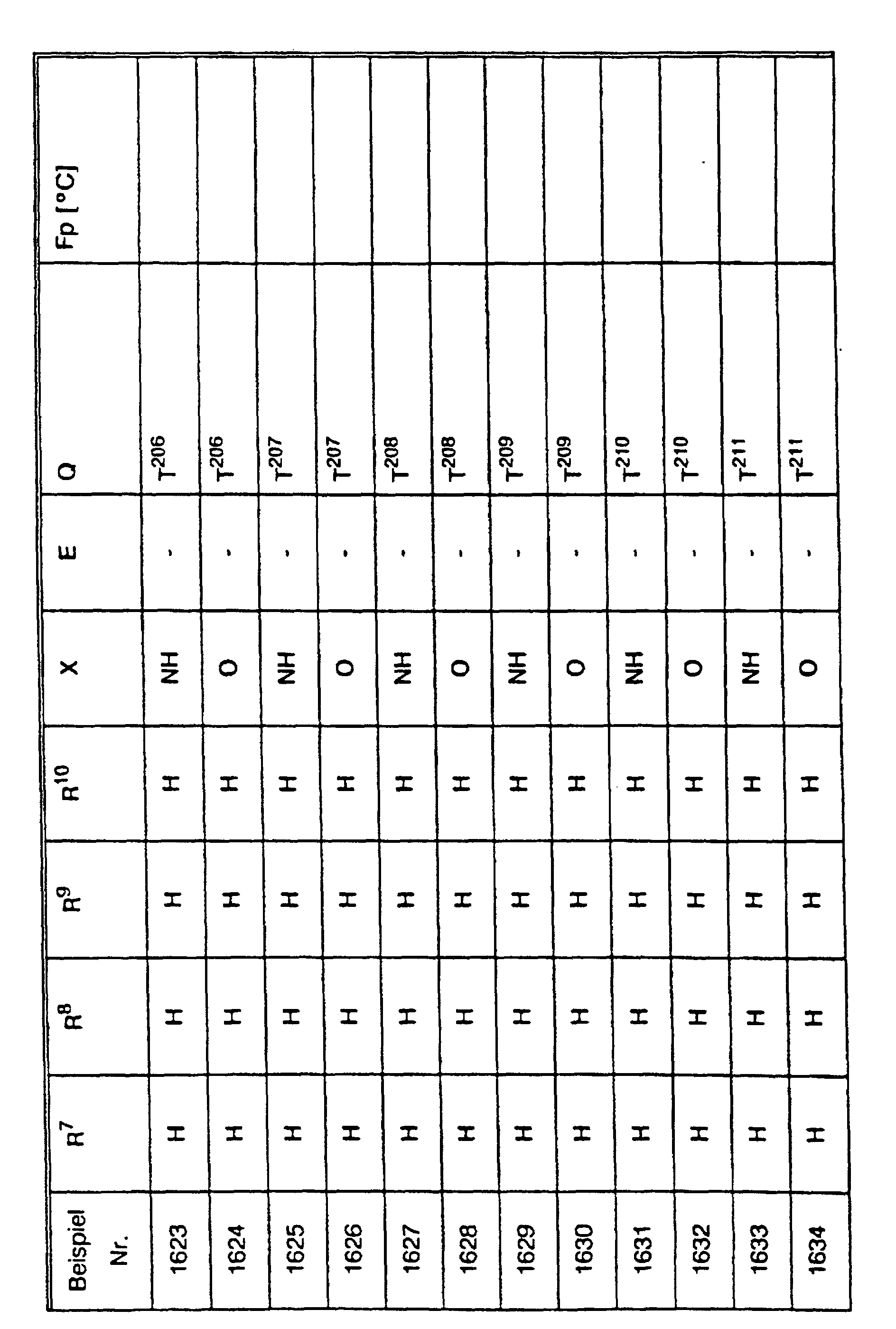

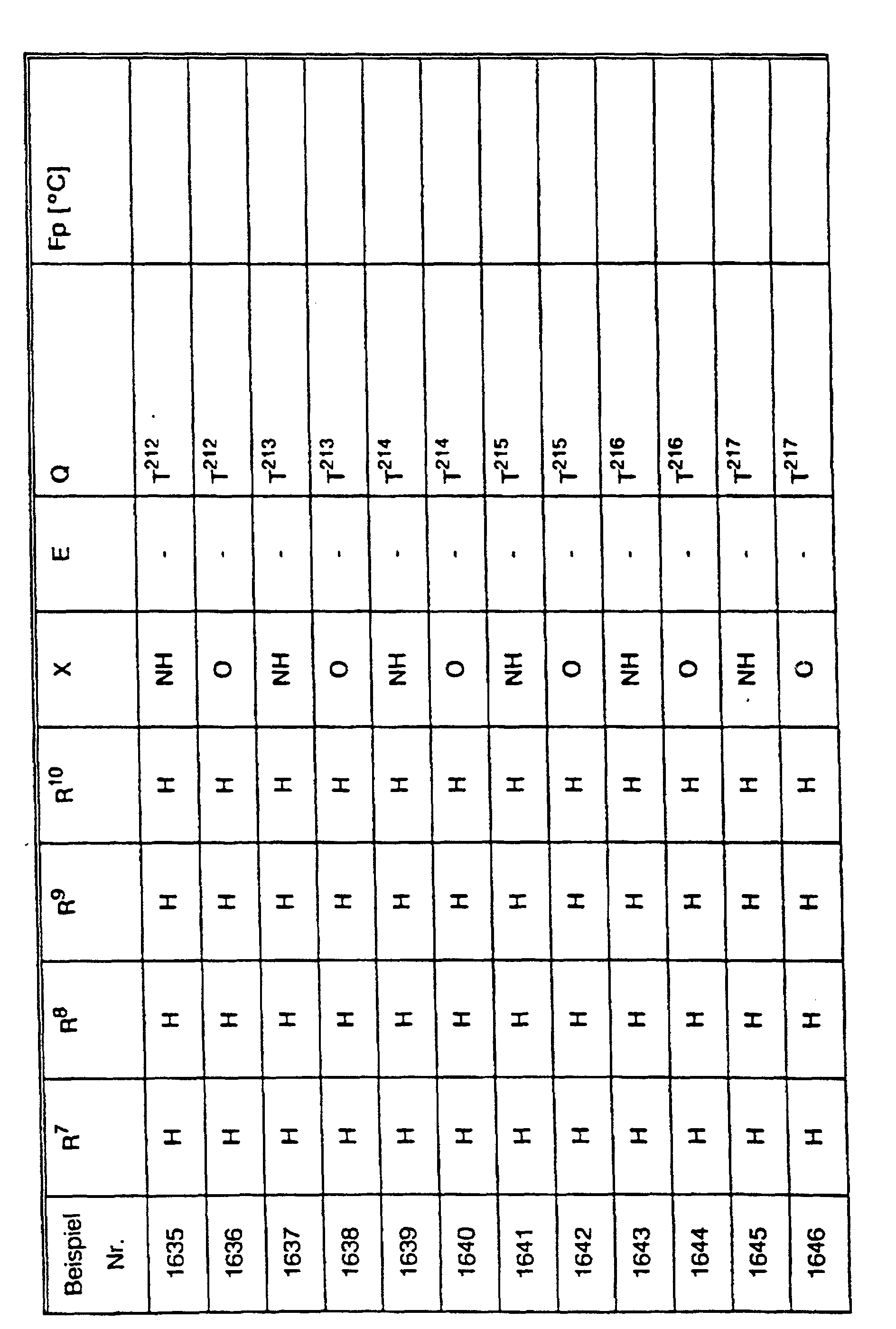

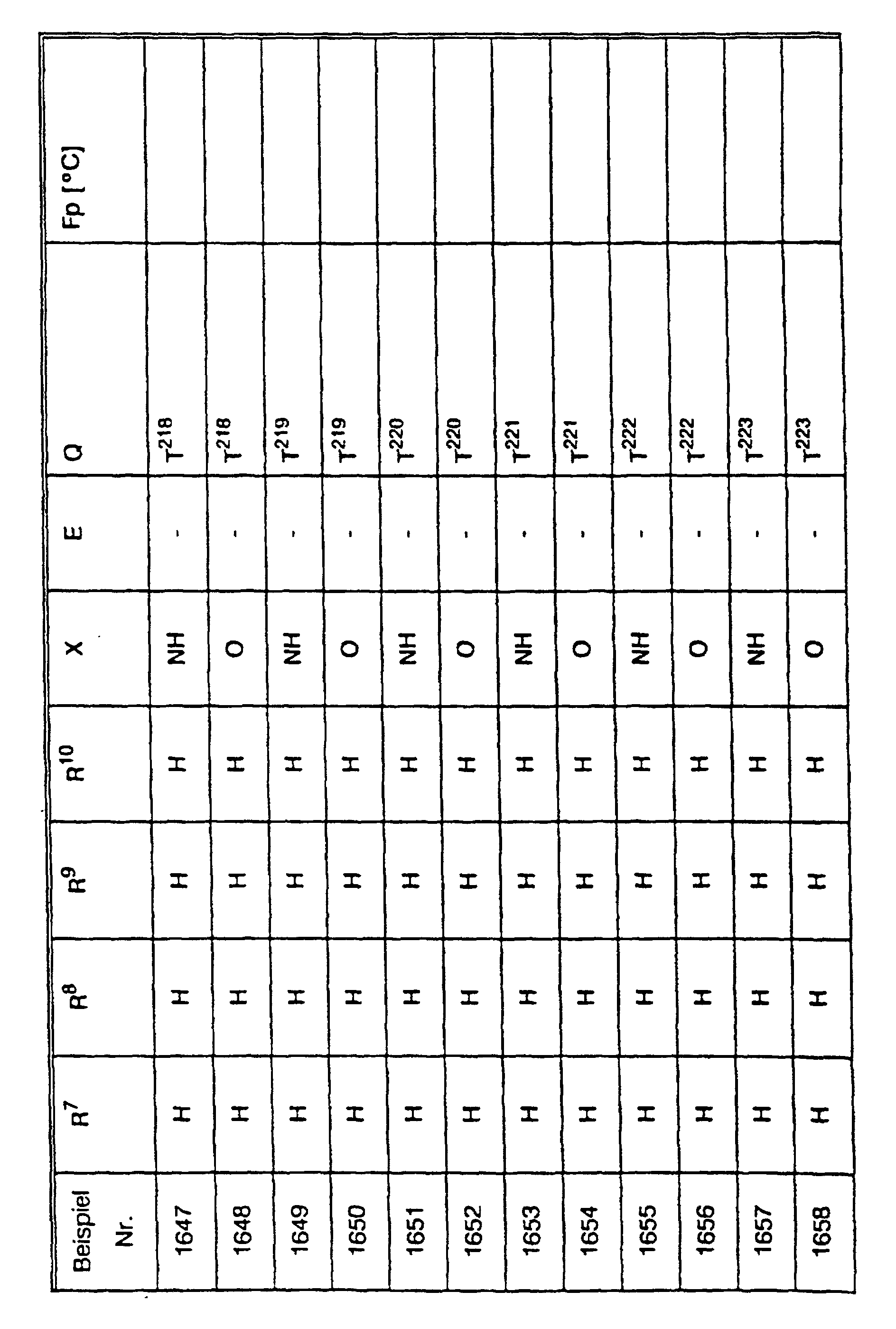

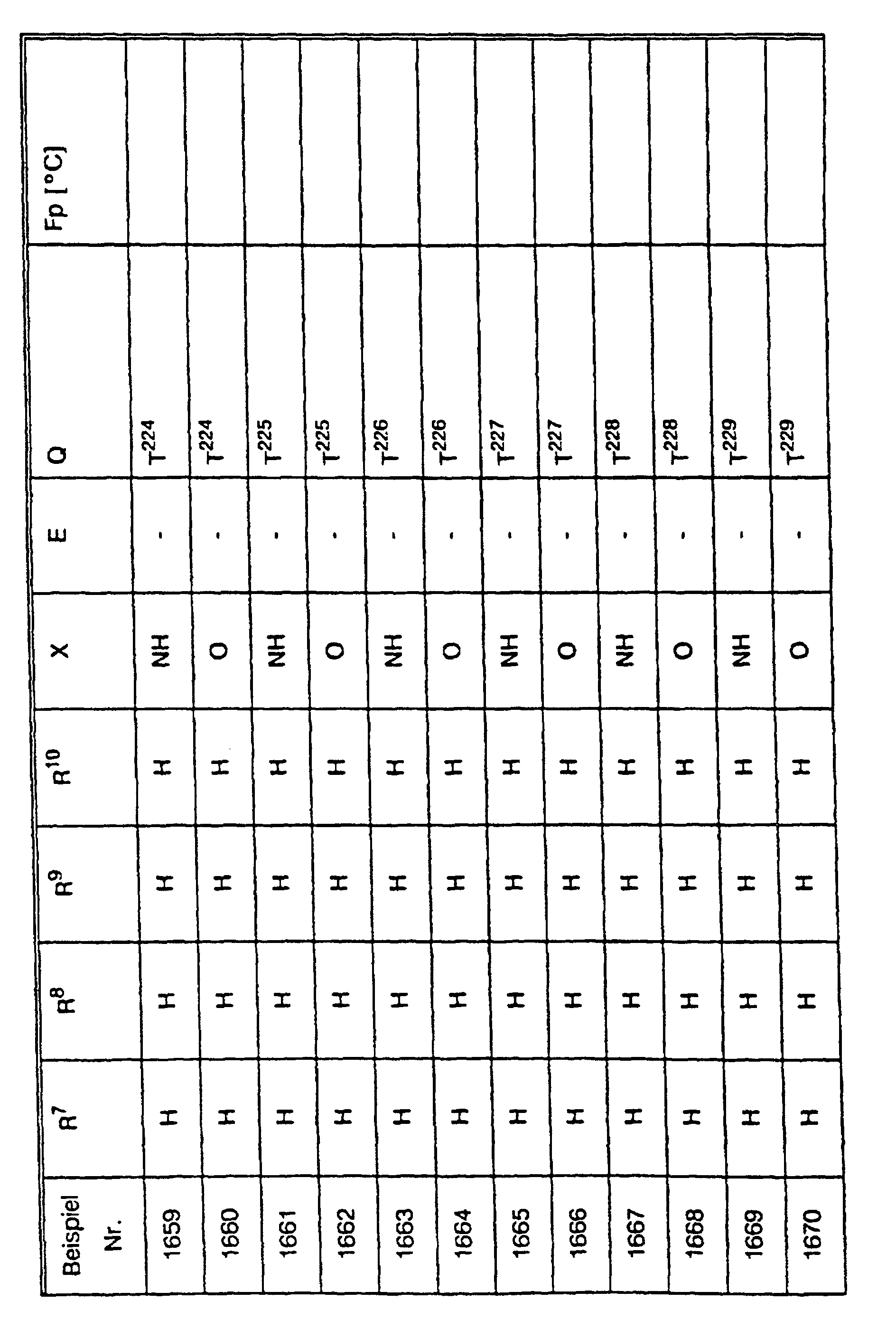

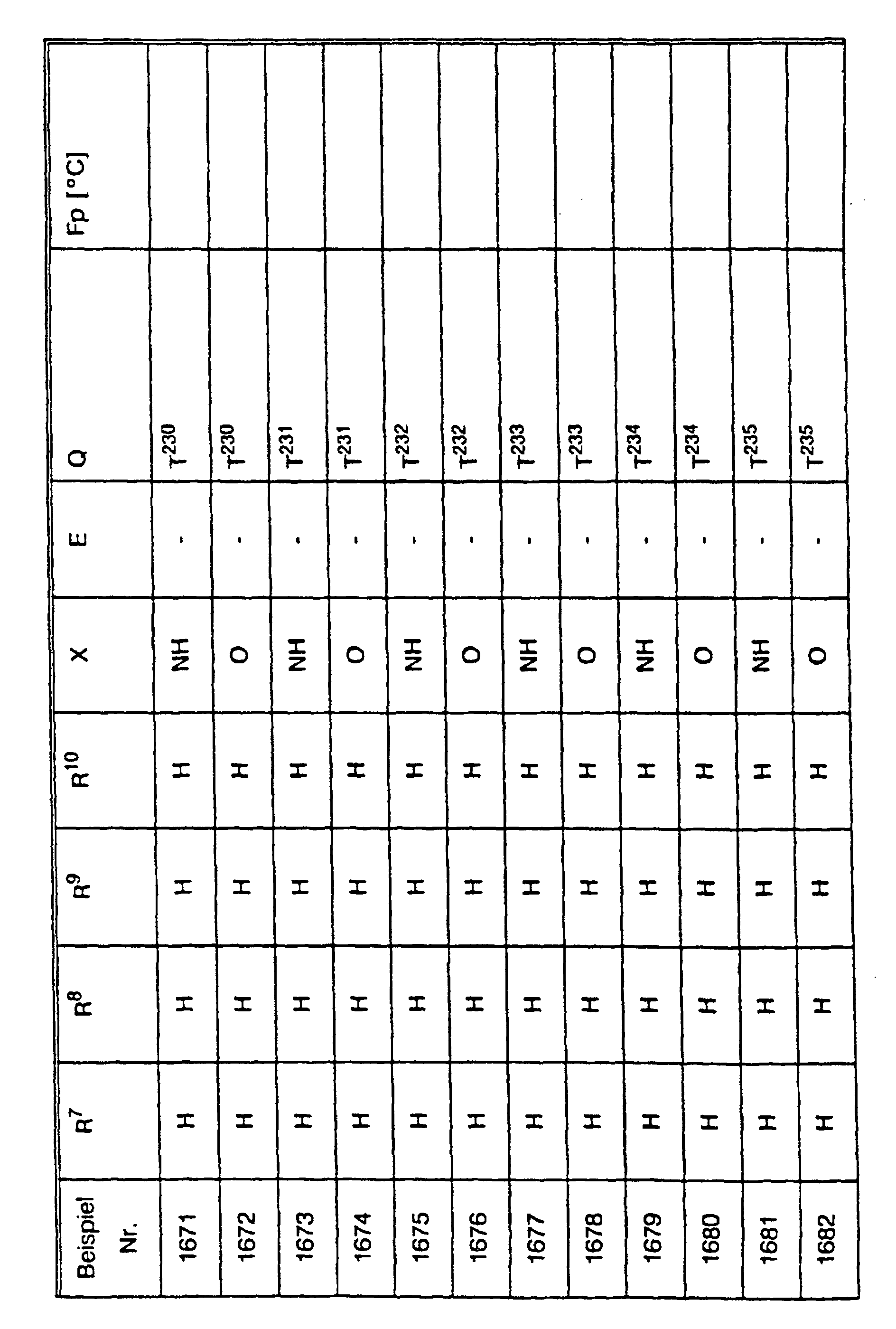

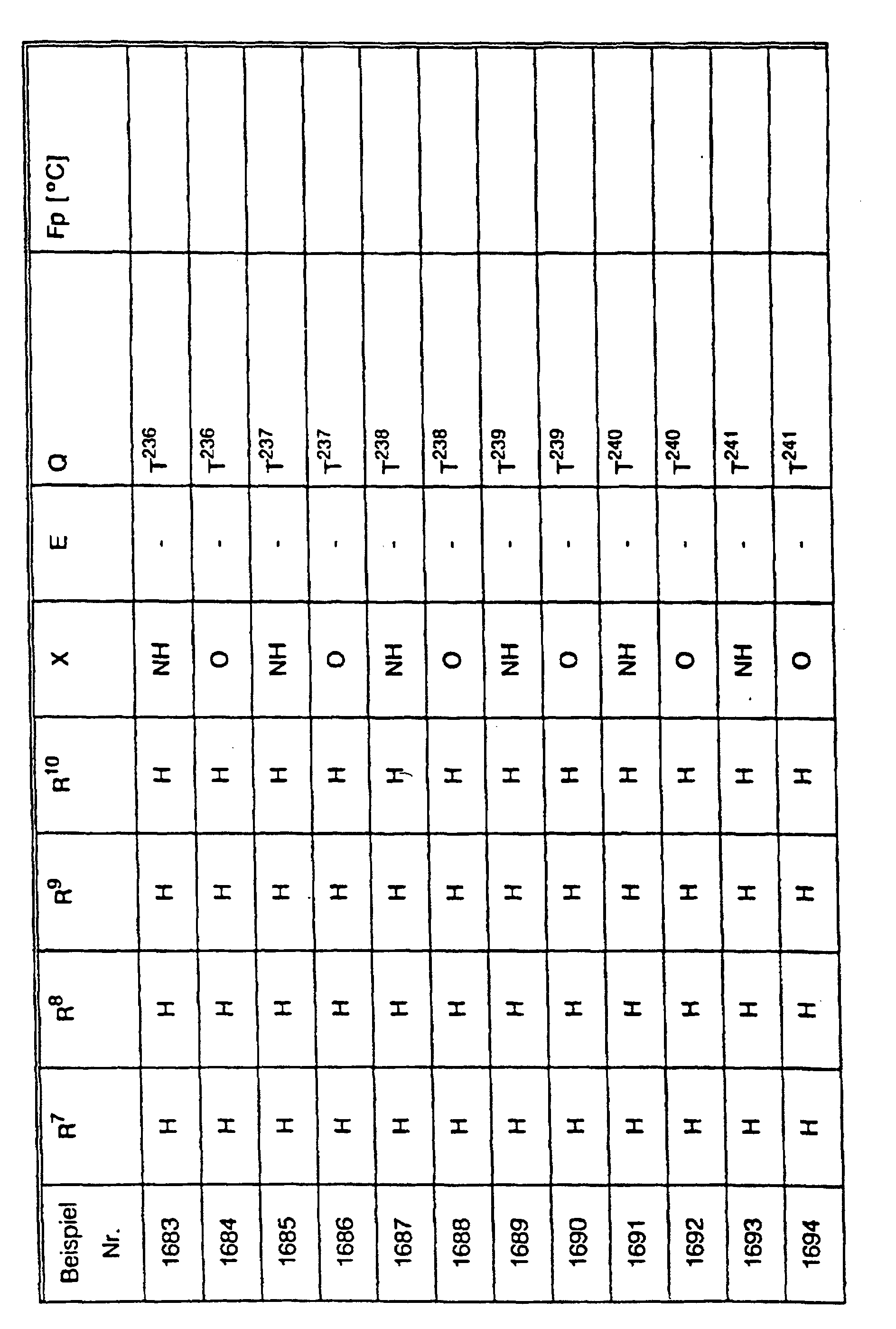

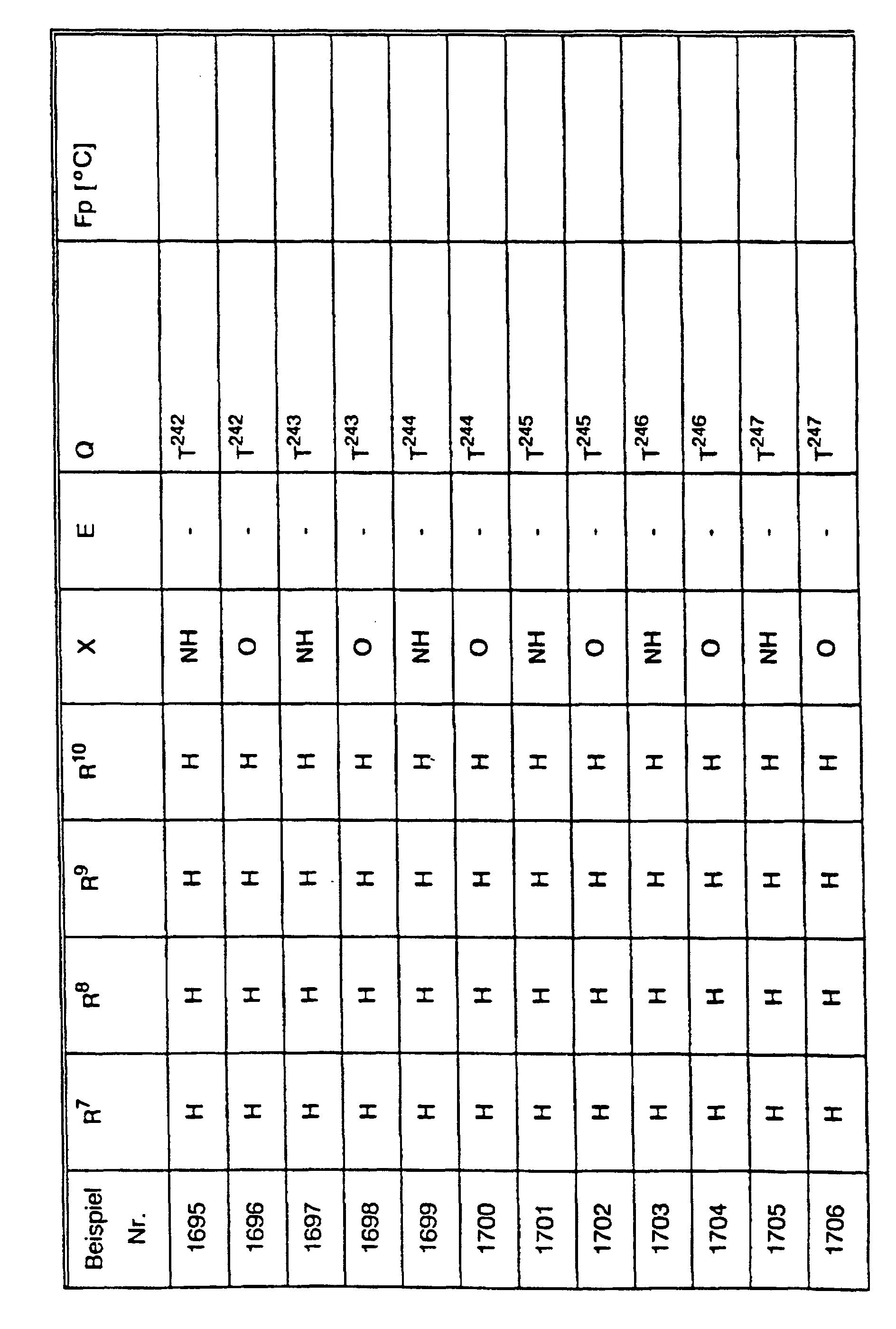

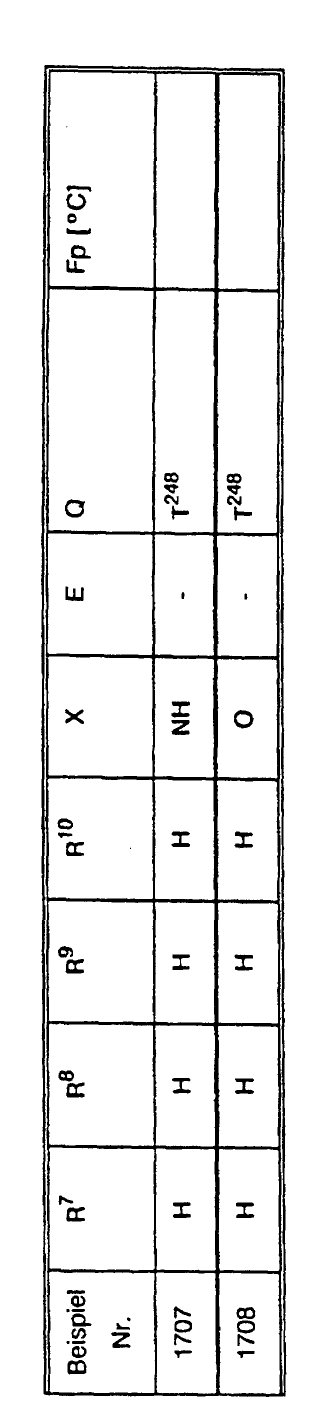

## Patentansprüche

1. Verbindungen der allgemeinen Formel I, 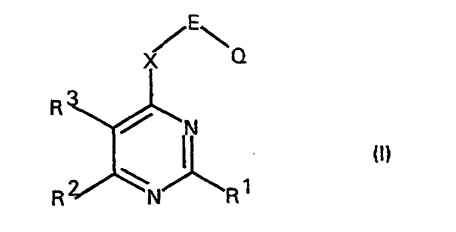 worin
R¹ Wasserstoff, Halogen, (C₁-C₄)-Alkyl oder (C₃-C₆)-Cycloalkyl bedeutet,
R² Wasserstoff, (C₁-C₄)-Alkyl, Halogen, (C₁-C₄)-Halogenalkyl, (C₁-C₄)-Alkoxy, (C₁-C₄)-Halogenalkoxy, (C₁-C₄)-Alkoxy-(C₁-C₄)-alkyl, (C₁-C₄)-Alkylthio, (C₁-C₄)-Alkylthio-(C₁-C₄)-alkyl, (C₁-C₄)-Alkylamino oder Di-(C₁-C₄)-alkylamino bedeutet,
R³ Wasserstoff, (C₁-C₄)-Alkyl, (C₁-C₄)-Alkoxy, (C₁-C₄)-Halogenalkoxy, Halogen, (C₁-C₄)-Alkylthio, Amino, (C₁-C₄)-Alkylamino oder Di-(C₁-C₄)-alkylamino bedeutet oder
R² und R³ zusammen mit den Kohlenstoffatomen, an die sie gebunden sind, einen ungesättigten 6-gliedrigen isocydischen Ring bilden, der gegebenenfalls durch 1, 2 oder 3 gleiche oder verschiedene Reste substituiert ist und diese Reste (C₁-C₄)-Alkyl, (C₁-C₄)-Alkoxy, (C₁-C₄)-Halogenalkyl, (C₁-C₄)-Halogenalkoxy und/oder Halogen bedeuten,
oder
R² und R³ zusammen mit den Kohlenstoffatomen, an die sie gebunden sind, einen gesättigten 6-gliedrigen isocyclischen Ring bilden, der gegebenenfalls durch 1, 2 oder 3 (C₁-C₄)-Alkylgruppen substituiert ist,
X NH oder Sauerstoff bedeutet,
E für eine direkte Bindung oder eine geradkettige oder verzweigte (C₁-C₄)-Alkandiylgruppe steht,
Q die Bedeutung Q¹ hat und
Q¹ eine Cycloalkylgruppe der allgemeinen Formel II bedeutet 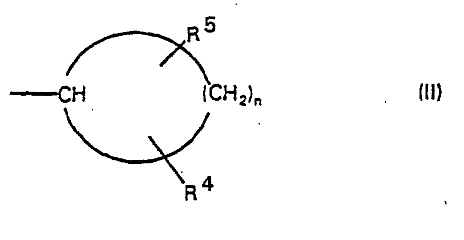 worin n eine ganze Zahl von 2 bis 7 ist,
R⁴ und R⁵ gleich oder verschieden sind und jeweils Wasserstoff, (C₁-C₁₂)-Alkyl, (C₃-C₈)-Cycloalkyl, (C₃-C₈)-Cycloalkyl-(C₁-C₄)-alkyl, (C₁-C₈)-Alkoxy, (C₃-C₈)-Cycloalkoxy, (C₁-C₄)-Alkoxy-(C₁-C₄)-alkyl, (C₃-C₈)-Cycloalkyl-(C₁-C₄)-alkoxy, Tri-(C₁-C₈)-alkylsilyl, Di-(C₁-C₈)-alkyl-(C₃-C₈)-cycloalkylsilyl, Di-(C₁-C₈)-alkyl-(phenyl-(C₁-C₄)-alkyl)-silyl, Di-(C₁-C₈)-alkyl-(C₁-C₄)-halogenalkylsilyl, Dimethylphenylsilyl, (C₁-C₄)-Halogenalkyl, Halogen, (C₁-C₄)-Halogenalkoxy, Heteroaryl, Phenyl, Phenyl-(C₁-C₄)-alkyl, Benzyloxy, Benzyloxy-(C₁-C₄-)-alkyl, Benzylthio, Phenylthio oder Phenoxy bedeuten, wobei die Phenylringe in den sieben letztgenannten Resten unsubstituiert oder mit einem oder zwei Substituenten versehen sein können und diese Substituenten gleich oder verschieden sind und jeweils (C₁-C₈)-Alkyl, (C₃-C₈)-Cycloalkyl, (C₁-C₄)-Halogenalkyl, Halogen, (C₁-C₄)-Dialkylamino, (C₁-C₄)-Alkylthio, (C₁-C₈)-Alkoxy, (C₁-C₄)-Halogenalkoxy. (C₁-C₄)-Alkoxy-(C₁-C₄)-alkoxy, H₅C₂-O-[CH₂-CH₂-O-]ₓ, 2-(Tetrahydro-2H-pyran-2-yloxy)-ethoxy, (C₂-C₈)-Alkenyl, (C₂-C₈)-Alkinyl, Benzyloxy, welches im Phenylrest gegebenenfalls einen oder zwei gleiche oder verschiedene Substituenten aus der Reihe (C₁-C₄)-Alkyl, (C₁-C₄)-Halogenalkyl, (C₁-C₄)-Alkoxy, (C₁-C₄)-Halogenalkoxy und Halogen trägt, Tri-(C₁-C₄)-alkylsilylmethoxy, (C₃-C₈)-Cycloalkyl-(C₁-C₄)-alkoxy, 1,3-Dioxolan-2-ylmethoxy, Tetrahydrofuran-2-ylmethoxy oder Tetrahydro-2H-pyran-2-ylmethoxy bedeuten können, wobei R⁴ und R⁵ nicht beide gleichzeitig Wasserstoff bedeuten können und wobei in zwei benachbarten Substituenten, die gleich oder verschieden sind und aus (C₁-C₈)-Alkyl und (C₁-C₈)-Alkoxy ausgewählt sind, jeweils ein Wasserstoffatom durch eine gemeinsame, diese beiden Substituenten verknüpfende C-C-Bindung ersetzt sein kann, und worin unter dem Ausdruck "Heteroaryl" einen Arylrest, worin mindestens eine CH-Gruppe durch N ersetzt ist und/oder mindestens zwei benachbarte CH-Gruppen durch D, NH oder O ersetzt sind, zu verstehen ist; oder
R⁴ und R⁵ zusammen mit der Cycloalkylgruppe ein 3-8-gliedriges spirocyclisches Ringsystem bilden, das an Stelle einer oder zweier CH₂-Gruppen Sauefstoff enthalten kann, oder
R⁴ und R⁵ zusammen mit den diese tragenden Kohlenstoffatomen einen ankondensierten 5- oder 6-gliedrigen Isocyclus bilden,
x 2,3 oder 4 ist,
oder
Q die Bedeutung Q² hat und
Q² eine Gruppe der allgemeinen Formel III bedeutet 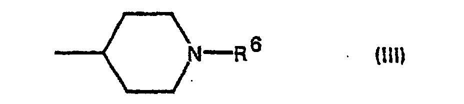
worin
R⁶ eine Gruppe der allgemeinen Formel Z-W bedeutet und Z eine direkte Verbindung oder Carbonyl oder Sulfonyl bedeutet und W eine Phenyl, Naphthyl, Biphenylyl oder eine dieser Gruppen worin mindestens eine CH-Gruppe durch N ersetzt ist und/oder mindestens zwei benachbarte CH-Gruppen durch S, NH oder O ersetzt sind bedeutet, die unsubstituiert oder mit einem oder zwei Substituenten versehen sein konnen und diese Substituenten gleich oder verschieden sind und jeweils (C₁-C₈)-Alkyl, (C₃-C₈)-Cycloalkyl, Trifluormethyl, Halogen, (C₁-C₄)-Alkoxy, (C₁-C₄)-Dialkylamino oder (C₁-C₄)-Alkylthio bedeuten,
oder deren Säureadditionssalze.

2. Verbindungen der Formel I gemäß Anspruch 1, in welcher
R¹ Wasserstoff, Methyl oder Cydopropyl ist,
R² (C₁-C₄)-Alkyl, Chlor, Methoxy, Ethoxy oder Methoxymethyl bedeutet,
R³ Wasserstoff, (C₁-C₃)-Alkyl, Methoxy, Ethoxy oder Halogen bedeutet oder
R² und R³ zusammen mit den Kohlenstoffatomen, an die sie gebunden sind, einen ungesitigten 6-gliedrigen Ring bilden,
Q die Bedeutung Q¹ oder Q² hat, oder deren Säureadditionssalze.

3. Verbindungen der Formel I gemäß Anspruch 1 oder 2, in welcher
R¹ Wasserstoff oder Methyl bedeutet,
R² Methyl, Ethyl; Methoxy, Ethoxy oder Methoxymethyl bedeutet,
R³ Methyl, Ethyl, Methoxy, Chlor oder Brom bedeutet, oder
R² und R³ zusammen mit den Kohlenstoffatomen, an die sie gebunden sind, das Chinazolin-System bilden, das mit Fluor, Chlor, Brom, Methyl und/oder Methoxy substituiert sein kann, und
Q die Bedeutung Q¹ oder Q² hat, oder deren Säureadditionssalze.

4. Verbindungen der Formel I gemäß einem der Ansprüche 1 bis 3, worin
E eine direkte Bindung bedeutet
R¹ Wasserstoff bedeutet,
R² Methyl, Ethyl oder Methoxymethyl bedeutet,
R³ Chlor, Brom oder Methoxy bedeutet oder
R² und R³ zusammen mit den Kohlenstoffatomen, an die sie gebunden sind, das Chinazolin-System bilden, das mit Fluor, Chlor, Brom oder Methyl substituiert sein kann oder
R² und R³ zusammen mit dem Pyrimidin-Ring das 5,6,7,8-Tetrahydrochinazolin-System bilden und
Q die Bedeutung Q¹ oder Q² hat, oder deren Säureadditionssalze.

5. Verbindungen der Formel I gemäß einem der Ansprüche 1 bis 4, worin
E eine direkte Bindung bedeutet,
R¹ Wasserstoff bedeutet,
R² Methoxymethyl und R³ Methoxy oder
R² Methyl oder Ethyl und R³ Chlor oder Brom bedeutet oder
R² und R³ zusammen mit dem Kohlenstoffatom, an das sie gebunden sind, ein Chinazolin-System bilden, das mit Fluor, Chlor oder Methyl substituiert ist oder ein 5,6,7,8 Tetrahydrochinazolin-System bilden und
Q die Bedeutung Q¹ hat, oder deren Säureadditionssalze.

6. Verbindungen der Formel I gemäß einem der Ansprüche 1 bis 5, worin
E eine direkte Verbindung bedeutet
R¹ Wasserstoff bedeutet,
R² Methoxymethyl und R³ Methoxy bedeutet oder
R² Ethyl und R³ Chlor bedeutet oder
R² und R³ zusammen mit dem Kohlenstoffatom, an das sie gebunden sind, ein Chinazolin, ein 5,6,7,8-Tetrahydrochinazolin-System bilden und
Q¹ eine in Position 3 oder 4 substituierte Cycloalkylgruppe der allgemeinen Formel II bedeutet, worin
n 4 oder 5 bedeutet,
R⁴ (C₃-C₈)-Alkyl, Cyclopentyl, Cyclohexyl, Phenyl oder Phenoxy bedeutet, wobei die beiden letztgenannten Reste unsubstituiert oder mit einem oder zwei Substituenten versehen sein können die gleich oder verschieden sein können und diese Fluor, Chlor, Brom,(C₁-C₄)-Alkyl, (C₁-C₄)-Alkoxy, Trifluormethyl, (C₁-C₂)-Halogenalkoxy, Cyclohexyl, 2-Ethoxyethoxy, Methylthio oder Dimethylamino bedeuten oder
R⁵ Wasserstoff bedeutet,
oder deren Säureadditionssalze.

7. Verbindungen der Formel I gemäß einem der Ansprüche 1 bis 6, worin
E eine direkte Bindung bedeutet,
R¹ Wasserstoff bedeutet,
R² Methoxymethyl und R³ Methoxy oder
R² Ethyl und R³ Chlor bedeuten, oder
R² und R³ zusammen mit den Kohlenstoffatomen, an die sie gebunden sind, ein Chinazolin- oder ein 5,6,7,8 Tetrahydrochinazolin-System bilden,
Q die Bedeutung Q¹ hat und
Q¹ in 4-Position substituiertes Cyclohexyl bedeutet, und E und der Substituent in 4-Position des Cyclohexyl zueinander cis-ständig sind,
oder deren Säureadditionssalze.

8. Verfahren zur Herstellung von Verbindungen der Formel I gemäß einem der Ansprüche 1-7, **dadurch gekennzeichnet, daß** man eine Verbindung der Formel IV, 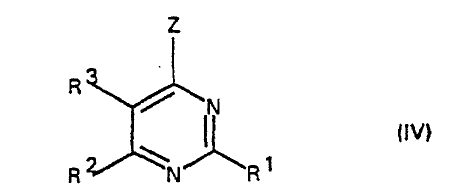 worin R¹, R² und R³ die unter Formel I angegebenen Bedeutungen haben und Z eine Abgangsgruppe, beispielsweise Halogen, Alkylthio, Alkansulfonyloxy oder Arylsulfonyloxy, Alkylsulfonyl oder Arylsulfonyl bedeutet, mit einem Nudeophil der Formel V
HX-E-Q (V)
worin X, E und Q die unter Formel I angegebenen Bedeutungen haben, umsetzt und die so erhaltenen Verbindungen der Formel I gegebenenfalls, falls R³ Wasserstoff bedeutet, an C⁵ des Pyrimidins chloriert oder bromiert, und die so erhaltenen Verbindungen der Formel I gegebenenfalls in ihre Säureadditionssalze überführt.

9. Insektizide oder akarizide Mittel, welche eine wirksame Menge einer Verbindung der Formel I gemäß einem der Ansprüche 1 bis 7 enthalten.

10. Fungizide Mittel, welche eine wirksame Menge einer Verbindung der Formel I gemäß einem der Ansprüche 1 bis 7 haben.

11. Nematozide Mittel, welche eine wirksame Menge einer Verbindung der Formel I gemäß einem der Ansprüche 1 bis 7 enthalten.

12. Verfahren zur Bekämpfung von Schadinsekten, Akariden, **dadurch gekennzeichnet, daß** man auf diese oder die von ihre befallenen Pflanzen, Flächen oder Substrate eine wirksame Menge einer Verbindung der Formel I gemäß einem der Ansprüche 1 bis 7 appliziert.

13. Verfahren zur Bekämpfung von Schadpilzen, **dadurch gekennzeichnet, daß** man auf diese oder die von ihre befallenen Pflanzen, Flächen oder Substrate eine wirksame Menge einer Verbindung der Formel I gemäß einem der Ansprüche 1 bis 7 appliziert.

14. Verfahren zur Bekämpfung von Nematoden, **dadurch gekennzeichnet, daß** man auf diese oder die von ihre befallenen Pflanzen, Flächen oder Substrate eine wirksame Menge einer Verbindung der Formel I gemäß einem der Ansprüche 1 bis 7 appliziert.

## Claims

1. A compound of the formula I 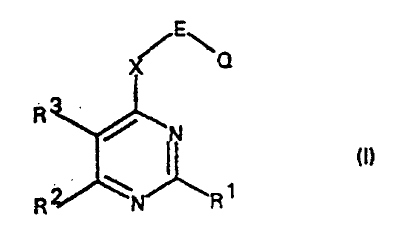 in which
R¹ is hydrogen, halogen, (C₁-C₄)-alkyl or (C₃-C₆)-cycloalkyl,
R² is hydrogen, (C₁-C₄)-alkyl, halogen, (C₁-C₄)-haloalkyl, (C₁-C₄)-alkoxy, (C₁-C₄)-haloalkoxy, (C₁-C₄)-alkoxy- (C₁-C₄)-alkyl, (C₁-C₄)-alkylthio, (C₁-C₄)-alkylthio- (C₁-C₄)-alkyl, (C₁-C₄)-alkylamino or di- (C₁-C₄) -alkylamino,
R³ is hydrogen, (C₁-C₄)-alkyl, (C₁-C₄)-alkoxy, (C₁-C₄)-haloalkoxy, halogen, (C₁-C₄) -alkylthio, amino, (C₁-C₄)-alkylamino or di-(C₁-C₄)-alkylamino or
R² and R³ together with the carbon atoms to which they are bonded form an unsaturated 6-membered isocyclic ring which is optionally substituted by 1, 2 or 3 identical or different radicals, these radicals being (C₁-C₄)-alkyl, (C₁-C₄)-alkoxy, (C₁-C₄)-haloalkyl, (C₁-C₄)-haloalkoxy and/or halogen,
or
R² and R³ together with the carbon atoms to which they are bonded form a saturated 6-membered isocyclic ring which is optionally substituted by 1, 2 or 3 (C₁-C₄)-alkyl groups,
X is NH or oxygen,
E is a direct bond or a straight-chain or branched (C₁-C₄)-alkanediyl group,
Q has the meaning Q¹ and
Q¹ is a cycloalkyl group of the formula II 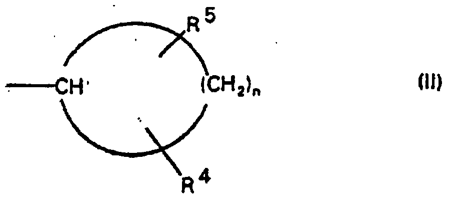 in which n is an integer from 2 to 7,
R⁴ and R⁵ are identical or different and are in each case hydrogen, (C₁-C₁₂)-alkyl, (C₃-C₈)-cycloalkyl, (C₃-C₈)-cycloalkyl- (C₁-C₄) -alkyl, (C₁-C₈)-alkoxy, (C₃-C₈) -cycloalkoxy, (C₁-C₄)-alkoxy- (C₁-C₄) -alkyl, (C₃-C₈) -cycloalkyl- (C₁-C₄)-alkoxy, tri-(C₁-C₈)-alkylsilyl, di-(C₁-C₈)-alkyl- (C₃-C₈)-cycloalkylsilyl, di- (C₁-C₈) -alkyl(phenyl- (C₁-C₄) -alkyl) -silyl, di- (C₁-C₈) -alkyl-(C₁-C₄)-haloalkylsilyl, dimethylphenyl silyl, (C₁-C₄)-haloalkyl, halogen, (C₁-C₄)-haloalkoxy, heteroaryl, phenyl, phenyl-(C₁-C₄)-alkyl, benzyloxy, benzyloxy-(C₁-C₄)-alkyl, benzylthio, phenylthio or phenoxy, it being possible for the phenyl rings in the seven last-mentioned radicals to be unsubstituted or provided with one or two substituents and these substituents are identical or different and can be in each case (C₁-C₈)-alkyl, (C₃-C₈)-cycloalkyl, (C₁-C₄)-haloalkyl, halogen, (C₁-C₄)-dialkylamino, (C₁-C₄)-alkylthio, (C₁-C₈)-alkoxy, (C₁-C₄)-haloalkoxy, (C₁-C₄)-alkoxy-(C₁-C₄)-alkoxy, H₅C₂-O-[CH₂-CH₂-O-]ₓ, 2- (tetrahydro-2H-pyran-2-yloxy)-ethoxy, (C₂-C₈)-alkenyl, (C₂-C₈)-alkynyl, benzyloxy which in the phenyl radical optionally carries one or two identical or different substituents selected from the group comprising (C₁-C₄)-alkyl, (C₁-C₄)-haloalkyl, (C₁-C₄)-alkoxy, (C₁-C₄)-haloalkoxy and halogen, or tri-(C₁-C₄)-alkylsilylmethoxy, (C₃-C₈)-cycloalkyl-(C₁-C₄)-alkoxy, 1,3-dioxolan-2-yl-methoxy, tetrahydrofuran-2-ylmethoxy or tetrahydro-2H-pyran-2-ylmethoxy, where R⁴ and R⁵ cannot both simultaneously be hydrogen, and where, in two adjacent substituents which are identical or different and selected from the group comprising (C₁-C₈)-alkyl and (C₁-C₈)-alkoxy, in each case one hydrogen atom can be replaced by a joint carbon-carbon bond which links these two substituents, and in which the term "heteroaryl" is to be understood as meaning an aryl radical in which at least one CH group is replaced by N and/or at least two adjacent CH groups are replaced by S, NH or O; or
R⁴ and R⁵ together with the cycloalkyl group form a 3-8-membered spirocyclic ring system which can contain oxygen in place of one or two CH₂ groups or
R⁴ and R⁵ together with the carbon atoms carrying them form a fused 5- or 6-membered isocycle,
x is 2, 3 or 4, or
Q has the meaning Q² and
Q² is a group of the formula III 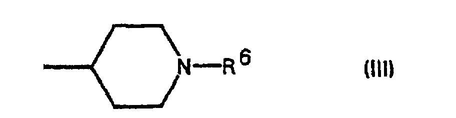
in which
R⁶ is a group of the formula Z-W and Z is a direct bond or carbonyl or sulfonyl and W is a phenyl, naphthyl or biphenylyl or one of these groups in which at least one CH group is replaced by N and/or at least two adjacent CH groups are replaced by S, NH or O, which can be unsubstituted or provided with one or two substituents and these substituents are identical or different and are in each case (C₁-C₈)-alkyl, (C₃-C₈) -cycloalkyl, trifluoromethyl, halogen, (C₁-C₄)-alkoxy, (C₁-C₄)-dialkylamino or (C₁-C₄)-alkylthio, or acid addition salts thereof.

2. A compound of the formula I as claimed in claim 1, in which
R¹ is hydrogen, methyl or cyclopropyl,
R² is (C₁-C₄)-alkyl, chlorine, methoxy, ethoxy or methoxymethyl,
R³ is hydrogen, (C₁-C₃)-alkyl, methoxy, ethoxy or halogen or
R² and R³ together with the carbon atoms to which they are bonded form an unsaturated 6-membered ring,
Q has the meaning Q¹ or Q², or acid addition salts thereof.

3. A compound of the formula I as claimed in claim 1 or 2, in which
R¹ is hydrogen or methyl,
R² is methyl, ethyl, methoxy, ethoxy or methoxymethyl,
R³ is methyl, ethyl, methoxy, chlorine or bromine, or
R² and R³ together with the carbon atoms to which they are bonded form the quinazoline system, which can be substituted by fluorine, chlorine, bromine, methyl and/or methoxy and
Q has the meaning Q¹ or Q², or acid addition salts thereof.

4. A compound of the formula I as claimed in any one of claims 1 to 3, in which
E is a direct bond,
R¹ is hydrogen,
R² is methyl, ethyl or methoxymethyl,
R³ is chlorine, bromine or methoxy or
R² and R³ together with the carbon atoms to which they are bonded form the quinazoline system which can be substituted by fluorine, chlorine, bromine or methyl, or
R² and R³ together with the pyrimidine ring form the 5,6,7,8-tetrahydroquinazoline system and
Q has the meaning Q¹ or Q², or acid addition salts thereof.

5. A compound of the formula I as claimed in any one of claims 1 to 4, in which
E is a direct bond,
R¹ is hydrogen,
R² is methoxymethyl and R³ is methoxy or
R² is methyl or ethyl and R³ is chlorine or bromine or
R² and R³ together with the carbon atom to which they are bonded form a quinazoline system which is substituted by fluorine, chlorine or methyl, or form a 5,6,7,8-tetrahydroquinazoline system and
Q has the meaning Q¹, or acid addition salts thereof.

6. A compound of the formula I as claimed in any one of claims 1 to 5, in which
E is a direct bond,
R¹ is hydrogen,
R² is methoxymethyl and R³ is methoxy or
R² is ethyl and R³ is chlorine or
R² and R³ together with the carbon atom to which they are bonded form a quinazoline or a 5,6,7,8-tetrahydroquinazoline system and
Q¹ is a cycloalkyl group of the formula II which is substituted in the 3- or 4-position and in which
n is 4 or 5,
R⁴ is (C₃-C₈)-alkyl, cyclopentyl, cyclohexyl, phenyl or phenoxy, it being possible for the two last-mentioned radicals to be unsubstituted or provided with one or two substituents which can be identical or different and which are fluorine, chlorine, bromine, (C₁-C₄)-alkyl, (C₁-C₄)-alkoxy, trifluoromethyl, (C₁-C₂)-haloalkoxy, cyclohexyl, 2-ethoxyethoxy, methylthio or dimethylamino, and
R⁵ is hydrogen,
or acid addition salts thereof.

7. A compound of the formula I as claimed in any one of claims 1 to 6, in which
E is a direct bond,
R¹ is hydrogen,
R² is methoxymethyl and R³ is methoxy or
R³ is ethyl and R³ is chlorine, or
R² and R³ together with the carbon atoms to which they are bonded form a quinazoline or a 5,6,7,8-tetrahydroquinazoline system,
Q has the meaning Q¹ and
Q¹ is cyclohexyl which is substituted in the 4-position, and E and the substituent in the 4-position of the cyclohexyl are in the cis-position relative to each other,
or acid addition salts thereof.

8. A process for the preparation of compounds of the formula I as claimed in any one of claims 1 to 7, which comprises reacting a compound of the formula IV 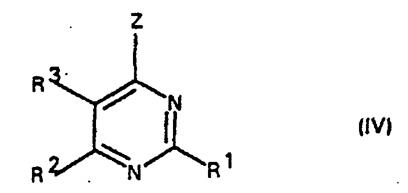 in which R¹, R² and R³ are defined as in formula I and Z is a leaving group, for example halogen, alkylthio, alkanesulfonyloxy or arylsulfonyloxy, alkylsulfonyl or arylsulfonyl, with a nucleophile of the formula V
HX - E - Q (V)
in which X, E and Q are as defined in formula I and, if R³ is hydrogen, the resulting compounds of the formula I are optionally chlorinated or brominated on carbon atom 5 of the pyrimidine, and, if appropriate, converting the resulting compounds of the formula I into their acid addition salts.

9. An insecticidal or acaricidal agent comprising an effective amount of a compound of the formula I as claimed in any one of claims 1 to 7.

10. A fungicidal agent comprising an effective amount of a compound of the formula I as claimed in any one of claims 1 to 7.

11. A nematocidal agent comprising an effective amount of a compound of the formula I as claimed in any one of claims 1 to 7.

12. A method of controlling insect pests and acarids, which comprises applying an effective amount of a compound of the formula I as claimed in any one of claims 1 to 7 to these insect pests or acarids or to the plants, areas or substrates which are infested with them.

13. A method of controlling harmful fungi, which comprises applying an effective amount of a compound of the formula I as claimed in any one of claims 1 to 7 to these harmful fungi or to the plants, areas or substrates infected by them.

14. A method of controlling nematodes, which comprises applying an effective amount of a compound of the formula I as claimed in any one of claims 1 to 7 to these nematodes or to the plants, areas or substrates infested with them.

## Revendications

1. Composés de formule générale I, 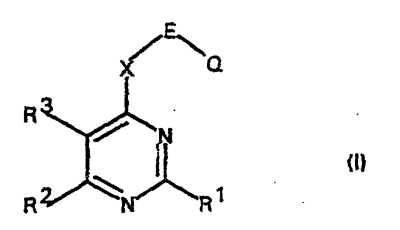 dans laquelle
R¹ représente un atome d'hydrogène, d'halogène, un groupe alkyle en C₁ à C₄ ou cycloalkyle en C₃ à C₆,
R² représente un atome d'hydrogène, un groupe alkyle en C₁ à C₄, un atome d'halogène, un groupe halogénoalkyle en C₁ à C₄, alcoxy en C₁ à C₄, halogénoalcoxy en C₁ à C₄, alcoxy en (C₁ à C₄)-alkyle en (C₁ à C₄), alkylthio en C₁ à C₄, alkylthio en (C₁ à C₄)-alkyle en (C₁ à C₄), alkylamino en C₁ à C₄ ou di-(alkylamino en C₁ à C₄),
R³ représente un atome d'hydrogène, un groupe alkyle en C₁ à C₄, alcoxy en C₁ à C₄, halogénoalcoxy en C₁ à C₄, un atome d'halogène, un groupe alkylthio en C₁ à C₄, amino, alkylamino en C₁ à C₄ ou di-(alkylamino en C₁ à C₄) ou
R² et R³ forment ensemble avec les atomes de carbone auxquels ils sont liés, un cycle isocyclique insaturé à 6 chaînons, qui est éventuellement substitué par 1, 2 ou 3 groupes identiques ou différents et ces groupes représentent un groupe alkyle en C₁ à C₄, alcoxy en C₁ à C₄, halogénoalkyle en C₁ à C₄, halogénoalcoxy en C₁ à C₄ et/ou un atome d'halogène, ou
R² et R³ forment ensemble avec les atomes de carbone auxquels ils sont liés, un cycle isocyclique insaturé à 6 chaînons, qui est éventuellement substitué par 1, 2 ou 3 groupes alkyle en C₁ à C₄,
X représente NH ou l'oxygène,
E représente une liaison directe ou un groupe alcandiyle en C₁ à C₄ à chaîne linéaire ou ramifiée,
Q a la signification Q¹ et
Q¹ représente un groupe cycloalkyle de formule générale II 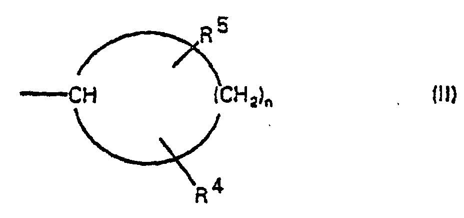
dans laquelle n est un nombre entier de 2 à 7,
R⁴ et R⁵ sont identiques ou différents et représentent respectivement un atome d'hydrogène, un groupe alkyle en C₁ à C₁₂, cycloalkyle en (C₃ à C₈)-alkyle en (C₁ à C₄), alcoxy en C₁ à C₈, cycloalcoxy en C₃ à C₈, alcoxy en (C₁ à C₄)-alkyle en (C₁ à C₄), cycloalkyle en (C₃ à C₈)-alcoxy en (C₁ à C₄), trialkyle en (C₁ à C₈)-silyle, di-alkyle en (C₁ à C₈)-cycloalkyle en (C₃ à C₈)-silyle, di-alkyle en (C₁ à C₈)-(phényl-alkyle en (C₁ à C₄))-silyle, di-alkyle en (C₁ à C₈)-halogénoalkyle en (C₁ à C₄)-silyle, diméthylphényl-silyle, halogénolalkyle en C₁ à C₄, halogène, halogénoalcoxy en C₁ à C₄, hétéroaryle, phényle, phényle-alkyle en C₁ à C₄, benzyloxy, benzyloxy-alkyle en C₁ à C₄, benzylthio, phénylthio ou phénoxy, les cycles phényle dans les sept derniers groupes cités pouvant être non substitués ou munis d'un ou deux substituants et ces substituants sont identiques ou différents et peuvent représenter respectivement un groupe alkyle en C₁ à C₈, cyclo-alkyle en C₃ à C₈, halcgénoalkyle en C₁ à C₄, halogène, dialkylamino en C₁ à C₄, alkylthio en C₁ à C₄, alcoxy en C₁ à C₈, halogénoalcoxy en C₁ à C₄, alcoxy en (C₁ à C₄)-alcoxy en (C₁ à C₄), H₅C₂-O-[CH₂-CH₂-O-]ₓ, 2-(tétrahydro-2H-pyran-2-yloxy)-éthoxy, alcényle en C₂ à C₈, alcynyle en C₂ à C₈, benzyloxy, qui porte éventuellement dans le groupe phényle un ou deux substituants identiques ou différents choisis parmi un groupe alkyle en C₁ à C₄, halogénoalkyle en C₁ à C₄, alcoxy en C₁ à C₄, halogénoalcoxy en C₁ à C₄ et halogène, trialkyle en (C₁ à C₄)-silylméthoxy, cycloalkyle en (C₃ à C₈)-alcoxy en (C₁ à C₄), 1,3-dioxolan-2-ylméthoxy, tétrahydrofuran-2-ylméthoxy ou tétrahydro-2H-pyran-2-ylméthoxy, dans laquelle R⁴ et R⁵ ne peuvent pas représenter en même temps tous les deux un atome d'hydrogène et dans laquelle dans deux substituants voisins qui sont identiques ou différents et sont choisis entre un groupe alkyle en C₁ à C₈ et alcoxy en C₁ à C₈, respectivement un atome d'hydrogène peut être remplacé par une liaison commune C-C liant ces deux substituants, et dans laquelle on entend par l'expression « hétéroaryle » un résidu aryle, dans lequel au moins un groupe CH est remplacé par N et/ou au moins deux groupes CH voisins sont remplacés par S, NH ou O ; ou
R⁴ et R⁵ forment ensemble avec le groupe cycloalkyle un système cyclique spirocyclique à 3-8 chaînons, qui peut contenir l'oxygène à la place d'un ou de deux groupes CH₂, ou
R⁴ et R⁵ forment ensemble avec les atomes de carbone les portant un isocycle condensé à 5 ou 6 chaînons,
x est 2, 3 ou 4,
ou
Q a la signification de Q² et
Q² représente un groupe de formule générale III 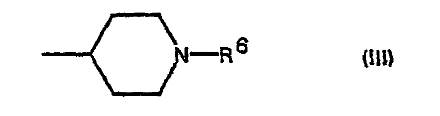
dans laquelle
R⁶ représente un groupe de formule générale Z-W et Z représente une liaison directe ou un groupe carbonyle ou sulfonyle et W un groupe phényle, naphtyle, biphénylyle ou un de ces groupes dans lesquels au moins un groupe CH est remplacé par N et/ou au moins deux groupes CH voisins sont remplacés par S, NH ou O, qui peuvent être non substitués ou munis d'un ou deux substituants et ces substituants sont identiques ou différents et représentent respectivement un groupe alkyle en C₁ à C₈, cycloalkyle en C₃ à C₈, trifluorométhyle, halogène, alcoxy en C₁ à C₄, dialkylamino en C₁ à C₄ ou alkylthio en C₁ à C₄,
ou leurs sels d'addition avec un acide.

2. Composés de formule I selon la revendication 1, dans lesquels
R¹ est un atome d'hydrogène, un groupe méthyle ou cyclopropyle,
R² représente un groupe alkyle en C₁ à C₄, un atome de chlore, un groupe méthoxy, éthoxy ou méthoxyméthyle,
R³ représente un atome d'hydrogène, un groupe alkyle en C₁ à C₃, méthoxy, éthoxy ou un atome d'halogène ou
R² et R³ forment ensemble avec les atomes de carbone auxquels ils sont liés un cycle insaturé à 6 chaînons,
Q a la signification Q¹ ou Q², ou leurs sels d'addition avec un acide.

3. Composés de formule I selon la revendication 1 ou 2, dans lesquels
R¹ représente un atome d'hydrogène ou un groupe méthyle,
R² représente un groupe méthyle, éthyle, méthoxy, éthoxy ou méthoxyméthyle,
R³ représente un groupe méthyle, éthyle, méthoxy, un atome de chlore ou de brome, ou
R² et R³ forment ensemble avec les atomes de carbone auxquels ils sont liés le système quinazoline, qui peut être substitué avec du fluor, du chlore, du brome, des groupes méthyle et/ou méthoxy, et
Q a la signification Q¹ ou Q², ou leurs sels d'addition avec un acide.

4. Composés de formule I selon l'une des revendications 1 à 3, dans lesquels
E représente une liaison directe,
R¹ représente un atome d'hydrogène,
R² représente un groupe méthyle, éthyle ou méthoxyméthyle,
R³ un atome de chlore, de brome ou un groupe méthoxy ou
R² et R³ forment ensemble avec les atomes de carbone auxquels ils sont liés le système quinazoline, qui peut être substitué avec du fluor, du chlore, du brome ou des groupes méthyle, ou
R² et R³ forment ensemble avec le cycle pyridine le système 5,6,7,8-tétrahydroquinazoline et
Q a la signification Q¹ ou Q², ou leurs sels d'addition avec un acide.

5. Composés de formule I selon l'une des revendications 1 à 4, dans lesquels
E représente une liaison directe,
R¹ représente un atome d'hydrogène,
R² représente un groupe méthoxyméthyle et R³ un groupe méthoxy ou
R² représente un groupe méthyle ou éthyle et R³ un atome de chlore ou de brome ou
R² et R³ forment ensemble avec l'atome de carbone auquel ils sont liés un système quinazoline, qui peut être substitué avec du fluor, du chlore ou des groupes méthyle, ou un système 5,6,7,8-tétrahydroquinazoline et
Q a la signification Q¹, ou leurs sels d'addition avec un acide.

6. Composés de formule I selon l'une des revendications 1 à 5, dans lesquels
E représente une liaison directe,
R¹ représente un atome d'hydrogène,
R² représente un groupe méthoxyméthyle et R³ un groupe méthoxy ou
R² représente un groupe éthyle et R³ un atome de chlore ou
R² et R³ forment ensemble avec l'atome de carbone auquel ils sont liés une quinazoline, un système 5,6,7,8-tétrahydroquinazoline et
Q¹ un groupe cycloalkyle substitué en position 3 ou 4 de formule générale II, dans laquelle
n représente 4 ou 5,
R⁴ représente un groupe alkyle en C₁ à C₄, cyclopentyle, cyclohexyle, phényle ou phénoxy, les deux derniers groupes cités pouvant être non substitués ou munis d'un ou de deux substituants, qui peuvent être identiques ou différents et ceux-ci représentent le fluor, le chlore, le brome, un groupe alkyle en C₁ à C₄, alcoxy en C₁ à C₄, trifluorométhyle, halogénoalcoxy en C₁ à C₂, cyclohexyle, 2-éthoxyéthoxy, méthylthio ou diméthylamino ou
R⁵ représente un atome d'hydrogène,
ou leurs sels d'addition avec un acide.

7. Composés de formule I selon l'une des revendications 1 à 6, dans lesquels
E représente une liaison directe,
R¹ représente un atome d'hydrogène,
R² représente un groupe méthoxyméthyle et R³ un groupe méthoxy ou
R² représente un groupe éthyle et R³ un atome de chlore, ou
R² et R³ forment ensemble avec les atomes de carbone auxquels ils sont liés un système quinazoline ou 5,6,7,8-tétrahydroquinazoline,
Q a la signification Q¹ et
Q¹ représente un groupe cyclohexyle substitué en position 4, et E et le substituant en position 4 du cyclohexyle sont situés en position cis l'un par rapport à l'autre,
ou leurs sels d'addition avec un acide.

8. Procédé pour la préparation de composés de formule I selon l'une des revendications 1 à 7, **caractérisé en ce que**, l'on met à réagir un composé de formule IV, 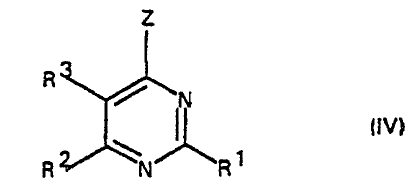 dans laquelle R¹, R² et R³ ont les significations indiquées à la formule I et Z représente un groupe partant, par exemple un atome d'halogène, un groupe alkylthio, alcanesulfonyloxy ou arylsulfonyloxy, alkylsulfonyle ou arylsulfonyle, avec un nucléophile de formule
HX-E-Q (IV)
dans lesquelles X, E et Q ont les significations indiquées à la formule I, et les composés de formule I ainsi obtenus éventuellement, au cas où R³ représente un atome d'hydrogène on réalise éventuellement une chloration ou une bromation en C⁵ de la pyridine, et on transforme éventuellement les composés de formule I ainsi obtenus en leurs sels d'addition avec un acide.

9. Agents insecticides ou acaricides, qui contiennent une quantité active d'un composé de formule I selon l'une des revendications 1 à 7.

10. Agents fongicides, qui ont une quantité efficace d'un composé de formule I selon l'une des revendications 1 à 7.

11. Agents nématocides, qui contiennent une quantité efficace d'un composé de formule I selon l'une des revendications 1 à 7.

12. Procédé de lutte contre les insectes nuisibles, les acariens, **caractérisé en ce qu'**on applique sur ceux-ci ou sur les plantes, les surfaces ou les substrats infestés par ceux-ci une quantité efficace d'un composé de formule I selon l'une des revendications 1 à 7.

13. Procédé de lutte contre les champignons nuisibles, **caractérisé en ce qu'**on applique sur ceux-ci ou sur les plantes, les surfaces ou les substrats infestés par ceux-ci une quantité efficace d'un composé de formule I selon l'une des revendications 1 à 7.

14. Procédé de lutte contre les nématodes, **caractérisé en ce qu'**on applique sur ceux-ci ou sur les plantes, les surfaces ou les substrats infestés par ceux-ci une quantité efficace d'un composé de formule I selon l'une des revendications 1 à 7.
